# EUROPÄISCHE PATENTSCHRIFT

(11) **EP 2 603 508 B1**
(45) Veröffentlichungstag und Bekanntmachung des Hinweises auf die Patenterteilung: **13.03.2019**
(21) Anmeldenummer: 11741248.6
(22) Anmeldetag: 10.08.2011
(51) Int. Cl.: C07D 417/14

(54) **HETEROARYLPIPERIDIN UND -PIPERAZINDERIVATE ALS FUNGIZIDE**
HETEROARYLPIPERIDINE AND -PIPERAZINE DERIVATIVES AS FUNGICIDES
DÉRIVÉS D'HÉTÉROARYLPIPÉRIDINE ET D'HÉTÉROARYLPIPÉRAZINE UTILISÉS COMME FONGICIDES

(30) Priorität: 11.08.2010 US 372615 P; 11.08.2010 EP 10172486
(43) Veröffentlichungstag der Anmeldung: 19.06.2013
(73) Patentinhaber: Bayer CropScience Aktiengesellschaft, 40789 Monheim (DE)
(72) Erfinder: TSUCHIYA, Tomoki, 69009 Lyon (FR); WASNAIRE, Pierre, 40225 Düsseldorf (DE); HOFFMANN, Sebastian, 41470 Neuss (DE); CRISTAU, Pierre, F-69009 Lyon (FR); SEITZ, Thomas, 40764 Langenfeld (DE); KLUTH, Joachim, 40764 Langenfeld (DE); BENTING, Jürgen, 42799 Leichlingen (DE); WACHENDORFF-NEUMANN, Ulrike, 56566 Neuwied (DE)
(74) Vertreter: BIP Patents
(86) Internationale Anmeldenummer: PCT/EP2011/063783
(87) Internationale Veröffentlichungsnummer: WO 2012/020060

(56) Entgegenhaltungen:
- WO-A2-2009/055514

## Beschreibung

Die Erfindung betrifft Heteroarylpiperidin und -piperazinderivate, deren agrochemisch wirksame Salze, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, in der Tiergesundheit, im Materialschutz sowie im Bereich Haushalt und Hygiene. Die vorliegende Erfindung betrifft weiterhin ein Verfahren zur Herstellung von Heteroarylpiperidin und -piperazinderivaten.

Es ist bereits bekannt, dass bestimmte heterocyclisch substituierte Thiazole als fungizide Pflanzenschutzmittel benutzt werden können (siehe WO 07/014290, WO 08/013925, WO 08/013622, WO 08/091594, WO 08/091580, WO 09/055514, WO 09/094407, WO 09/094445, WO 09/132785, WO 10/037479, WO 10/065579, WO 11/018401, WO 11/018415, WO 11/076510, WO 11/076699). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Heteroarylpiperidin und -piperazinderivate die genannten Aufgaben zumindest in Teilaspekten lösen und sich als Pflanzenschutzmittel, insbesondere als Fungizide eignen.

Gegenstand der Erfindung sind Verbindungen der Formel **(I),** 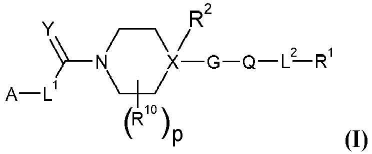 in welcher die Restedefinitionen folgende Bedeutungen haben:
A steht für Phenyl, das bis zu fünf Substituenten enthalten kann,
   wobei die Substituenten unabhängig voneinander ausgewählt sind aus Z^{A-1},
   oder
A steht für ein gegebenenfalls benzokondensiertes, unsubstituiertes oder substituiertes 5- oder 6-gliedriges Heteroaryl, das bis zu vier Substituenten enthalten kann, wobei die Substituenten am Kohlenstoff unabhängig voneinander ausgewählt sind aus Z^{A-2} und die Substituenten am Stickstoff unabhängig voneinander ausgewählt sind aus Z^{A-3},
Z^{A-1} stehen gleich oder verschieden und unabhängig voneinander für Wasserstoff, Halogen, Hydroxy, Thioxyl, Nitro, Cyano, -C(=O)H, -C(=O)OH,, Alkyl, Alkenyl, Alkinyl, Halogenalkyl, Halogenalkenyl, Halogenalkinyl, Cycloalkyl, Cycloalkenyl, Halogencycloalkyl, Halogencycloalkenyl, Hydroxyalkyl, Cyanoalkyl, Formylalkyl, Alkoxyalkyl, Halogenalkoxyalkyl, Cycloalkoxyalkyl, Alkinyloxyalkyl, Alkylthioalkyl, Alkylsulfinylalkyl, Alkylaminoalkyl, Halogenalkylaminoalkyl, Cycloalkylaminoalkyl, Dialkylaminoalkyl, Alkylcarbonylalkyl, Alkylsulfonylalkyl, Alkylcycloalkyl, Alkylcycloalkenyl, Alkoxy, Alkylcycloalkylalkyl, Halogencycloalkoxy, Alkylthio, Halogenalkylthio, Cycloalkylthio, Alkinylthio, Alkenyloxy, Alkinyloxy, Halogenalkoxy, Halogenalkenyloxy, Halogenalkinyloxy, Cycloalkoxy, Alkoxyalkoxy, Cycloalkylalkoxy, Alkylcarbonyloxy, Halogenalkylcarbonyloxy, Cycloalkylcarbonyloxy, Cycloalkylamino, Alkylcarbonylamino, Cycloalkylcarbonylamino, Alkoxycarbonylamino, Alkylsulfonylamino, Halogenalkylsulfonylamino, Phenylsulfonylamino, Cycloalkylalkyl, Halogencycloalkylalkyl, Cycloalkylcycloalkyl, Alkoxyalkoxyalkyl, Alkylaminocarbonyloxy, Alkylcarbonylalkoxy, Cycloalkylaminocarbonyl, Cycloalkylalkoxycarbonyl, Alkylsulfinyl, Halogenalkylsulfinyl, Alkylsulfonyl, Halogenalkylsulfonyl, Cycloalkylsulfonyl, Alkylcarbonyl, Halogenalkylcarbonyl, Cycloalkylcarbonyl, Alkoxycarbonyl, Cycloalkoxycarbonyl, Trialkylsilyl, -SF₅, Phenyl,-C(=O)NR³R⁴ oder -NR³R⁴,
Z^{A-2} und R^{G1} stehen gleich oder verschieden und unabhängig voneinander für Wasserstoff, Halogen, Hydroxy, Thioxyl, Nitro, Cyano, -C(=O)H, -C(=O)OH, Alkyl, Alkenyl, Alkinyl, Halogenalkyl, Halogenalkenyl, Halogenalkinyl, Cycloalkyl, Halogencycloalkyl, Hydroxyalkyl, Formylalkyl, Alkoxyalkyl, Alkylcarbonylalkyl, Alkylcycloalkyl, Alkoxy, Alkylcycloalkylalkyl, Alkylthio, Halogenalkylthio, Alkinylthio, Alkenyloxy, Alkinyloxy, Halogenalkoxy, Alkoxyalkoxy, Alkylcarbonyloxy, Halogenalkylcarbonyloxy, Cycloalkylcarbonylamino, Alkylsulfonylamino, Halogenalkylsulfonylamino, Phenylsulfonylamino, Cycloalkylalkyl, Halogencycloalkylalkyl, Cycloalkylcycloalkyl, Alkoxycarbonyloxy, Alkylcarbonylthio, Alkylsulfinyl, Halogenalkylsulfinyl, Alkylsulfonyl, Halogenalkylsulfonyl, Alkylcarbonyl, Halogenalkylcarbonyl, Alkoxycarbonyl, Alkylamionocarbonyloxy, -C(=O)NR³R⁴ oder -NR³R⁴,
Z^{A-3}, R^{G2} und Z² stehen gleich oder verschieden und unabhängig voneinander für Wasserstoff, -C(=O)H, -C(=O)NR³R⁴, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Alkoxyalkyl, Alkylsulfonyl, Halogenalkylsulfonyl, Cycloalkylsulfonyl, Phenylsulfonyl, Alkylcarbonyl, Halogenalkylcarbonyl, Alkoxycarbonyl, Halogenalkoxycarbonyl, Cycloalkoxycarbonyl, Phenyl oder Benzyl,
R³ und R⁴ stehen gleich oder verschieden und unabhängig voneinander für Wasserstoff, Alkyl, Alkenyl, Alkinyl, Halogenalkyl, Cycloalkyl, Benzyl oder Phenyl,
L¹ steht für NR^{L12} oder C(R^{L11})₂,
R^{L11} steht gleich oder verschieden und unabhängig voneinander für Wasserstoff, Halogen, Hydroxy, Cyano, -C(=O)H, -C(=O)OH, Alkyl, Alkenyl, Alkinyl, Halogenalkyl, Halogenalkenyl, Halogenalkinyl, Alkoxyalkyl, Alkylthioalkyl, Alkylaminoalkyl, Dialkylaminoalkyl, Alkoxy, Alkylthio, Halogenalkylthio, Halogenalkoxy, Alkylcarbonyloxy, Alkylcarbonylamino, Alkylcarbonylthio, Alkylsulfonyl, Halogenalkylsulfonyl, Alkylcarbonyl, Halogenalkylcarbonyl, Alkoxycarbonyl, Trialkylsilyloxy, -NR³R⁴ oder -C(=O)NR³R⁴,
   oder die beiden Reste R^{L11} bilden zusammen mit dem Kohlenstoffatom, an das sie gebunden sind, einen Cyclopropylring, oder
   die beiden Reste R^{L11} stehen für =CH₂, =COR³, =NOR³ oder =CHN(R⁹)₂,
R^{L12} steht für Wasserstoff, -C(=O)H, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Cycloalkylaminocarbonyl, Halogenalkylaminocarbonyl, Alkylsulfonyl, Halogenalkylsulfonyl, Cycloalkylsulfonyl, Alkylcarbonyl, Halogenalkylcarbonyl, Alkoxycarbonyl, Halogenalkoxycarbonyl, Cycloalkoxycarbonyl, Alkylaminocarbonyl, Dialkylaminocarbonyl, Phenyl oder Benzyl,
R⁹ steht für Alkyl, Alkenyl, Alkinyl, Halogenalkyl, Cycloalkyl, Benzyl oder Phenyl,
Y steht für Schwefel oder Sauerstoff,
X steht für Kohlenstoff oder Stickstoff,
R² steht für Wasserstoff, Alkyl, Alkenyl, Halogenalkyl, Alkoxy, Halogen, Cyano oder Hydroxy,
R¹⁰ steht für Oxo, Alkyl, Alkenyl, Halogenalkyl, Alkoxy, Halogen, Cyano oder Hydroxy,
p steht für 0 bis 2,
G steht für ein unsubstituiertes oder substituiertes 5-gliedriges Heteroaryl, wobei die Substituenten am Kohlenstoff unabhängig voneinander ausgewählt sind aus R^{G1} und die Substituenten am Stickstoff unabhängig voneinander ausgewählt sind aus R^{G2},
Q steht für gesättigtes oder teilweise oder vollständig ungesättigtes, unsubstituiertes oder substituiertes 5-gliedriges Heterocyclyl, wobei ein oder mehrere Substituenten R⁵ gleich oder verschieden unabhängig voneinander ausgewählt sind aus,
R⁵ steht gleich oder verschieden unabhängig voneinander für:
   verbunden mit Kohlenstoff des 5-gliedrigen Heterocyclyl von Q:
      Wasserstoff, Oxo, Halogen, Cyano, Hydroxy, Nitro, -CHO, -C(=O)OH, -C(=O)NH₂,-C(=O)NR³R⁴, -NR³R⁴, Alkyl, Alkenyl, Alkinyl., Halogenalkyl, Halogenalkenyl, Halogenalkinyl, Cycloalkyl, Halogencycloalkyl, Alkylcycloalkyl, Cycloalkylalkyl, Cycloalkylcycloalkyl, Halogencycloalkylalkyl, Alkylcycloalkylalkyl, Cycloalkenyl, Halogencycloalkenyl, Alkoxyalkyl, Halogenalkoxyalkyl, Cycloalkoxyalkyl, Alkoxyalkoxyalkyl, Alkylthioalkyl, Formylalkyl, Alkylcarbonylalkyl, Alkylsulfinylalkyl, Alkylsulfonylalkyl, Alkylaminoalkyl, Dialkylaminoalkyl, Halogenalkylaminoalkyl, Cycloalkylaminoalkyl, Alkylcarbonyl, Halogenalkylcarbonyl, Cycloalkylcarbonyl, Alkoxycarbonyl, Cycloalkoxycarbonyl, Cycloalkylalkoxycarbonyl, Cycloalkylaminocarbonyl, Hydroxyalkyl, Alkoxy, Halogenalkoxy, Cycloalkoxy, Halogencycloalkoxy, Cycloalkylalkoxy, Alkenyloxy, Halogenalkenyloxy, Alkinyloxy, Halogenalkinyloxy, Alkoxyalkoxy, Alkylcarbonyloxy, Halogenalkylcarbonyloxy, Cycloalkylcarbonyloxy, Alkylcarbonylalkoxy, Alkylthio, Halogenalkylthio, Cycloalkylthio, Alkylsulfinyl, Halogenalkylsulfinyl, Alkylsulfonyl, Halogenalkylsulfonyl, Cycloalkylsulfonyl, Trialkylsilyl, Alkylsulfonylamino, Halogenalkylsulfonylamino,
   verbunden mit Stickstoff, des 5-gliedrigen Heterocyclyl von Q:
      Wasserstoff, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Phenyl, Benzyl, Alkylsulfonyl, -C(=O)H, Alkoxycarbonyl oder Alkylcarbonyl,
L² steht für 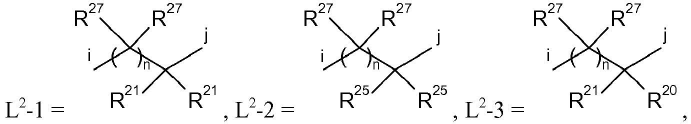 oder L²-4 = 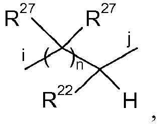 wobei die Bindung, die mit "i" identifiziert ist, direkt an Q gebunden ist, und wobei die Bindung, die mit "j" identifiziert ist, direkt an R¹ gebunden ist,
m steht für 0 bis 2,
n steht für 0 bis 4,
R²⁰ steht für Halogen oder Hydroxy,
R²¹ steht gleich oder verschieden und unabhängig voneinander für Alkyl, Halogenalkyl, Alkoxy, Halogenalkoxy, Alkenyl, Halogenalkenyl, Alkinyl, Halogenalkinyl, Alkenyloxy, Halogenalkenyloxy, Alkinyloxy, Halogenalkinyloxy, oder Alkylcarbonyloxy,
R²² steht für Hydroxy, Halogen, Alkoxy, Halogenalkoxy, Alkenyl, Halogenalkenyl, Alkinyl, Halogenalkinyl, Alkenyloxy, Halogenalkenyloxy, Alkinyloxy, Halogenalkinyloxy, Alkylcarbonyloxy oder Cycloalkyl,
R²⁵ steht gleich oder verschieden unabhängig voneinander für Fluor, Brom, Chlor,
R²⁷ steht jeweils unabhängig voneinander und gleich oder verschieden für Wasserstoff, Alkyl, Alkoxy, Alkenyloxy oder Alkinyloxy,
unter der Voraussetzung, daß L² maximal zwei R²⁷ enhalten darf, die von Wasserstoff verschieden sind,
   - R¹: steht für ein gegebenenfalls ein- oder mehrfach und gleich oder verschieden durch Z¹ substituiertes Phenyl, Benzyl oder Naphthalenyl, oder für ein gegebenenfalls benzokondensiertes, unsubstituiertes oder substituiertes 5- oder 6-gliedriges Heteroaryl, wobei die Substituenten am Kohlenstoff unabhängig voneinander ausgewählt aus Z¹ sind und die Substituenten am Stickstoff unabhängig voneinander ausgewählt sind aus Z²,
   oder
   - R¹: steht für einen 3- bis 8-gliedrigen nicht-aromatischen (gesättigten bzw. partiell gesättigten) carbocyclischen Ring, für einen 5-, 6- oder 7-gliedrigen nicht-aromatischen Heterocyclylrest oder für einen von 8- bis 11-gliedrigen carbocyclischen oder heterocyclischen bicyclischen Ring, wobei die Substituenten am Kohlenstoff unabhängig voneinander ausgewählt sind aus Oxo, Thio oder Z¹ und die Substituenten am Stickstoff unabhängig voneinander ausgewählt sind aus Z²,
   - Z¹: stehen für Wasserstoff, Halogen, Hydroxy, Thio, Nitro, Cyano, -C(=O)H, -C(=O)OH, Alkyl, Alkenyl, Alkinyl, Halogenalkyl, Halogenalkenyl, Halogenalkinyl, Cycloalkyl, Cycloalkenyl, Halogencycloalkyl, Halogencycloalkenyl, Hydroxyalkyl, Alkoxyalkyl, Halogenalkoxyalkyl, Cycloalkoxyalkyl, Alkylthioalkyl, Alkylsulfinylalkyl, Alkylaminoalkyl, Halogenalkylaminoalkyl, Cycloalkylaminoalkyl, Dialkylaminoalkyl, Alkoxycarbonylalkyl, Alkylsulfonylalkyl, Alkylcycloalkyl, Alkoxy, Alkylcycloalkylalkyl, Halogencycloalkoxy, Alkylthio, Halogenalkylthio, Alkinylthio, Alkenyloxy, Alkinyloxy, Halogenalkoxy, Halogenalkenyloxy, Halogenalkinyloxy, Cycloalkoxy, Alkoxyalkoxy, Cycloalkylalkoxy, Alkylcarbonyloxy, Halogenalkylcarbonyloxy, Cycloalkylcarbonyloxy, Halogencycloalkylcarbonyloxy, Alkylsulfonyloxy, Halogenalkylsulfonyloxy, Phenylsulfonyloxy, Alkylcarbonylamino, Halogenalkylcarbonylamino, Alkylsulfonylamino, Halogenalkylsulfonylamino, Phenylsulfonylamino, Cycloalkylalkyl, Halogencycloalkylalkyl, Cycloalkylcycloalkyl, Alkoxyalkoxyalkyl, Alkylcarbonylalkoxy, Cycloalkylcarbonyl, Cycloalkylthio, Cycloalkylaminocarbonyl, Cycloalkylalkoxycarbonyl, Alkenylthio, Alkylcarbonylthio, Alkylsulfinyl, Halogenalkylsulfinyl, Alkylsulfonyl, Halogenalkylsulfonyl, Cycloalkylsulfonyl, Alkylcarbonyl, Halogenalkylcarbonyl, Alkoxycarbonyl, Halogenalkoxycarbonyl, Cycloalkoxycarbonyl, Trialkylsilyl, Trialkylsilyloxy, -SF₅, Cycloalkylalkylamino, Dialkylaminothiocarbonyl, Alkoxycarbonylamino, Alkoxyalkylaminocarbonyl, Halogenalkoxyamino, Cycloalkylalkylaminoalkyl, Dialkylaminocarbonylamino, Alkoxyhalogenalkoxy, Alkylthiocarbonyloxy, Halogenalkoxyalkoxy, Dialkylaminosulfonyl, Alkoxyhalogenalkyl, Alkylaminosulfonyl, Dialkoxyalkyl, Halogenalkoxyhalogenalkyl, -NHC(=O)H, Halogencycloalkenyloxyalkyl, Alkylthiocarbonyl, Alkoxyalkenyl, -SO₂NHCN, -NHCN, Halogenalkylsulfonylaminocarbonyl, Alkylaminothiocarbonylamino, Alkylaminothiocarbonyl, Alkylaminocarbonylalkylamino, Alkoxyalkinyl, Halogendialkylaminoalkyl, Cyanoalkyl, Alkoxyalkylcarbonyl, Alkoxycarbonylalkoxy, Alkoxyamino, Alkoxyalkoxycarbonyl, Cycloalkenyloxyalkyl, Dialkylaminothiocarbonylamino, Alkylsulfonylaminocarbonyl, Halogenalkoxyhalogenalkoxy, Halogencycloalkoxyalkyl, -C(=O)NHCN, -N=C(R⁹)₂, -NR³R⁴, -C(=O)NR³R⁴, -C(=N-OR⁷)R⁸ oder-L³Z³,
   - R⁷: steht für Wasserstoff, Alkyl, Halogenalkyl, Benzyl oder Z³,
   - R⁸: steht für Wasserstoff, Alkyl, Halogenalkyl, Cycloalkylalkyl, Cycloalkyl, Alkylcycloalkyl, Halogenalkylcycloalkyl, Alkoxylalkyl, Halogenalkoxyalkyl, Benzyl oder Phenyl,
   - L³: steht für eine direkte Bindung, -CH₂-, -C(=O)-, Schwefel, Sauerstoff, -C(=O)O-, -C(=O)NH-,-OC(=O)- oder -NHC(=O)-,
   - Z³: steht für einen Phenylrest oder einen 5- oder 6-gliedrigen Heteroarylrest, der bis zu drei Substituenten enthalten kann, wobei die Substituenten unabhängig voneinander ausgewählt sind aus folgender Liste:
   Substituenten am Kohlenstoff: Halogen, Cyano, Nitro, Hydroxy, Amino, -SH, Alkyl, Alkenyl, Alkinyl, Halogenalkyl, Halogenalkenyl, Halogenalkinyl, Cycloalkyl, Halogencycloalkyl, Alkoxyalkyl, Alkylcarbonyl, Halogenalkylcarbonyl, Alkoxycarbonyl, Alkoxy, Halogenalkoxy, Cycloalkoxy, Halogencycloalkoxy, Alkenyloxy, Alkinyloxy, Alkoxyalkoxy, Alkylthio, Halogenalkylthio, Alkylsulfinyl, Halogenalkylsulfinyl, Alkylsulfonyl, Halogenalkylsulfonyl, Trisilylalkyl oder Phenyl,
   Substituenten am Stickstoff: Wasserstoff, -C(=O)H, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Alkoxyalkyl, Alkylsulfonyl, Halogenalkylsulfonyl, Cycloalkylsulfonyl, Phenylsulfonyl, Alkylcarbonyl, Halogenalkylcarbonyl, Alkoxycarbonyl, Halogenalkoxycarbonyl, Cycloalkoxycarbonyl, -C(=O)NR³R⁴, Phenyl oder Benzyl
   sowie Salze, Metallkomplexe und N-Oxide der Verbindungen der Formel **(I).**

Ein weiterer Gegenstand ist die Verwendung der Verbindungen der Formel **(I)** als Fungizide.

Erfindungsgemäße Heteroarylpiperidin und -piperazinderivate der Formel **(I)** sowie deren Salze, Metallkomplexe und N-Oxide eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel **(I)** können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Restedefinitionen der erfindungsgemäßen Verbindungen der Formel **(I)** haben bevorzugt, besonders bevorzugt und ganz besonders bevorzugt folgende Bedeutungen:
- A: steht bevorzugt für Phenyl, das bis zu zwei Substituenten enthalten kann, wobei die Substituenten unabhängig voneinander ausgewählt sind aus folgender Liste,
Halogen, Cyano, Hydroxy, -NR³R⁴, -C(=O)NR³R⁴, Nitro, C₁-C₆-Alkyl, C₂-C₆-Alkenyl, C₂-C₆-Alkinyl, C₃-C₈-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkenyloxy, C₁-C₄-Alkinyloxy, C₁-C₄-Alkylthio, C₁-C₄-Alkylsulfonyl, C₁-C₄-Halogenalkylthio, C₁-C₄-Halogenalkylsulfonyl, C₁-C₄-Alkoxy-C₁-C₆-alkyl, Hydroxy-C₁-C₄-alkyl, C₁-C₆-Alkylcarbonyl, C₁-C₆-Alkoxycarbonyl, C₁-C₆-Alkylcarbonyloxy oder -C(=O)H, oder,
- A: steht bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrazin-2-yl, Pyrazin-3-yl, Pyrimidin-2-yl, Pyrimidin-4-yl oder Pyrimidin-5-yl, der bis zu zwei Substituenten enthalten kann, wobei die Substituenten unabhängig voneinander ausgewählt sind aus folgender Liste,
Substituenten am Kohlenstoff:
Halogen, Cyano, Hydroxy, Nitro, -NR³R⁴, C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkyl-sulfonyl, C₁-C₄-Halogenalkylthio, C₁-C₄-Halogenalkylsulfonyl, C₁-C₄-Alkoxy-C₁-C₄-alkyl, Hydroxy-C₁-C₄-alkyl, C₁-C₆-Alkylcarbonyl, C₁-C₆-Alkoxycarbonyl, C₁-C₆-Alkylcarbonyloxy oder Phenyl,
Substituenten am Stickstoff:
C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₁₀-Cycloalkyl-C₁-C₆-alkyl, C₁-C₆-Halogenalkylcarbonyl, Phenyl, Benzyl, C₁-C₄-Alkylsulfonyl, C₁-C₄-Halogenalkylsulfonyl, Phenylsulfonyl, -C(=O)H, oder C₁-C₆-Alkylcarbonyl,
- A: steht besonders bevorzugt für Phenyl, das bis zu zwei Substituenten enthalten kann, wobei die Substituenten unabhängig voneinander ausgewählt sind aus folgender Liste: Fluor, Brom, Iod, Chlor, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio oder Trifluormethylthio, oder
- A: steht besonders bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten gleich oder verschieden und unabhängig voneinander ausgewählt sind aus folgender Liste:
Substituenten am Kohlenstoff:
Fluor, Chlor, Brom, Iod, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio, Trifluormethylthio oder Phenyl,

### Substituenten am Stickstoff:

Methyl, Ethyl, Propyl, 1-Methylethyl, Methylsulfonyl, Trifluormethylsulfonyl, Methylcarbonyl, Trifluormethylcarbonyl, Chlormethylcarbonyl, 2,2-Trifluorethyl, 2,2-Difluorethyl, 2,2-Dichlor-2-fluorethyl, 2-Chlor-2-difluorethyl oder 2-Chlor-2-fluorethyl.

A steht ganz besonders bevorzugt für Pyrazol-1-yl, der bis zu zwei Substituenten enthalten kann, wobei die Substituenten unabhängig voneinander ausgewählt sind aus folgender Liste:
Methyl, Ethyl, Propyl, 1-Methylethyl, Chlor, Brom, Fluor, Monofluormethyl, Difluormethyl oder Trifluormethyl, oder

A steht ganz besonders bevorzugt für Phenyl, der bis zu zwei Substituenten enthalten kann, wobei die Substituenten unabhängig voneinander ausgewählt sind aus folgender Liste:
Methyl, Ethyl, Iod, Chlor, Brom, Fluor, Methoxy, Ethoxy, Difluormethyl oder Trifluormethyl.
R³ und R⁴ stehen bevorzugt gleich oder verschieden unabhängig voneinander für Wasserstoff, C₁-C₆-Alkyl, C₂-C₆-Alkenyl, C₂-C₆-Alkinyl, C₁-C₆-Halogenalkyl, C₃-C₈-Cycloalkyl, Benzyl oder Phenyl, und besonders bevorzugt für Methyl, Ethyl, Propyl, 1-Methylethyl, Butyl oder 1,1-Dimethylethyl,
L¹ steht bevorzugt für C(R^{L11})₂ (insbesondere CHR^{L11}) oder NR^{L12} und besonders bevorzugt für CH₂,
R^{L11} steht bevorzugt für Wasserstoff, Methyl, Ethyl oder Cyclopropyl, oder die beiden Reste R^{L11} bilden zusammen mit dem Kohlenstoffatom, an das sie gebunden sind, einen Cyclopropylring oder
   die beiden Reste R^{L11} stehen für =CHN(R⁹)₂,
R^{L11} steht besonders bevorzugt für Wasserstoff oder Methyl,
R^{L12} steht bevorzugt für Wasserstoff, C₁-C₄-Alkyl, C₁-C₄-Halogenalkyl, C₃-C₈-Cycloalkyl, C₁-C₄-Alkylsulfonyl, C₁-C₄-Alkoxycarbonyl, und besonders bevorzugt für Wasserstoff oder Methyl, und ganz besonders bevorzugt für Wasserstoff,
R⁹ steht bevorzugt gleich oder verschieden unabhängig voneinander für C₁-C₆-Alkyl, C₂-C₆-Alkenyl, C₂-C₆-Alkinyl, C₁-C₆-Halogenalkyl, C₃-C₈-Cycloalkyl, Benzyl oder Phenyl, und besonders bevorzugt für Wasserstoff,. Methyl, Ethyl, Propyl,, 1-Methylethyl, Butyl oder 1,1-Dimethylethyl,
Y steht bevorzugt für Sauerstoff oder Schwefel und besonders bevorzugt für Sauerstoff,
X steht für Kohlenstoff oder Stickstoff und vorzugsweise für Kohlenstoff
R² steht bevorzugt für Wasserstoff, C₁-C₄-Alkyl, C₁-C₄-Alkenyl, C₁-C₄-Halogenalkyl, C₁-C₄-Alkoxy, Halogen, Cyano oder Hydroxy, und besonders bevorzugt für Wasserstoff, Fluor, Chlor, Brom oder Hydroxy, und ganz besonders bevorzugt für Wasserstoff oder Fluor,
R¹⁰ steht bevorzugt für Oxo, C₁-C₄-Alkyl, C₁-C₄-Alkenyl, C₁-C₄-Halogenalkyl, C₁-C₄-Alkoxy, Halogen, Cyano oder Hydroxy, und besonders bevorzugt für Fluor, Chlor, Brom oder Hydroxy, und ganz besonders bevorzugt für Fluor,
p steht bevorzugt für 0 bis 1, und besonders bevorzugt für 0,
G steht bevorzugt für 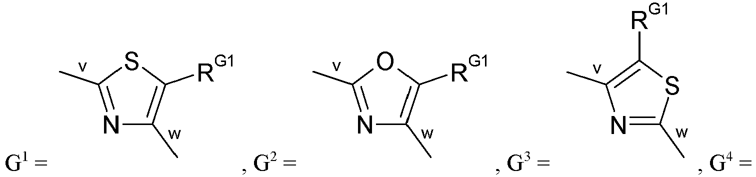 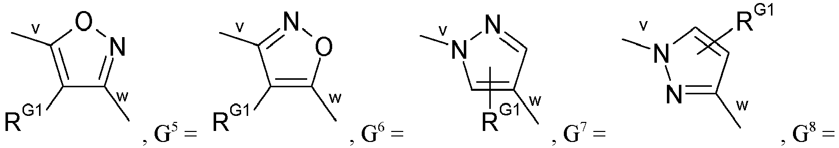 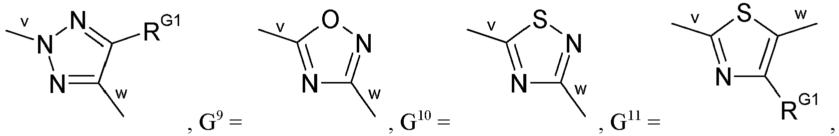 wobei die Bindung, die mit "v" identifiziert ist, direkt an X gebunden ist, und wobei die Bindung, die mit "w" identifiziert ist, direkt an Q gebunden ist,
G steht besonders bevorzugt für G¹, G² oder G³, und ganz besonders bevorzugt für G¹,
R^{G1} steht bevorzugt für Wasserstoff oder Halogen und besonders bevorzugt für Wasserstoff,
Q steht bevorzugt für 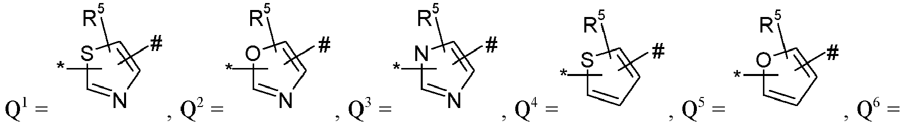 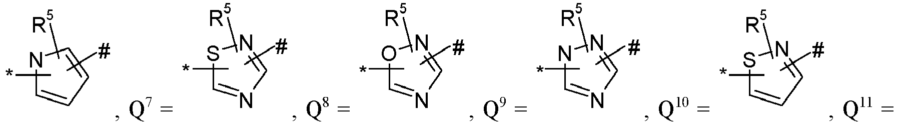 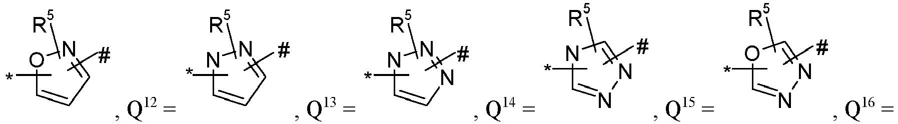 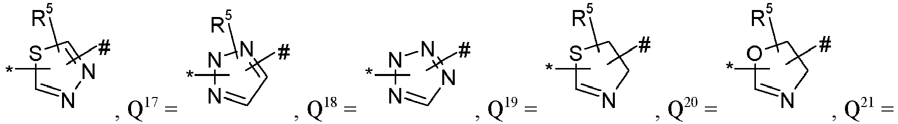 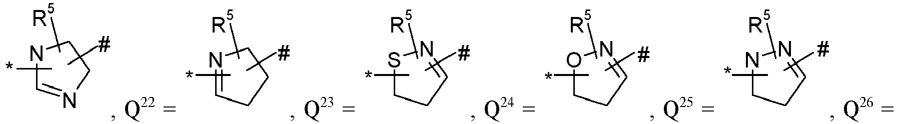 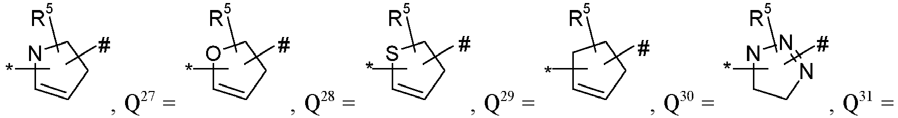 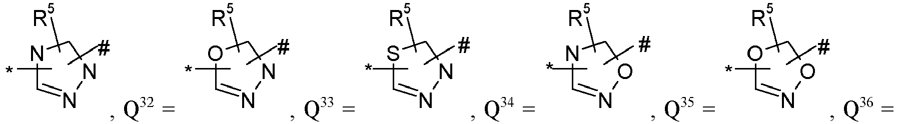 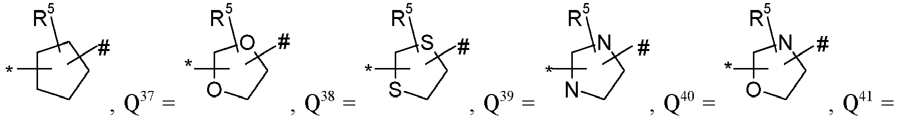 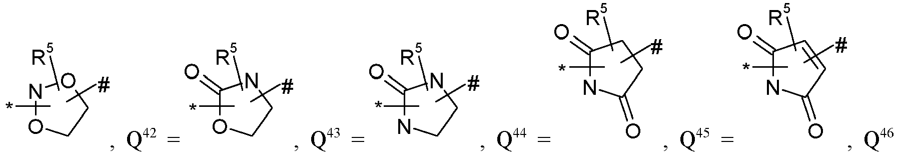 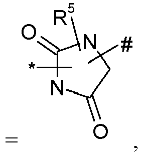 wobei die Bindung, die mit "*" identifiziert ist, direkt an G ist und gleichzeitig die Bindung, die mit "#" identifiziert ist, direkt an L² gebunden ist oder wobei die Bindung, die mit "*" identifiziert ist, direkt an L² gebunden ist, und gleichzeitig die Bindung, die mit "#" identifiziert ist, direkt an G gebunden ist,
Q steht besonders bevorzugt für 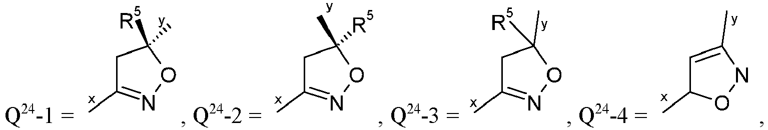 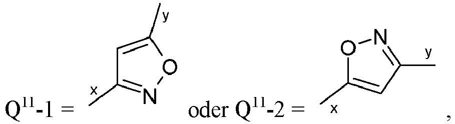 wobei die Bindung, die mit "x" identifiziert ist, direkt an G gebunden ist, und wobei die Bindung, die mit "y" identifiziert ist, direkt an L² gebunden ist,
Q steht insbesondere bevorzugt für 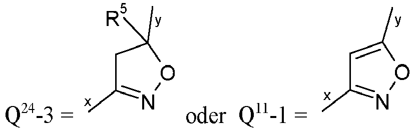 wobei die Bindung, die mit "x" identifiziert ist, direkt an G gebunden ist, und wobei die Bindung, die mit "y" identifiziert ist, direkt an L² gebunden ist,
R⁵ steht bevorzugt gleich oder verschieden unabhängig voneinander für
   verbunden mit Kohlenstoff des 5-gliedriges Heterocyclyl von Q:
   Wasserstoff, Cyano, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₃-Cg-Halogencycloalkyl, C₁-C₄-Alkyl-C₃-C₈-cycloalkyl, C₃-C₈-Cycloalkyl-C₁-C₄-alkyl, C₁-C₄-Alkoxy-C₁-C₄-alkyl, C₃-C₈-Cycloalkoxy-C₁-C₄-alkyl, C₁-C₄-Alkoxy-C₁-C₄-alkoxy-C₁-C₄-alkyl, C₁-C₄-Alkylthio-C₁-C₄-alkyl, C₁-C₆-Alkoxy, C₁-C₆-Halogenalkoxy, C₃-C₈-Cycloalkoxy, C₃-C₈-Halogencycloalkoxy, C₃-C₈-Cycloalkyl-C₁-C₄-alkoxy, C₂-C₆-Alkenyloxy, C₂-C₆-Halogenalkenyloxy, C₂-C₆-Alkinyloxy, C₂-C₆-Halogenalkinyloxy, C₁-C₆-Alkoxy-C₁-C₄-alkoxy, C₁-C₆-Alkylcarbonyloxy, C₁-C₆-Halogenalkylcarbonyloxy, C₃-C₈-Cycloalkylcarbonyloxy, C₁-C₆-Alkylcarbonyl-C₁-C₆-alkoxy, C₁-C₆-Alkylthio, C₁-C₆-Halogenalkylthio, C₃-C₈-Cycloalkylthio,
   verbunden mit Stickstoff des 5-gliedriges Heterocyclyl von Q:
   Wasserstoff, -C(=O)H, C₁-C₃-Alkyl, C₁-C₆-Alkylcarbonyl, C₁-C₆-Alkoxycarbonyl oder Benzyl,
R⁵ besonders bevorzugt für Wasserstoff, Cyano, Methyl, Trifluormethyl, Difluormethyl oder Methoxymethyl,
R⁵ ganz besonders bevorzugt für Wasserstoff,
L² steht bevorzugt für L²-1, L²-2, L²-3, L²-4, L²-5, L²-6, L²-7, L²-8 oder L²-9,
m steht bevorzugt für 0 oder 2,
n steht bevorzugt für 0 bis 2, und besonders bevorzugt für 0 oder 1,
R²⁰ steht bevorzugt für Halogen oder Hydroxy, besonders bevorzugt für Hydroxy, Chlor, Fluor,
R²¹ steht gleich oder verschieden und unabhängig voneinander bevorzugt für C₁-C₄-Alkyl, C₁-C₄-Halogenalkyl, C₂-C₄-Alkenyl, C₂-C₄-Alkinyl, C₁-C₄-Alkoxy, C₁-C₄-Halogenalkoxy, C₂-C₄-Alkenyloxy C₂-C₄-Halogenalkenyloxy, C₂-C₄-Alkinyloxy, C₂-C₄-Halogenalkinyloxy, C₁-C₄-Alkylcarbonyloxy, und besonders bevorzugt für Methyl, Ethyl, Ethenyl, Ethinyl, Trifluormethyl, Difluormethyl, Methylcarbonyloxy, Ethylcarbonyloxy, Methoxy, Ethoxy, Propoxy, 1-Methylethoxy, 2-Propenyloxy, 2-Propinyloxy oder Trifluormethoxy,
R²² steht bevorzugt für Hydroxy, Halogen, C₁-C₄-Alkoxy, C₁-C₄-Halogenalkoxy, C₂-C₄-Alkenyloxy C₂-C₄-Halogenalkenyloxy, C₂-C₄-Alkinyloxy, C₂-C₄-Halogenalkinyloxy, C₁-C₄-Alkylcarbonyloxy, C₃-C₆-Cycloalkyl und besonders bevorzugt für Hydroxy, Chlor, Fluor, Brom, Iod, Methoxy, Ethoxy, Propoxy, 1-Methylethoxy, Butoxy, Trifluormethyl, Difluormethyl, Methylcarbonyloxy, Ethylcarbonyloxy, 1-Ethenyloxy, 2-Propenyloxy, 2-Propinyloxy oder Trifluormethoxy,
R²⁵ steht bevorzugt für Fluor,
R²⁷ steht gleich oder verschieden und unabhängig voneinander bevorzugt für Wasserstoff, C₁-C₄-Alkyl, C₁-C₄-Alkoxy, und besonders bevorzugt Wasserstoff,
R¹ steht bevorzugt für unsubstituiertes oder substituiertes C₅-C₆-Cycloalkenyl, C₃-C₈-Cycloalkyl, wobei die Substituenten unabhängig voneinander ausgewählt sind aus Z¹⁻¹, und besonders bevorzugt für Cyclopentenyl, Cyclohexenyl, Cyclopentyl, Cyclohexyl oder Cycloheptyl, welche jeweils bis zu zwei Substituenten enthalten kann, wobei die Substituenten unabhängig voneinander ausgewählt sind aus folgender Liste: Methyl, Ethyl, Methoxy, Ethoxy, Trifluormethoxy, Ethinyl, 2-Propenyloxy, 2-Propinyloxy, Methylcarbonyloxy, Ethylcarbonyloxy, Trifluoralkylcarbonyloxy, Methylthio, Ethylthio oder Trifluormethylthio und ganz besonders bevorzugt für Cyclopent-2-en-1-yl, Cyclopent-3-en-1-yl, Cyclohex-1-en-1-yl, Cyclohex-2-en-1-yl, Cyclohex-3-en-1-yl, Cyclopentyl, oder Cyclohexyl, oder
R¹ steht bevorzugt für unsubstituiertes oder substituiertes Phenyl, wobei die Substituenten unabhängig voneinander ausgewählt sind aus Z¹⁻² und besonders bevorzugt für Phenyl, das bis zu drei Substituenten enthalten kann, wobei die Substituenten unabhängig voneinander ausgewählt sind aus folgender Liste: Fluor, Chlor, Brom, Iod, Cyano, Nitro, Hydroxy, Amino, Thio, -(C=O)H, Methyl, Ethyl, Propyl, 1-Methylethyl, Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylcarbonylamino, Trifluoralkylcarbonylamino, Chlormethylcarbonylamino, Methylthio, Ethylthio, Methylsulfinyl, Methylsulfonyl, Methylsulfonyloxy, Trifluorsulfonyloxy, Methylsulfonylamino, Trifluoromethylsulfonylamino, -C(=N-OH)H, -C(=N-OCH₃)H, -C(=N-OCH₂CH₃)H,-C(=N-OH)CH₃, -C(=N-OCH₃)CH₃, -C(=N-OCH₂CH₃)CH₃ oder Trimethylsilyloxy, ganz besonders bevorzugt für Phenyl, das bis zu drei Substituenten enthalten kann, wobei die Substituenten unabhängig voneinander ausgewählt sind aus folgender Liste: Fluor, Chlor, Brom, Iod, Cyano, Amino, Hydroxy, -(C=O)H, Methyl, Ethyl, Propyl, 1-Methylethyl, Ethenyl, Ethinyl, Trifluormethyl, Difluormethyl, Methoxy, Ethoxy, Methylcarbonyl, Trifluormethylcarbonyl, Methoxycarbonyl, Ethoxycarbonyl, 1-Ethenyloxy, 2-Propenyloxy, 2-Propenyloxy, 2-Propinyloxy, Methylcarbonyloxy, Trifluoralkylcarbonyloxy, Chlormethylcarbonyloxy, Methylcarbonylamino, Methylthio, Ethylthio, Methylsulfonyl, Methylsulfonyloxy, Trifluorsulfonyloxy, Methylsulfonylamino, Trifluoromethylsulfonylamino, -C(=N-OH)H,-C(=N-OCH₃)H, -C(=N-OCH₂CH₃)H, -C(=N-OH)CH₃, -C(=N-OCH₃)CH₃, -C(=N-OCH₂CH₃)CH₃ oder Trimethylsilyloxy, oder
R¹ steht bevorzugt für unsubstituiertes oder substituiertes, Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydro-naphthalen-1-yl, 5,6,7,8-Tetrahydronaphthalen-2-yl, Decalin-1-yl, Decalin-2-yl, 1H-Inden-1-yl, 2,3-Dihydro-1H-inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl,
wobei die Substituenten unabhängig voneinander ausgewählt aus Z¹⁻³ sind und besonders bevorzugt, wo die Substituenten unabhängig voneinander aus folgender Liste ausgewählt sind: Methyl, Methoxy, Cyano, Fluor, Chlor, Brom, Iod, wobei bei der besonders bevorzugten Variante höchstens drei Substituenten enthalten sind und ganz besonders bevorzugt keine Substitutenten vorliegen, oder
R¹ steht bevorzugt für einen unsubstituierten oder substituierten 5- oder 6-gliedrigen Heteroarylrest, wobei die Substituenten am Kohlenstoff unabhängig voneinander ausgewählt sind aus Z¹⁻⁴, und die Substituenten am Stickstoff unabhängig voneinander ausgewählt sind aus Z²
und steht besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Cyano, Nitro, Hydroxy, Amino, Thio, -(C=O)H, Methyl, Ethyl, Propyl, 1-Methylethyl, Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylcarbonylamino, Trifluoralkylcarbonylamino, Chlormethylcarbonylamino, Methylthio, Ethylthio, Methylsulfinyl, Methylsulfonyl, Methylsulfonyloxy, Trifluorsulfonyloxy, Methylsulfonylamino, oder Trifluoromethylsulfonylamino,
Substituenten am Stickstoff: Methyl, Ethyl, Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl,
wobei ganz besonders bevorzugt keine Substitutenten am Stickstoff vorliegen, oder
R¹ steht bevorzugt für benzokondensiertes unsubstituiertes oder substituiertes 5- oder 6- gliedriges Heteroaryl, wobei die Substituenten am Kohlenstoff unabhängig voneinander ausgewählt aus Z¹⁻⁵ sind, und die Substituenten am Stickstoff unabhängig voneinander ausgewählt sind aus Z² und besonders bevorzugt für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl, und ganz besonders bevorzugt keine Substitutenten an den benzokondensierten Heteroarylresten vorliegen, oder
R¹ steht bevorzugt für unsubstituiertes oder substituiertes C₅-C₁₅-Heterocyclyl, wobei die Substituenten am Kohlenstoff unabhängig voneinander ausgewählt sind aus Z¹⁻⁶ und die Substituenten am Stickstoff unabhängig voneinander ausgewählt sind aus Z² und besonders bevorzugt für Piperidin-1-yl, Piperidin-2-yl, Piperidin-3-yl, Piperidin-4-yl, Piperazin-1-yl, Piperazin-2-yl, Piperazin-3-yl, Morpholin-1-yl, Morpholin-2-yl, Morpholin-3-yl, Tetrahydropyran-2-yl, Tetrahydropyran-3-yl, Tetrahydropyran-4-yl, 1,2,3,4-Tetrahydrochinolin-1-yl, 1,2,3,4-Tetrahydroisochinolin-2-yl, 1,2,3,4-Tetrahydrochinoxalin-1-yl, Indolin-1-yl, Isoindolin-2-yl, Decahydrochinolin-1-yl oder Decahydroisochinolin-2-yl, welche jeweils bis zu zwei Substituenten enthalten kann, wobei die Substituenten unabhängig voneinander ausgewählt sind aus folgender Liste:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl, und ganz besonders bevorzugt keine Substitutenten an den Heterocyclylresten vorliegen,
Z¹⁻¹ stehen gleich oder verschieden und unabhängig voneinander für Cyano, Halogen, -C(=O)H, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylcarbonyloxy, C₁-C₆-Halogenalkylcarbonyloxy, C₁-C₆-Alkylthio oder C₁-C₆-Halogenalkylthio,
Z¹⁻² stehen gleich oder verschieden und unabhängig voneinander für Halogen, Cyano, Hydroxy, Thio, Nitro, -C(=O)H, -COOH, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₃-C₈-Cycloalkenyl, C₃-C₈-Halogencycloalkenyl, C₁-C₆-Alkoxy-C₁-C₆-alkyl, C₁-C₆-Alkylcarbonyl, C₁-C₆-Halogenalkylcarbonyl, C₃-C₈-Cycloalkylcarbonyl, C₁-C₆-Alkoxycarbonyl, C₃-C₈-Cycloalkoxycarbonyl, C₃-C₈-Cycloalkylaminocarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Halogenalkenyloxy, C₂-C₆-Alkinyloxy, C₂-C₆-Halogenalkinyloxy, C₁-C₄-Alkoxy-C₁-C₄-alkoxy, C₁-C₆-Alkylcarbonyloxy, C₁-C₆-Halogenalkylcarbonyloxy, C₃-C₈-Cycloalkylcarbonyloxy, C₁-C₆-Alkylcarbonyl-C₁-C₄-alkoxy, C₁-C₆-Alkylthio, C₁-C₆-Halogenalkylthio, C₃-C₆-Cycloalkylthio, C₁-C₆-Alkylsulfonyl, C₁-C₆-Halogenalkylsulfonyl, C₃-C₈-Cycloalkylsulfonyl, C₁-C₆-Alkylsulfonyloxy, C₁-C₆-Halogenalkylsulfonyloxy, Phenylsulfonyloxy, C₁-C₆-Alkylsulfonylamino, C₁-C₆-Halogenalkylsulfonylamino, Tri(C₁-C₄)alkylsilyl, Tri(C₁-C₄)alkylsilyloxy oder-C(=N-OR⁷)R⁸,
Z¹⁻³ und Z¹⁻⁵ stehen gleich oder verschieden und unabhängig voneinander_für Halogen, Cyano, Nitro,-C(=O)H, -NR³R⁴, -C(=O)NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C-C₄-Alkoxy- C₁-C₄-alkyl, C₁-C₆-Alkylcarbonyl, C₁-C₆-Alkoxycarbonyl, C₁-C₄-Alkoxy, C₁-C₄-Halogenalkoxy, C₁-C₆-Alkylcarbonyloxy, C₁-C₆-Alkylcarbonylthio, C₁-C₄-Alkylthio, C₁-C₄-Halogenalkylthio, C₁-C₄-Alkylsulfonyl, C₁-C₄-Halogenalkylsulfonyl,
Z¹⁻⁴ stehen gleich oder verschieden und unabhängig voneinander für Halogen, Cyano, Hydroxy, Thio, Nitro, -C(=O)H, -C(=O)OH, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₃-C₈-Cycloalkenyl, C₃-C₈-Halogencycloalkenyl, C₁-C₆-Alkoxy-C₁-C₆-alkyl, C₁-C₆-Alkylcarbonyl, C₁-C₆-Halogenalkylcarbonyl, C₃-C₈-Cycloalkylcarbonyl, C₁-C₆-Alkoxycarbonyl, C₃-C₈-Cycloalkoxycarbonyl, C₃-C₁₀-Cycloalkylaminocarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Halogenalkenyloxy, C₂-C₆-Alkinyloxy, C₂-C₆-Halogenalkinyloxy, C₁-C₄-Alkoxy-C₁-C₄-alkoxy, C₁-C₆-Alkylcarbonyloxy, C₁-C₆-Halogenalkylcarbonyloxy, C₃-C₈-Cycloalkylcarbonyloxy, C₁-C₆-Alkylcarbonyl-C₁-C₄-alkoxy, C₁-C₆-Alkylthio, C₁-C₆-Halogenalkylthio, C₃-C₆-Cycloalkylthio, C₁-C₆-Alkylsulfonyl, C₁-C₆-Halogenalkylsulfonyl, C₃-C₈-Cycloalkylsulfonyl, C₁-C₆-Alkylsulfonyloxy, C₁-C₆-Halogenalkylsulfonyloxy, Phenylsulfonyloxy, C₁-C₆-Alkylsulfonylamino oder C₁-C₆-Halogenalkylsulfonylamino,
Z¹⁻⁶ stehen gleich oder verschieden und unabhängig voneinander für Cyano, Halogen, -C(=O)H,-C(=O)NR³R⁴, Phenyl, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, C₁-C₆-Alkoxy, C₁-C₆-Halogenalkoxy, C₂-C₆-Alkenyloxy, C₂-C₆-Alkinyloxy, C₁-C₆-Alkylthio, -NR³R⁴, C₁-C₆-Alkylcarbonyl, C₁-C₆-Alkoxycarbonyl, C₁-C₆-Alkylcarbonyloxy oder C₁-C₆-Halogenalkylthio,
Z² steht gleich oder verschieden unabhängig voneinander bevorzugt für Wasserstoff, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₄-alkoxy-C₁-C₄-alkyl, Phenyl, Benzyl, C₁-C₄-Halogenalkylsulfonyl, C₁-C₆-Alkoxycarbonyl, C₁-C₆-Halogenalkoxycarbonyl, Phenylsulfonyl, C₁-C₄-Alkylsulfonyl, -C(=O)H, oder C₁-C₃-Alkylcarbonyl,
R⁷ steht bevorzugt für Wasserstoff, C₁-C₆-Alkyl, C₁-C₆-Halogenalkyl, Benzyl oder Z³⁻¹, besonders bevorzugt für Wasserstoff, Methyl oder Ethyl,
R⁸ steht bevorzugt für Wasserstoff, C₁-C₆-Alkyl, C₁-C₆-Halogenalkyl, C₃-C₈-Cycloalkyl-C₁-C₄-alkyl, C₃-C₈-Cycloalkyl, C₁-C₄-Alkyl-C₃-C₈-cycloalkyl, C₁-C₄-Halogenalkyl-C₃-C₈-cycloalkyl, C₁-C₄-Alkoxyl-C₁-C₄-alkyl, C₁-C₄-Halogenalkoxy-C₁-C₄-alkyl, Benzyl oder Phenyl,
L³ steht bevorzugt für eine direkte Bindung, -CH₂-, Schwefel, Sauerstoff, -C(=O)O-, -C(=O)NH-, - OC(=O)- oder -NHC(=O)-, besonders bevorzugt für eine direke Bindung,
Z³⁻¹ steht bevorzugt für einen Phenylrest oder einen 5- oder 6-gliedrigen Heteroarylrest, der bis zu zwei Substituenten enthalten kann, wobei die Substituenten unabhängig voneinander ausgewählt sind aus folgender Liste:

### Substituenten am Kohlenstoff:

Halogen, Cyano, Nitro, Hydroxy, Amino, -SH, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₁-C₄-Alkoxyalkyl, C₁-C₆-Alkylcarbonyl, C₁-C₆-Halogenalkylcarbonyl, C₁-C₆-Alkoxycarbonyl, C₁-C₄-Alkoxy, C₁-C₄-Halogenalkoxy, C₂-C₆-Alkenyloxy, C₂-C₆-Alkinyloxy, C₁-C₄-Alkylthio, C₁-C₄-Halogenalkylthio, C₁-C₄-Alkylsulfonyl, C₁-C₄-Halogenalkylsulfonyl oder C₁-C₄-Alkylamino, Di(C₁-C₄-alkyl)amino,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C1-C4-alkoxy-C₁-C₄-alkyl, Phenyl, Benzyl, C₁-C₄-Halogenalkylsulfonyl, C₁-C₆-Alkoxycarbonyl, C₁-C₆-Halogenalkoxycarbonyl, Phenylsulfonyl, C₁-C₄-Alkylsulfonyl, -C(=O)H, oder C₁-C₃-Alkylcarbonyl.

Die erfindungsgemäß verwendbaren Heteroarylpiperidin und -piperazinderivate sind durch die Formel **(I)** allgemein definiert. Die Restedefinitionen der vorstehenden und nachfolgend genannten Reste der Formel **(I)** gelten für die Endprodukte der Formel **(I)** wie für alle Zwischenprodukte (siehe auch unten unter "Erläuterungen der Verfahren und Zwischenprodukte") gleichermaßen.

Die oben aufgeführten bzw. nachfolgenden allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel **(I),** in welcher alle Reste jeweils die oben genannten bevorzugten Bedeutungen haben.

Besonders bevorzugt sind solche Verbindungen der Formel **(I),** in welcher alle Reste jeweils die oben genannten besonders bevorzugten Bedeutungen haben.

Ganz besonders bevorzugt sind solche Verbindungen der Formel **(I),** in welcher alle Reste jeweils die oben genannten ganz besonders bevorzugten Bedeutungen haben.

Weiterhin bevorzugt sind Verbindungen der Formel **(I),** in welcher A für 3,5-Bis(difluormethyl)-1H-pyrazol-1-yl steht,
sowie agrochemisch wirksame Salze, Metallkomplexe und N-Oxide davon.

Weiterhin bevorzugt sind Verbindungen der Formel **(I),** in welcher A für 2,5-Dichlorphenyl steht,
sowie agrochemisch wirksame Salze, Metallkomplexe und N-Oxide davon.

Weiterhin bevorzugt sind Verbindungen der Formel **(I),** in welcher A für 2,5-Bis(difluormethyl)phenyl steht,
sowie agrochemisch wirksame Salze, Metallkomplexe und N-Oxide davon.

Weiterhin bevorzugt sind Verbindungen der Formel **(I),** in welcher A für 5-Methyl-3-(trifluormethyl)-1H-pyrazol-1-yl steht,
sowie agrochemisch wirksame Salze, Metallkomplexe und N-Oxide davon.

Weiterhin bevorzugt sind Verbindungen der Formel **(I),** in welcher A für 2,5-Dimethylphenyl steht,
sowie agrochemisch wirksame Salze, Metallkomplexe und N-Oxide davon.

Weiterhin bevorzugt sind Verbindungen der Formel **(I),** in welcher A für 2-Methoxy-5-methylphenyl steht,
sowie agrochemisch wirksame Salze, Metallkomplexe und N-Oxide davon.

Weiterhin bevorzugt sind Verbindungen der Formel **(I),** in welcher A für 5-Chlor-2-methylphenyl steht,
sowie agrochemisch wirksame Salze, Metallkomplexe und N-Oxide davon.

Weiterhin bevorzugt sind Verbindungen der Formel **(I),** in welcher A für 3-(Difluormethyl)-5-methyl-1H-pyrazol-1-yl steht,

Weiterhin bevorzugt sind Verbindungen der Formel **(I),** in welcher A für 3 -Isopropyl-5-(trifluormethyl)-1H-pyrazol-1-yl steht,

Weiterhin bevorzugt sind Verbindungen der Formel **(I),** in welcher L¹ für -CH₂- steht,
sowie agrochemisch wirksame Salze, Metallkomplexe und N-Oxide davon.

Weiterhin bevorzugt sind Verbindungen der Formel **(I),** in welcher L¹ für -NH- steht,
sowie agrochemisch wirksame Salze, Metallkomplexe und N-Oxide davon.

Weiterhin bevorzugt sind Verbindungen der Formel **(I),** in welcher Y für Schwefel steht,
sowie agrochemisch wirksame Salze, Metallkomplexe und N-Oxide davon.

Weiterhin bevorzugt sind Verbindungen der Formel **(I),** in welcher Y für Sauerstoff steht,
sowie agrochemisch wirksame Salze, Metallkomplexe und N-Oxide davon.

Weiterhin bevorzugt sind Verbindungen der Formel **(I),** in welcher X für Kohlenstoff steht,
sowie agrochemisch wirksame Salze, Metallkomplexe und N-Oxide davon.

Weiterhin bevorzugt sind Verbindungen der Formel **(I),** in welcher R² für Wasserstoff oder Fluor steht,
sowie agrochemisch wirksame Salze, Metallkomplexe und N-Oxide davon.

Weiterhin bevorzugt sind Verbindungen der Formel **(I),** in welcher G für G¹ steht,
sowie agrochemisch wirksame Salze, Metallkomplexe und N-Oxide davon.

Weiterhin bevorzugt sind Verbindungen der Formel **(I),** in welcher Q für Q²⁴-3 steht,
sowie agrochemisch wirksame Salze, Metallkomplexe und N-Oxide davon.

Weiterhin bevorzugt sind Verbindungen der Formel **(I),** in welcher Q für Q¹¹-1 steht,
sowie agrochemisch wirksame Salze, Metallkomplexe und N-Oxide davon.

Weiterhin bevorzugt sind Verbindungen der Formel **(I),** in welcher R^{G1} für Wasserstoff steht,
sowie agrochemisch wirksame Salze, Metallkomplexe und N-Oxide davon.

Weiterhin bevorzugt sind Verbindungen der Formel **(I),** in welcher R⁵ für Wasserstoff steht,
sowie agrochemisch wirksame Salze, Metallkomplexe und N-Oxide davon.

Weiterhin bevorzugt sind Verbindungen der Formel **(I),** in welcher L² für -CH₂O- steht,
sowie agrochemisch wirksame Salze, Metallkomplexe und N-Oxide davon.

Weiterhin bevorzugt sind Verbindungen der Formel **(I),** in welcher L² für -CH₂S- steht,
sowie agrochemisch wirksame Salze, Metallkomplexe und N-Oxide davon.

Weiterhin bevorzugt sind Verbindungen der Formel **(I),** in welcher L² für -C(CH₃)₂- steht,
sowie agrochemisch wirksame Salze, Metallkomplexe und N-Oxide davon.

Weiterhin bevorzugt sind Verbindungen der Formel **(I),** in welcher L² für -CH₂S(O)₂- steht,
sowie agrochemisch wirksame Salze, Metallkomplexe und N-Oxide davon.

Weiterhin bevorzugt sind Verbindungen der Formel **(I),** in welcher L² für -CH₂(OCH₃)- steht,
sowie agrochemisch wirksame Salze, Metallkomplexe und N-Oxide davon.

Weiterhin bevorzugt sind Verbindungen der Formel **(I),** in welcher L² für -Si(CH₃)₂- steht,
sowie agrochemisch wirksame Salze, Metallkomplexe und N-Oxide davon.

Weiterhin bevorzugt sind Verbindungen der Formel **(I),** in welcher L² für -CH₂CH₂(C=O)- steht,
sowie agrochemisch wirksame Salze, Metallkomplexe und N-Oxide davon.

Weiterhin bevorzugt sind Verbindungen der Formel **(I),** in welcher L² für -CF₂- steht,
sowie agrochemisch wirksame Salze, Metallkomplexe und N-Oxide davon.

Weiterhin bevorzugt sind Verbindungen der Formel **(I),** in welcher L² für -CHF- steht,
sowie agrochemisch wirksame Salze, Metallkomplexe und N-Oxide davon.

Weiterhin bevorzugt sind Verbindungen der Formel **(I),** in welcher L² für -CH(OH)- steht,
sowie agrochemisch wirksame Salze, Metallkomplexe und N-Oxide davon.

Weiterhin bevorzugt sind Verbindungen der Formel **(I),** in welcher L² für -CH₂N(CH₃)₂- steht,
sowie agrochemisch wirksame Salze, Metallkomplexe und N-Oxide davon.

Weiterhin bevorzugt sind Verbindungen der Formel **(I),** in welcher L² für -C(CH₃)OH- steht,
sowie agrochemisch wirksame Salze, Metallkomplexe und N-Oxide davon.

Weiterhin bevorzugt sind Verbindungen der Formel **(I),** in welcher L² für -C(CF₃)OH- steht,
sowie agrochemisch wirksame Salze, Metallkomplexe und N-Oxide davon.

Weiterhin bevorzugt sind Verbindungen der Formel **(I),** in welcher R¹ für Phenyl steht,
sowie agrochemisch wirksame Salze, Metallkomplexe und N-Oxide davon.

Weiterhin bevorzugt sind Verbindungen der Formel **(I),** in welcher R¹ für 2-Acetylphenyl steht,
sowie agrochemisch wirksame Salze, Metallkomplexe und N-Oxide davon.

Weiterhin bevorzugt sind Verbindungen der Formel **(I),** in welcher R¹ für 2,6-Difluorphenyl steht,
sowie agrochemisch wirksame Salze, Metallkomplexe und N-Oxide davon.

Weiterhin bevorzugt sind Verbindungen der Formel **(I),** in welcher R¹ für 2,6-Dibromphenyl steht,
sowie agrochemisch wirksame Salze, Metallkomplexe und N-Oxide davon.

Weiterhin bevorzugt sind Verbindungen der Formel **(I),** in welcher R¹ für 2-Methoxyphenyl steht,
sowie agrochemisch wirksame Salze, Metallkomplexe und N-Oxide davon.

Weiterhin bevorzugt sind Verbindungen der Formel **(I),** in welcher R¹ für 2-Methylphenyl steht,
sowie agrochemisch wirksame Salze, Metallkomplexe und N-Oxide davon.

Weiterhin bevorzugt sind Verbindungen der Formel **(I),** in welcher R¹ für 2-Fluorphenyl steht,
sowie agrochemisch wirksame Salze, Metallkomplexe und N-Oxide davon.

Weiterhin bevorzugt sind Verbindungen der Formel **(I),** in welcher R¹ für 2,6-Dichlorphenyl steht,
sowie agrochemisch wirksame Salze, Metallkomplexe und N-Oxide davon.

Weiterhin bevorzugt sind Verbindungen der Formel **(I),** in welcher R¹ für 2-Chlor-6-fluorphenyl steht, sowie agrochemisch wirksame Salze, Metallkomplexe und N-Oxide davon.

Weiterhin bevorzugt sind Verbindungen der Formel **(I),** in welcher R¹ für Thiophen-2-yl steht, sowie agrochemisch wirksame Salze, Metallkomplexe und N-Oxide davon.

Weiterhin bevorzugt sind Verbindungen der Formel **(I),** in welcher R¹ für 2-[(E/Z)-(Methoxyimino)methyl]phenyl steht, sowie agrochemisch wirksame Salze, Metallkomplexe und N-Oxide davon.

Weiterhin bevorzugt sind Verbindungen der Formel **(I),** in welcher R¹ für 2-Chlorphenyl steht, sowie agrochemisch wirksame Salze, Metallkomplexe und N-Oxide davon.

Weiterhin bevorzugt sind Verbindungen der Formel **(I),** in welcher R¹ für 2-Bromphenyl steht, sowie agrochemisch wirksame Salze, Metallkomplexe und N-Oxide davon.

Weiterhin bevorzugt sind Verbindungen der Formel **(I),** in welcher R¹ für 2-[(E/Z)-(Hydroxyimino)methyl]phenyl steht, sowie agrochemisch wirksame Salze, Metallkomplexe und N-Oxide davon.

Weiterhin bevorzugt sind Verbindungen der Formel **(I),** in welcher R¹ für 2-Formyl-6-methoxyphenyl steht, sowie agrochemisch wirksame Salze, Metallkomplexe und N-Oxide davon.

Weiterhin bevorzugt sind Verbindungen der Formel **(I),** in welcher R¹ für 2-Formylphenyl steht, sowie agrochemisch wirksame Salze, Metallkomplexe und N-Oxide davon.

Weiterhin bevorzugt sind Verbindungen der Formel **(I),** in welcher R¹ für 2-Brom-6-fluorphenyl steht, sowie agrochemisch wirksame Salze, Metallkomplexe und N-Oxide davon.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Iod und vorzugsweise Fluor, Chlor, Brom und noch bevorzugter Fluor, Chlor.
Alkyl: gesättigte, geradkettige oder verzweigte Kohlenwasserstoffreste mit 1 bis 8 vorzugsweise 1 bis 6 und noch bevorzugter 1 bis 3 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Diese Definition gilt auch für Alkyl as Bestandteil eines zusammengesetzen Substitutenten wie z.B. Cycloalkylalkyl, Hydroxyalkyl etc. sofern an anderer Stelle nicht definiert wie z.B. Alkylthio, Alkylsufinyl, Alkylsulfonyl, Halogenalkyl bzw. Halogenalkylthio. Steht das Alkyl am Ende eines zusammengesetzten Substituenten,. wie z.B. bei Alkylcycloalkyl, so kann der am Anfang stehende Bestandteil des zusammengesetzten Substituenten, z.B. das Cycloalkyl, ein- bzw. mehrfach, gleich oder verschieden und unabhängig voneinander mit Alkyl substituiert sein. Das gleiche gilt auch für zusammengesetzte Substituenten bei denen andere Reste wie z.B. Alkenyl, Alkinyl, Hydroxy, Halogen, Formyl etc. am Ende stehen.
**Alkenyl:** ungesättigte, geradkettige oder verzweigte Kohlenwasserstoffreste mit 2 bis 8 vorzugsweise 2 bis 6 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-l-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-l-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl. Diese Definition gilt auch für Alkenyl als Bestandteil eines zusammengesetzen Substitutenten wie z.B. Halogenalkenyl etc. sofern an anderer Stelle nicht definiert.
Alkinyl: geradkettige oder verzweigte Kohlenwasserstoffgruppen mit 2 bis 8 vorzugsweise 2 bis 6 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-l-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl. Diese Definition gilt auch für Alkinyl als Bestandteil eines zusammengesetzen Substitutenten wie z.B. Halogenalkinyl etc. sofern an anderer Stelle nicht definiert.
**Alkoxy:** gesättigte, geradkettige oder verzweigte Alkoxyreste mit 1 bis 8 vorzugsweise 1 bis 6 und noch bevorzugter 1 bis 3 Kohlenstoffatomen, z.B. (aber nicht beschränkt auf) C1-C6-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Diese Definition gilt auch für Alkoxy als Bestandteil eines zusammengesetzen Substitutenten wie z.B. Halogenalkoxy, Alkinylalkoxy etc. sofern an anderer Stelle nicht definiert.
**Alkylthio:** gesättigte, geradkettige oder verzweigte Alkylthioreste mit 1 bis 8 vorzugsweise 1 bis 6 und noch bevorzugter 1 bis 3 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Di-methylpropylthio, 1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methyl-propylthio. Diese Definition gilt auch für Alkylthio als Bestandteil eines zusammengesetzen Substitutenten wie z.B. Halogenalkylthio etc. sofern an anderer Stelle nicht definiert.
**Alkoxycarbonyl:** eine Alkoxygruppe mit 1 bis 6 bevorzugt 1 bis 3 Kohlenstoffatomen (wie vorstehend genannt), welche über eine Carbonylgruppe (-CO-) an das Gerüst gebunden ist. Diese Definition gilt auch für Alkoxycarbonyl als Bestandteil eines zusammengesetzen Substitutenten wie z.B. Cycloalkylalkoxycarbonyl etc. sofern an anderer Stelle nicht definiert.
**Alkylsulfinyl:** gesättigte, geradkettige oder verzweigte Alkylsulfinylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl, 1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl. Diese Definition gilt auch für Alkylsulfinyl als Bestandteil eines zusammengesetzen Substitutenten wie z.B. Halogenalkylsulfinyl etc. sofern an anderer Stelle nicht definiert.
**Alkylsulfonyl:** gesättigte, geradkettige oder verzweigte Alkylsulfonylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, Hexylsulfonyl, 1,1-Dimethylpropylsulfonyl, 1,2-Dimethylpropylsulfonyl, 1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl. Diese Definition gilt auch für Alkylsulfonyl als Bestandteil eines zusammengesetzen Substitutenten wie z.B. Alkylsulfonylalkyl etc. sofern an anderer Stelle nicht definiert.
**Cycloalkyl:** monocyclische, gesättigte Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropyl, Cyclopentyl und Cyclohexyl. Diese Definition gilt auch für Cycloalkyl als Bestandteil eines zusammengesetzen Substitutenten wie z.B. Cycloalkylalkyl etc. sofern an anderer Stelle nicht definiert.
**Cycloalkenyl:** monocyclische, partiell ungesättigter Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropenyl, Cyclopentyl und Cyclohexenyl. Diese Definition gilt auch für Cycloalkenyl als Bestandteil eines zusammengesetzen Substitutenten wie z.B. Cycloalkylalkyl etc. sofern an anderer Stelle nicht definiert.
**Cycloalkoxy:** monocyclische, gesättigte Cycloalkyloxyreste mit 3 bis 10 vorzugsweise 3 bis 8 und noch bevorzugter 3 bis 6 Kohlenstoffringgliedern, z.B. (aber nicht beschränkt auf) Cyclopropyloxy, Cyclopentyloxy und Cyclohexyloxy. Diese Definition gilt auch für Cycloalkoxy als Bestandteil eines zusammengesetzen Substitutenten wie z.B. Cycloalkoxyalkyl etc. sofern an anderer Stelle nicht definiert.
**Halogenalkyl:** geradkettige oder verzweigte Alkylgruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl. Diese Definition gilt auch für Halogenalkyl als Bestandteil eines zusammengesetzen Substitutenten wie z.B. Halogenalkylaminoalkyl etc. sofern an anderer Stelle nicht definiert.

Halogenalkenyl und Halogenalkinyl sind analog wie Halogenalkyl definiert, wobei anstatt Alkylgruppen, Alkenyl und Alkinylgruppen als Bestandteil des Substituenten vorliegen.
**Halogenalkoxy:** geradkettige oder verzweigte Alkoxygruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2-difluorethoxy, 2,2-Dichlor-2-fluorethoxy, 2,2,2-Trichlorethoxy, Pentafluor-ethoxy und 1,1,1-Trifluorprop-2-oxy. Diese Definition gilt auch für Halogenalkoxy als Bestandteil eines zusammengesetzen Substitutenten wie z.B. Halogenalkoxyalkyl etc. sofern an anderer Stelle nicht definiert.
**Halogenalkylthio:** geradkettige oder verzweigte Alkylthiogruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor-2-difluorethylthio, 2,2-Dichlor-2-fluorethylthio, 2,2,2-Trichlorethylthio, Pentafluorethylthio und 1,1,1-Trifluorprop-2-ylthio. Diese Definition gilt auch für Halogenalkylthio als Bestandteil eines zusammengesetzen Substitutenten wie z.B. Halogenalkylthioalkyl etc. sofern an anderer Stelle nicht definiert.
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, l-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome:** : z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;

Diese Definition gilt auch für Heteroaryl als Bestandteil eines zusammengesetzen Substitutenten wie z.B. Heteroarylalkyl etc. sofern an anderer Stelle nicht definiert;
**Heterocyclyl:** drei- bis fünfzehngliedriger vorzugsweise ein drei- bis neungliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl. Diese Definition gilt auch für Heterocyclyl als Bestandteil eines zusammengesetzen Substitutenten wie z.B. Heterocyclylalkyl etc. sofern an anderer Stelle nicht definiert;
**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph,-OSO₂PhMe);

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Herstellverfahren und Zwischenprodukte

Die Heteroarylpiperidin und -piperazinderivate der Formel **(I)** lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

Die erfindungsgemäßen Verfahren zur Herstellung von Verbindungen der Formel **(I)** werden gegebenenfalls unter Verwendung eines oder mehrerer Reaktionshilfsmittel durchgeführt.

Als Reaktionshilfsmittel kommen gegebenenfalls anorganische oder organische Basen oder Säureakzeptoren in Betracht. Hierzu gehören vorzugsweise Alkalimetall- oder Erdalkalimetall- -acetate, - amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU). Die erfindungsgemäßen Verfahren werden gegebenenfalls unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im Allgemeinen arbeitet man bei Temperaturen zwischen 0 °C und 250 °C, vorzugsweise bei Temperaturen zwischen 10 °C und 185 °C.

Die Reaktionszeit variiert in Abhängigkeit vom Maßstab der Reaktion und der Reaktionstemperatur, liegt aber im Allgemeinen zwischen einigen Minuten und 48 Stunden.

Die erfindungsgemäßen Verfahren werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden.

### Verfahren A

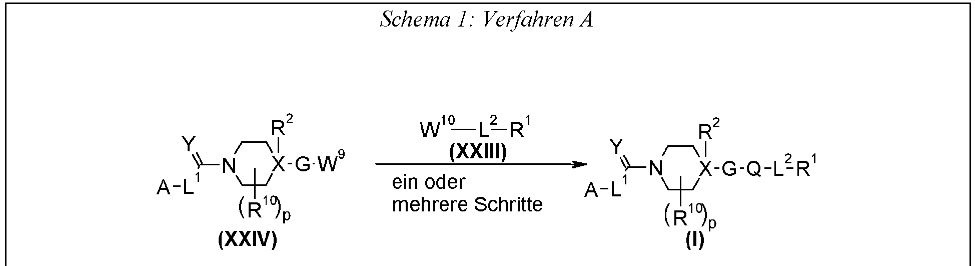

W⁹ und W¹⁰ sind funktionelle Gruppen geeignet für das Bilden des gewünschten Heterocyclus

Im Allgemeinen ist es möglich Verbindungen der Formel **(I)** aus entsprechenden Verbindungen **(XXIII)** und **(XXIV)** mit geeigneten funktionellen Gruppen W⁹ und W¹⁰ **(I)** herzustellen (s. Schema 1, Verfahren A). Die möglichen funktionellen Gruppen für W⁹ und W¹⁰ sind z.B. Aldehyde, Ketone, Ester, Carbonsäuren, Amide, Thioamide, Nitrile, Alkohole, Thiole, Hydrazine, Oxime, Amidine, Amidoxime, Olefine, Acetylene, Halide, Alkylhalide, Methansulfonate, Trifluormethansulfonate, Boronsäuren, Boronate u.s.w., die unter geeigneten Reaktionsbedingungen den gewünschten Heterocyclus **Q** bilden können. In der Literatur finden sich zahlreiche Methoden für die Darstellung von Heterocyclen (s. WO 2008/013622; Comprehensive Heterocyclic Chemistry Vol. 4-6, A. R. Katritzky and C. W. Rees editors, Pergamon Press, New York, 1984; Comprehensive Heterocyclic Chemistry II, Vol 2-4, A. R. Katritzky, C. W. Rees and E. F: Scriven editors, Pergamon Press, New York, 1996; The Chemistry of Heterocyclic Compounds, E. C. Taylor, editor, Wiley, New York; Rodd's Chemistry of Carbon Compounds, Vol. 2-4, Elsevier, New York; Synthesis, 1982, 6, 508-509; Tetrahedron, 2000, 56, 1057-1094; und darin zitierte Literatur).

### Verfahren B

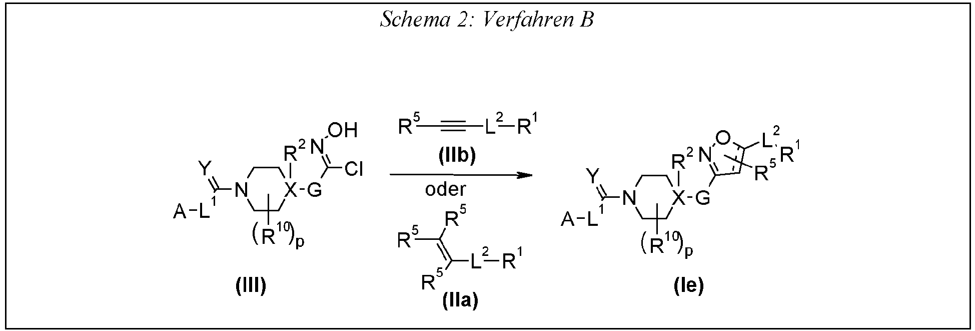

Eine bestimmte Möglichkeit, Verbindungen der Formel **(Ie)** aus entsprechenden Verbindungen **(III)** durch Reaktion mit den Verbindungen **(IIa)** oder **(IIb)** darzustellen, ist in Schema 2 gezeigt.

Die Alkene und Alkine **(IIa)** und **(IIb)** sind kommerziell verfügbar oder können aus kommerziell erhältlichen Vorstufen nach literaturbeschriebenen Vorschriften hergestellt werden (**Abbildung 1** und Schema 2). 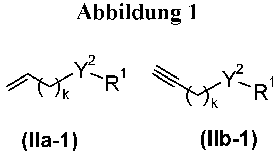
Y² = O, S, NR²³, Si(R²⁶)₂
k = 1-5,

Der Alken **(IIa-2)** wird beispielsweise aus dem entsprechenden Keton **(IIc)** durch Fluorierung mit (Diethylamino)schwefeltrifluorid (DAST) oder Bis(2-methoxyethyl)aminoschwefeltrifluorid (Deoxofluor) dargestellt (siehe z.B. Advances in Organic Synthesis, 2006, 2, 291-326, Singh, R. P., Meshri, D. T., Shreeve, J. M., Bentham Science Publishers Ltd; Singh, R. P., Shreeve, J. M. Synthesis 2002, 17, 2561-2578; Chang, Y., Tewari, A., Adi, A., Bae, C. Tetrahedron 2008, 64, 9837-9842; und darin zitierte Literatur). Das Alken **(IIa-3), (IIa-4)** oder **(IIa-5)** kann auch durch Acetalisierung, reduktive Aminierung, nukleophile Addition oder Reduktion dargestellt werden (siehe Schema 3). Die Alkene **(IIa-6)** und **(IIa-7)** können aus Verbindungen **(IIa-5)** durch Halogenierung und Schwefelung herstellt werden (s. z.B. für Acetalisirung: Meskens, F. A. J. Synthesis 1981, 501; für reduktive Aminierung: Nugent, T. C., El-Shazly, M. Adv. Synth. Catal. 2010, 352, 753-819; Margaretha, P. Science of Synthesis 2008, Vol. 40, 65-89, Georg Thieme Verlag; für nukleophile Addition, Reduzierung, Halogenierung und Schwefelung: Smith. M. B., March J. March's Advanced Organic Chemistry, 6th Ed. 2007, Wiley-Interscience; WO 2006/11761 A1; Shandala, M. Y., Khalil, S. M.; Al-Dabbagh, M. S Tetrahedron 1984, 40, 1195-8; Yang, X.; Mague, J. T.; Li, C. J. Org. Chem. 2001, 66, 739-747; und darin zitierte Literatur). 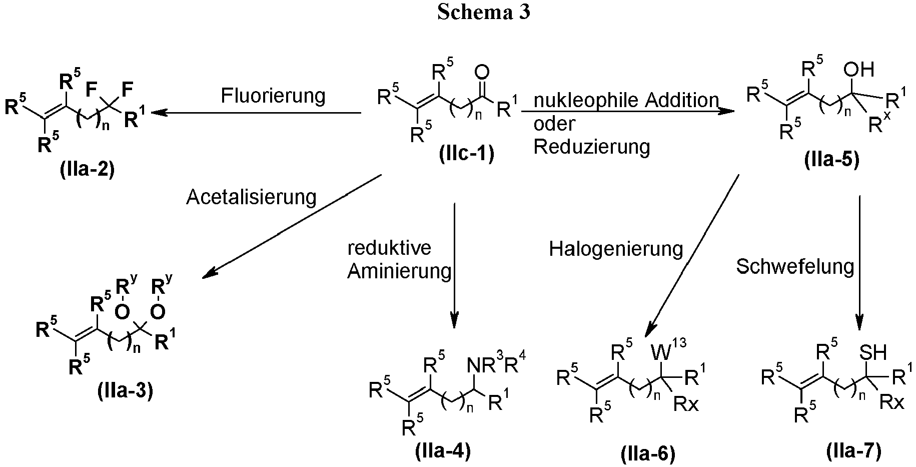
n = 0, 1, 2, 3, oder 4,
R^{y} = Alkyl, Halogenalkyl, Alkylcarbonyl
R^{x} = Alkyl, Halogenalkyl, Alkenyl, Alkinyl,
W¹³ = Halogen, Toluolsulfonyloxy, Methylsulfonyloxy,

Weitere Umsetzungen mit Verbindungen **(IIa-4), (IIa-5)** und **(IIa-7)** sind durchführbar. Beispielsweise lassen sich durch Substitutionsreaktion mit elektrophilen Reagenzien **(XXII)** Verbindungen **(IIa-9)** darstellen (Schema 4). In der Literatur finden sich zahlreiche Methoden für die Darstellung aus Aminen, Thioalkoholen und Alkoholen (s. Smith. M. B., March J. March's Advanced Organic Chemistry, 6th Ed. 2007, Wiley-Interscience; Matsuda, H., Ando, S., Morikawa, T., Kataoka, S., Yoshikawa, M. Bioorg. Med. Chem. Lett. 2005, 15, 1949-1953; US 2005/0234040 A1; Masquelin, T. Obrecht, D. Tetrahedron Lett. 1994, 35, 9387-90; Bondzic, B. P., Eilbracht, P. Org. Lett. 2008, 10, 3433-3436; Bondzic, B. P., Farwick, A., Liebich, J., Eilbracht, P.v. Org. Biomol. Chem. 2008, 6, 3723-3731; und darin zitierte Literatur). 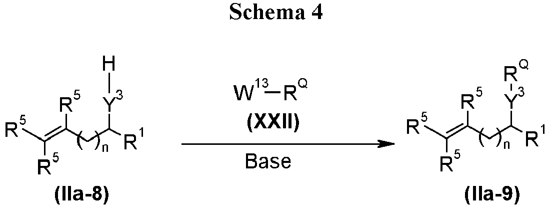
W¹³ = Halogen, Toluolsulfonyloxy, Methylsulfonyloxy,
Y³ = O, S, NR²³, und
R^{Q} = C₁-C₄-Alkyl, C₁-C₄-Halogenalkyl, C₃-C₄-Alkenyl, C₃-C₄-Halogenalkenyl, C₃-C₄-Alkinyl, C₃-C₄-Halogenalkinyl, C₂-C₄-Alkylcarbonyl

Analog zu dem in Schema 2 gezeigten Verfahren, werden Verbindungen der Formel **(IIb)** dargestellt.

Eine Verbindung der allgemeinen Formel **(Ie)** wird aus einem Alken der allgemeinen Formel **(IIa)** oder aus einem Alkin der Formel **(IIb)** und Verbindung **(III)** durch eine Zykloadditionsreaktion erhalten (s. z.B. WO 08/013622 und Synthesis, 1987, 11, 998-1001).

Das *Verfahren B* wird in Gegenwart einer geeigneten Base durchgeführt. Bevorzugte Basen sind tertiäre Amine (z.B. Triethylamin), Alkalimetall- oder Erdalkalimetallcarbonate, Hydrogencarbonate und Phosphate.

Das *Verfahren B* wird vorzugsweise unter Verwendung eines oder mehrer Verdünnungsmittel durchgeführt. Bei der Durchführung des *Verfahrens B* kommen vorzugsweise inerte organische Lösungsmittel infrage (wie z.B. Toluol und Hexan). Ebenso kommt Wasser als Lösungsmittel infrage. Alternativ kann das *Verfahren B* in einem Überschuß des Alkens **(IIa)** oder des Alkins **(IIb)** durchgeführt werden.

Die Aufarbeitung erfolgt nach üblichen Methoden. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

### Verfahren C

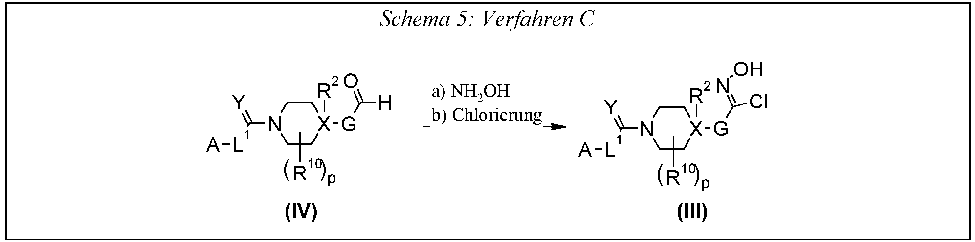

Eine Möglichkeit, das Zwischenprodukt **(III)** aus Verbindung **(IV)** herzustellen, ist in Schema 5 (*Verfahren C*) gezeigt

Eine Verbindung der allgemeinen Formel **(III)** wird durch Kondensation eines Aldehyds der Formel **(IV)** mit Hydroxylamin und anschließender Chlorierung erhalten (s. z.B. WO 05/0040159, WO 08/013622 und Synthesis, 1987, 11, 998-1001).

Im *Verfahren* C werden zunächst Aldehyd **(IV)** und Hydroxylamin zur Reaktion gebracht (Schema 5, Schritt (a)). Das korrespondierende Oxim wird anschließend in Gegenwart eines geeingeneten Chlorierungsmittels chloriert. Bevorzugte Chlorierungsreagenzien sind N-Chlorsuccinimid, HClO und Chlor. Nach dem Schritt (a) des *Verfahrens C* kann die Reaktionsmischung nach üblichen Methoden aufgearbeitet oder direkt im Schritt (b) weiter umgesetzt werden.

Das *Verfahren C* wird vorzugsweise unter Verwendung eines oder mehrer Verdünnungsmittel durchgeführt. Im Schritt (a) des erfindungsgemäßen *Verfahrens C* werden bevorzugt protische Lösungsmittel, wie z.B. Ethanol, als Lösungsmittel verwendet. Nach der Bildung des korrespondierenden Oxims aus Verbindung **(IV)** wird die Reaktionsmischung im Schritt (b) mit einem weiteren Lösungsmittel verdünnt, z.B. Tetrahydrofuran, und anschließend mit wässrigem Natriumhypochlorit versehen. Ebenso kann die Chlorierung mit Hilfe von N-Chlorsuccinimid in DMF erfolgen.

Die Aufarbeitung erfolgt nach üblichen Methoden. Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren D

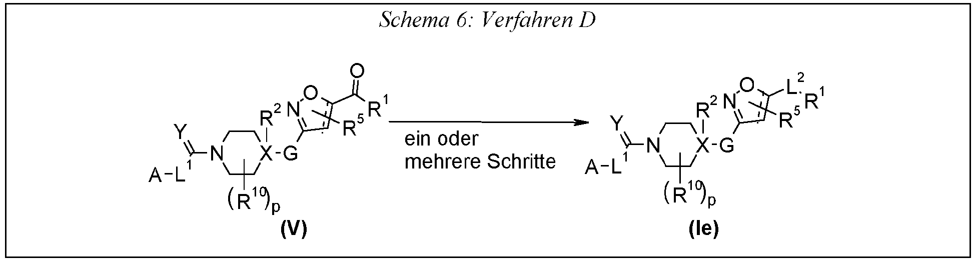

Eine bestimmte Möglichkeit, Verbindungen der Formel **(Ie)** aus entsprechenden Verbindungen **(V)** herzustellen, ist in Schema 6 (*Verfahren D)* gezeigt. *Verfahren D* wird analog *Verfahren B* (in Schema 3 und 4) durchgeführt.

### Verfahren E

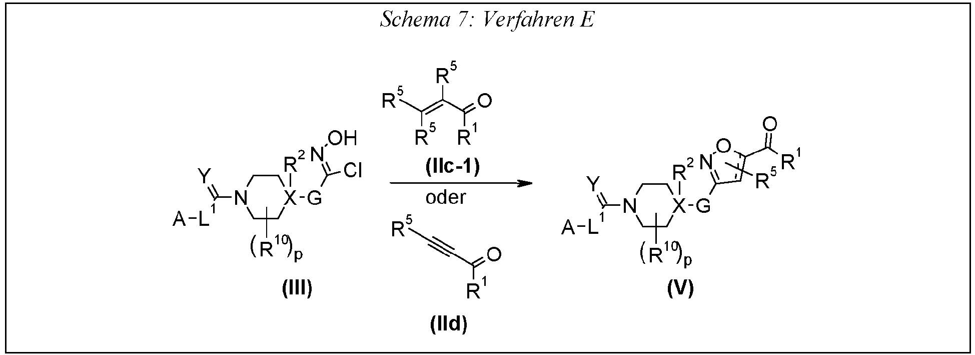

Eine bestimmte Möglichkeit, das Zwischenprodukt **(V)** aus Verbindung **(III)** mit den Verbindungen **(IIc-1)** oder **(IId)** herzustellen, ist in Schema 7 (*Verfahren E*) gezeigt. *Verfahren E* wird analog *Verfahren B* (Schema 2) durchgeführt.

### Verfahren F

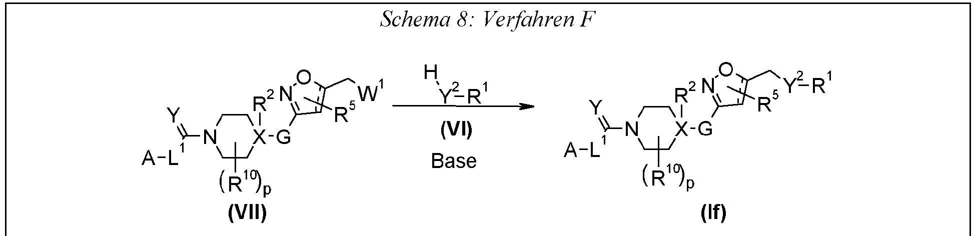

W¹ steht für Chlor, Brom, Iod, Toluolsulfonyloxy,
Y² steht für O, S, NR²³,

Eine bestimmte Möglichkeit, Verbindungen der Formel **(If)** aus entsprechenden Verbindungen **(VII)** mit den Verbindungen **(VI)** herzustellen, ist in Schema 8 (*Verfahren F*) gezeigt.

In Anwesenheit einer organischen oder anorganischen Base wie z.B K₂CO₃ oder Cs₂CO₃, werden Verbindungen der Formel **(If)** aus entsprechenden Verbindungen **(VII)** mit den Verbindungen **(VI)** hergestellt. Die Reaktionstemperatur für die Substitution kann in einem größeren Bereich variiert werden und es können verschiedene inerte Lösungsmittel verwendet werden, wie z.B DMF, CH₃CN und Aceton. Für die Reaktion kann auch die Anwesenheit eines Cokatalysators hilfreich sein, wie z.B. Kaliumiodid oder Tetrabutylammoniumiodid (s z.B. Mao, J., Yuan, H., Wang, Y. et al Biorg. Med. Chem. Lett. 2010, 20, 1263-1268; Mirzae, Y. R., Tabrizi, S. B., Edjlali, L. Acta Chim. Slov. 2008, 55, 554-561; Huang Q. et al J. Med. Chem. 2009, 52, 6757-6767; WO 2006/114272 und darin zitierte Literatur).

Die substituierten Verbidungen der Formel **(VI)** sind entweder kommerziell verfügbar oder können durch literaturbekannte Methoden aus kommerziell verfügbaren Vorstufen hergestellt werden.

### Verfahren G

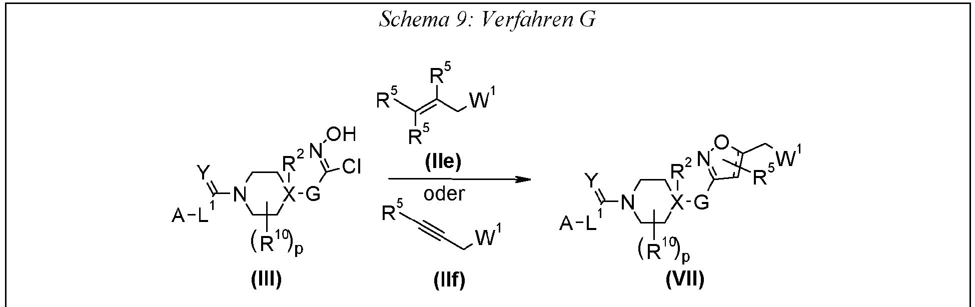

W¹ steht für Chlor, Brom, Iod, Toluolsulfonyloxy

Eine bestimmte Möglichkeit, das Zwischenprodukt **(VII)** aus entsprechenden Verbindungen **(III)** mit den Verbindungen **(IIe)** oder **(IIf)** herzustellen, ist in Schema 9 (*Verfahren G*) gezeigt. Die Alkene **(IIe)** und Alkine **(IIf)** sind kommerziell verfügbar oder können aus kommerziell erhältlichen Vorstufen nach literaturbeschriebenen Vorschriften hergestellt werden (z.B. aus Allylalkoholen oder Propargylalkoholen, oder aus nukleophilen Additionsprodukten von Vinyl- oder Acetylenyl-Nukleophilen an Ketone oder Aldehyde, siehe z.B. Smith. M. B., March J. March 's Advanced Organic chemistry, 6th Ed. 2007, Wiley-Interscience; für Nukleophile Addition von Vinyl- oder Acetylenyl-Nukleophilen: Palin, R. et al Bioorg. Med. Chem. Lett. 2005, 15, 589-593; Kanady, J. S., Nguyen, J. D., Ziller, J. W., Vanderwal, C. D. J. Org. Chem. 2009, 74, 2175-2178; für Halogenierung: Yamakoshi, S., Hayashi, N., Suzuki, T., Nakada, M. Tetrahedron Lett. 2009, 50, 5372-5375; Niggemann, M., Jelonek, A., Biber, N., Wuchrer, M., Plietker, B. J. Org. Chem. 2008, 73, 7028-7036; Forbeck, E. M., Evans, C. D., Gilleran, J. A., Li, P., Joullie, Madeleine M. J. Am. Chem. Soc. 2007, 129, 14463-14469; Gichinga, M., Striegler, S. Tetrahedron 2009, 65, 4917-492; und darin zitierte Literatur). *Verfahren G* wird analog *Verfahren B* (Schema 2) durchgeführt.

### Verfahren H

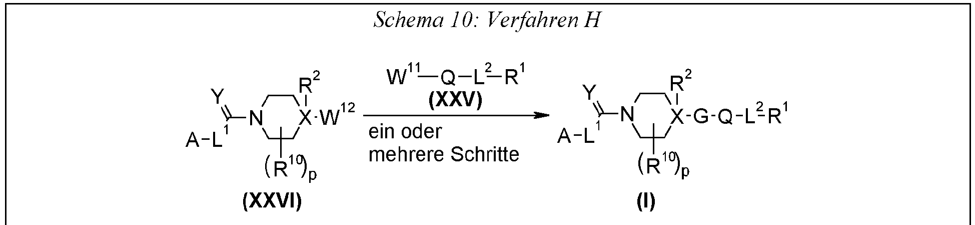

W¹¹ und W¹² sind funktionelle Gruppen geeignet für das Bilden des gewünschten Heterocyclus

Im Allgemeinen ist es möglich die Verbindugen der Formel **(I)** aus entsprechenden Verbindungen **(XXVI)** und **(XXV)** mit geeigneten funktionellen Gruppen, W¹¹ und W¹², **(I)** herzustellen (s. Schema 10, *Verfahren H*). Mögliche funktionelle Gruppen für W¹¹ und W¹² sind z.B. Aldehyde, Ketone, Ester, Carbonsäuren, Amide, Thioamide, Nitrile, Alkohole, Thiole, Hydrazine, Oxime, Amidine, Amidoxime, Olefine, Acetylene, Halide, Alkylhalide, Methansulfonate, Trifluormethansulfonate, Boronsäuren, Boronate u.s.w. Sie können unter geeigneten Reaktionsbedingungen den gewünschten 5-gliedrigen Heterocyclus G bilden. In der Literatur finden sich zahlreiche Methoden für die Darstellung von Heterocyclen (s. WO 2008/013622; Comprehensive Heterocyclic Chemistry Vol. 4-6, A. R. Katritzky and C. W. Rees editors, Pergamon Press, New York, 1984; Comprehensive Heterocyclic Chemistry II, Vol 2-4, A. R. Katritzky, C. W. Rees and E. F: Scriven editors, Pergamon Press, New York, 1996; The Chemistry of Heterocyclic Compounds, E. C. Taylor, editor, Wiley, New York; Rodd's Chemistry of Carbon Compounds, Vol. 2-4, Elsevier, New York; und darin zitierte Literatur).

### Verfahren I

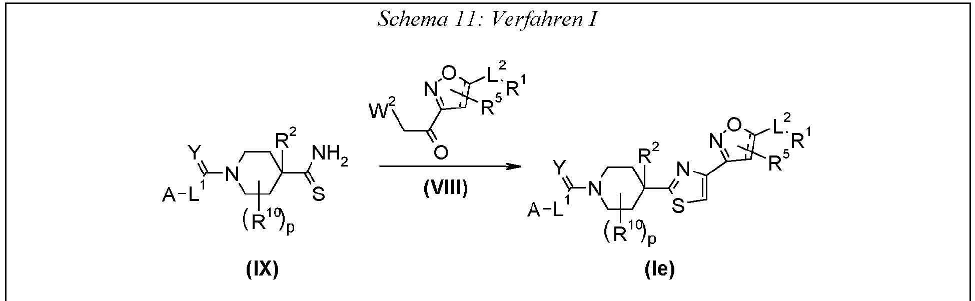

W² steht für Chlor, Brom, Iod, Toluolsulfonyloxy

Eine bestimmte Möglichkeit zur Synthese von Verbindungen der Formel **(Ie)** aus Verbindungen **(IX)** mit den Verbindungen **(VIII)** ist in Schema 11 (*Verfahren 1*) gezeigt.

Thiocarboxamide **(IX)** sind nach literaturbekannten Methoden erhältlich, beispielsweise durch Schwefelung des kommerziell erhältlichen entsprechenden Carboxamids durch Verwendung von z.B. Lawesson's Reagenz (WO2008/013622, Org. Synth. Vol. 7, 1990, 372).

α-Haloketone oder entsprechende Äquivalente (z.B. Toluolsulfonyloxy) sind auch nach literaturbekannten Methoden erhältlich (Beispiele s. WO2008/013622), (Schema 12). 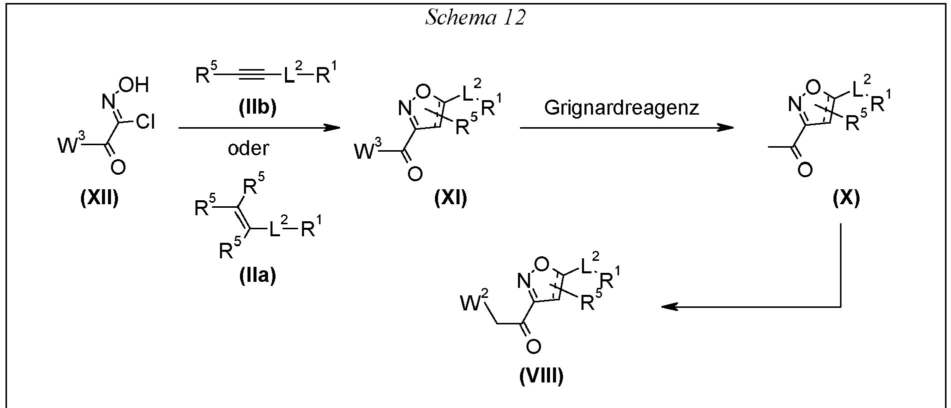 W³ steht für N,N-Dimethylamino, N-Methoxy-N-methylamino oder Morpholin-1-yl,

Die Thiazole **(Ie)** werden durch eine Hantzsch-Thiazol-Synthese aus den Thiocarboxamiden **(IX)** und α-Halogenketonen oder entsprechende Äquivalente **(VIII)** erhalten (siehe z.B. "Comprehensive Heterocyclic Chemistry", Pergamon Press, 1984; Vol 6, Seite 235-363, "Comprehensive Heterocyclic Chemistry II", Pergamon Press, 1996; Vol 3, Seite 373-474 und darin zitierte Referenzen, und WO 07/014290).

Das *Verfahren I* wird vorzugsweise unter Verwendung eines oder mehrer Verdünnungsmittel durchgeführt. Bei der Durchführung des *Verfahrens I* kommen vorzugsweise inerte organische Lösungsmittel infrage (wie z.B. sind N,N-Dimethylformamid und Ethanol).

Gegebenenfalls wird eine Hilfsbase, wie beispielsweise Triethylamin, verwendet.

Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren J

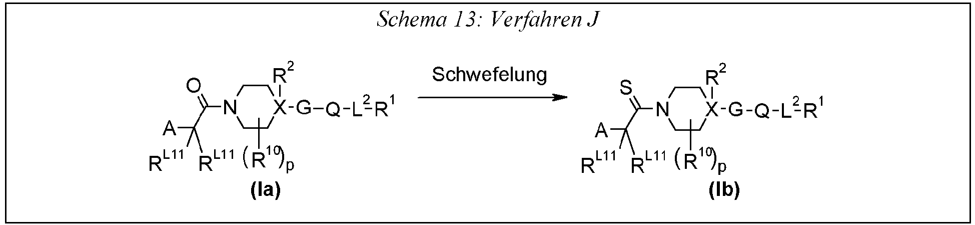

Die bei der Durchführung des erfindungsgemäßen *Verfahrens J* (Schema 13) erhaltenen Amide **(Ia)** lassen sich mittels literaturbeschriebener Methoden in die korrespondierenden Thioamide **(Ib)** überführen (z.B. Bioorganic & Medicinal Chemistry Letters, 2009, 19(2), 462-468). Hierbei werden die Verbindungen der Formel **(Ia)** typischerweise mit Phosphorpentasulfid oder 2,4-Bis(4-methoxyphenyl)-1,3-dithia-2,4-diphosphetan-2,4-disulfid (Lawesson Reagenz) umgesetzt.

Das erfindungsgemäße *Verfahren J* wird vorzugsweise unter Verwendung eines oder mehrerer Verdünnungsmittel durchgeführt. Die bevorzugten Lösungsmittel sind Toluol, Tetrahydrofuran und 1,2-Dimethoxyethan.

Nach Beendigung der Reaktion werden die Verbindungen **(Ib)** von der Reaktionsmischung durch eine der üblichen Trenntechniken getrennt. Falls notwendig werden die Verbindungen durch Umkristallisation oder Chromatographie gereinigt.

### Verfahren K

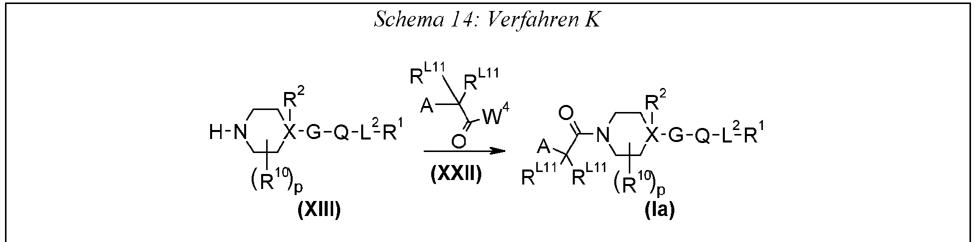

W⁴ = OH oder Cl

Eine Möglichkeit, Verbindungen der Formel **(Ia)** aus entsprechenden Verbindungen **(XIII)** mit den Verbindungen **(XXII)** darzustellen, ist in Schema 14 (*Verfahren K*) gezeigt

Verbindungen **(XXII)** sind entweder kommerziell erhältlich oder durch in der Literatur beschriebene Prozesse darstellbar (s. z.B. WO 2008/013622 und WO 2008/013925).

Eine Verbindung mit der allgemeinen Formel **(Ia)** kann analog zu in der Literatur beschriebenen Vorschriften (s. z.B. WO 2007/147336) durch eine Kupplungsreaktion einer Verbindung mit der entsprechenden allgemeinen Formel **(XIII)** mit einem Substrat der allgemeinen Formel **(XXII),** wobei W⁴ für Chlor steht, gegebenenfalls in der Gegenwart eines Säurefängers / Base synthetisiert werden.

Wenigstens ein Äquivalent eines Säurefängers / einer Base (z.B. Hünig Base, Triethylamin oder kommerziell erhältliche polymere Säurefänger) wird im Verhältnis zum Startmaterial der allgemeinen Formel **(XIII)** verwendet. Ist das Startmaterial ein Salz, werden wenigstens zwei Äquivalente des Säurefängers benötigt.

Alternativ kann eine Verbindung der Formel **(Ia),** auch aus der entsprechenden Verbindung der Formel **(XIII)** mit einem Substrat der Formel **(XXII),** wobei W⁴ für Hydroxy steht, in Gegenwart eines Kupplungsreagenzes analog zu in der Literatur beschriebenen Vorschriften synthetisiert werden (z.B. Tetrahedron 2005, 61, 10827-10852, und darin zitierte Referenzen).

Geeignete Kupplungsreagenzien sind beispielsweise Peptidkupplungsreagenzien zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.

Nach Beendigung der Reaktion, werden die Verbindungen **(Ia)** von der Reaktionsmischung durch eine der üblichen Trenntechniken getrennt. Falls notwendig werden die Verbindungen durch Umkristallisation oder Chromatographie gereinigt.

### Verfahren L

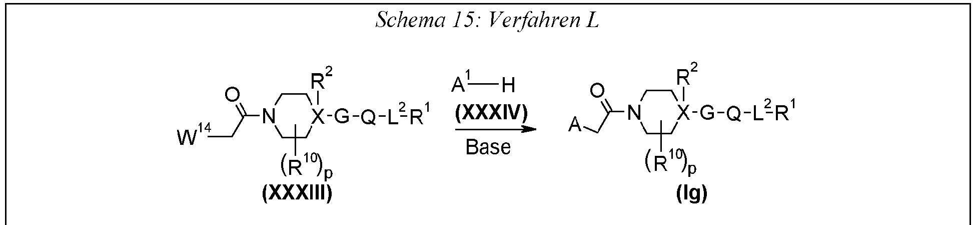

W¹⁴ = OSO₂CH₃, OSO₂C₆H₄CH₃, I, Br oder Cl

In A¹-H ist der Wasserstoff über ein Stickstoffatom angebunden,

Eine Möglichkeit, Verbindungen der Formel **(Ig)** aus entsprechenden Verbindungen **(XXXIII)** mit den Verbindungen **(XXXIV)** darzustellen, ist in Schema 15 (*Verfahren L*) gezeigt

Die Ausgangstoffe, α-Halogeacetamid oder die äquivalente Abgangsgruppe **(XXXIII),** lassen sich mittels literaturbeschriebener Methoden aus Verbindungen **(XXXV), (XVIIe) (Abbildung 2)** oder aus **(XIII)** herstellen (s. z.B. Mesylierung: Organic Letters, 2003, 2539-2541; Tosylierung: JP60156601; Halogenierung: Australian Journal of Chemistry, 1983, 2095-2110;). Typischerweise werden die Verbindungen der Formel **(XXXIII,** W¹⁴ = Chlor) ausgehend von einem Amid der Formel **(XIII)** und Chloracetylchlorid hergestellt. Die Verbindungen **(XXXV)** in **(Abbildung 2)** werden analog zu *Verfahren K* mit Glycolsäure oder Hydroxyacetylchlorid aus **(XIII)** hergestellt (s. z.B. WO2007103187, WO 2006117521, Bioorganic & Medicinal Chemistry Letters, 2007, 6326-6329).

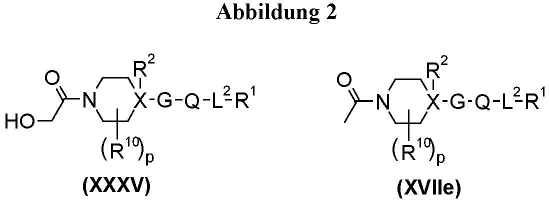

Wenigstens ein Äquivalent einer Base (z.B. Natriumhydrid, Kaliumcarbonat) wird im Verhältnis zum Startmaterial der allgemeinen Formel **(XXXIV)** verwendet.

Nach Beendigung der Reaktion werden die Verbindungen **(Ig)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden.

### Verfahren M

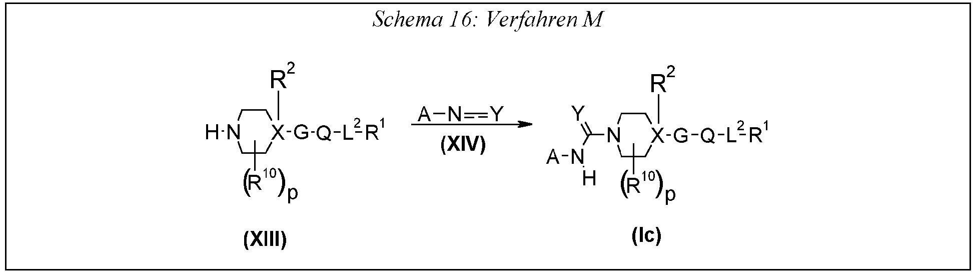

Eine Möglichkeit, Verbindungen der Formel **(Ic)** aus entsprechenden Verbindungen **(XIII)** mit den Verbindungen **(XIV)** darzustellen, ist in Schema 16 (*Verfahren M*) gezeigt.

Eine Verbindung mit der allgemeinen Formel **(Ic)** kann analog zu in der Literatur beschriebenen Vorschriften (s. z.B. WO 2009/055514) durch eine Kupplungsreaktion einer Verbindung mit der entsprechenden allgemeinen Formel **(XIII)** mit einem Substrat der allgemeinen Formel **(XIV),** gegebenenfalls in der Gegenwart eines Säurefängers / Base, wie z.B. Triethylamin, 1,8-Diazabicyclo[5.4.0]undec-7-en oder Hünig-Base synthetisiert werden.

Nach Beendigung der Reaktion, werden die Verbindungen **(Ic)** von der Reaktionsmischung durch eine der üblichen Trenntechniken getrennt. Falls notwendig werden die Verbindungen durch Umkristallisation oder Chromatographie gereinigt.

### Verfahren N

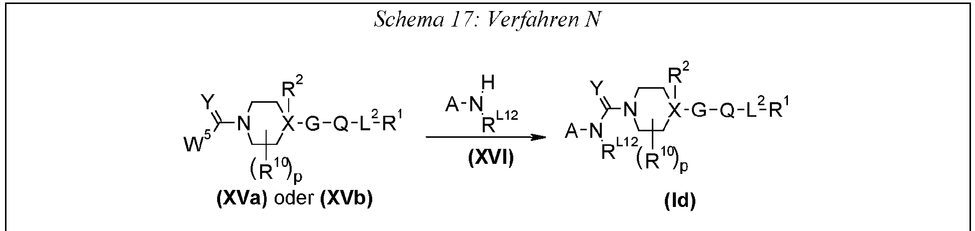

W⁵ steht für Chlor oder Imidazol-1-yl

Eine Möglichkeit, Verbindungen der Formel **(Id)** aus entsprechenden Verbindungen **(XVa)** oder **(XVb)** (s. Abbildung 3) mit den Verbindungen **(XVI)** darzustellen, ist in Schema 17 (*Verfahren N*) gezeigt.

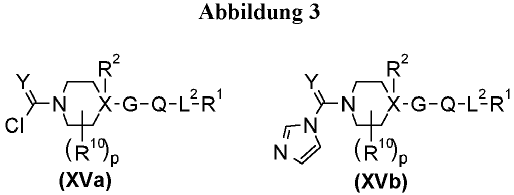

Die Ausgangstoffe, Carbamoyl- und Thiocarbamoylchloride der Formel (**XVa,** W⁵ = Chlor), lassen sich mittels literaturbeschriebener Methoden aus Verbindungen **(XIII)** herstellen (s. z.B. Tetrahedron, 2008, 7605; Journal of Organic Chemistry, 2004, 3787; Journal of Organic Chemistry, 1983, 4750; European Journal of Organic Chemistry, 2006, 1177). Typischerweise werden die Verbindungen der Formel (**XVa,** W⁵ = Chlor) ausgehend von Aminen der Formel **(XIII)** und Phosgen, Thiophosgen oder deren Äquivalenten hergestellt.

Die alternativen Ausgangstoffe, Carbamoyl- und Thiocarbamoylimidazole der Formel (**XVb,** W⁵ = imidazol-1-yl), lassen sich mittels literaturbeschriebener Methoden herstellen (s. z.B. Tetrahedron Letters, 2008, 5279; Tetrahedron, 2005, 7153). Typischerweise werden die Verbindungen der Formel (**XVb,** W5 = imidazol-1-yl) ausgehend von Aminen der Formel **(XIII)** und 1,1'-Carbonyldiimidazole oder 1,1'-Thiocarbonyldimidazole hergestellt.

Das *Verfahren N* wird gegebenenfalls in Gegenwart eines geeigneten Säureakzeptors durchgeführt.

Die bei der Durchführung des erfindungsgemäßen *Verfahren N* erhaltenen Verbindungen **(Id)** können alternativ teilweise auch ohne Verwendung eines Säureakzeptors als korrespondierende Säurechloride [**(Id)**-HCl] erhalten werden. Bei Bedarf erfolgt die Freisetzung der Verbindungen **(Id)** nach üblichen Methoden.

Nach Beendigung der Reaktion, werden die Verbindungen **(Id)** von der Reaktionsmischung durch eine der üblichen Trenntechniken getrennt. Falls notwendig werden die Verbindungen durch Umkristallisation oder Chromatographie gereinigt.

### Verfahren O

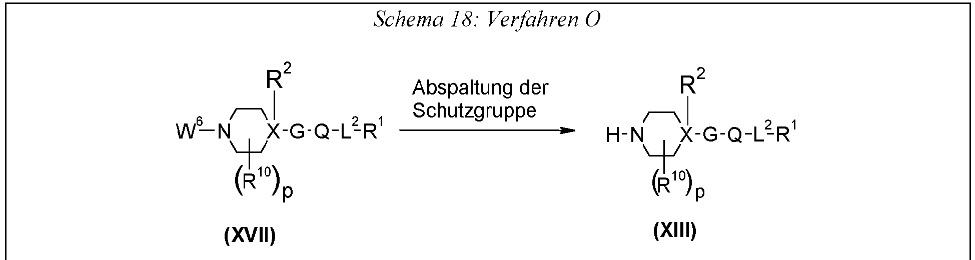

W⁶ steht für Acetyl, C₁-C₄-Alkoxycarbonyl, Benzyl oder Benzyloxycarbonyl

Eine Möglichkeit Verbindungen der Formel **(XIII)** aus entsprechenden Verbindungen **(XVII)** darzustellen ist in Schema 18 (*Verfahren O*) gezeigt.

Eine Verbindung der Formel **(XVII)** wird in eine Verbindung der Formel **(XIII)** durch geeignete Methoden zur Entfernung von Schutzgruppen, die in der Literatur beschrieben sind ("Protective Groups in Organic Synthesis"; Third Edition; 494-653, und dort zitierte Literatur), überführt.

*tert*-Butoxycarbonyl- und Benzyloxycarbonylschutzgruppen können im sauren Medium entfernt werden (z.B mit Salzsäure oder Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Nach Beendigung der Reaktion werden die Verbindungen **(XIII)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden. Es ist außerdem möglich, die Verbindung der allgemeinen Formel **(XIII)** als Salz zu isolieren, z.B. als Salz der Salzsäure oder der Trifluoressigsäure.

### Verfahren P

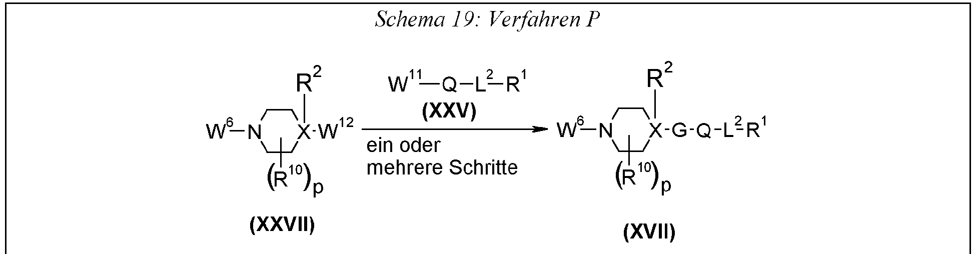

W⁶ steht für Acetyl, C₁-C₄-Alkoxycarbonyl, Benzyl oder Benzyloxycarbonyl,
W¹¹ und W¹² sind funktionelle Gruppen geeignet für das Bilden des gewünschten Heterocyclus

Im Allgemeinen ist es möglich, das Zwischenprodukt **(XVII)** aus entsprechenden Verbindungen **(XXVII)** mit Verbindungen **(XXV)** herzustellen. *Verfahren P* (Schema 19) wird analog *Verfahren H* (Schema 10) durchgeführt.

### Verfahren Q

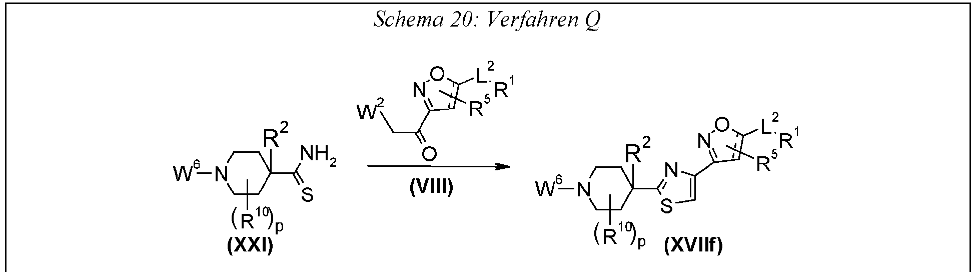

W² steht für Chlor, Brom, Iod, Toluolsulfonyloxy
W⁶ steht für Acetyl, C₁-C₄-Alkoxycarbonyl, Benzyl oder Benzyloxycarbonyl,

Eine andere Möglichkeit, das Zwischenprodukt der Formel **(XVIIf)** aus entsprechenden Verbindungen **(XXI)** herzustellen, ist in Schema 20 (*Verfahren Q*) gezeigt. Verbindungen **(XXI)** sind entweder kommerziell erhältlich oder durch in der Literatur beschriebene Prozesse darstellbar (s. z.B. WO 2008/013622 und WO 2007/014290). *Verfahren* Q wird analog *Verfahren I* (Schema 11) durchgeführt.

### Verfahren R

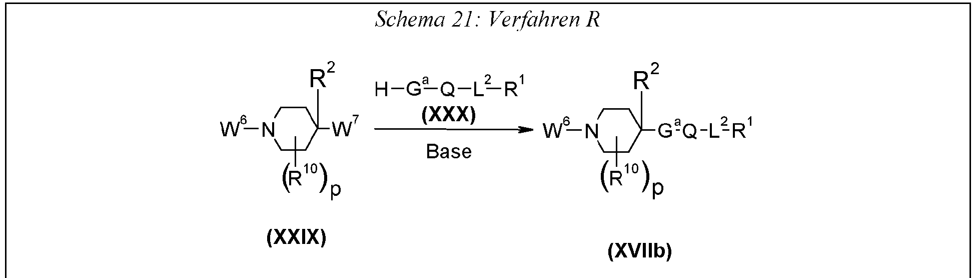

G^{a}: Der Piperidin-Rest ist über ein Stickstoffatom oder Kohlenstoffatom verbunden,
W⁶ steht für Acetyl, C₁-C₄-Alkoxycarbonyl, Benzyl oder Benzyloxycarbonyl,
W⁷ steht für Brom, Iod, Methylsulfonyloxy oder Trifluormethylsulfonyloxy

Eine Verbindung mit der allgemeinen Formel **(XVIIb)** kann analog zu in der Literatur beschriebenen Vorschriften durch eine Kupplungsreaktion einer Verbindung mit der entsprechenden allgemeinen Formel **(XXIX)** mit einem Substrat der allgemeinen Formel **(XXX)** gegebenenfalls in der Gegenwart einer Base synthetisiert werden (Schema 21, *Verfahren R*). (s. z.B. für nukleophile Substitutionen: WO 2008/104077; WO 2006/084015 (Pyrazole mit N-Substitution); für Zn/Pd Kupplung: WO2008/147831, WO2006/106423 (Pyridin), Shakespeare, W. C. et al Chem. Biol. Drug Design 2008, 71, 97-105 (Pyrimidin-Derivate), Pasternak, A. et al Bioorg. Med. Chem. Lett. 2008, 18, 994-998 (Diazine); Coleridge, B. M.; Bello, C. S.; Leitner, A. Tetrahedron Lett. 2009, 50, 4475-4477; Bach, T., Heuser, S. Angew. Chem. Int. Ed. 2001, 40, 3184-3185. (Thiazole)).

Für nukleophile Substitutionen wird mindestens ein Äquivalent einer Base (z.B. Natriumhydrid, Kaliumcarbonat) im Verhältnis zum Startmaterial der allgemeinen Formel **(XXIX)** verwendet.

Nach Beendigung der Reaktion werden die Verbindungen **(XVIIb)** von der Reaktionsmischung durch eine der üblichen Trenntechniken getrennt. Falls notwendig werden die Verbindungen durch Umkristallisation oder Chromatographie gereinigt oder können, wenn gewünscht, im nächsten Schritt auch ohne vorhergehende Reinigung eingesetzt werden.

### Verfahren S

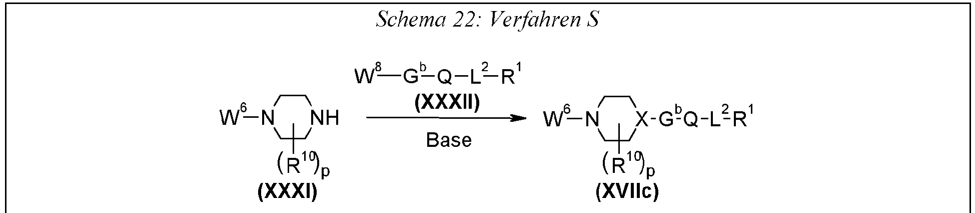

G^{b}: Der Piperazin-Ring ist über Kohlenstoffatom verbunden.,
W⁶ steht für Acetyl, C₁-C₄-Alkoxycarbonyl, Benzyl oder Benzyloxycarbonyl,
W⁸ steht für Brom, Iod, Methylsulfonyloxy oder Trifluormethylsulfonyloxy

Eine Verbindung mit der allgemeinen Formel **(XVIIc)** kann analog zu in der Literatur beschriebenen Vorschriften (s. z.B. für nukleophile Substitutionen: Li, C. S., Belair, L., Guay, J. et al Bioorg. Med. Chem. Lett. 2009, 19, 5214-5217; WO2008/062276; für Kupferkupplungen: Yeh, V. S. C.; Wiedeman, P. E. Tetrahedron Lett. 2006, 47, 6011-6016; für Palladiumkupplungen: WO2008/157500) durch eine Kupplungsreaktion einer Verbindung mit der entsprechenden allgemeinen Formel **(XXXI)** mit einem Substrat der allgemeinen Formel **(XXXII)** gegebenenfalls in der Gegenwart einer Base synthetisiert werden (Schema 22, *Verfahren S*).

Für nukleophile Substitutionen wird mindestens ein Äquivalent einer Base (z.B. Natriumhydrid, Kaliumcarbonat) im Verhältnis zum Startmaterial der allgemeinen Formel **(XXXI)** verwendet.

Nach Beendigung der Reaktion werden die Verbindungen **(XVIIc)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden.

### Verfahren T

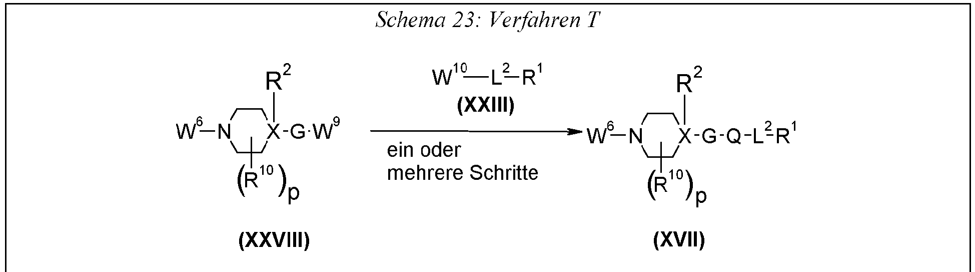

W⁶ steht für Acetyl, C₁-C₄-Alkoxycarbonyl, Benzyl oder Benzyloxycarbonyl,
W⁹ und W¹⁰ sind funktionelle Gruppen, die für das Bilden des gewünschten Heterocyclus geeignet sind

Im Allgemeinen ist es möglich, das Zwischenprodukt **(XVII)** aus entsprechenden Verbindungen **(XXVIII)** und **(XXIII)** herzustellen. *Verfahren T* wird analog *Verfahren A* (Schema 1) durchgeführt.

### Verfahren U

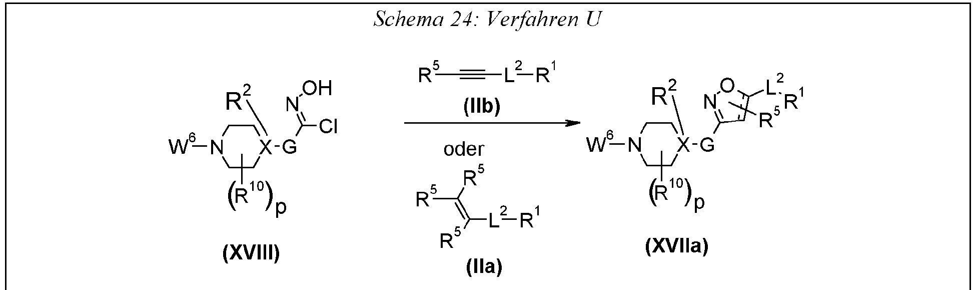

W⁶ steht für Acetyl, C₁-C₄-Alkoxycarbonyl, Benzyl oder Benzyloxycarbonyl

Eine bestimmte Möglichkeit, das Zwischenprodukt **(XVIIa)** aus entsprechenden Verbindungen **(XVIII)** herzustellen, ist in Schema 24 (*Verfahren U*) gezeigt. Die Ausgangstoffe der Formel **(XVIII)** lassen sich mittels literaturbeschriebener Methoden herstellen (s. z.B. WO 2008/013622), und *Verfahren U* wird analog *Verfahren B* (Schema 2) durchgeführt.

### Verfahren V

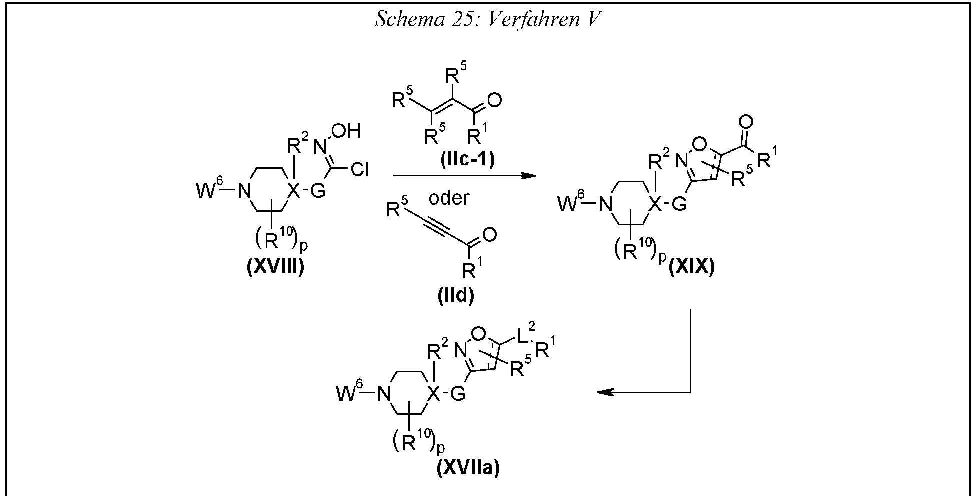

W⁶ steht für Acetyl, C₁-C₄-Alkoxycarbonyl, Benzyl oder Benzyloxycarbonyl

Eine andere bestimmte Möglichkeit, das Zwischenprodukt **(XVIIa)** aus entsprechenden Verbindungen **(XVIII)** herzustellen, ist in Schema 25 (*Verfahren V*) gezeigt. *Verfahren V* wird analog *Verfahren E* (Schema 7) und *Verfahren D* (Schema 6) durchgeführt.

### Verfahren W

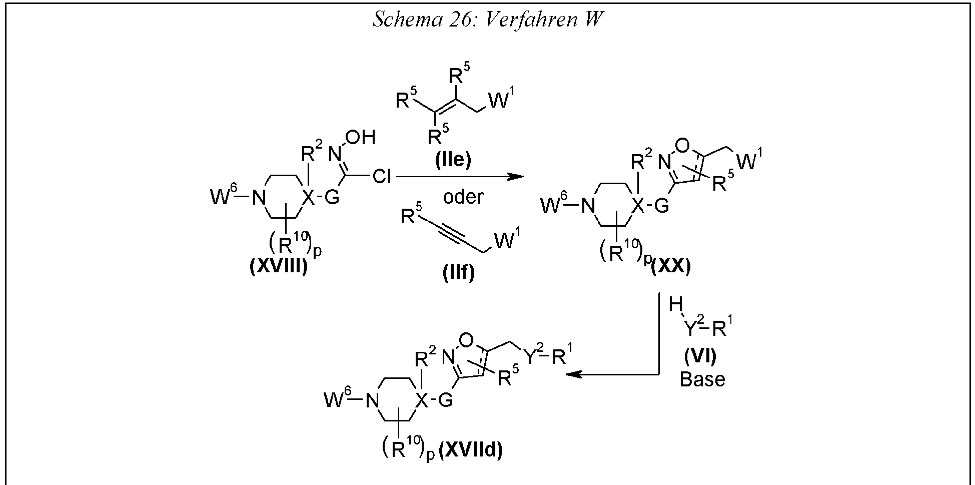

W¹ steht für Chlor, Brom, Iod, Toluolsulfonyloxy,
W⁶ steht für Acetyl, C₁-C₄-Alkoxycarbonyl, Benzyl oder Benzyloxycarbonyl,
Y² steht für O, S, NR²³,

Eine andere bestimmte Möglichkeit, das Zwischenprodukt der Formel **(XVIId)** aus entsprechenden Verbindungen *(XVIII)* herzustellen, ist in Schema 26 (*Verfahren W*) gezeigt. *Verfahren W* wird analog *Verfahren* G (Schema 9) und *Verfahren F* (Schema 8) durchgeführt.

### Verfahren X

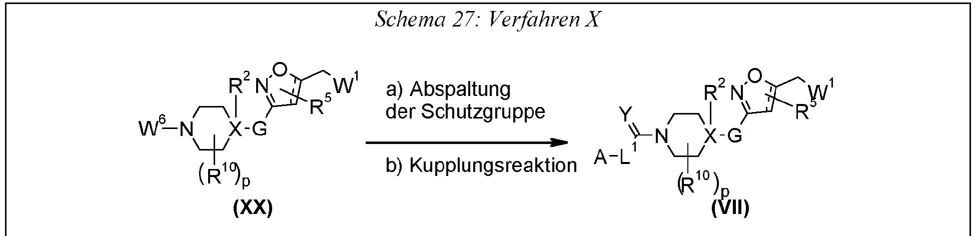

W¹ steht für Chlor, Brom, Iod, Toluolsulfonyloxy,
W⁶ steht für Acetyl, C₁-C₄-Alkoxycarbonyl, Benzyl oder Benzyloxycarbonyl,

Eine andere bestimmte Möglichkeit, das Zwischenprodukt der Formel *(VII)* aus entsprechenden Verbindungen *(XX)* herzustellen, ist in Schema 27 (*Verfahren X*) gezeigt. *Verfahren X* wird analog *Verfahren* **O** (Schema 18, Schritt a) und dann *Verfahren K* (Schema 14), *Verfahren M* (Schema 16) oder *Verfahren N* (Schema 17, Schritt b) durchgeführt.

### Verfahren Y

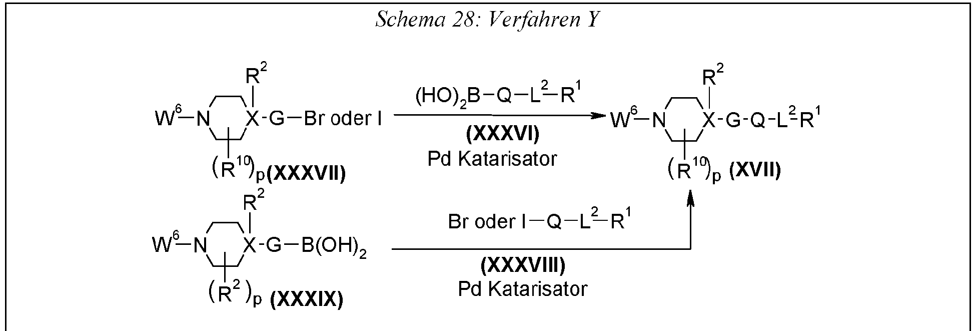

W⁶ steht für Acetyl, C₁-C₄-Alkoxycarbonyl, Benzyl oder Benzyloxycarbonyl

Eine Verbindung der allgemeinen Formel **(XVII)** kann aus Verbindungen **(XXXVII)** oder aus **(XXXIX)** durch Palladium-katalysierte Kupplungsreaktionen, wie zum Beispiel der Suzuki-Reaktion *(*Angew. Chem. Int. Ed. Engl., 1998, 27, 2046; A. Syn. Commun., 1981, 11, 7, 513) hergestellt werden (s. Schema *28, Verfahren Y*).

Als Lösungsmittel können alle üblichen, unter den Reaktionsbedingungen inerten Lösungsmittel verwendet werden und die Reaktion kann in Mischungen von zwei oder mehrerer dieser Lösungsmittel ausgeführt werden. Die bevorzugten Lösungsmittel sind *N,N-*Dimethylformamid, Dichlormethan, DMSO und Tetrahydrofuran.

Die Reaktion kann in Gegenwart der folgenden Additive ausgeführt werden: Phosphine, wie z.B. 2-Dicyclohexylphosphinobiphenyl, Trocknungsmitteln, z.B. 4 Å Molekularsieb, und geeigneten Basen, z.B. Triethylamin, Pyridin, Natriumcarbonat, Natriumethoxid oder Kaliumphosphat.

Es können zahlreiche kommerziell erhältliche Kupfer(II)-Katalysatoren, Palladium(0)-katalysatoren oder Palladium(II)katalysatoren in der Reaktion verwendet werden, aber bevorzugt wird in der Reaktion Kupfer(II)acetat, Tetrakistriphenylphosphinpalladium(0), 1,1-Bis(diphenylphosphino)ferrocene]dichloropalladium(II), oder Palladium(II)acetat verwendet. Die Aufwandmenge an Katalysator beträgt mindestens 1% bis zu einem Überschuss in Abhängigkeit von der Ausgangsverbindung **(XXXVII)** oder **(XXXIX).**

Nach Beendigung der Reaktion, werden die Verbindungen **(XVII)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

Es wird erkannt, daß einige Reagenzien und Reaktionsbegindungen, beschrieben vorstehend zum Herstellen von Verbindungen der Formel **(I)**, nicht mit bestimmten Funktionalitäten, vorhanden in den Zwischenverbindungen, kompatibel sein können. In diesen Fällen hilft die Einbringung von Schutz-/Entschützungssequenzen oder gegenseitiger Umwandlungen funktioneller Gruppen in die Synthese, die gewünschten Produkte zu erhalten. Die Verwendung und Auswahl der schützenden Gruppen ist für den Fachmann in der cshmischen Synthese offensichtlich (s. z.B. *"*Protective Groups in Organic Synthesis"; Third Edition; 494-653, und dort zitierte Literatur). Der Fachmann wird erkennen, daß es, in einigen Fällen, nach der Einführung eines gebenen Reagenzes, wie es in einem individuellen Schema dargestellt ist, notwendig sein kann, zusätzliche routinemäßige Syntheseschritte durchzuführen, die nicht im einzelnen beschrieben sind, um die Synthese von Verbindungen der Formel **(I)** zu vervollständigen. Der Fachmann wird ebenfalls erkennen, daß es notwendig sein kann, eine Kombination der Schritte, veranschaulicht in den vorstehenden Schemata, in einer anderen Reihenfolge als der durch die spezielle dargestellte implizierten Seqenz durchzuführen, um die Verbindungen der Formel **(I)** herzustellen.

### Neu sind Verbindungen der Formel (VIIIa)

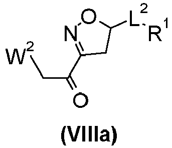

sowie Salze davon, in denen die Symbole W², L² und R¹ die oben angegebenen allgemeinen, bevorzugten, besonders bevorzugten oder ganz besonders bevorzugten Bedeutungen haben.

### Neu sind Verbindungen der Formel (XVIIa),

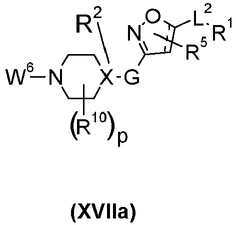

wie zum Beispiel **(XVIIa-1),** 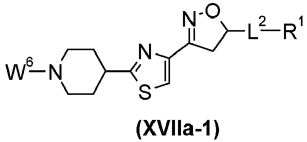 sowie Salze Metallkomplexe und N-Oxide davon, in denen die Symbole W⁶, X, G, L², p, R¹, R², R⁵ und R¹⁰ die oben angegebenen allgemeinen, bevorzugten, besonders bevorzugten oder ganz besonders bevorzugten Bedeutungen haben.

### Neu sind Verbindungen der Formel (XXXIII),

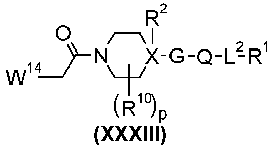

wie zum Beispiel **(XXXIII-1),** 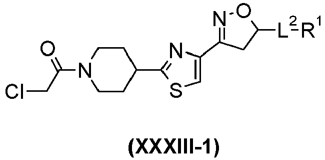 sowie Salze davon, in denen die Symbole W¹⁴, X, L², R¹, R², R¹⁰, Q, p und G die oben angegebenen allgemeinen, bevorzugten, besonders bevorzugten oder ganz besonders bevorzugten Bedeutungen haben.

### Neu sind Verbindungen der Formel (XIIIa),

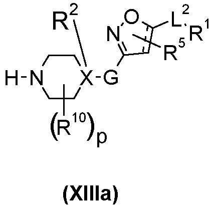

wie zum Beispiel **(XIIIa-1),** 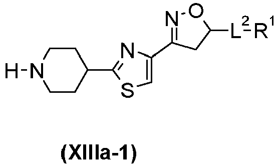 sowie Salze, Metallkomplexe und N-Oxide davon, in denen die Symbole X, L², p, G, R¹, R², R⁵ und R¹⁰ die oben angegebenen allgemeinen, bevorzugten, besonders bevorzugten oder ganz besonders bevorzugten Bedeutungen haben.

Ein weiterer Gegenstand der Erfindung betrifft die nichtmedizinische Verwendung der erfindungsgemäßen Heteroarylpiperidin und -piperazinderivate der Formel **(I)** zum Bekämpfen unerwünschter Mikroorganismen.

Ein weiterer Gegenstand der Erfindung betrifft ein Mittel zum Bekämpfen unerwünschter Mikroorganismen, umfassend wenigstens ein Heteroarylpiperidin und -piperazinderivate gemäß der vorliegenden Erfindung.

Außerdem betrifft die Erfindung ein Verfahren zum Bekämpfen unerwünschter Mikroorganismen, dadurch gekennzeichnet, dass die erfindungsgemäßen Heteroarylpiperidin und -piperazinderivate auf die Mikroorganismen und/oder in deren Lebensraum ausgebracht werden.

Weiterhin betrifft die Erfindung ein Saatgut, welches mit wenigstens einem erfindungsgemäßen Heteroarylpiperidin und -piperazinderivate behandelt wurde.

Ein letzter Gegenstand der Erfindung betrifft ein Verfahren zum Schutz von Saatgut vor unerwünschten Mikroorganismen durch Verwendung eines mit wenigstens einem Heteroarylpiperidin und - piperazinderivate gemäß der vorliegenden Erfindung behandelten Saatgutes.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Heteroarylpiperidin und -piperazinderivate der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Altemaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe bzw. Mittel können außerdem im Materialschutz zum Schutz von technischen Materialien gegen Befall und Zerstörung durch unerwünschten Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Heteroarylpiperidin und - piperazinderivate. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe,

Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die vorliegende Erfindung umfasst nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Heteroarylpiperidin und -piperazinderivate auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Erfindung umfasst weiterhin ein Verfahren zur Behandlung von Saatgut.

Ein weiterer Aspekt der vorliegenden Erfindung betrifft insbesondere ein Saatgut, welches mit wenigstens einem der erfindungsgemäßen Heteroarylpiperidin und -piperazinderivate behandelt ist. Die erfindungsgemäßen Saatgüter finden Anwendung in Verfahren zum Schutz von Saatgut vor pflanzenpathogenen Schadpilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417, US 4,245,432, US4,808,430, US 5,876,739, US 2003/0176428, WO 2002/080675, WO 2002/028186.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel **(I)** auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe der Formel **(I)** können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel **(I)** als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen CrylAb, CrylAc, CrylF, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins aus *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bbl in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel **(I)** bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln **(I)** geht aus den folgenden Beispielen hervor. Die Erfindung ist jedoch nicht auf diese Beispiele beschränkt.

Allgemeines: Wenn nicht anders angegeben, werden alle chromatografischen Reinigungs- bzw. Trennungsschritte an Kieselgel und mit einem Lösungsmittelgradienten von 0:100 Essigsäure-ethylester/Cyclohexan zu 100:0 Essigsäure-ethylester/Cyclohexan durchgeführt.

### Herstellung von Verbindungen der Formel (1-15)

### Schritt 1

### tert-Butyl-4-(4-brom-1,3-thiazol-2-yl)-4-hydroxypiperidin-1-carboxylat

Zu einer Lösung von 2,4-Dibrom-1,3-thiazol (8.8 g) in Dichlormethan (180 mL) wurde bei -78 °C unter Argon n-Butyllithium (1.6M in Tetrahydrofuran, 25 mL) getropft. Das Reaktionsgemisch wurde 20 Minuten bei -78 °C gerührt und dann wurde *tert*-Butyl-4-oxopiperidin-1-carboxylat zugegeben. Das Gemisch wurde bei Raumtemperatur für 30 Minuten gerührt. Das Reaktionsgemisch wurde anschließend mit gesättigter Ammoniumchloridlösung bei -30°C versetzt und die wässrige Phase abgetrennt. Nach Extraktion der wässrigen Phase mit Dichlormethan wurden die vereinigten organischen Phasen über Natriumsulfat getrocknet und unter vermindertem Druck eingeengt. Der Rückstand wurde chromatographisch gereinigt. Man erhielt *tert*-Butyl-4-(4-brom-1,3-thiazol-2-yl)-4-hydroxypiperidin-1-carboxylat (15.3 g).

### Schritt 2

### tert-Butyl-4-(4-brom-1,3-thiazol-2-yl)-4-fluorpiperidin-1-carboxylat

*tert*-Butyl-4-(4-brom-1,3-thiazol-2-yl)-4-hydroxypiperidin-1-carboxylat (17.7 g) wurde unter Argon bei 0 °C in Dichlormethan in einem PE-Kolben vorgelegt und Diethylaminoschwefeltrifluorid (DAST) (7.08 mL) wurden zugetropft. Die Kühlung wurde entfernt. Nach Rühren über Nacht wurde mit gesättigter wässriger Natriumhydrogencarbonatlösung versetzt und mit Dichlormethan extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-(4-brom-1,3-thiazol-2-yl)-4-fluorpiperidin-1-carboxylat (18.0 g).

### Schritt 3

### tert-Butyl-4-fluor-4-(4-formyl-1,3-thiazol-2-yl)piperidin-1-carboxylat

Zu einer Lösung von *tert*-Butyl-4-(4-brom-1,3-thiazol-2-yl)-4-fluorpiperidin-1-carboxylat (245 mg) in Dichlormethan (5 mL) wurde bei -78 °C nButyllithium (1.6 M in Tetrahydrofuran, 0.42 mL) getropft. Nach 20 min wurde N,N-Dimethylformamid (0.16 mL) zugetropft. Nach 30-minütigem Rühren bei -78 °C wurde mit gesättigter Ammoniumchloridlösung versetzt und mit Dichlormethan extrahiert. Die organischen Extrakte wurden über Natriumsulfat getrocknet und unter vermindertem Druck eingeengt.

Der Rückstand wurde chromatographisch gereinigt. Man erhielt *tert*-Butyl-4-fluor-4-(4-formyl-1,3-thiazol-2-yl)piperidin-1-carboxylat (75 mg).

### Schritt 4

### tert-Butyl-4-fluor-4-{4-[(E/Z)-(hydroxyimino)methyl]-1,3-thiazol-2-yl}piperidin-1-carboxylat (XVIII-1)

Zu einer Lösung von *tert*-Butyl-4-fluor-4-(4-formyl-1,3-thiazol-2-yl)piperidin-1-carboxylat (3.49 g) in Ethanol (50 mL) wurde Hydroxylamin (50% in Wasser, 0.81 mL) bei Raumtemperatur getropft. Die Reaktionsmischung wurde für 1 Stunde bei 60 °C gerührt, dann wurde das Lösungsmittel unter vermindertem Druck entfernt. Man erhielt *tert*-Butyl-4-fluor-4-{4-[(E/Z)-(hydroxyimino)methyl]-1,3-thiazol-2-yl}piperidin-1-carboxylat (3.49 g).

### Schritt 5

### tert-Butyl-4-fluor-4-(4-{5-[methoxy(phenyl)methyl]-4,5-dihydro-1,2-oxazol-3-yl}-1,3-thiazol-2-yl)piperidin-1-carboxylat (XVIIa-1)

Zu einer Lösung von *tert*-Butyl-4-fluor-4-{4-[(E/Z)-(hydroxyimino)methyl]-1,3-thiazol-2-yl}piperidin-1-carboxylat (200 mg) und (1-Methoxyprop-2-en-1-yl)benzol (99 mg) in Ethylacetat (3 mL) wurde bei Raumtemperatur Kaliumhydrogencarbonat (304 mg) und N-Chlorsuccinimid (97 mg) gegeben und anschließend ein Tropfen Wasser. Nach Rühren über Nacht bei Rückfluß wurde die Reaktionsmischung mit Ethylacetat und Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-fluor-4-(4-{5-[methoxy(phenyl)methyl]-4,5-dihydro-1,2-oxazol-3-yl}-1,3-thiazol-2-yl)piperidin-1-carboxylat (150 mg).

### Schritt 6

### 4-Fluor-4-(4-{5-[methoxy(phenyl)methyl]-4,5-dihydro-1,2-oxazol-3-yl}-1,3-thiazol-2-yl)piperidiniumchlorid (XIII-1)

Zu einer Lösung *tert*-Butyl-4-fluor-4-(4-{5-[methoxy(phenyl)methyl]-4,5-dihydro-1,2-oxazol-3-yl}-1,3-thiazol-2-yl)piperidin-1-carboxylat (150 mg) wurde bei 0 °C eine 4-molare Lösung von Chlorwasserstoff in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-Fluor-4-(4-{5-[methoxy(phenyl)methyl]-4,5-dihydro-1,2-oxazol-3-yl}-1,3-thiazol-2-yl)piperidiniumchlorid (150 mg).

### Schritt 7

### 2-[3,5-Bis(difluormethyl)-1H-pyrazol-1-yl]-1-[4-fluor-4-(4-{5-[methoxy(phenyl)methyl]-4,5-dihydro-1,2-oxazol-3-yl}-1,3-thiazol-2-yl)piperidin-1-yl]ethanon (I-15)

Zu einer Suspension von 4-Fluor-4-(4-{5-[methoxy(phenyl)methyl]-4,5-dihydro-1,2-oxazol-3-yl}-1,3-thiazol-2-yl)piperidiniumchlorid (153 mg) in Dichlormethan (2 mL) und Triethylamin (0.05 mL) wurde bei Raumtemperatur Bis-3,5-(difluormethyl)-1H-pyrazol-1-yl]essigsäure (70 mg), 4-Dimethylaminopyridin (4 mg) und 1-Ethyl-3-(3'-dimethylaminopropyl)carbodiimide (65 mg) gegeben. Das Gemisch wurde für 2 Stunden bei Raumtemperatur gerührt und dann mit Wasser versetzt. Die wässrige Phase wurde abgetrennt und mit Ethylacetat extrahiert. Die vereinigten organischen Phasen wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-[4-fluor-4-(4-{5-[methoxy(phenyl)methyl]-4,5-dihydro-1,2-oxazol-3-yl}-1,3-thiazol-2-yl)piperidin-1-yl]ethanon (30 mg).

### Herstellung von Verbindungen der Formel (1-9)

### Schritt 1

### tert-Butyl-4-[4-(5-benzoyl-4,5-dihydro-1,2-oxazol-3-yl)-1,3-thiazol-2-yl]piperidin-1-carboxylat (XIX-1)

*tert*-Butyl-4-{4-[(hydroxyimino)methyl]-1,3-thiazol-2-yl}piperidin-1-carboxylat (1.0 g) wurde analog zu **I-15** (Schritt 5) mit 1-Phenylprop-2-en-1-on (637 mg) umgesetzt. Man erhielt *tert*-Butyl-4-[4-(5-benzoyl-4,5-dihydro-1,2-oxazol-3-yl)-1,3-thiazol-2-yl]piperidin-1-carboxylat (1.12 g).

### Schritt 2

### tert-Butyl-4-(4-{5-[difluor(phenyl)methyl]-4,5-dihydro-1,2-oxazol-3-yl}-1,3-thiazol-2-yl)piperidin-1-carboxylat (XVIIa-2)

*tert*-Butyl-4-[4-(5-benzoyl-4,5-dihydro-1,2-oxazol-3-yl)-1,3-thiazol-2-yl]piperidin-1-carboxylat (2.0 g) wurde unter Argon bei Raumtemperatur in Dichlormethan (20 mL) in einem PE-Kolben vorgelegt und (Diethylamino)schwefeltrifluorid (DAST) (1.50 mL) wurden zugetropft. Nach Rühren über Nacht bei Rückflußtemparatur wurde mit gesättigter wässriger Natriumhydrogencarbonatlösung versetzt und mit Dichlromethan extrahiert. Die organischen Extrakte wurden über Natriumsulfat getrocknet und unter vermindertem Druck eingeengt. Der Rückstand wurde chromatographisch gereinigt. Man erhielt *tert-*Butyl-4-(4-{5-[difluor(phenyl)methyl]-4,5-dihydro-1,2-oxazol-3-yl}-1,3-thiazol-2-yl)piperidin-1-carboxylat (1.8 g).

### Schritt 3

### 4-(4-15-[Difluor(phenyl)methyl]-4,5-dihydro-1,2-oxazol-3-yl}-1,3-thiazol-2-yl)piperidiniumchlorid (XIII-2)

*tert*-Butyl-4-(4-{5-[difluor(phenyl)methyl]-4,5-dihydro-1,2-oxazol-3-yl}-1,3-thiazol-2-yl)piperidin-1-carboxylat (1.8 g) wurde analog zu **I-15** (Schritt 6) umgesetzt. Man erhielt 4-(4-{5-[Difluor(phenyl)methyl]-4,5-dihydro-1,2-oxazol-3-yl}-1,3-thiazol-2-yl)piperidiniumchlorid (1.7 g).

### Schritt 4

### 2-[3,5-Bis(difluormethyl)-1H-pyrazol-1-yl]-1-[4-(4-{5-[difluor(phenyl)methyl]-4,5-dihydro-1,2-oxazol-3-yl}-1,3-thiazol-2-yl)piperidin-1-yl]ethanon (I-9)

Zu einer Suspension von 4-(4-{5-[Difluor(phenyl)methyl]-4,5-dihydro-1,2-oxazol-3-yl}-1,3-thiazol-2-yl)piperidiniumchlorid (407 mg) in Dichlormethan (4 mL) und Triethylamin (0.43 mL) wurde bei Raumtemperatur Bis-3,5-(difluormethyl)-1H-pyrazol-1-yl]essigsäure (230 mg) und Benzotriazol-1-yl-oxytripyrrolidinophosphoniumhexafluorophosphat (570 mg) gegeben. Das Gemisch wurde für 2 Stunden bei Raumtemperatur gerührt und dann mit Wasser versetzt. Die wässrige Phase wurde abgetrennt und mit Ethylacetat extrahiert. Die vereinigten organischen Phasen wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-[4-(4-{5-[difluor(phenyl)methyl]-4,5-dihydro-1,2-oxazol-3-yl}-1,3-thiazol-2-yl)piperidin-1-yl]ethanon (455 mg).

### Herstellung von Verbindungen der Formel (I-31)

### 2-[3,5-Bis(difluormethyl)-1H-pyrazol-1-yl]-1-[4-(4-{5-[difluor(phenyl)methyl]-4,5-dihydro-1,2-oxazol-3-yl}-1,3-thiazol-2-yl)piperidin-1-yl]ethanthion (I-31)

Zu einer Lösung von 2-[3,5-Bis(difluormethyl)-1H-pyrazol-1-yl]-1-[4-(4-{5-[difluor(phenyl)methyl]-4,5-dihydro-1,2-oxazol-3-yl}-1,3-thiazol-2-yl)piperidin-1-yl]ethanon (455 mg) in Toluol (10 mL) wurde bei Raumtemperatur 2,4-Bis-(4-methoxyphenyl)-1,3,2,4-dithiadiphosphetan-2,4-disulfid (Lawessons Reagenz) (213 mg) gegeben. Das Gemisch wurde für 2 Stunden bei 70 °C gerührt und dann mit Wasser versetzt. Die wässrige Phase wurde abgetrennt und mit Ethylacetat extrahiert. Die vereinigten organischen Phasen wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-[4-(4-{5-[difluor(phenyl)methyl]-4,5-dihydro-1,2-oxazol-3-yl}-1,3-thiazol-2-yl)piperidin-1-yl]ethanthion (350 mg).

### Herstellung von Verbindungen der Formel (I-16)

### N-[2,5-Bis(difluormethyl)phenyl]-4-(4-{5-[difluor(phenyl)methyl]-4,5-dihydro-1,2-oxazol-3-yl}-1,3-thiazol-2-yl)piperidin-1-carboxamid (I-16)

Zu einer Suspension von 4-(4-{5-[Difluor(phenyl)methyl]-4,5-dihydro-1,2-oxazol-3-yl}-1,3-thiazol-2-yl)piperidiniumchlorid (411 mg) in Dichlormethan (5 mL) und Triethylamin (0.16 mL) wurde bei Raumtemperatur 1,4-Bis(difluormethyl)-2-isocyanatobenzol (225 mg) und ein Tropfen 1,8-Diazabicyclo[5.4.0]undec-7-en (DBU) gegeben. Das Gemisch wurde für 2 Stunden bei Raumtemperatur gerührt und dann mit gesättigter Ammoniumchloridlösung versetzt. Die wässrige Phase wurde abgetrennt und mit Ethylacetat extrahiert. Die vereinigten organischen Phasen wurden über Natriumsulfat getrocknet und unter vermindertem Druck eingeengt. Der Rückstand wurde chromatographisch gereinigt. Man erhielt N-[2,5-Bis(difluormethyl)phenyl]-4-(4-{5-[difluor-(phenyl)methyl]-4,5-dihydro-1,2-oxazol-3-yl}-1,3-thiazol-2-yl)piperidin-1-carboxamid (388 mg).

### Herstellung von Verbindungen der Formel (I-2)

### Schritt 1

### tert-Butyl-4-{4-[5-(phenoxymethyl)-4,5-dihydro-1,2-oxazol-3-yl]-1,3-thiazol-2-yl}piperidin-1-carboxylat (XVIIa-3)

Zu einer Lösung von *tert*-Butyl-4-{4-[(E/Z)-(hydroxyimino)methyl]-1,3-thiazol-2-yl}piperidin-1-carboxylat (3.2 g) und Allyl-phenylether (2.21 g) in Ethylacetat (80 mL) wurde bei Raumtemperatur Kaliumhydrogencarbonat (5.65 g) und N-Chlorsuccinimid (2.34 g) gegeben und anschließend ein Tropfen Wasser. Nach Rühren über Nacht bei Rückfluß wurde die Reaktionsmischung mit Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt tert-Butyl-4-{4-[5-(phenoxymethyl)-4,5-dihydro-1,2-oxazol-3-yl]-1,3-thiazol-2-yl}piperidin-1-carboxylat (2.34 g).

### Schritt 2

### 2-[3,5-Bis(difluormethyl)-1H-pyrazol-1-yl]-1-(4-{4-[5-(phenoxymethyl)-4,5-dihydro-1,2-oxazol-3-yl]-1,3-thiazol-2-yl}piperidin-1-yl)ethanon (I-2)

Zu einer Lösung tert-Butyl-4-{4-[5-(phenoxymethyl)-4,5-dihydro-1,2-oxazol-3-yl]-1,3-thiazol-2-yl}piperidin-1-carboxylat (4.27 g) in Dichlormethan (20 ml) wurde bei 0 °C eine 4-molare Lösung von Chlorwasserstoff in 1,4-Dioxan getropft. Das Reaktionsgemisch wurde bei 0 °C gerührt und dann langsam auf Raumtemperatur erwärmt. Nach 5-stündigem Rühren wurden das Lösungsmittel und überschüssiger Chlorwasserstoff entfernt. Der Rückstand wurde wieder in Dichlormethan (50 ml) gelöst (Lösung A).

Gleichzeitig wurde zu einer Lösung von 3,5-Bis(difluormethyl)-1H-pyrazol-1-yl]essigsäure (2.89 g) in Dichlormethan (30 ml) bei Raumtemperatur ein Tropfen N,N-Dimethylformamid und Oxalylchlorid (3.74 g) getropft. Nach 5-stündigem Rühren bei Raumtemperatur wurde das Lösungsmittel entfernt und der Rückstand wurde wieder in Dichlormethan (20 ml) gelöst (Lösung B).

Zu Lösung A wurde bei Raumtemperatur Diisopropylethylamin (3.77 ml) zugesetzt. Nach 15 Minuten wurde die Lösung B zugetropft. Nach Rühren über Nacht bei Raumtemperatur wurde die Reaktionsmischung mit Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-(4-{4-[5-(phenoxymethyl)-4,5-dihydro-1,2-oxazol-3-yl]-1,3-thiazol-2-yl}piperidin-1-yl)ethanon (3.81 g).

### Herstellung von Verbindungen der Formel (I-43)

### Schritt 1

### tert-Butyl-4-{4-[5-(brommethyl)-4,5-dihydro-1,2-oxazol-3-yl]-1,3-thiazol-2-yl}piperidin-1-carboxylat (XX-1)

Zu einer Lösung von *tert*-Butyl-4-{4-[(E/Z)-(hydroxyimino)methyl]-1,3-thiazol-2-yl}piperidin-1-carboxylat (250 mg) und Allylbromid (107 mg) in Ethylacetat (10 mL) wurde bei Raumtemperatur Kaliumhydrogencarbonat (402 mg) und N-Chlorsuccinimid (129 mg) gegeben und anschließend ein Tropfen Wasser. Nach Rühren über Nacht bei Rückfluß wurde die Reaktionsmischung mit Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt *tert*-Butyl-4-{4-[5-(brommethyl)-4,5-dihydro-1,2-oxazol-3-yl]-1,3-thiazol-2-yl}piperidin-1-carboxylat (136 mg)

### Schritt 2

### 2-[3,5-Bis(difluormethyl)-1H-pyrazol-1-yl]-1-(4-{4-[5-(brommethyl)-4,5-dihydro-1,2-oxazol-3-yl]-1,3-thiazol-2-yl}piperidin-1-yl)ethanon (VII-1)

Zu einer Lösung *tert*-Butyl-4-{4-[5-(brommethyl)-4,5-dihydro-1,2-oxazol-3-yl]-1,3-thiazol-2-yl}-piperidin-1-carboxylat (720 mg) in Dichlormethan (5 ml) wurde bei 0 °C eine 4-molare Lösung von Chlorwasserstoff in 1,4-Dioxan getropft. Das Reaktionsgemisch wurde bei 0 °C gerührt und dann langsam auf Raumtemperatur erwärmt. Nach 2-stündigem Rühren wurden das Lösungsmittel und überschüssiger Chlorwasserstoff entfernt. Die Rückstand würde wieder in Dichlormethan (30 ml) gelöst (Lösung A).

Gleichzeitig wurde zu einer Lösung von 3,5-Bis(difluormethyl)-1H-pyrazol-1-yl]essigsäure (397 mg) in Dichlormethan (10 ml) bei Raumtemperatur ein Tropfen N,N-Dimethylformamid und Oxalylchlorid (650 mg)getropft. Nach 2-stündigem Rühren bei Raumtemperatur wurde das Lösungsmittel entfernt und die Rückstand würde wieder in Dichlormethan (10 ml) gelöst (Lösung B).

Zu Lösung A wurde bei Raumtemperatur Diisopropylethylamin (0.66 ml) zugesetzt. Nach 15 Minuten wurde die Lösung B zugetropft. Nach Rühren über Nacht bei Raumtemperatur wurde die Reaktionsmischung mit Wasser versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-(4-{4-[5-(brommethyl)-4,5-dihydro-1,2-oxazol-3-yl]-1,3-thiazol-2-yl}piperidin-1-yl)ethanon (335 mg).

### Schritt 3

### 2-[3,5-Bis(difluormethyl)-1H-pyrazol-1-yl]-1-[4-(4-{5-[(2,6-difluorphenoxy)methyl]-4,5-dihydro-1,2-oxazol-3-yl}-1,3-thiazol-2-yl)piperidin-1-yl]ethanon (I-43)

Zur einer Lösung von 2-[3,5-Bis(difluormethyl)-1H-pyrazol-1-yl]-1-(4-{4-[5-(brommethyl)-4,5-dihydro-1,2-oxazol-3-yl]-1,3-thiazol-2-yl}piperidin-1-yl)ethanon (55 mg) und 2,6 Difluorphenol (13.3 mg) wurden Tetra-n-butylammoniumbromid (33 mg) und Natriumhydroxid (13 mg fein gemahlen) zugegeben. Nach 48-stündigem Rühren bei Rückfluß wurde die Reaktionsmischung mit Ethylacetat und Salzsaüre (pH2) versetzt und mit Ethylacetat extrahiert. Die organischen Extrakte wurden über Natriumsulfat getrocknet und unter vermindertem Druck eingeengt. Der Rückstand wurde chromatographisch gereinigt. Man erhielt 2-[3,5-Bis(difluormethyl)-1H-pyrazol-1-yl]-1-[4-(4-{5-[(2,6-difluorphenoxy)methyl]-4,5-dihydro-1,2-oxazol-3-yl}-1,3-thiazol-2-yl)piperidin-1-yl]ethanon. (10 mg)

### Beispiele

Folgende Verbindungen können mit einem oder mehreren der oben genannten Verfahren hergestellt werden.

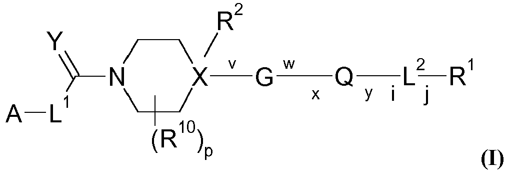

Die in Tablle 1 aufgelisteteten Strukturelemente G¹ und Q²⁴-3 sind wie folgt definiert:

| | | | | | |
|---|---|---|---|---|---|
| G¹= | 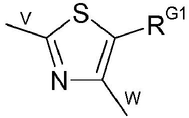 | Q²⁴-3 = | 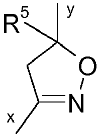 | Q¹¹-1 = | 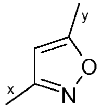 |

Für alle in Tabelle 1 aufgeführten Verbindungen ist p=0

**Tabelle 1:**

| **Bsp** | **A** | **L¹** | **Y** | **X(R)²** | **G** | **R^{G1}** | **Q** | **R⁵** | **L²** | **R¹** | **Log P** |
|---|---|---|---|---|---|---|---|---|---|---|---|
| I-1* | 3,5-Bis(difluormethyl)-1H-pyrazol-1-yl | CH₂ | O | CH | G¹ | H | Q²⁴-3 | H | CH₂O | 2,6-Dichlorphenyl | 3,7^{[a]}; 3,66^{[b]} |
| I-2* | 3,5-Bis(difluormethyl)-1H-pyrazol-1-yl | CH₂ | O | CH | G¹ | H | Q²⁴-3 | H | CH₂O | Phenyl | 3,22^{[a]}; 3,25^{[b]} |
| I-3* | 3,5-Bis(difluormethyl)-1H-pyrazol-1-yl | CH₂ | O | CH | G¹ | H | Q²⁴-3 | H | CH₂O | 2-Fluorphenyl | 3.19^{[a]}; 3.2^{[b]} |
| I-4 | 3,5-Bis(difluormethyl)-1H-pyrazol-1-yl | CH₂ | O | CH | G¹ | H | Q²⁴-3 | H | C(CH₃)₂ | Phenyl | 3.79^{[a]} |
| I-5 | 3,5-Bis(difluormethyl)-1H-pyrazol-1-yl | CH₂ | O | CH | G¹ | H | Q²⁴-3 | H | CHF | Phenyl | 3.23^{[a]} |
| I-6* | 3,5-Bis(difluormethyl)-1H-pyrazol-1-yl | CH₂ | O | CH | G¹ | H | Q²⁴-3 | H | CH₂CH₂C(O) | Phenyl | 3.16^{[a]}; 3.13^{[b]} |
| I-7* | 3,5-Bis(difluormethyl)-1H-pyrazol-1-yl | CH₂ | O | CH | G¹ | H | Q²⁴-3 | H | CH₂SO₂ | Phenyl | 2.65^{[a]}; 2.65^{[b]} |
| I-8 | 3,5-Bis(difluormethyl)-1H-pyrazol-1-yl | CH₂ | O | CH | G¹ | H | Q²⁴-3 | H | CHOH | Phenyl | 2.53^{[a]} |
| I-9 | 3,5-Bis(difluormethyl)-1H-pyrazol-1-yl | CH₂ | O | CH | G¹ | H | Q²⁴-3 | H | CF₂ | Phenyl | 3.41^{[a]} |
| I-10* | 3,5-Bis(difluormethyl)-1H-pyrazol-1-yl | CH₂ | O | CH | G¹ | H | Q²⁴-3 | H | CH₂S | Phenyl | 3.46^{[a]}; 3.51^{[b]} |
| I-11 | 3,5-Bis(difluormethyl)-1H-pyrazol-1-yl | CH₂ | O | CF | G¹ | H | Q²⁴-3 | H | C(CH₃)₂ | Phenyl | 4.03^{[a]} |
| I-12* | 3,5-Bis(difluormethyl)-1H-pyrazol-1-yl | CH₂ | O | CH | G¹ | H | Q²⁴-3 | H | CH₂O | 2-Acetylphenyl | 2.93^{[a]}; 2.89^{[b]} |
| I-13* | 3,5-Bis(difluormethyl)-1H-pyrazol-1-yl | CH₂ | O | CH | G¹ | H | Q²⁴-3 | H | CH₂O | 2-Methylphenyl | 3.5^{[a]}; 3.46^{[b]} |
| I-14* | 3,5-Bis(difluormethyl)-1H-pyrazol-1-yl | CH₂ | O | CH | G¹ | H | Q²⁴-3 | H | CH₂N(CH₃) | Phenyl | 3.47^{[a]}; 3.64^{[b]} |
| I-15 | 3,5-Bis(difluormethyl)-1H-pyrazol-1-yl | CH₂ | O | CF | G¹ | H | Q²⁴-3 | H | CH(OCH₃) | Phenyl | 3.56^{[a]} |
| I-16 | 2,5-Bis(difluormethyl)phenyl | NH | O | CH | G¹ | H | Q²⁴-3 | H | CF₂ | Phenyl | 3.64^{[a]} |
| I-17 | 3,5-Bis(difluormethyl)-1H-pyrazol-1-yl | CH₂ | O | CH | G¹ | H | Q²⁴-3 | H | CHF | 2,6-Difluorphenyl | 3.36^{[a]} |
| I-18 | 3,5-Bis(difluormethyl)-1H-pyrazol-1-yl | CH₂ | S | CH | G¹ | H | Q²⁴-3 | H | CHF | 2,6-Difluorphenyl | 3.81^{[a]} |
| I-19 | 5-Methyl-3-(trifluormethyl)-1H-pyrazol-1-yl | CH₂ | O | CH | G¹ | H | Q²⁴-3 | H | CHF | Phenyl | 3.3^{[a]} |
| I-20 | 5-Methyl-3-(trifluormethyl)-1H-pyrazol-1-yl | CH₂ | O | CH | G¹ | H | Q²⁴-3 | H | CHF | 2,6-Difluorphenyl | 3.48^{[a]} |
| I-21 | 2,5-Dimethylphenyl | NH | S | CH | G¹ | H | Q²⁴-3 | H | CF₂ | Phenyl | 3.86^{[a]} |
| I-22 | 3,5-Bis(difluormethyl)-1H-pyrazol-1-yl | CH₂ | O | CH | G¹ | H | Q²⁴-3 | H | CF₂ | 2,6-Difluorphenyl | 3.47^{[a]} |
| I-23* | 2,5-Dichlorphenyl | CH₂ | O | CH | G¹ | H | Q²⁴-3 | H | CH₂O | Phenyl | 3.89^{[a]}; 3.82^{[b]} |
| I-24 | 2,5-Bis(difluormethyl)phenyl | NH | O | CH | G¹ | H | Q²⁴-3 | H | CHF | Phenyl | 3.41^{[a]} |
| I-25 | 5-Methyl-3-(trifluormethyl)-1H-pyrazol-1-yl | CH₂ | O | CH | G¹ | H | Q²⁴-3 | H | CF₂ | Phenyl | 3.55^{[al} |
| I-26* | 3,5-Bis(difluormethyl)-1H-pyrazol-1-yl | CH₂ | O | CH | G¹ | H | Q²⁴-3 | H | CH₂O | 2-Methoxyphenyl | 3.01^{[a]}; 3.03^{[b]} |
| I-27 | 2,5-Dimethylphenyl | NH | S | CH | G¹ | H | Q²⁴-3 | H | CF₂ | 2,6-Difluorphenyl | 3.81^{[a]} |
| I-28 | 5-Methyl-3-(trifluormethyl)-1H-pyrazol-1-yl | CH₂ | O | CH | G¹ | H | Q²⁴-3 | H | CF₂ | 2,6-Difluorphenyl | 3.51^{[a]} |
| I-29* | 2,5-Dimethylphenyl | CH₂ | O | CH | G¹ | H | Q²⁴-3 | H | CH₂O | Phenyl | 3.71^{[a]}; 3.67^{[b]} |
| I-30 | 2,5-Bis(difluormethyl)phenyl | NH | O | CH | G¹ | H | Q²⁴-3 | H | CHF | 2,6-Difluorphenyl | 3,60^{[a]} |
| I-31 | 3,5-Bis(difluormethyl)-1H-pyrazol-1-yl | CH₂ | S | CH | G¹ | H | Q²⁴-3 | H | CF₂ | Phenyl | 3.88^{[a]} |
| I-32 | 2,5-Dimethylphenyl | NH | S | CH | G¹ | H | Q²⁴-3 | H | CHF | 2,6-Difluorphenyl | 3,80^{[a]} |
| I-33* | 5-Chlor-2-methylphenyl | NH | O | CH | G¹ | H | Q²⁴-3 | H | CH₂O | Phenyl | 3.54^{[a]}; 3.5^{[b]} |
| I-34* | 3,5-Bis(difluormethyl)-1H-pyrazol-1-yl | CH₂ | S | CH | G¹ | H | Q²⁴-3 | H | CH₂O | Phenyl | 3.73^{[a]}; 3.66^{[b]} |
| I-35 | 2,5-Bis(difluormethyl)phenyl | NH | O | CH | G¹ | H | Q²⁴-3 | H | CF₂ | 2,6-Difluorphenyl | 3,61^{[a]} |
| I-36* | 3,5-Bis(difluormethyl)-1H-pyrazol-1-yl | CH₂ | O | CH | G¹ | H | Q²⁴-3 | H | CH₂O | 2,6-Dibromphenyl | 3.9^{[a]}; 3.82^{[b]} |
| I-37* | 5-Methyl-3-(trifluormethyl)-1H-pyrazol-1-yl | CH₂ | O | CH | G¹ | H | Q²⁴-3 | H | CH₂O | Phenyl | 3.27^{[a]}; 3.28^{[b]} |
| I-38* | 2-Methoxy-5-methylphenyl | NH | O | CH | G¹ | H | Q²⁴-3 | H | CH₂O | Phenyl | 3.59^{[a]}; 3.55^{[b]} |
| I-39 | 2,5-dimethylphenyl | NH | S | CH | G¹ | H | Q²⁴-3 | H | CHF | Phenyl | 3.61^{[a]} |
| I-40* | 3,5-Bis(difluormethyl)-1H-pyrazol-1-yl | CH₂ | O | CH | G¹ | H | Q²⁴-3 | H | Si(CH₃)₂ | Phenyl | 4.01^{[a]}; 3.99^{[b]} |
| I-41* | 2,5-Dimethylphenyl | NH | O | CH | G¹ | H | Q²⁴-3 | H | CH₂O | Phenyl | 3.36^{[a]}; 3.28^{[b]} |
| I-42 | 3,5-Bis(difluormethyl)-1H-pyrazol-1-yl | CH₂ | S | CH | G¹ | H | Q²⁴-3 | H | CF₂ | 2,6-Difluorphenyl | 3.94^{[a]} |
| I-43* | 3,5-Bis(difluormethyl)-1H-pyrazol-1-yl | CH₂ | O | CH | G¹ | H | Q²⁴-3 | H | CH₂O | 2,6-Difluorphenyl | 3.3^{[a]}; 3.23^{[b]} |
| I-44* | 3,5-Bis(difluormethyl)-1H-pyrazol-1-yl | CH₂ | O | CH | G¹ | H | Q¹¹-1 | - | CH₂O | Phenyl | 3.63^{[a]}; 3.52^{[b]} |
| I-45* | 3-(Difluormethyl)-5-methyl-1H-pyrazol-1-yl | CH₂ | O | CH | G¹ | H | Q²⁴-3 | H | CH₂O | 2,6-Dichlorphenyl | 3.35^{[a]}; 3.4^{[b]} |
| I-46* | 5-Methyl-3-(trifluormethyl)-1H-pyrazol-1-yl | CH₂ | O | CH | G¹ | H | Q²⁴-3 | H | CH₂O | 2-Chlor-6-fluorphenyl | 3.54^{[a]}; 3.56^{[b]} |
| I-47 | 3,5-Bis(difluormethyl)-1H-pyrazol-1-yl | CH₂ | O | CH | G¹ | H | Q²⁴-3 | H | C(CH₃)OH | 2,6-Difluorphenyl | 3.03^{[a]}; 2.94^{[b]} |
| I-48* | 3-(Difluormethyl)-5-methyl-1H-pyrazol-1-yl | CH₂ | O | CH | G¹ | H | Q²⁴-3 | H | CH₂O | 2,6-Difluorphenyl | 3.01^{[a]}; 2.93^{[b]} |
| I-49 | 3,5-Bis(difluormethyl)-1H-pyrazol-1-yl | CH₂ | O | CH | G¹ | H | Q²⁴-3 | H | C(CF₃)OH | Phenyl | 3.2^{[a]}; 3.13^{[b]} |
| I-50* | 5-Chlor-2-methylphenyl | NH | O | CH | G¹ | H | Q²⁴-3 | H | CH₂O | 2,6-Difluorphenyl | 3.66^{[a]}; 3.54^{[b]} |
| I-51* | 5-Chlor-2-methylphenyl | NH | O | CH | G¹ | H | Q²⁴-3 | H | CH₂O | 2-Chlor-6-fluorphenyl | 3.81^{[a]}; 3.78^{[b]} |
| I-52 | 3-Isopropyl-5-(trifluormethyl)-1H-pyrazol-1-yl | CH₂ | O | CH | G¹ | H | Q²⁴-3 | H | C(CH₃)OH | 2,6-Difluorphenyl | 3.66^{[a]}; 3.56^{[b]} |
| I-53 | 2,5-Bis(difluormethyl)phenyl | NH | O | CH | G¹ | H | Q²⁴-3 | H | C(CH₃)₂ | 2-Chlor-6-fluorphenyl | 4.53^{[a]}; 4.43^{[b]} |
| I-54* | 5-Methyl-3-(trifluormethyl)-1H-pyrazol-1-yl | CH₂ | O | CH | G¹ | H | Q²⁴-3 | H | CH₂O | 2,6-Difluorphenyl | 3.34^{[a]}; 3.32^{[b]} |
| I-55* | 3,5-Bis(difluormethyl)-1H-pyrazol-1-yl | CH₂ | O | CH | G¹ | H | Q²⁴-3 | H | CH₂S | 2-Fluorphenyl | 3.54^{[a]}; 3.47^{[b]} |
| I-56* | 2,5-Dimethylphenyl | NH | O | CH | G¹ | H | Q²⁴-3 | H | CH₂O | 2,6-Dichlorphenyl | 3.89^{[a]}; 3.9^{[b]} |
| I-57 | 3,5-Bis(difluormethyl)-1H-pyrazol-1-yl | CH₂ | O | CH | G¹ | H | Q²⁴-3 | H | CF₂ | Thiophen-2-yl | 3.33^{[a]} |
| I-58* | 3,5-Bis(difluormethyl)-1H-pyrazol-1-yl | CH₂ | O | CH | G¹ | H | Q²⁴-3 | H | CH₂S | 2,6-Dichlorphenyl | 3.99^{[a]}; 3.96^{[b]} |
| I-59* | 5-Chlor-2-methylphenyl | NH | O | CH | G¹ | H | Q²⁴-3 | H | CH₂O | 2,6-Dichlorphenyl | 4.08^{[a]}; 4.08^{[b]} |
| I-60* | 3,5-Bis(difluormethyl)-1H-pyrazol-1-yl | CH₂ | O | CH | G¹ | H | Q²⁴-3 | H | CH₂O | 2-[(*E*/*Z*)-(Methoxyimino)methyl]phenyl | 3.39^{[a]}; 3.39^{[b]} |
| I-61* | 3,5-Bis(difluormethyl)-1H-pyrazol-1-yl | CH₂ | O | CH | G¹ | H | Q²⁴-3 | H | CH₂S | 2-Chlorphenyl | 3.68^{[a]}; 3.7^{[b]} |
| I-62 | 5-Methyl-3-(trifluormethyl)-1H-pyrazol-1-yl | CH₂ | O | CH | G¹ | H | Q²⁴-3 | H | C(CH₃)₂ | 2,6-Difluorphenyl | 4.09^{[a]}; 4.15^{[b]} |
| I-63* | 3,5-Bis(difluormethyl)-1H-pyrazol-1-yl | CH₂ | O | CH | G¹ | H | Q²⁴-3 | H | CH₂S | 2-Bromphenyl | 3.72^{[a]}; 3.71^{[b]} |
| I-64* | 3,5-Bis(difluormethyl)-1H-pyrazol-1-yl | CH₂ | O | CH | G¹ | H | Q²⁴-3 | H | CH₂O | 2-[(*E*/*Z*)-(Hydroxyimino)methyl]phenyl | 2.62^{[a]}; 2.59^{[b]} |
| I-65* | 5-Methyl-3-(trifluormethyl)-1H-pyrazol-1-yl | CH₂ | O | CH | G¹ | H | Q²⁴-3 | H | CH₂O | 2,6-Dichlorphenyl | 3.9^{[a]}; 3.8^{[b]} |
| I-66 | 3,5-Bis(difluormethyl)-1H-pyrazol-1-yl | CH₂ | O | CH | G¹ | H | Q²⁴-3 | H | C(CH₃)₂ | 2,6-Difluorphenyl | 4.05^{[a]}; 3.94^{[b]} |
| I-67 | 5-Methyl-3-(trifluormethyl)-1H-pyrazol-1-yl | CH₂ | O | CH | G¹ | H | Q²⁴-3 | H | C(CH₃)₂ | 2-Chlor-6-fluorphenyl | 4.43^{[a]}; 4.36^{[b]} |
| I-68 | 2,5-Bis(difluormethyl)phenyl | NH | O | CH | G¹ | H | Q²⁴-3 | H | C(CH₃)₂ | 2,6-Difluorphenyl | 4.29^{[a]}; 4.04^{[b]} |
| I-69 | 3,5-Bis(difluormethyl)-1H-pyrazol-1-yl | CH₂ | O | CH | G¹ | H | Q²⁴-3 | H | C(CH₃)₂ | 2-Chlor-6-fluorphenyl | 4.32^{[a]}; 4.21^{[b]} |
| I-70* | 3,5-Bis(difluormethyl)-1H-pyrazol-1-yl | CH₂ | O | CH | G¹ | H | Q²⁴-3 | H | CH₂S | 2-Methylphenyl | 3.78^{[a]}; 3.75^{[b]} |
| I-71* | 3,5-Bis(difluormethyl)-1H-pyrazol-1-yl | CH₂ | O | CH | G¹ | H | Q²⁴-3 | H | CH₂O | 2-Chlorphenyl | 3.35^{[a]}; 3.31^{[b]} |
| I-72* | 3,5-Bis(difluormethyl)-1H-pyrazol-1-yl | CH₂ | O | CH | G¹ | H | Q²⁴-3 | H | CH₂O | 2-Formyl-6-methoxyphenyl | 3.02^{[a]}; 2.94^{[b]} |
| I-73* | 3,5-Bis(difluormethyl)-1H-pyrazol-1-yl | CH₂ | O | CH | G¹ | H | Q²⁴-3 | H | CH₂O | 2-Formylphenyl | 2.83^{[a]}; 2.83^{[b]} |
| I-74* | 3,5-Bis(difluormethyl)-1H-pyrazol-1-yl | CH₂ | O | CH | G¹ | H | Q²⁴-3 | H | CH₂O | 2-Chlor-6-fluorphenyl | 3.44^{[a]}; 3.39^{[b]} |
| I-75* | 3,5-Bis(difluormethyl)-1H-pyrazol-1-yl | CH₂ | O | CH | G¹ | H | Q²⁴-3 | H | CH₂O | 2-Brom-6-fluorphenyl | 3.67^{[a]}; 3.65^{[b]} |

| | | | | | | | | | | | |
|---|---|---|---|---|---|---|---|---|---|---|---|
| *: nicht erfindungsgemäßes Beispiel Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden: ^{[a]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril ^{[b]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. | | | | | | | | | | | |

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen). Die lambda-maX Werte wurden an Hand der UV-Spektren von 200 nm bis 400 nm in den Maxima der chromatographischen Signale ermittelt.

### NMR-Daten ausgewählter Beispiele

### NMR-Peak-Listenverfahren

Die 1H-NMR-Daten der Beispiele I-1 bis I-43 sind in Form von ¹H-NMR-Peaklisten notiert. Zu jedem Signalpeak sind der δ-Wert in ppm und die Signalintensität in Klammern aufgeführt:

| **Bsp. I-1, Solvent: DMSO-d₆** |
|---|
| 8,7789 (0,96); 8,0181 (10,19); 7,5119 (9,1); 7,4916 (10,83); 7,3082 (1,65); 7,2169 (3,43); 7,1971 (4,32); 7,1961 (3,93); 7,1761 (5,27); 7,1595 (1,95); 7,0416 (1,79); 7,0235 (4,31); 6,8992 (4,11); 6,8877 (2,32); 5,7476 (5,36); 5,4539 (0,75); 5,412 (2,71); 5,3675 (2,99); 5,325 (0,81); 5,3067 (0,37); 5,1157 (0,4); 5,1032 (0,7); 5,0973 (0,72); 5,0843 (1,01); 5,0753 (1,09); 5,058 (0,72); 5,0462 (0,45); 4,363 (0,86); 4,3302 (0,97); 4,1787 (0,37); 4,1682 (0,55); 4,1524 (4,14); 4,1498 (4,08); 4,1426 (3,51); 4,1368 (3,15); 4,123 (0,38); 4,1104 (0,44); 4,0568 (1,19); 4,039 (3,63); 4,0212 (3,65); 4,0035 (1,41); 3,9866 (0,85); 3,9503 (0,96); 3,6413 (1,12); 3,6136 (1,22); 3,598 (2,09); 3,5705 (1,86); 3,4989 (2,06); 3,48 (2,06); 3,4557 (1,32); 3,4367 (1,32); 3,4161 (0,89); 3,3966 (1,11); 3,3875 (1,58); 3,3776 (1,15); 3,3676 (1,06); 3,359 (1,49); 3,3506 (1,4); 3,2991 (837,78); 3,2776 (5,93); 3,2439 (1,17); 3,0369 (0,71); 2,8735 (0,7); 2,8496 (1,48); 2,8175 (0,66); 2,6733 (1,51); 2,6687 (1,94); 2,6641 (1,4); 2,5928 (0,4); 2,5387 (2,37); 2,5219 (9,51); 2,5085 (118,32); 2,5042 (214,59); 2,4997 (276,14); 2,4953 (189,37); 2,4909 (89,72); 2,3309 (1,45); 2,3263 (1,89); 2,3216 (1,36); 2,1348 (0,9); 2,1032 (1,72); 2,0695 (3,22); 1,9867 (16); 1,8391 (0,41); 1,8184 (0,8); 1,7889 (0,74); 1,7558 (0,33); 1,6154 (0,43); 1,5793 (0,83); 1,5592 (0,85); 1,5288 (0,37); 1,3988 (0,71); 1,2351 (0,51); 1,1926 (4,73); 1,1748 (9,4); 1,157 (4,55); 0,7834 (0,49); 0,008 (1,76); -0,0002 (35,77); -0,0084 (1,31) |

| **Bsp. I-2, Solvent: DMSO-d₆** |
|---|
| 8,7784 (1,46); 8,4332 (0,32); 8,0177 (13,14); 7,3122 (4,56); 7,293 (5,64); 7,2901 (4,38); 7,2799 (1,11); 7,2721 (4,78); 7,2658 (0,67); 7,1758 (5,2); 7,1598 (2,55); 7,0427 (2,58); 7,0369 (0,73); 7,0238 (5,91); 6,9671 (9,39); 6,9604 (2,61); 6,9504 (7,44); 6,9458 (5,79); 6,9436 (5,22); 6,932 (1,82); 6,8998 (5,29); 6,8879 (2,93); 5,7473 (1,69); 5,4551 (1,04); 5,4119 (3,69); 5,396 (0,77); 5,3693 (3,98); 5,3265 (1,07); 5,0802 (0,58); 5,0674 (0,83); 5,0616 (0,85); 5,0509 (1,14); 5,0478 (1,16); 5,0393 (1,19); 5,0287 (0,86); 5,0207 (0,89); 5,0095 (0,64); 4,3632 (1,17); 4,3321 (1,27); 4,33 (1,24); 4,1518 (0,33); 4,1397 (0,71); 4,1232 (6,29); 4,113 (4,31); 4,1082 (4,2); 4,0955 (0,47); 4,0818 (0,52); 4,0569 (1,29); 4,039 (3,67); 4,0212 (3,78); 4,0035 (1,48); 3,9856 (1,19); 3,9517 (1,3); 3,6073 (1,85); 3,5799 (2,11); 3,564 (2,73); 3,5366 (2,33); 3,4542 (0,42); 3,4458 (0,48); 3,4184 (1,34); 3,3986 (1,5); 3,3897 (2,32); 3,3798 (1,71); 3,3719 (1,41); 3,361 (1,95); 3,3444 (4,05); 3,325 (6,91); 3,3006 (1060,06); 3,2447 (1,16); 2,8741 (0,86); 2,8438 (1,48); 2,8168 (0,85); 2,6734 (1,9); 2,6687 (2,44); 2,6641 (1,88); 2,6597 (0,99); 2,6468 (0,33); 2,6335 (0,32); 2,5387 (2,85); 2,522 (9,76); 2,5086 (140,38); 2,5042 (264,61); 2,4997 (348,18); 2,4953 (240,49); 2,4909 (113,32); 2,3355 (0,84); 2,3311 (1,7); 2,3263 (2,21); 2,3218 (1,56); 2,1455 (1,09); 2,1414 (1,11); 2,1058 (2,18); 2,0693 (3,84); 1,9867 (16); 1,8472 (0,45); 1,8233 (1,05); 1,8153 (1,01); 1,7943 (0,95); 1,7642 (0,41); 1,6218 (0,44); 1,6141 (0,52); 1,583 (1,05); 1,5624 (0,98); 1,553 (0,97); 1,5341 (0,41); 1,4081 (1,71); 1,3985 (2,92); 1,3017 (0,49); 1,2367 (0,57); 1,1926 (4,37); 1,1748 (8,96); 1,157 (4,31); 0,8902 (0,35); 0,0079 (1,2); -0,0002 (27,08); -0,0085 (0,9) |

| **Bsp. I-3, Solvent: DMSO-d₆** |
|---|
| 8,018 (14,6); 7,309 (2,51); 7,2336 (1,85); 7,2297 (2,13); 7,226 (1,76); 7,2221 (1,72); 7,2134 (2,44); 7,2094 (2,88); 7,2047 (4,81); 7,2008 (4,9); 7,1839 (4,61); 7,1799 (5,33); 7,1757 (6,35); 7,1594 (2,91); 7,1469 (2,1); 7,1282 (3,25); 7,1078 (1,56); 7,0424 (2,78); 7,0234 (6,26); 6,989 (1,26); 6,9851 (1,34); 6,9775 (1,43); 6,9731 (1,62); 6,9692 (1,83); 6,9661 (1,78); 6,9584 (1,73); 6,9538 (1,67); 6,9462 (0,99); 6,939 (0,82); 6,9347 (0,88); 6,9002 (5,56); 6,8875 (3,21); 5,7463 (16); 5,4543 (1,15); 5,4118 (4,26); 5,369 (4,19); 5,3272 (1,11); 5,1002 (0,59); 5,0863 (1,03); 5,0819 (0,99); 5,0726 (1,44); 5,0677 (1,32); 5,0585 (1,59); 5,0485 (1,02); 5,044 (0,94); 5,0404 (0,95); 5,0302 (0,63); 4,3654 (1,3); 4,3326 (1,37); 4,2343 (0,65); 4,2239 (0,95); 4,2072 (4,86); 4,2007 (5,8); 4,1974 (5,32); 4,1868 (4,21); 4,174 (0,81); 4,1597 (0,8); 4,0392 (0,55); 4,0213 (0,64); 4,0032 (0,49); 3,9854 (1,21); 3,9528 (1,32); 3,6171 (2,01); 3,5896 (2,28); 3,5739 (3,05); 3,5467 (2,58); 3,4279 (0,8); 3,4184 (1,3); 3,4087 (1,15); 3,3997 (1,7); 3,3897 (2,64); 3,3807 (2,08); 3,3579 (5,39); 3,3387 (8,4); 3,3098 (1358,34); 3,2435 (2,19); 3,2218 (1,32); 3,1444 (0,37); 3,1345 (0,34); 2,8759 (0,99); 2,8475 (1,71); 2,8187 (0,97); 2,6739 (1,13); 2,6694 (1,5); 2,6649 (1,12); 2,6604 (0,61); 2,5394 (2,36); 2,5224 (6,94); 2,5091 (85,13); 2,5048 (157,27); 2,5004 (204,86); 2,496 (145,08); 2,4916 (71,76); 2,3924 (0,35); 2,336 (0,7); 2,3317 (1,18); 2,327 (1,5); 2,3225 (1,15); 2,3182 (0,66); 2,1408 (1,26); 2,1051 (2,51); 2,0846 (1,2); 2,0691 (3,78); 1,9867 (2,4); 1,8544 (0,5); 1,8453 (0,55); 1,8217 (1,1); 1,8154 (1,16); 1,7931 (1,06); 1,7847 (0,99); 1,7641 (0,48); 1,6129 (0,54); 1,5913 (1,08); 1,5831 (1,12); 1,5601 (1,06); 1,5521 (1,01); 1,5306 (0,45); 1,5208 (0,39); 1,3984 (0,5); 1,2365 (0,43); 1,1927 (0,68); 1,1749 (1,31); 1,1571 (0,65); 0,8903 (0,34); 0,0079 (0,59); -0,0002 (11,53); -0,0085 (0,53) |

| **Bsp. I-4, Solvent: DMSO-d₆** |
|---|
| 7,8948 (11,69); 7,4657 (3,19); 7,4625 (4,18); 7,4443 (5,4); 7,4422 (4,93); 7,3519 (3,15); 7,3471 (1,2); 7,3333 (5,39); 7,3133 (3,22); 7,3003 (1,95); 7,2342 (1,99); 7,216 (2,9); 7,2006 (0,78); 7,1978 (1,14); 7,167 (4,23); 7,1563 (2,09); 7,0338 (2,09); 7,0203 (4,86); 6,8962 (3,9); 6,8844 (2,46); 5,7465 (7,98); 5,4414 (0,77); 5,3991 (2,88); 5,356 (2,84); 5,3139 (0,79); 4,9693 (1,71); 4,9472 (2,2); 4,9414 (2,04); 4,9192 (1,76); 4,35 (0,86); 4,3178 (0,89); 4,057 (0,58); 4,0392 (1,67); 4,0214 (1,69); 4,0036 (0,6); 3,9638 (0,79); 3,93 (0,89); 3,3758 (0,6); 3,3674 (0,96); 3,3573 (0,85); 3,3477 (1,32); 3,3383 (2,18); 3,3062 (607,62); 3,2816 (4,07); 3,2528 (2,1); 3,2371 (2,83); 3,209 (2,17); 2,9596 (1,83); 2,9375 (1,74); 2,9156 (1,44); 2,8936 (1,43); 2,8409 (0,64); 2,8131 (1,06); 2,7833 (0,6); 2,6737 (0,56); 2,6692 (0,7); 2,6646 (0,54); 2,5392 (0,93); 2,5224 (2,18); 2,5177 (3,43); 2,509 (39,04); 2,5046 (75,34); 2,5001 (100,62); 2,4957 (69,15); 2,4912 (32,6); 2,3314 (0,52); 2,3268 (0,67); 2,3223 (0,47); 2,0991 (0,78); 2,069 (2,9); 2,0307 (0,87); 1,9867 (7,51); 1,7791 (0,69); 1,7494 (0,64); 1,5739 (0,32); 1,5429 (0,71); 1,513 (0,66); 1,3399 (16); 1,2731 (15,91); 1,2361 (0,45); 1,2307 (0,44); 1,1927 (2,11); 1,1749 (4,34); 1,1571 (2,09); -0,0002 (1,98) |

| **Bsp. I-5, Solvent: DMSO-d₆** |
|---|
| 8,7779 (0,36); 8,0112 (16); 8,0008 (2,37); 7,5131 (0,47); 7,4949 (0,98); 7,4838 (1,49); 7,4786 (2,4); 7,4585 (12,69); 7,442 (7,85); 7,4227 (3,62); 7,4138 (2,68); 7,409 (2,95); 7,401 (1,78); 7,3924 (2,63); 7,3836 (0,72); 7,3803 (0,73); 7,3759 (0,84); 7,3069 (2,88); 7,1737 (6,44); 7,1597 (3,25); 7,1523 (0,9); 7,0404 (3,14); 7,0237 (7,34); 7,0161 (1,52); 6,8994 (6,62); 6,8879 (4,11); 6,8803 (0,87); 5,8553 (2,32); 5,8454 (2,32); 5,747 (5,71); 5,7374 (2,32); 5,7275 (2,41); 5,6879 (0,32); 5,5853 (0,32); 5,5699 (0,34); 5,451 (1,28); 5,4083 (4,72); 5,3653 (4,58); 5,3227 (1,38); 5,217 (1,94); 5,1711 (0,68); 5,1609 (0,73); 5,152 (0,89); 5,1426 (1,39); 5,1335 (0,8); 5,1243 (0,84); 5,1192 (0,93); 5,1144 (0,95); 5,1099 (0,81); 5,1004 (0,92); 5,0912 (1,3); 5,0818 (0,72); 5,0727 (0,74); 5,063 (0,63); 4,5906 (0,52); 4,3598 (1,4); 4,3273 (1,47); 3,9779 (1,29); 3,9444 (1,4); 3,7205 (0,34); 3,7153 (0,34); 3,7096 (0,92); 3,7055 (0,75); 3,6981 (1,17); 3,696 (1,24); 3,6812 (1,37); 3,6791 (1,09); 3,6717 (0,78); 3,6682 (0,94); 3,6621 (0,39); 3,6568 (0,34); 3,5224 (0,68); 3,5053 (1,5); 3,498 (2,16); 3,4879 (1,22); 3,4743 (1,41); 3,4701 (1,54); 3,4639 (1,37); 3,4544 (3,17); 3,4269 (2,83); 3,4103 (0,69); 3,394 (4,02); 3,3752 (4,64); 3,3618 (1,64); 3,351 (2,34); 3,3426 (1,48); 3,3318 (2,7); 3,3018 (604,73); 3,263 (2,65); 3,2332 (1,3); 3,15 (0,53); 3,1309 (0,46); 3,1067 (0,4); 3,0871 (0,34); 2,8628 (0,96); 2,8319 (1,73); 2,8051 (0,94); 2,6779 (0,39); 2,6734 (0,8); 2,6688 (1,08); 2,6643 (0,79); 2,6596 (0,39); 2,5388 (1,24); 2,5221 (3,17); 2,5173 (4,97); 2,5087 (61,05); 2,5043 (118,37); 2,4998 (158,05); 2,4954 (110,05); 2,4909 (52,81); 2,3358 (0,49); 2,3311 (0,87); 2,3265 (1,16); 2,3219 (0,87); 2,3173 (0,45); 2,1242 (1,26); 2,0888 (2,62); 2,0691 (1,82); 2,0564 (1,47); 1,9866 (0,33); 1,9407 (0,5); 1,9241 (0,83); 1,9077 (0,68); 1,8296 (0,73); 1,8104 (1,54); 1,7952 (1,61); 1,7782 (1,53); 1,7484 (0,51); 1,5966 (0,52); 1,5758 (1,05); 1,5673 (1,17); 1,5451 (1,07); 1,5368 (1,08); 1,5164 (0,48); 1,5064 (0,44); 1,4065 (1,23); 1,3983 (1,77); 1,2362 (0,49); 1,1083 (0,33); 1,0909 (0,63); 1,0733 (0,33); 0,008 (0,7); -0,0002 (18,53); -0,0085 (0,73) |

| **Bsp. I-6, Solvent: DMSO-d₆** |
|---|
| 7,9948 (5,21); 7,9771 (6,01); 7,9736 (4,77); 7,9474 (11,68); 7,6622 (1,19); 7,659 (0,77); 7,6438 (3,02); 7,6392 (1,1); 7,6285 (1,47); 7,6254 (2,25); 7,6221 (1,27); 7,5544 (4,18); 7,5349 (6,04); 7,5165 (2,74); 7,3091 (1,94); 7,1756 (4,37); 7,1597 (2,13); 7,0425 (2,19); 7,0237 (4,84); 6,9003 (4,31); 6,8879 (2,42); 5,7467 (16); 5,4536 (0,89); 5,4105 (3,21); 5,3676 (3,19); 5,3254 (0,86); 4,7927 (0,51); 4,7764 (0,94); 4,7674 (0,73); 4,7579 (1,26); 4,7521 (1,17); 4,7405 (0,73); 4,7332 (1); 4,7151 (0,43); 4,3659 (1,04); 4,3316 (1,12); 3,9855 (0,99); 3,9482 (1,11); 3,5676 (0,71); 3,5614 (0,38); 3,537 (1,8); 3,511 (2,08); 3,4943 (2,58); 3,4685 (2,19); 3,4081 (1,6); 3,3879 (2,12); 3,3791 (2,87); 3,369 (2,71); 3,3066 (1246,75); 3,2408 (1,38); 3,1894 (3,26); 3,1772 (3,6); 3,1722 (4,94); 3,1577 (3,08); 3,153 (3,57); 3,1348 (1,79); 3,1152 (1,73); 2,8708 (0,74); 2,8417 (1,32); 2,8131 (0,78); 2,6942 (0,44); 2,6737 (1,38); 2,6691 (1,85); 2,6645 (1,38); 2,6259 (0,39); 2,579 (0,7); 2,5392 (3,68); 2,5222 (9,49); 2,509 (106,46); 2,5046 (194,2); 2,5001 (250,65); 2,4957 (174,09); 2,4913 (83,35); 2,3314 (1,22); 2,3269 (1,65); 2,3222 (1,19); 2,1317 (1); 2,0976 (1,89); 2,0692 (2); 2,0497 (0,76); 2,0143 (0,37); 1,9998 (1,13); 1,9916 (1,11); 1,9824 (2,09); 1,9766 (2,09); 1,9634 (2,31); 1,9468 (1,03); 1,9396 (1); 1,8468 (0,35); 1,8383 (0,39); 1,8086 (0,86); 1,7849 (0,82); 1,6082 (0,39); 1,5872 (0,82); 1,5798 (0,84); 1,5581 (0,8); 1,5281 (0,35); 1,2365 (0,52); -0,0002 (3,32) |

| **Bsp. I-7, Solvent: DMSO-d₆** |
|---|
| 7,9748 (10,13); 7,9587 (3,46); 7,9559 (4,09); 7,943 (1,43); 7,938 (5,32); 7,9346 (3,96); 7,7961 (0,56); 7,7934 (0,94); 7,7903 (0,61); 7,7801 (0,78); 7,775 (2,5); 7,77 (0,92); 7,7595 (1,27); 7,7563 (2,06); 7,7531 (1,09); 7,6979 (3,47); 7,6941 (1,64); 7,6812 (2,95); 7,6782 (4,96); 7,664 (0,96); 7,6599 (2); 7,3064 (1,46); 7,1731 (3,48); 7,1587 (1,69); 7,0399 (1,71); 7,0227 (4,01); 6,8989 (3,26); 6,8868 (1,99); 5,4503 (0,66); 5,4074 (2,49); 5,3643 (2,45); 5,3216 (0,68); 5,0161 (0,36); 5,0002 (0,78); 4,9889 (0,59); 4,9827 (1); 4,9742 (0,97); 4,9563 (0,83); 4,9401 (0,35); 4,3529 (0,77); 4,32 (0,78); 4,0566 (1,21); 4,0388 (3,55); 4,0211 (3,55); 4,0033 (1,29); 3,9732 (0,7); 3,9405 (0,79); 3,878 (0,53); 3,862 (0,56); 3,8417 (2,78); 3,8286 (3,34); 3,8257 (3,32); 3,8143 (2,66); 3,7924 (0,56); 3,7778 (0,48); 3,6418 (1,21); 3,6151 (1,42); 3,5985 (1,79); 3,572 (1,49); 3,407 (0,51); 3,398 (0,81); 3,3786 (0,96); 3,3688 (1,52); 3,3604 (1,12); 3,351 (1,08); 3,3409 (1,53); 3,3171 (5,32); 3,2965 (828,91); 3,2746 (5); 3,2564 (2,6); 3,2319 (0,74); 2,8643 (0,56); 2,8366 (0,97); 2,8067 (0,59); 2,6775 (1,02); 2,673 (1,98); 2,6684 (2,62); 2,6638 (1,94); 2,6591 (0,99); 2,5385 (3,43); 2,5217 (10,36); 2,5169 (16,24); 2,5083 (147,02); 2,5038 (275,75); 2,4993 (362,1); 2,4949 (247,06); 2,4904 (115,63); 2,3352 (0,97); 2,3307 (1,87); 2,3261 (2,47); 2,3215 (1,75); 2,3169 (0,84); 2,1126 (0,66); 2,082 (1,39); 2,0695 (3); 2,0492 (0,86); 2,0391 (1,09); 1,9866 (16); 1,9072 (0,42); 1,823 (0,35); 1,7918 (0,66); 1,7697 (0,59); 1,5549 (0,62); 1,535 (0,58); 1,2364 (0,7); 1,1925 (4,7); 1,1747 (9,31); 1,1569 (4,59); 0,008 (2,61); -0,0002 (63,6); -0,0085 (2,11) |

| **Bsp. I-8, Solvent: DMSO-d₆** |
|---|
| 7,9579 (3,77); 7,9086 (16); 7,4331 (5,22); 7,4154 (7,99); 7,3777 (1,39); 7,3732 (0,66); 7,3597 (6,08); 7,3418 (8,65); 7,3229 (4,35); 7,3043 (3,02); 7,2854 (2,23); 7,282 (3,2); 7,2786 (1,92); 7,2696 (1,97); 7,264 (3,9); 7,2581 (1,12); 7,2492 (1,12); 7,2458 (1,28); 7,1709 (6,49); 7,1582 (3,33); 7,0377 (3,2); 7,0221 (7,39); 7,0162 (1,07); 6,8978 (6,85); 6,8863 (3,96); 5,7467 (12,05); 5,7383 (0,96); 5,7268 (0,81); 5,651 (2,28); 5,6388 (2,22); 5,4469 (1,21); 5,4041 (4,42); 5,361 (4,23); 5,3189 (1,25); 5,2164 (0,98); 4,8728 (1,17); 4,859 (1,29); 4,8521 (1,67); 4,8458 (1,36); 4,838 (1,74); 4,8317 (1,76); 4,8248 (1,26); 4,8184 (0,6); 4,8109 (1,61); 4,7991 (0,49); 4,7916 (0,48); 4,7731 (0,43); 4,7632 (0,57); 4,7514 (0,84); 4,7404 (0,36); 4,6652 (1,76); 4,6528 (2,67); 4,6407 (1,57); 4,3539 (1,4); 4,3207 (1,42); 4,0567 (0,39); 4,039 (1,23); 4,0213 (1,23); 4,0033 (0,52); 3,9706 (1,28); 3,9379 (1,42); 3,5053 (0,65); 3,4885 (0,4); 3,4113 (0,63); 3,392 (1,06); 3,3815 (1,27); 3,3681 (1,59); 3,3632 (1,71); 3,3527 (2,57); 3,3441 (2,09); 3,3032 (915,47); 3,2697 (4,08); 3,254 (5,65); 3,2266 (3,86); 3,1879 (0,63); 3,1689 (0,34); 3,1478 (2,97); 3,1267 (2,96); 3,1047 (1,92); 3,0837 (1,79); 2,8545 (0,99); 2,8267 (1,6); 2,7973 (0,94); 2,6782 (0,6); 2,6733 (1,19); 2,6688 (1,61); 2,6643 (1,2); 2,6595 (0,56); 2,5542 (0,33); 2,5388 (2,11); 2,522 (5,65); 2,5172 (8,99); 2,5086 (94,87); 2,5042 (180,06); 2,4997 (237,82); 2,4953 (161,9); 2,4908 (75,43); 2,4273 (0,35); 2,3358 (0,64); 2,331 (1,27); 2,3264 (1,68); 2,3218 (1,24); 2,1052 (1,45); 2,0759 (2,54); 2,069 (3,09); 2,047 (1,37); 1,9865 (5,52); 1,9234 (0,45); 1,9076 (1,04); 1,823 (0,6); 1,8135 (0,95); 1,7937 (1,45); 1,7848 (1,28); 1,7631 (1,09); 1,7562 (1,03); 1,7334 (0,44); 1,5813 (0,68); 1,5496 (1,18); 1,5276 (0,96); 1,4971 (0,4); 1,399 (0,33); 1,2369 (0,52); 1,1925 (1,54); 1,1747 (3,13); 1,157 (1,53); 0,008 (1,16); -0,0002 (30,07); - 0,0085 (0,94) |

| **Bsp. I-9, Solvent: DMSO-d₆** |
|---|
| 8,0673 (16); 8,0335 (0,47); 7,6238 (3,09); 7,6196 (3,66); 7,6064 (4,82); 7,6001 (5,56); 7,5834 (0,44); 7,5773 (0,53); 7,5575 (2,22); 7,5457 (9,82); 7,5294 (4,28); 7,5221 (1,24); 7,507 (0,66); 7,3063 (2,61); 7,191 (0,34); 7,173 (5,94); 7,1592 (2,99); 7,0547 (0,6); 7,0397 (2,95); 7,0232 (6,92); 6,9172 (0,39); 6,8996 (5,69); 6,8873 (3,45); 5,7468 (8,71); 5,4503 (1,46); 5,4353 (0,75); 5,4224 (1,12); 5,4067 (5,06); 5,391 (1,24); 5,3651 (4,35); 5,346 (0,69); 5,3226 (1,1); 4,5924 (0,35); 4,3582 (1,25); 4,3267 (1,31); 3,9766 (1,17); 3,9441 (1,28); 3,7153 (0,34); 3,709 (0,69); 3,7021 (2); 3,6985 (1,07); 3,6961 (0,9); 3,6814 (0,91); 3,6792 (0,84); 3,6733 (2,37); 3,6686 (0,85); 3,6576 (3,19); 3,6291 (2,58); 3,5677 (0,47); 3,5118 (0,4); 3,4981 (0,65); 3,4866 (3,17); 3,4705 (3,01); 3,4526 (0,49); 3,4421 (2,18); 3,4261 (2,19); 3,4107 (0,78); 3,4014 (1,21); 3,3915 (1,04); 3,3818 (1,55); 3,3728 (2,31); 3,3633 (1,63); 3,3534 (1,41); 3,3438 (1,89); 3,3058 (797,64); 3,2847 (4,81); 3,2617 (1,87); 3,2324 (0,85); 2,8619 (0,88); 2,8498 (0,46); 2,8347 (1,56); 2,8049 (0,89); 2,6782 (0,41); 2,6738 (0,81); 2,6691 (1,09); 2,6646 (0,78); 2,5392 (1,63); 2,5224 (4,11); 2,5177 (6,46); 2,5091 (60,47); 2,5047 (113,02); 2,5002 (147,94); 2,4957 (99,66); 2,4913 (45,49); 2,3315 (0,74); 2,3269 (0,97); 2,3224 (0,67); 2,1254 (1,06); 2,0917 (2,24); 2,0692 (1,38); 2,0578 (1,21); 1,9867 (0,66); 1,8275 (0,46); 1,8071 (1); 1,7992 (1); 1,7769 (0,94); 1,7464 (0,38); 1,6013 (0,38); 1,5933 (0,45); 1,5749 (0,96); 1,5651 (0,98); 1,5438 (0,95); 1,5342 (0,83); 1,5134 (0,37); 1,3982 (0,39); 1,237 (0,33); 1,1749 (0,45); 0,008 (0,79); -0,0002 (19,93); -0,0085 (0,6) |

| **Bsp. I-10, Solvent: DMSO-d₆** |
|---|
| 8,7783 (1,32); 7,9848 (16); 7,4231 (4,67); 7,4198 (6,3); 7,4148 (2,02); 7,4018 (8,93); 7,3997 (8,43); 7,3507 (5,5); 7,346 (1,88); 7,3322 (8,79); 7,3164 (2,4); 7,3123 (5,44); 7,2397 (1,92); 7,2366 (3,1); 7,2334 (1,72); 7,2228 (1,65); 7,2183 (4,21); 7,203 (1,05); 7,2001 (1,67); 7,1762 (6,25); 7,1606 (3,15); 7,1492 (0,46); 7,0429 (3,06); 7,0245 (6,92); 6,9006 (6,35); 6,8886 (3,63); 5,7472 (10,81); 5,4546 (1,18); 5,4113 (4,45); 5,3685 (4,82); 5,3266 (1,31); 5,307 (0,59); 4,8733 (0,58); 4,8571 (1,57); 4,8413 (1,73); 4,8311 (1,81); 4,8161 (1,63); 4,7992 (0,71); 4,3599 (1,39); 4,3296 (1,44); 4,039 (0,55); 4,0212 (0,42); 3,9817 (1,39); 3,9472 (1,51); 3,581 (2,32); 3,5549 (2,63); 3,5379 (3,39); 3,5117 (2,92); 3,4319 (0,41); 3,4068 (1,29); 3,3972 (1,13); 3,3876 (1,69); 3,3784 (2,56); 3,3683 (1,83); 3,3594 (1,72); 3,3499 (2,28); 3,3372 (4,07); 3,3226 (7,22); 3,3004 (1081,4); 3,2796 (11,86); 3,2543 (3,75); 3,2395 (5,77); 3,2236 (4,22); 3,2055 (2,26); 3,1893 (2,08); 3,0369 (1,13); 2,8711 (1,07); 2,849 (2,13); 2,8422 (1,88); 2,8131 (1,05); 2,6778 (0,97); 2,6732 (1,78); 2,6688 (2,43); 2,6643 (1,77); 2,5388 (2,46); 2,522 (8,87); 2,5086 (135,1); 2,5042 (255,37); 2,4998 (336,11); 2,4954 (233,23); 2,491 (110,97); 2,331 (1,72); 2,3265 (2,3); 2,3218 (1,58); 2,1325 (1,32); 2,0956 (2,61); 2,0693 (2); 2,0611 (1,49); 1,9866 (1,89); 1,8407 (0,52); 1,8355 (0,59); 1,8128 (1,14); 1,8068 (1,19); 1,7823 (1,18); 1,776 (1,08); 1,7542 (0,44); 1,7432 (0,43); 1,6133 (0,51); 1,6029 (0,6); 1,581 (1,18); 1,5729 (1,15); 1,5515 (1,11); 1,521 (0,45); 1,51 (0,42); 1,2363 (0,45); 1,1927 (0,52); 1,1748 (1); 1,1571 (0,49); -0,0002 (15,5) |

| **Bsp. I-12, Solvent: DMSO-d₆** |
|---|
| 8,0094 (5,02); 7,5797 (1,05); 7,5753 (1,3); 7,5605 (1,23); 7,5558 (1,56); 7,5496 (0,57); 7,5357 (0,93); 7,533 (1,01); 7,5286 (0,77); 7,5149 (0,84); 7,5103 (0,62); 7,3082 (0,8); 7,1973 (1,42); 7,1753 (2,88); 7,1592 (0,9); 7,0589 (0,91); 7,0567 (0,89); 7,0406 (2,09); 7,0228 (2,41); 6,8997 (1,8); 6,8872 (1,03); 5,4548 (0,4); 5,413 (1,34); 5,3683 (1,34); 5,3264 (0,38); 5,1469 (0,51); 5,1334 (0,58); 5,1153 (0,32); 4,3631 (0,45); 4,3316 (0,53); 4,3194 (0,72); 4,3105 (0,53); 4,2926 (1,14); 4,2836 (1,06); 4,2658 (1,07); 4,2525 (1,04); 4,2392 (0,51); 4,2258 (0,45); 4,0568 (0,71); 4,039 (2,03); 4,0212 (2,05); 4,0032 (0,77); 3,9835 (0,43); 3,961 (0,37); 3,951 (0,47); 3,6559 (0,65); 3,6282 (0,73); 3,6128 (1,02); 3,5848 (0,84); 3,4224 (1,36); 3,404 (1,47); 3,3917 (1,06); 3,3792 (1,34); 3,3611 (1,51); 3,3013 (476,96); 3,2467 (0,64); 2,8796 (0,67); 2,8678 (0,51); 2,8508 (0,56); 2,82 (0,33); 2,6734 (0,83); 2,6689 (1,07); 2,6643 (0,79); 2,6095 (0,34); 2,5388 (1,84); 2,5219 (5,94); 2,5086 (63,99); 2,5043 (114,23); 2,4998 (145,47); 2,4954 (99,54); 2,491 (47,06); 2,4509 (16); 2,3311 (0,74); 2,3264 (0,99); 2,3218 (0,67); 2,14 (0,39); 2,1054 (0,8); 2,0694 (0,5); 1,9867 (8,88); 1,8231 (0,36); 1,8119 (0,36); 1,7914 (0,36); 1,5926 (0,35); 1,5834 (0,38); 1,5632 (0,36); 1,5547 (0,35); 1,1926 (2,49); 1,1748 (5,05); 1,157 (2,44); 0,0079 (0,56); -0,0002 (11,07); -0,0085 (0,42) |

| **Bsp. I-13, Solvent: DMSO-d₆** |
|---|
| 8,0118 (8,81); 7,3082 (1,51); 7,1748 (3,46); 7,1583 (2,4); 7,1373 (1,94); 7,1231 (2,51); 7,1054 (2,06); 7,0416 (1,74); 7,0225 (3,77); 6,9441 (2,54); 6,9248 (2,21); 6,8997 (3,36); 6,8865 (1,95); 6,8612 (1,49); 6,8426 (2,4); 6,8245 (1,12); 5,7463 (10,1); 5,4553 (0,72); 5,412 (2,58); 5,3675 (2,48); 5,3254 (0,74); 5,0994 (0,43); 5,0825 (0,67); 5,0727 (0,92); 5,06 (1,04); 5,0509 (0,64); 5,0426 (0,62); 5,029 (0,71); 4,3643 (0,8); 4,3288 (0,85); 4,1569 (0,9); 4,1482 (0,98); 4,1298 (2,25); 4,1211 (2,1); 4,1028 (2,2); 4,0903 (2,14); 4,076 (0,9); 4,0638 (0,92); 3,9829 (0,8); 3,9516 (0,85); 3,6102 (1,27); 3,5827 (1,38); 3,5675 (2,06); 3,54 (1,73); 3,4719 (0,38); 3,4194 (1,24); 3,4073 (2,83); 3,4009 (1,86); 3,3901 (3,8); 3,3638 (3,81); 3,3467 (5,47); 3,3095 (1099,49); 3,2454 (1,43); 2,8775 (0,64); 2,851 (1,03); 2,819 (0,6); 2,6951 (0,34); 2,6738 (1,1); 2,6692 (1,43); 2,6648 (1,09); 2,5393 (2,63); 2,5091 (83,19); 2,5048 (152,23); 2,5003 (196,93); 2,4959 (138,75); 2,4916 (67,78); 2,4342 (0,37); 2,4274 (0,36); 2,3314 (1,04); 2,3269 (1,33); 2,3224 (0,96); 2,2521 (0,94); 2,1382 (0,76); 2,1028 (1,57); 2,069 (1,54); 2,0486 (16); 1,8137 (0,7); 1,79 (0,64); 1,6108 (0,35); 1,583 (0,68); 1,56 (0,67); 1,2364 (0,38); -0,0002 (1,69) |

| **Bsp. I-14, Solvent: CD₃CN** |
|---|
| 7,5913 (6,28); 7,2623 (12,81); 7,2542 (0,36); 7,2501 (2,47); 7,2475 (2,49); 7,2389 (0,69); 7,2354 (2,02); 6,9715 (1,06); 6,8797 (2,25); 6,788 (1,14); 6,7625 (4,16); 6,7607 (3,61); 6,7548 (1,23); 6,747 (2,69); 6,7415 (2,2); 6,7294 (0,85); 6,6713 (2,65); 6,5799 (1,28); 5,3017 (13,63); 5,1586 (2,57); 5,1469 (2,57); 5,1196 (0,32); 5,0547 (0,57); 5,0509 (0,36); 5,0461 (0,49); 5,0416 (0,7); 5,0373 (0,69); 5,0328 (0,52); 5,028 (0,38); 5,0242 (0,62); 4,588 (0,5); 4,5659 (0,52); 3,922 (0,48); 3,8985 (0,51); 3,5991 (3,75); 3,5898 (3,89); 3,4854 (1,03); 3,468 (1,08); 3,4572 (1,34); 3,4398 (1,22); 3,3537 (0,51); 3,3476 (0,57); 3,3418 (0,67); 3,3351 (1,03); 3,3286 (1); 3,3243 (0,87); 3,3166 (0,61); 3,3097 (0,37); 3,3063 (0,47); 3,3018 (0,38); 3,2217 (1,33); 3,2086 (1,31); 3,1935 (1,1); 3,1804 (1,09); 3,0639 (16); 3,0568 (0,58); 2,9359 (0,35); 2,9159 (0,61); 2,8955 (0,36); 2,2792 (0,43); 2,259 (0,5); 2,2004 (0,44); 2,1747 (0,69); 1,8991 (0,86); 1,8856 (0,92); 1,882 (0,44); 1,8765 (0,45); 1,8606 (0,44); 1,8555 (0,44); 1,8344 (0,39); 1,8147 (0,5); 1,808 (0,55); 1,7928 (0,55); 1,7863 (0,52); 1,7736 (0,36); 1,7665 (0,36); 1,2531 (0,82); 0,0052 (0,38); -0,0002 (13,69); -0,0057 (0,45) |

| **Bsp. I-15, Solvent: DMSO-d₆** |
|---|
| 8,6125 (0,43); 8,4815 (0,4); 8,1857 (0,45); 8,1796 (5,87); 8,1262 (2,3); 7,8855 (1,12); 7,8776 (1,89); 7,8711 (1,65); 7,8511 (4,04); 7,8431 (2,38); 7,4285 (0,63); 7,4082 (4,86); 7,3968 (7,52); 7,3942 (7,05); 7,3902 (5,82); 7,3744 (1,23); 7,3628 (0,62); 7,3545 (1,14); 7,3503 (1,16); 7,3411 (1,22); 7,3333 (1,23); 7,3241 (0,67); 7,3174 (1,93); 7,3093 (0,73); 7,1842 (3,18); 7,1759 (1,24); 7,1671 (1,33); 7,1623 (1,56); 7,0511 (1,62); 7,0427 (0,67); 7,0312 (2,94); 7,0262 (3,17); 6,911 (4,79); 6,8954 (1,42); 6,8904 (1,52); 5,7457 (15,25); 5,5303 (0,5); 5,5157 (0,4); 5,5093 (0,56); 5,4901 (1,21); 5,466 (1,57); 5,4166 (2,89); 5,3741 (0,94); 4,9239 (0,36); 4,8679 (0,47); 4,8551 (0,51); 4,8482 (0,56); 4,8408 (0,72); 4,8356 (0,6); 4,8281 (0,73); 4,8212 (0,51); 4,8086 (0,48); 4,4144 (1,75); 4,4017 (1,64); 4,3381 (1); 4,3217 (0,96); 4,2794 (0,55); 4,2399 (0,84); 4,2004 (0,49); 3,9117 (0,9); 3,5678 (0,53); 3,5097 (0,58); 3,4988 (0,59); 3,4803 (0,98); 3,4604 (1); 3,4407 (0,9); 3,4167 (1,39); 3,3963 (1,47); 3,3865 (1); 3,3614 (1,17); 3,3138 (787,45); 3,2785 (0,64); 3,2698 (0,36); 3,2113 (16); 3,181 (0,51); 3,1693 (7,55); 3,1505 (0,34); 3,1399 (0,35); 3,1165 (0,74); 3,0872 (0,9); 3,0794 (0,93); 3,0559 (0,5); 2,6744 (0,46); 2,6697 (0,64); 2,6652 (0,48); 2,5397 (1,12); 2,5231 (1,95); 2,5183 (2,97); 2,5097 (35,11); 2,5053 (68,02); 2,5007 (90,85); 2,4963 (61,93); 2,4918 (28,84); 2,4616 (0,42); 2,4343 (0,42); 2,4081 (0,33); 2,3699 (0,39); 2,3367 (0,64); 2,3322 (0,82); 2,3274 (0,88); 2,3227 (0,68); 2,3185 (0,47); 2,2355 (0,67); 2,2033 (1,21); 2,1742 (2,11); 2,1497 (1,24); 2,1155 (0,42); 2,0973 (0,38); 2,0783 (0,35); 2,0688 (0,71) |

| **Bsp. I-16, Solvent: DMSO-d₆** |
|---|
| 8,6106 (6,92); 8,0668 (16); 7,7227 (0,38); 7,7034 (3,86); 7,6833 (4,57); 7,6258 (4,01); 7,6216 (4,72); 7,6084 (6,05); 7,6022 (6,7); 7,5858 (0,7); 7,5781 (0,83); 7,5728 (0,83); 7,5456 (12,63); 7,5329 (9,9); 7,5069 (0,95); 7,485 (3,76); 7,465 (3,04); 7,2205 (3); 7,2134 (2,43); 7,0814 (6,6); 7,0756 (5,45); 6,9422 (3,48); 6,938 (2,87); 5,7471 (10,76); 5,4521 (0,56); 5,4361 (0,7); 5,4241 (1,09); 5,4088 (1,17); 5,4018 (1); 5,3977 (1,03); 5,3917 (1,19); 5,376 (1,13); 5,3635 (0,7); 5,3472 (0,57); 4,1729 (3,64); 4,1395 (3,76); 4,0574 (0,52); 4,0397 (1,42); 4,0218 (1,42); 4,0041 (0,51); 3,7064 (2,25); 3,6776 (2,47); 3,6618 (3,55); 3,6332 (2,98); 3,5681 (0,56); 3,4936 (3,34); 3,4775 (3,4); 3,4491 (2,45); 3,4331 (2,37); 3,355 (1,85); 3,3067 (282,1); 3,0619 (2,49); 3,0326 (4,52); 3,0035 (2,46); 2,6742 (0,4); 2,6696 (0,52); 2,6652 (0,4); 2,5394 (1,28); 2,5092 (31,19); 2,505 (54,68); 2,5006 (68,48); 2,4963 (47,2); 2,332 (0,33); 2,3272 (0,46); 2,3226 (0,35); 2,1042 (2,9); 2,0982 (3); 2,0697 (4,01); 1,9871 (5,92); 1,7193 (1,05); 1,713 (1,17); 1,689 (2,62); 1,6825 (2,69); 1,6595 (2,52); 1,6303 (0,91); 1,1929 (1,61); 1,1751 (3,22); 1,1573 (1,53); -0,0002 (6,37) |

| **Bsp. I-18, Solvent: DMSO-d₆** |
|---|
| 8,8065 (0,37); 8,1082 (7,01); 8,0642 (2,58); 7,6069 (0,45); 7,5951 (0,73); 7,5818 (0,87); 7,5703 (0,59); 7,2965 (0,96); 7,2889 (0,42); 7,2636 (0,58); 7,2487 (1,09); 7,2394 (1,57); 7,2348 (0,78); 7,2253 (2,8); 7,2082 (2,59); 7,2005 (0,96); 7,1773 (0,35); 7,1302 (1,16); 7,1249 (0,54); 7,1197 (1,2); 7,1121 (0,48); 7,0398 (2,82); 7,0346 (1,04); 7,028 (0,47); 6,9495 (1,32); 6,9443 (0,5); 6,9131 (3,05); 6,9007 (0,39); 5,888 (0,95); 5,8755 (0,99); 5,8659 (0,35); 5,8528 (0,35); 5,8136 (0,96); 5,8011 (1,02); 5,7888 (0,33); 5,7757 (0,38); 5,7617 (16); 5,6067 (0,43); 5,578 (2,82); 5,5701 (3,19); 5,5604 (1,49); 5,5419 (0,41); 5,4185 (0,84); 5,3285 (0,75); 5,3162 (0,57); 5,3061 (0,73); 5,2435 (0,36); 5,2386 (0,44); 5,2273 (0,65); 5,2156 (0,42); 5,2107 (0,37); 4,4699 (0,55); 4,4486 (0,73); 4,0344 (0,91); 4,0226 (0,91); 3,8026 (0,43); 3,7902 (0,34); 3,7487 (0,69); 3,7311 (0,76); 3,7198 (1,24); 3,7023 (1,08); 3,6813 (0,37); 3,6396 (1,04); 3,6293 (1,06); 3,6108 (0,69); 3,6005 (0,68); 3,5881 (0,63); 3,5826 (0,8); 3,5748 (0,46); 3,5632 (1,95); 3,5562 (0,88); 3,5441 (1,41); 3,5376 (1,07); 3,5301 (0,32); 3,5247 (0,36); 3,5195 (0,55); 3,3795 (0,77); 3,3488 (43,09); 3,3201 (0,53); 2,6152 (0,34); 2,5245 (0,65); 2,5214 (0,81); 2,5183 (0,79); 2,5094 (18,14); 2,5065 (39,22); 2,5034 (53,9); 2,5004 (39,02); 2,4974 (17,97); 2,3876 (0,34); 2,211 (0,92); 2,2039 (0,84); 2,1887 (1,31); 2,0413 (0,4); 1,9903 (4,08); 1,9214 (0,55); 1,9105 (1,44); 1,8986 (0,73); 1,8721 (0,32); 1,7405 (0,54); 1,7203 (0,62); 1,6954 (0,33); 1,1865 (1,11); 1,1746 (2,17); 1,1628 (1,1); -0,0002 (9,35) |

| **Bsp. I-19, Solvent: DMSO-d₆** |
|---|
| 8,0092 (7,59); 7,999 (1,95); 7,5135 (0,5); 7,4955 (0,98); 7,4836 (1,02); 7,4787 (1,51); 7,4585 (6,94); 7,4415 (4,39); 7,4221 (2,14); 7,4135 (1,62); 7,4086 (1,72); 7,4007 (1,06); 7,3921 (1,49); 7,3832 (0,47); 7,3797 (0,46); 7,3755 (0,49); 6,4914 (4,78); 5,8548 (1,23); 5,8449 (1,22); 5,7368 (1,2); 5,7271 (1,26); 5,3421 (0,86); 5,2994 (2,74); 5,2467 (2,55); 5,2036 (0,99); 5,1702 (0,45); 5,1602 (0,52); 5,1516 (0,58); 5,142 (0,84); 5,133 (0,49); 5,1233 (0,53); 5,119 (0,6); 5,1137 (0,62); 5,1092 (0,57); 5,0997 (1,63); 5,0907 (0,79); 5,0815 (0,43); 5,0722 (0,4); 5,0623 (0,35); 4,3825 (0,84); 4,3495 (0,92); 4,0572 (0,87); 4,0394 (2,6); 4,0216 (2,65); 4,0037 (1,08); 3,9867 (0,82); 3,9527 (0,88); 3,6983 (0,38); 3,6963 (0,41); 3,6818 (0,41); 3,5678 (1,07); 3,5321 (0,35); 3,5154 (0,73); 3,5055 (0,51); 3,4983 (1,12); 3,475 (0,6); 3,4706 (0,85); 3,4649 (0,59); 3,4547 (1,7); 3,4273 (1,63); 3,3947 (2,46); 3,3757 (2,66); 3,3659 (1,75); 3,3514 (1,92); 3,3113 (116,41); 3,2633 (1,52); 3,2329 (0,79); 3,1512 (0,43); 3,1318 (0,39); 2,8629 (0,59); 2,8328 (1,04); 2,8049 (0,57); 2,8001 (0,56); 2,5393 (0,58); 2,5223 (1,32); 2,509 (16,52); 2,5048 (30,02); 2,5003 (38,56); 2,496 (27,04); 2,4918 (13,33); 2,2155 (3,97); 2,2094 (16); 2,1943 (1,86); 2,1231 (0,85); 2,0907 (1,7); 2,0688 (0,99); 1,9867 (11,23); 1,9229 (0,47); 1,9081 (0,46); 1,8299 (0,4); 1,8232 (0,38); 1,8116 (0,96); 1,7944 (0,84); 1,7768 (0,89); 1,7522 (0,33); 1,595 (0,33); 1,5723 (0,62); 1,5645 (0,72); 1,5429 (0,63); 1,5336 (0,68); 1,398 (0,36); 1,1927 (3,06); 1,1749 (6,08); 1,1571 (2,97); -0,0002 (1,99) |

| **Bsp. I-20, Solvent: DMSO-d₆** |
|---|
| 8,0774 (8,72); 7,5961 (0,77); 7,5783 (1,07); 7,5753 (1,08); 7,5579 (0,78); 7,2359 (2,04); 7,2147 (3,56); 7,1933 (1,66); 6,4936 (4,25); 5,9037 (1,2); 5,8853 (1,27); 5,7919 (1,19); 5,7734 (1,26); 5,7461 (2,03); 5,3481 (0,79); 5,3053 (2,42); 5,2506 (2,73); 5,2212 (0,98); 5,2077 (1,14); 5,0984 (1,35); 4,3902 (0,71); 4,3556 (0,77); 4,057 (0,64); 4,0393 (1,89); 4,0215 (1,92); 4,0037 (1,1); 3,9951 (0,73); 3,9605 (0,79); 3,7563 (0,81); 3,73 (0,85); 3,7125 (1,73); 3,6865 (1,55); 3,6391 (1,48); 3,6232 (1,47); 3,5953 (0,8); 3,5794 (0,77); 3,568 (0,42); 3,5321 (0,56); 3,5153 (0,9); 3,4981 (0,6); 3,4187 (0,96); 3,4091 (0,86); 3,3991 (1,21); 3,3896 (1,76); 3,3803 (1,44); 3,3611 (2,06); 3,314 (823,15); 3,2454 (0,73); 2,8749 (0,5); 2,8472 (0,87); 2,8171 (0,49); 2,6745 (0,6); 2,6699 (0,8); 2,6652 (0,6); 2,5398 (1,27); 2,5229 (3,35); 2,5097 (46,25); 2,5053 (86,72); 2,5008 (113,85); 2,4964 (78,37); 2,4919 (37,11); 2,3321 (0,56); 2,3275 (0,73); 2,3229 (0,53); 2,2128 (16); 2,1964 (0,97); 2,1397 (0,68); 2,1091 (1,46); 2,0787 (0,84); 2,0692 (0,91); 1,9869 (8,1); 1,9233 (0,5); 1,9081 (0,91); 1,8284 (0,69); 1,8192 (0,65); 1,8124 (0,68); 1,7953 (1,02); 1,778 (0,45); 1,6149 (0,37); 1,5794 (0,63); 1,5573 (0,57); 1,1929 (2,25); 1,1751 (4,55); 1,1573 (2,25); -0,0002 (3,63) |

| **Bsp. I-21, Solvent: DMSO-d₆** |
|---|
| 9,0409 (2,65); 8,068 (6,88); 7,6266 (1,65); 7,6225 (1,95); 7,6093 (2,53); 7,603 (2,87); 7,579 (0,33); 7,5469 (5,02); 7,5307 (2,23); 7,5234 (0,63); 7,5081 (0,34); 7,0898 (1,84); 7,0706 (2,38); 6,9585 (1,6); 6,9391 (1,22); 6,8671 (2,64); 5,4248 (0,45); 5,409 (0,5); 5,3982 (0,44); 5,392 (0,51); 5,3764 (0,5); 4,7973 (1,43); 4,7644 (1,47); 4,0571 (1,25); 4,0393 (3,72); 4,0215 (3,75); 4,0037 (1,27); 3,7079 (0,93); 3,6792 (1,04); 3,6634 (1,49); 3,6349 (1,28); 3,5677 (0,94); 3,4934 (1,45); 3,4772 (1,51); 3,4691 (0,47); 3,4577 (0,63); 3,4488 (1,4); 3,4398 (0,83); 3,4326 (1,66); 3,4203 (0,68); 3,4117 (0,51); 3,4017 (0,62); 3,3924 (0,41); 3,3629 (0,36); 3,3061 (168,87); 3,2656 (2,26); 3,2366 (1,16); 2,539 (0,7); 2,5087 (17,42); 2,5045 (31,21); 2,5001 (39,62); 2,4958 (27,56); 2,2544 (10,98); 2,1146 (12,12); 2,0847 (1,53); 1,9868 (16); 1,7601 (0,46); 1,7514 (0,52); 1,7229 (1,17); 1,6978 (1,11); 1,6695 (0,41); 1,6623 (0,34); 1,1927 (4,4); 1,1749 (8,74); 1,1571 (4,25); -0,0002 (3,25) |

| **Bsp. I-22, Solvent: DMSO-d₆** |
|---|
| 8,7773 (0,37); 8,1319 (5,01); 7,7175 (0,44); 7,7129 (0,45); 7,6971 (0,76); 7,6807 (0,46); 7,6761 (0,44); 7,3167 (1,41); 7,3086 (1,24); 7,2931 (1,73); 7,2702 (1,05); 7,1753 (2,18); 7,1598 (1,07); 7,0421 (1,09); 7,0238 (2,35); 7,016 (0,43); 6,9002 (2,39); 6,888 (1,27); 5,7465 (16); 5,4541 (0,46); 5,4113 (1,79); 5,369 (1,98); 5,3475 (0,33); 5,3276 (0,51); 5,217 (0,75); 4,3657 (0,53); 4,3325 (0,54); 3,9848 (0,52); 3,9508 (0,56); 3,8142 (0,61); 3,7855 (0,68); 3,7693 (0,96); 3,7409 (0,85); 3,7096 (0,41); 3,7059 (0,35); 3,6965 (0,53); 3,6819 (0,55); 3,6724 (0,35); 3,6687 (0,4); 3,5993 (1,01); 3,585 (1,01); 3,5546 (0,75); 3,5405 (0,72); 3,5234 (0,39); 3,5058 (0,65); 3,499 (0,51); 3,4882 (0,6); 3,4764 (0,6); 3,4646 (0,54); 3,4207 (0,59); 3,4107 (0,52); 3,4007 (0,7); 3,3912 (1,01); 3,3817 (0,78); 3,3707 (0,69); 3,3625 (0,93); 3,3524 (0,94); 3,3067 (225,38); 3,2838 (2,97); 3,2411 (0,43); 2,8706 (0,39); 2,8417 (0,65); 2,8127 (0,36); 2,6697 (0,35); 2,5396 (0,78); 2,5093 (20,82); 2,5051 (36,76); 2,5006 (46,42); 2,4963 (32,28); 2,1391 (0,51); 2,1049 (1); 2,0695 (0,77); 1,987 (0,7); 1,8132 (0,65); 1,7945 (0,65); 1,5889 (0,41); 1,5812 (0,44); 1,5587 (0,4); 1,5514 (0,4); 1,1752 (0,37); -0,0002 (1,67) |

| **Bsp. I-23**, **Solvent: DMSO-d₆** |
|---|
| 9,8983 (0,54); 8,7716 (0,39); 8,6417 (0,48); 8,0108 (16); 7,4632 (6,45); 7,4416 (15,56); 7,4349 (8,11); 7,3627 (5,42); 7,3562 (4,62); 7,3414 (3,68); 7,3349 (3,3); 7,3126 (4,9); 7,2933 (7,2); 7,2906 (5,75); 7,2802 (1,67); 7,2725 (5,97); 7,2665 (0,96); 6,9982 (0,36); 6,9672 (11,48); 6,9604 (3,73); 6,9507 (9,22); 6,9459 (7,58); 6,9436 (7,04); 6,9349 (2,35); 6,9325 (2,45); 5,7458 (12,3); 5,0801 (0,72); 5,0664 (1,17); 5,0616 (1,09); 5,0512 (1,5); 5,0477 (1,56); 5,039 (1,67); 5,0283 (1,1); 5,0204 (1,13); 5,0092 (0,71); 4,4347 (1,44); 4,404 (1,51); 4,1506 (0,53); 4,1396 (0,94); 4,1225 (8,17); 4,113 (6,4); 4,1077 (6,75); 4,0808 (1,4); 4,0667 (1,61); 4,0571 (1,83); 4,0392 (2,3); 4,0214 (2,19); 4,0036 (0,8); 3,9219 (0,79); 3,8822 (6,31); 3,8638 (6,24); 3,8235 (0,78); 3,6056 (2,28); 3,5782 (2,61); 3,5624 (3,42); 3,5351 (2,96); 3,4192 (1,4); 3,4101 (2,06); 3,4002 (1,97); 3,3912 (2,73); 3,3816 (3,98); 3,3719 (3,79); 3,3425 (12,82); 3,3146 (1762,05); 3,2816 (10,18); 3,253 (2,92); 2,8407 (1,12); 2,8121 (1,86); 2,7827 (1,08); 2,6954 (0,49); 2,6742 (1,25); 2,6698 (1,63); 2,6653 (1,27); 2,5398 (4,13); 2,5228 (8,11); 2,5095 (91,71); 2,5052 (168,54); 2,5007 (219,29); 2,4963 (156,07); 2,4919 (77,43); 2,4192 (0,46); 2,3367 (0,74); 2,3321 (1,25); 2,3274 (1,59); 2,3228 (1,17); 2,3184 (0,68); 2,1377 (1,43); 2,1016 (2,25); 2,069 (3); 1,9868 (9,07); 1,9082 (0,68); 1,8581 (0,43); 1,8537 (0,42); 1,841 (0,43); 1,8367 (0,43); 1,7746 (0,61); 1,7515 (1,25); 1,7454 (1,32); 1,7214 (1,19); 1,7143 (1,16); 1,6932 (0,52); 1,5872 (0,64); 1,5667 (1,21); 1,5578 (1,26); 1,5352 (1,17); 1,5285 (1,11); 1,5055 (0,51); 1,2367 (0,68); 1,2169 (0,49); 1,1992 (0,88); 1,1928 (2,64); 1,1812 (0,69); 1,175 (5,05); 1,1572 (2,5); -0,0002 (10,01) |

| **Bsp. I-24, Solvent: DMSO-d₆** |
|---|
| 8,6102 (3,25); 8,0095 (7,64); 7,9989 (1,98); 7,7027 (2,05); 7,6823 (2,42); 7,5339 (3,15); 7,5142 (0,77); 7,496 (1,33); 7,4811 (3,25); 7,4602 (7,87); 7,4419 (4,43); 7,4228 (2,15); 7,4137 (1,6); 7,409 (1,73); 7,4037 (1,01); 7,3925 (1,51); 7,3832 (0,46); 7,3797 (0,44); 7,3755 (0,49); 7,2203 (1,7); 7,2133 (1,32); 7,081 (3,6); 7,0756 (2,77); 6,9419 (1,9); 5,8563 (1,24); 5,8465 (1,2); 5,7384 (1,21); 5,7286 (1,24); 5,7055 (0,32); 5,1706 (0,42); 5,1604 (0,48); 5,1516 (0,53); 5,142 (0,82); 5,1331 (0,48); 5,1236 (0,52); 5,119 (0,57); 5,1139 (0,59); 5,1094 (0,51); 5,1001 (0,51); 5,0909 (0,7); 5,0817 (0,39); 5,0727 (0,38); 5,0628 (0,33); 4,1733 (1,97); 4,1396 (2,06); 4,0571 (1,25); 4,0392 (3,7); 4,0215 (3,73); 4,0037 (1,33); 3,5677 (1,55); 3,5013 (0,82); 3,4742 (0,92); 3,4578 (1,76); 3,4304 (1,67); 3,4003 (1,96); 3,3926 (0,89); 3,3812 (1,99); 3,3641 (1,12); 3,3566 (1,67); 3,3373 (2,18); 3,3043 (180,29); 3,1567 (0,35); 3,1369 (0,34); 3,0626 (1,32); 3,0331 (2,39); 3,0044 (1,35); 2,6735 (0,32); 2,669 (0,41); 2,539 (1,02); 2,5087 (23,16); 2,5044 (40,63); 2,5 (51,1); 2,4956 (35,01); 2,4913 (16,57); 2,3265 (0,35); 2,2176 (0,6); 2,0977 (1,71); 2,0693 (1,96); 1,9867 (16); 1,7129 (0,68); 1,6826 (1,57); 1,6584 (1,32); 1,6522 (1,37); 1,63 (0,51); 1,6219 (0,47); 1,398 (1,94); 1,1927 (4,49); 1,1748 (8,9); 1,1571 (4,31); -0,0002 (3,48) |

| **Bsp. I-25, Solvent: DMSO-d₆** |
|---|
| 8,0662 (7,79); 7,6237 (1,93); 7,6196 (2,28); 7,6064 (2,93); 7,6002 (3,29); 7,5832 (0,34); 7,5774 (0,39); 7,5726 (0,38); 7,5453 (5,81); 7,5289 (2,5); 7,5217 (0,67); 7,5067 (0,36); 6,4913 (4,18); 5,4346 (0,36); 5,4225 (0,55); 5,4072 (0,59); 5,3963 (0,53); 5,3897 (0,6); 5,3741 (0,59); 5,3617 (0,4); 5,3409 (0,8); 5,2978 (2,43); 5,2464 (2,37); 5,204 (0,73); 5,0993 (1,13); 4,3814 (0,75); 4,3479 (0,79); 4,0572 (1,28); 4,0394 (3,75); 4,0216 (3,8); 4,0038 (1,44); 3,9847 (0,76); 3,9511 (0,79); 3,7021 (1,08); 3,6733 (1,19); 3,6575 (1,7); 3,6288 (1,46); 3,5321 (0,41); 3,5153 (0,72); 3,4981 (0,54); 3,4863 (1,71); 3,4702 (1,69); 3,4417 (1,28); 3,4258 (1,31); 3,4051 (0,57); 3,3963 (0,83); 3,3864 (0,76); 3,3766 (1,05); 3,3674 (1,57); 3,3578 (1,28); 3,3067 (213,67); 3,2616 (1,1); 3,2317 (0,53); 2,8614 (0,53); 2,8336 (0,93); 2,8037 (0,53); 2,6693 (0,4); 2,5393 (0,99); 2,509 (22,17); 2,5048 (39,42); 2,5004 (49,88); 2,496 (34,5); 2,4919 (16,6); 2,3271 (0,34); 2,2156 (3,78); 2,2092 (15,56); 2,1951 (0,94); 2,1245 (0,72); 2,0963 (1,43); 2,0633 (0,79); 1,9869 (16); 1,923 (0,42); 1,9073 (0,36); 1,8292 (0,35); 1,8239 (0,33); 1,8118 (0,87); 1,8025 (0,67); 1,7949 (0,73); 1,7778 (0,77); 1,572 (0,57); 1,5629 (0,6); 1,5414 (0,56); 1,5329 (0,53); 1,1928 (4,44); 1,175 (8,67); 1,1572 (4,28); -0,0002 (2,03) |

| **Bsp. I-26, Solvent: DMSO-d₆** |
|---|
| 8,0125 (5,61); 7,3094 (0,99); 7,176 (2,23); 7,1598 (1,09); 7,0428 (1,12); 7,0237 (2,5); 7,0093 (1,23); 7,0051 (1,29); 6,99 (1,7); 6,9855 (1,97); 6,9788 (1,09); 6,9635 (1,87); 6,959 (1,96); 6,9439 (0,88); 6,9397 (0,96); 6,9256 (1,67); 6,9214 (1,38); 6,9013 (2,8); 6,893 (1,89); 6,888 (2,42); 6,8739 (1,32); 6,8693 (1,24); 6,8552 (0,48); 6,8507 (0,43); 5,4544 (0,48); 5,4128 (1,71); 5,3692 (1,68); 5,3267 (0,47); 5,0558 (0,44); 5,0516 (0,42); 5,042 (0,6); 5,0282 (0,63); 5,0171 (0,41); 5,0131 (0,39); 4,3647 (0,57); 4,3324 (0,58); 4,1084 (0,44); 4,0921 (2); 4,0859 (2,29); 4,0818 (2,07); 4,0719 (1,71); 4,0594 (0,35); 4,0448 (0,36); 3,9864 (0,54); 3,9506 (0,58); 3,7196 (16); 3,5963 (0,85); 3,5689 (0,98); 3,5533 (1,3); 3,5262 (1,14); 3,4195 (0,69); 3,4094 (0,63); 3,3994 (0,85); 3,3905 (1,25); 3,381 (1,02); 3,3624 (2,32); 3,3438 (2,62); 3,3052 (335,58); 3,2474 (0,63); 2,8903 (1,3); 2,8762 (0,39); 2,8494 (0,67); 2,8183 (0,4); 2,7315 (1); 2,6733 (0,46); 2,6689 (0,59); 2,6651 (0,44); 2,5391 (1,5); 2,5087 (34,08); 2,5045 (60,85); 2,5001 (77,38); 2,4958 (54,01); 2,3311 (0,36); 2,3267 (0,49); 2,3222 (0,36); 2,142 (0,49); 2,108 (1); 2,0692 (8,08); 1,825 (0,41); 1,817 (0,44); 1,794 (0,41); 1,5918 (0,41); 1,5842 (0,45); 1,5618 (0,4); 1,5525 (0,38); 0,008 (0,41); -0,0002 (7,11) |

| **Bsp. I-27**, **Solvent: DMSO-d₆** |
|---|
| 9,0457 (2,78); 8,1319 (10,41); 7,7342 (0,33); 7,7182 (0,76); 7,7134 (0,79); 7,6975 (1,39); 7,6818 (0,82); 7,6769 (0,81); 7,6613 (0,36); 7,3176 (2,39); 7,2935 (3,05); 7,2708 (1,93); 7,0908 (2,52); 7,0715 (3,26); 6,9585 (2,13); 6,9391 (1,61); 6,8706 (3,52); 5,7455 (9,76); 5,4138 (0,34); 5,402 (0,42); 5,3871 (0,64); 5,377 (0,54); 5,3626 (0,67); 5,3489 (0,44); 5,3364 (0,34); 4,8049 (1,84); 4,7718 (1,92); 4,0573 (0,96); 4,0395 (2,83); 4,0217 (2,87); 4,0039 (0,96); 3,8192 (1,12); 3,7907 (1,24); 3,7745 (1,8); 3,7461 (1,56); 3,6962 (0,4); 3,6819 (0,38); 3,6048 (1,83); 3,5904 (1,87); 3,5681 (0,75); 3,5602 (1,35); 3,5459 (1,32); 3,4988 (0,42); 3,4869 (0,63); 3,4765 (1,04); 3,4649 (0,82); 3,4584 (0,97); 3,4484 (1,47); 3,4386 (0,95); 3,4296 (0,72); 3,4201 (0,91); 3,4103 (0,63); 3,3148 (596,34); 3,2916 (5,41); 3,2731 (3,05); 3,2442 (1,54); 2,6745 (0,4); 2,6699 (0,54); 2,6653 (0,4); 2,5399 (0,73); 2,5231 (2,23); 2,5098 (30,74); 2,5054 (57,48); 2,5009 (75,17); 2,4965 (51,99); 2,4921 (24,84); 2,3322 (0,4); 2,3277 (0,51); 2,3228 (0,39); 2,255 (14,88); 2,1178 (16); 2,0995 (2,1); 2,069 (0,45); 1,987 (12,44); 1,7667 (0,6); 1,7381 (1,52); 1,7093 (1,41); 1,6812 (0,48); 1,193 (3,41); 1,1752 (6,83); 1,1574 (3,33); -0,0002 (3,29) |

| **Bsp. I-28, Solvent: DMSO-d₆** |
|---|
| 8,1299 (8,46); 7,7169 (0,67); 7,7123 (0,65); 7,6962 (1,2); 7,6807 (0,68); 7,6749 (0,68); 7,3164 (2,02); 7,2921 (2,57); 7,2699 (1,6); 6,4922 (4,16); 6,4424 (0,33); 5,7461 (12,32); 5,3978 (0,37); 5,3848 (0,56); 5,372 (0,49); 5,3592 (0,64); 5,344 (1,06); 5,3187 (0,38); 5,3011 (2,41); 5,2486 (2,39); 5,2066 (0,73); 5,0986 (0,47); 4,3855 (0,72); 4,3541 (0,75); 4,0572 (0,61); 4,0394 (1,79); 4,0216 (1,84); 4,0038 (0,97); 3,9911 (0,71); 3,9597 (0,79); 3,8135 (0,98); 3,7847 (1,06); 3,7685 (1,54); 3,7399 (1,33); 3,5977 (1,62); 3,5833 (1,6); 3,5532 (1,2); 3,5389 (1,19); 3,5161 (0,47); 3,4986 (0,36); 3,4146 (1,08); 3,4044 (1,03); 3,3951 (1,39); 3,3856 (1,98); 3,3761 (1,77); 3,3116 (294,6); 3,2397 (0,95); 2,8699 (0,49); 2,8409 (0,88); 2,81 (0,49); 2,6745 (0,52); 2,6697 (0,69); 2,6653 (0,52); 2,5397 (0,99); 2,5229 (3,04); 2,5096 (41,56); 2,5052 (77,43); 2,5008 (101,04); 2,4964 (69,82); 2,492 (33,24); 2,3319 (0,47); 2,3274 (0,64); 2,3232 (0,46); 2,2214 (2,68); 2,2113 (16); 2,1959 (0,62); 2,138 (0,67); 2,109 (1,39); 2,0693 (1,01); 1,9869 (7,6); 1,8259 (0,59); 1,8174 (0,61); 1,7943 (0,71); 1,586 (0,56); 1,5772 (0,57); 1,555 (0,55); 1,5492 (0,52); 1,193 (2,09); 1,1751 (4,15); 1,1574 (2,02); -0,0002 (4,74) |

| **Bsp. I-29**, **Solvent: DMSO-d₆** |
|---|
| 9,89 (0,4); 8,7611 (0,88); 8,631 (0,39); 8,0008 (8,78); 7,3113 (2,73); 7,3029 (0,72); 7,2919 (4,02); 7,2798 (0,83); 7,2711 (3,33); 7,2649 (0,53); 7,037 (2,39); 7,018 (3,4); 6,9651 (5,82); 6,9582 (2,13); 6,9495 (4,7); 6,9434 (4,98); 6,9414 (5,57); 6,9344 (3,21); 6,9174 (1,72); 6,8928 (3,54); 5,7468 (4,93); 5,0757 (0,38); 5,0621 (0,68); 5,057 (0,61); 5,0436 (0,86); 5,0347 (0,93); 5,0239 (0,59); 5,0165 (0,64); 5,005 (0,42); 4,4937 (0,81); 4,4611 (0,87); 4,1352 (0,49); 4,1186 (4,85); 4,1084 (3,35); 4,1042 (3,24); 4,0771 (0,35); 4,0567 (0,44); 4,039 (1,14); 4,0212 (1,17); 4,0033 (0,6); 3,9842 (0,85); 3,9508 (0,89); 3,7125 (0,46); 3,6733 (3,39); 3,6545 (3,43); 3,6136 (0,52); 3,5973 (1,34); 3,5699 (1,48); 3,5542 (1,9); 3,5268 (1,65); 3,3739 (1,53); 3,3641 (1,63); 3,354 (2,34); 3,3445 (3,55); 3,3354 (5,77); 3,3045 (806,31); 3,2739 (3,71); 3,2317 (0,97); 3,2008 (1,18); 3,1713 (0,67); 2,8194 (0,63); 2,789 (1,05); 2,76 (0,61); 2,6735 (0,9); 2,6689 (1,2); 2,6645 (0,91); 2,5389 (2,08); 2,5219 (6,03); 2,5086 (66,99); 2,5043 (123,34); 2,4998 (160,5); 2,4954 (113,45); 2,4911 (55,69); 2,3312 (0,87); 2,3266 (1,13); 2,3221 (0,83); 2,2293 (16); 2,1477 (15,22); 2,0853 (0,91); 2,0691 (3,21); 2,0498 (1,58); 2,0156 (0,93); 1,9866 (4,77); 1,9077 (0,34); 1,6213 (0,38); 1,5903 (0,97); 1,5592 (1,26); 1,5277 (0,87); 1,236 (0,55); 1,1926 (1,34); 1,1748 (2,61); 1,157 (1,29); 0,0079 (1,09); -0,0002 (20,51); -0,0084 (0,88) |

| **Bsp. I-30**, **Solvent: DMSO-d₆** |
|---|
| 8,7761 (1,28); 8,6165 (5,41); 8,6062 (2,47); 8,0776 (12,69); 8,0349 (4,7); 7,7031 (3,54); 7,6828 (4,18); 7,615 (0,65); 7,5967 (1,54); 7,5792 (1,95); 7,5586 (1,36); 7,5336 (4,83); 7,4848 (3,66); 7,4648 (3,06); 7,2586 (1,12); 7,2367 (4,98); 7,2156 (8,65); 7,1935 (2,68); 7,0795 (7,18); 7,068 (1,8); 6,9406 (4,08); 6,9308 (0,91); 5,9057 (1,8); 5,8866 (2,4); 5,8658 (0,76); 5,7936 (1,83); 5,775 (2,05); 5,746 (8,02); 5,4744 (0,34); 5,355 (0,53); 5,3237 (0,32); 5,2667 (0,39); 5,2488 (0,74); 5,2412 (0,83); 5,2233 (1,31); 5,2064 (0,87); 5,1996 (0,74); 5,181 (0,35); 4,1804 (2,81); 4,1464 (3,02); 4,0571 (1,26); 4,0393 (3,71); 4,0215 (3,73); 4,0038 (1,32); 3,76 (1,23); 3,7337 (1,36); 3,7162 (2,7); 3,6901 (2,34); 3,6721 (0,4); 3,6628 (0,35); 3,6435 (2,25); 3,6273 (2,28); 3,6 (1,3); 3,5842 (1,26); 3,5775 (0,89); 3,5678 (0,48); 3,5492 (0,85); 3,532 (0,94); 3,5046 (0,95); 3,4739 (0,6); 3,4644 (0,64); 3,4515 (0,6); 3,4419 (0,62); 3,3746 (2,64); 3,3145 (1557,26); 3,2614 (0,79); 3,2262 (0,33); 3,0795 (1,53); 3,074 (1,79); 3,0448 (3,38); 3,0262 (2,35); 3,0169 (2,27); 3,0103 (2,14); 2,9974 (0,99); 2,984 (0,74); 2,9656 (0,53); 2,6744 (1,13); 2,6699 (1,49); 2,6654 (1,13); 2,6608 (0,6); 2,5399 (2,5); 2,523 (6,43); 2,5097 (85,96); 2,5054 (160,56); 2,5009 (209,76); 2,4965 (144,61); 2,4921 (68,47); 2,3321 (1,03); 2,3275 (1,38); 2,323 (0,99); 2,3182 (0,49); 2,2225 (0,58); 2,1129 (2,49); 2,0874 (3,37); 2,0692 (5,14); 1,9869 (16); 1,9083 (1,73); 1,7353 (0,8); 1,7263 (0,95); 1,6962 (2,47); 1,6747 (2,76); 1,6481 (1,56); 1,6227 (0,67); 1,2364 (1,18); 1,1929 (4,5); 1,1751 (8,89); 1,1573 (4,41); 1,1205 (0,32); -0,0002 (4,43) |

| **Bsp. I-31**, **Solvent: DMSO-d₆** |
|---|
| 8,0778 (16); 7,6241 (3,81); 7,6201 (4,53); 7,6069 (5,93); 7,6007 (6,72); 7,5843 (0,66); 7,5772 (0,8); 7,5456 (11,72); 7,5293 (5,17); 7,5068 (0,79); 7,3345 (2,68); 7,2014 (5,85); 7,1753 (0,61); 7,1598 (3,01); 7,1504 (0,42); 7,0683 (2,92); 7,0418 (0,44); 7,0238 (7,03); 7,0145 (0,8); 6,8983 (6,52); 6,888 (4,21); 5,6026 (0,52); 5,5586 (10,22); 5,5132 (0,53); 5,4542 (0,54); 5,438 (0,73); 5,4261 (1,12); 5,4095 (2,18); 5,3932 (1,22); 5,3774 (1,21); 5,3653 (0,74); 5,3486 (0,63); 5,3127 (1,67); 5,2792 (1,69); 4,4646 (1,56); 4,4301 (1,65); 4,0573 (1,09); 4,0395 (3,27); 4,0217 (3,28); 4,0039 (1,13); 3,801 (0,58); 3,7864 (0,65); 3,7741 (0,35); 3,7063 (2,11); 3,6777 (2,29); 3,6618 (3,19); 3,6332 (2,78); 3,586 (1,44); 3,5766 (1); 3,5562 (3,05); 3,5382 (2,57); 3,5278 (2,6); 3,5098 (1,45); 3,4989 (1,08); 3,4905 (3,44); 3,4744 (3,39); 3,446 (2,53); 3,43 (2,51); 3,3879 (1,85); 3,3566 (3,74); 3,3103 (148,24); 2,6741 (0,48); 2,6695 (0,61); 2,6652 (0,45); 2,5395 (1,36); 2,5225 (3,18); 2,5092 (37,41); 2,5049 (67,29); 2,5005 (85,79); 2,4961 (59,65); 2,4919 (29,05); 2,4509 (0,4); 2,3319 (0,52); 2,3272 (0,62); 2,3229 (0,47); 2,2028 (2,66); 2,1718 (3,05); 2,0692 (0,48); 2,0606 (0,33); 2,0436 (0,5); 2,0256 (0,42); 1,9869 (14,24); 1,9276 (0,95); 1,9052 (1,43); 1,8959 (1,67); 1,8725 (1,31); 1,8429 (0,55); 1,8338 (0,46); 1,7726 (0,57); 1,7624 (0,7); 1,7389 (1,31); 1,7331 (1,4); 1,7094 (1,33); 1,7025 (1,22); 1,6799 (0,57); 1,6703 (0,46); 1,3981 (8,63); 1,2368 (0,33); 1,1928 (3,93); 1,175 (7,64); 1,1572 (3,83); -0,0002 (3,87) |

| **Bsp. I-32**, **Solvent: DMSO-d₆** |
|---|
| 9,0484 (3,42); 9,0375 (1,56); 8,7766 (0,88); 8,0787 (8,46); 8,0346 (3,42); 7,6174 (0,45); 7,5977 (0,95); 7,5798 (1,25); 7,5593 (0,81); 7,261 (0,85); 7,2383 (3,13); 7,2163 (3,98); 7,1947 (1,66); 7,0919 (2,56); 7,0724 (3,39); 6,9596 (2,64); 6,9402 (1,97); 6,8666 (3,23); 5,9067 (1,14); 5,8877 (1,5); 5,8665 (0,55); 5,7947 (1,14); 5,7761 (1,28); 5,7452 (6,11); 5,3569 (0,36); 5,2422 (0,55); 5,225 (0,83); 5,2078 (0,54); 5,2 (0,47); 4,8049 (1,75); 4,7722 (1,85); 4,0572 (0,79); 4,0394 (2,4); 4,0216 (2,42); 4,0038 (0,82); 3,7623 (0,72); 3,7364 (0,78); 3,7191 (1,63); 3,6929 (1,45); 3,6455 (1,35); 3,6297 (1,36); 3,6021 (0,72); 3,5863 (0,73); 3,5797 (0,54); 3,568 (0,96); 3,5517 (0,48); 3,5353 (0,56); 3,5078 (0,58); 3,49 (0,43); 3,4807 (0,7); 3,4621 (0,88); 3,4525 (1,33); 3,4427 (0,98); 3,4359 (0,83); 3,4254 (1,08); 3,3989 (0,68); 3,3208 (964,66); 3,2857 (3,65); 3,2811 (3,68); 3,2514 (1,96); 3,2309 (0,93); 3,0297 (0,43); 3,0107 (0,43); 2,9852 (0,39); 2,9678 (0,36); 2,6749 (0,48); 2,6702 (0,65); 2,6656 (0,5); 2,5402 (0,9); 2,5233 (2,56); 2,51 (38,44); 2,5056 (72,22); 2,5011 (94,66); 2,4967 (65,78); 2,4923 (31,57); 2,3324 (0,56); 2,3278 (0,72); 2,3232 (0,52); 2,2552 (16); 2,1201 (13,74); 2,107 (7,19); 2,0688 (1,3); 1,9869 (10,72); 1,9085 (0,33); 1,77 (0,62); 1,7404 (1,52); 1,7156 (1,73); 1,6952 (0,98); 1,689 (1,05); 1,6575 (0,51); 1,2363 (0,69); 1,1929 (2,99); 1,1751 (6,03); 1,1573 (3,02); -0,0002 (1,42) |

| **Bsp. I-33**, **Solvent: DMSO-d₆** |
|---|
| 8,7735 (0,39); 8,1276 (3,43); 8,0111 (8,23); 7,3352 (3,24); 7,3296 (3,4); 7,3183 (0,52); 7,3113 (2,55); 7,292 (3,58); 7,289 (3,01); 7,2781 (0,94); 7,2712 (3,09); 7,2648 (0,46); 7,1991 (2,26); 7,1785 (3,06); 7,0836 (2,35); 7,078 (2,21); 7,0633 (1,69); 7,0576 (1,6); 6,9668 (7,11); 6,9496 (5,26); 6,9459 (4,34); 6,9334 (1,11); 6,931 (1,21); 5,7474 (13,56); 5,0789 (0,35); 5,0655 (0,63); 5,0603 (0,58); 5,0497 (0,79); 5,0467 (0,78); 5,0375 (0,86); 5,0271 (0,58); 5,0192 (0,57); 5,0079 (0,37); 4,1734 (1,84); 4,1397 (2,32); 4,1227 (4,54); 4,1126 (3,12); 4,1077 (2,93); 4,095 (0,34); 4,0808 (0,33); 4,0568 (0,43); 4,039 (1,24); 4,0212 (1,24); 4,0034 (0,45); 3,6083 (1,19); 3,5809 (1,34); 3,5651 (1,75); 3,5378 (1,49); 3,3592 (0,95); 3,3456 (2,38); 3,3268 (3,27); 3,2985 (242,68); 3,284 (3,41); 3,2753 (1,76); 3,0429 (1,24); 3,0129 (2,26); 2,9846 (1,25); 2,6734 (0,51); 2,6687 (0,64); 2,6641 (0,5); 2,5386 (1,32); 2,5083 (39,8); 2,504 (71,66); 2,4996 (91,54); 2,4952 (63,53); 2,4908 (30,43); 2,3309 (0,51); 2,3263 (0,63); 2,322 (0,43); 2,1572 (16); 2,0976 (1,58); 2,0695 (2,22); 1,9867 (5,34); 1,7228 (0,54); 1,7125 (0,68); 1,6898 (1,33); 1,6824 (1,51); 1,66 (1,37); 1,6525 (1,31); 1,6306 (0,55); 1,6212 (0,45); 1,1926 (1,48); 1,1748 (2,92); 1,157 (1,42); 0,0077 (0,62); -0,0002 (11,11); -0,0081 (0,46) |

| **Bsp. I-34**, **Solvent: DMSO-d₆** |
|---|
| 8,0282 (16); 7,3359 (2,71); 7,3123 (4,83); 7,2929 (7,15); 7,2902 (5,8); 7,28 (1,6); 7,2721 (5,94); 7,2659 (1); 7,2028 (5,7); 7,1604 (2,96); 7,0698 (2,88); 7,0244 (6,79); 6,9974 (0,44); 6,9666 (11,24); 6,9598 (3,78); 6,9503 (8,94); 6,9453 (7,58); 6,9431 (7,11); 6,9346 (2,41); 6,9321 (2,53); 6,8987 (6,25); 6,8886 (3,79); 5,7468 (14,78); 5,6071 (0,51); 5,5628 (9,63); 5,5169 (0,59); 5,3162 (1,61); 5,2841 (1,66); 5,0825 (0,69); 5,0688 (1,18); 5,0635 (1,07); 5,0534 (1,5); 5,0501 (1,55); 5,0412 (1,68); 5,0304 (1,12); 5,0229 (1,14); 5,0116 (0,72); 4,4711 (1,47); 4,4362 (1,59); 4,1511 (0,46); 4,1401 (0,85); 4,1229 (7,68); 4,1135 (5,68); 4,1081 (5,36); 4,0956 (0,64); 4,0811 (0,62); 4,0568 (0,74); 4,039 (2,13); 4,0212 (2,16); 4,0034 (0,75); 3,6097 (2,66); 3,5988 (1,6); 3,5826 (3,82); 3,5669 (6,18); 3,5554 (2,78); 3,5396 (4,61); 3,5273 (1,62); 3,5173 (0,81); 3,4023 (1,59); 3,3713 (3); 3,3478 (6); 3,3287 (9,44); 3,3051 (1062,1); 3,2417 (0,99); 3,2042 (0,46); 3,1807 (0,36); 2,6778 (0,62); 2,6737 (1,14); 2,669 (1,51); 2,6645 (1,09); 2,5391 (2,45); 2,5221 (7,27); 2,5088 (84,55); 2,5045 (157,29); 2,5 (206,45); 2,4956 (148,03); 2,4912 (74,31); 2,3313 (1,15); 2,3267 (1,5); 2,3221 (1,13); 2,3178 (0,62); 2,2173 (2,66); 2,1859 (3,08); 2,0693 (0,85); 1,9867 (9,37); 1,9501 (0,56); 1,94 (0,69); 1,9079 (1,47); 1,886 (1,28); 1,8579 (0,54); 1,8484 (0,45); 1,7914 (0,56); 1,7828 (0,72); 1,753 (1,39); 1,7298 (1,35); 1,7214 (1,22); 1,7004 (0,56); 1,6918 (0,46); 1,3983 (2,52); 1,2364 (0,54); 1,1927 (2,61); 1,1749 (5,19); 1,1571 (2,56); 0,0079 (1,28); -0,0002 (24,6); -0,0084 (1,16) |

| **Bsp. I-35**, **Solvent: DMSO-d₆** |
|---|
| 8,6138 (5,08); 8,1304 (14,08); 7,733 (0,59); 7,712 (1,36); 7,7031 (3,62); 7,6826 (4,23); 7,6596 (0,51); 7,5345 (4,3); 7,4849 (2,66); 7,4651 (2,2); 7,3163 (3,2); 7,292 (4); 7,2697 (2,53); 7,2194 (2,41); 7,2143 (1,94); 7,0801 (5,54); 7,0767 (4,58); 6,9407 (3,11); 5,7462 (12,57); 5,4264 (0,36); 5,4121 (0,44); 5,3991 (0,54); 5,3852 (0,81); 5,3741 (0,69); 5,3619 (0,88); 5,3477 (0,58); 5,3348 (0,48); 5,3202 (0,37); 4,1786 (2,61); 4,1448 (2,72); 4,0573 (1,24); 4,0395 (3,63); 4,0218 (3,65); 4,004 (1,28); 3,8168 (1,44); 3,7879 (1,63); 3,7719 (2,4); 3,7435 (2,03); 3,6983 (0,33); 3,6818 (0,34); 3,68 (0,32); 3,6036 (2,44); 3,5892 (2,42); 3,5589 (1,76); 3,5447 (1,71); 3,4986 (0,32); 3,4871 (0,33); 3,4757 (0,44); 3,4649 (0,42); 3,4246 (0,37); 3,41 (0,47); 3,3828 (1,11); 3,373 (1,68); 3,363 (1,74); 3,3537 (2,48); 3,3443 (3,99); 3,3112 (792,76); 3,2878 (6,77); 3,069 (1,75); 3,0395 (3,22); 3,0107 (1,79); 2,6747 (0,58); 2,6698 (0,79); 2,6653 (0,61); 2,5399 (1,12); 2,5231 (3,4); 2,5097 (46,79); 2,5054 (87,29); 2,5009 (114,05); 2,4964 (78,33); 2,492 (37,06); 2,3321 (0,54); 2,3276 (0,75); 2,323 (0,54); 2,1176 (2,11); 2,1113 (2,21); 2,0853 (2,5); 2,0695 (1,41); 1,987 (16); 1,7247 (0,81); 1,6959 (1,94); 1,672 (1,78); 1,6433 (0,65); 1,2364 (0,33); 1,193 (4,38); 1,1752 (8,84); 1,1574 (4,29); -0,0002 (6,48) |

| **Bsp. I-36**, **Solvent: DMSO-d₆** |
|---|
| 8,0211 (14,29); 7,6895 (14,84); 7,6694 (16); 7,3078 (2,43); 7,1745 (5,6); 7,159 (2,78); 7,0833 (4,26); 7,0632 (7,34); 7,0429 (5,5); 7,023 (6,31); 6,8999 (5,83); 6,8872 (3,33); 5,7459 (3,42); 5,4534 (1,14); 5,4112 (4,22); 5,3671 (4,27); 5,3238 (1,2); 5,1462 (0,63); 5,1345 (1,07); 5,1272 (1,1); 5,1169 (1,71); 5,1081 (1,49); 5,0895 (1,2); 5,0766 (0,69); 4,3628 (1,36); 4,3301 (1,43); 4,1512 (0,86); 4,138 (1); 4,1256 (4,85); 4,1177 (5,51); 4,1129 (5,75); 4,1073 (5,12); 4,0924 (1,09); 4,0816 (0,73); 4,057 (1,23); 4,0392 (3,43); 4,0214 (3,48); 4,0036 (1,51); 3,9852 (1,37); 3,9537 (1,5); 3,6571 (1,44); 3,6296 (1,58); 3,6137 (3,66); 3,5866 (3,2); 3,5622 (3,44); 3,5431 (3,52); 3,5188 (1,71); 3,5 (1,59); 3,4646 (0,66); 3,418 (2,01); 3,409 (1,93); 3,3892 (3,81); 3,3792 (3,55); 3,3147 (1485,38); 3,2912 (22,56); 3,2443 (2,32); 3,1601 (0,45); 3,1453 (0,42); 2,8755 (1,09); 2,848 (1,81); 2,8175 (1,05); 2,6743 (1,35); 2,6699 (1,71); 2,6653 (1,31); 2,5399 (3,31); 2,5096 (94,11); 2,5053 (168,4); 2,5009 (215,71); 2,4965 (152,9); 2,4923 (75,9); 2,3322 (1,07); 2,3277 (1,4); 2,323 (1,04); 2,139 (1,31); 2,1054 (2,61); 2,0692 (3,56); 1,9869 (14,45); 1,8389 (0,59); 1,812 (1,17); 1,7877 (1,11); 1,7596 (0,47); 1,613 (0,6); 1,5822 (1,16); 1,5606 (1,06); 1,5311 (0,44); 1,3979 (0,48); 1,236 (0,46); 1,1929 (4,05); 1,1751 (7,85); 1,1573 (3,89); -0,0002 (13,93) |

| **Bsp. I-37**, **Solvent: DMSO-d₆** |
|---|
| 8,0162 (8,27); 7,312 (2,54); 7,2927 (3,86); 7,29 (3,1); 7,2796 (0,88); 7,2719 (3,15); 7,2658 (0,52); 6,9667 (6,29); 6,96 (1,97); 6,9501 (4,98); 6,9455 (4,13); 6,9434 (3,76); 6,9342 (1,24); 6,9319 (1,3); 6,4927 (4,15); 5,3443 (0,77); 5,3024 (2,57); 5,2514 (2,5); 5,2088 (0,76); 5,0797 (0,37); 5,066 (0,64); 5,0611 (0,6); 5,0506 (0,81); 5,0475 (0,82); 5,0387 (0,89); 5,0276 (0,59); 5,0203 (0,62); 5,0091 (0,4); 4,3876 (0,76); 4,3545 (0,83); 4,139 (0,45); 4,1223 (4,36); 4,1125 (3,11); 4,1075 (2,97); 4,0805 (0,35); 4,0569 (0,65); 4,0391 (1,88); 4,0213 (1,93); 4,0035 (1,04); 3,9932 (0,76); 3,9591 (0,8); 3,607 (1,2); 3,5796 (1,36); 3,5638 (1,81); 3,5365 (1,54); 3,4224 (0,48); 3,4135 (0,76); 3,4039 (0,68); 3,3941 (1); 3,3848 (1,56); 3,3752 (1,23); 3,3556 (1,66); 3,3445 (3,4); 3,3055 (567,39); 3,283 (5,2); 3,2447 (1,14); 2,8751 (0,59); 2,8459 (0,99); 2,8177 (0,57); 2,6738 (0,65); 2,6691 (0,82); 2,6647 (0,63); 2,5652 (0,48); 2,5391 (1,68); 2,5088 (48,27); 2,5045 (89); 2,5001 (115,88); 2,4957 (82,88); 2,4914 (41,54); 2,3314 (0,65); 2,3267 (0,84); 2,3222 (0,64); 2,2111 (16); 2,1409 (0,78); 2,1095 (1,55); 2,0692 (3,01); 1,9867 (8,01); 1,8189 (0,67); 1,7966 (0,63); 1,7898 (0,58); 1,5912 (0,62); 1,5815 (0,68); 1,5595 (0,63); 1,5521 (0,61); 1,1927 (2,22); 1,1749 (4,3); 1,1571 (2,15); -0,0002 (5,27) |

| **Bsp. I-38**, **Solvent: DMSO-d₆** |
|---|
| 8,0073 (5,72); 7,6232 (2,48); 7,4841 (2,04); 7,4794 (2,02); 7,3099 (1,76); 7,2906 (2,58); 7,2876 (2,17); 7,2768 (0,67); 7,2697 (2,19); 6,9653 (4,89); 6,9481 (3,7); 6,9444 (3,07); 6,9318 (0,79); 6,9296 (0,87); 6,8769 (1,74); 6,8562 (3,1); 6,8102 (1,43); 6,8065 (1,39); 6,7897 (0,81); 6,7859 (0,78); 5,7473 (9,49); 5,0623 (0,45); 5,0575 (0,41); 5,0483 (0,56); 5,0347 (0,62); 5,0234 (0,4); 5,0165 (0,42); 4,1316 (1,51); 4,12 (3,7); 4,1095 (2,91); 4,1048 (3,07); 4,0787 (0,33); 4,039 (0,5); 4,0212 (0,48); 3,7702 (16); 3,6067 (0,83); 3,5793 (0,96); 3,5635 (1,25); 3,5362 (1,07); 3,3439 (1,76); 3,325 (2,53); 3,2992 (241,38); 3,2828 (3,48); 3,026 (0,93); 2,9969 (1,65); 2,9687 (0,94); 2,6732 (0,42); 2,6686 (0,58); 2,6641 (0,41); 2,5387 (1,03); 2,5217 (2,92); 2,5083 (34,75); 2,504 (63,51); 2,4996 (82,11); 2,4952 (58,08); 2,4909 (28,57); 2,331 (0,49); 2,3264 (0,6); 2,322 (0,43); 2,2104 (11,48); 2,0865 (1,13); 2,0693 (1,15); 2,0593 (1,3); 1,9866 (2,15); 1,7156 (0,41); 1,7058 (0,49); 1,6851 (0,98); 1,6758 (1,1); 1,6541 (1); 1,6457 (0,93); 1,6234 (0,4); 1,6139 (0,34); 1,1926 (0,6); 1,1748 (1,18); 1,1569 (0,59); 0,0079 (0,53); -0,0002 (9,86); -0,0085 (0,44) |

| **Bsp. I-39**, **Solvent: DMSO-d₆** |
|---|
| 9,0412 (3,68); 8,0083 (8,33); 7,9969 (2,15); 7,514 (0,56); 7,4966 (1,13); 7,4813 (1,65); 7,461 (7,35); 7,4436 (4,79); 7,4243 (2,32); 7,4155 (1,71); 7,4109 (1,91); 7,3941 (1,64); 7,385 (0,54); 7,3816 (0,51); 7,3774 (0,53); 7,0899 (2,67); 7,0706 (3,43); 6,9581 (2,44); 6,9394 (1,84); 6,8638 (3,7); 5,8557 (1,31); 5,8458 (1,31); 5,7377 (1,28); 5,7279 (1,34); 5,7034 (0,33); 5,6887 (0,34); 5,5705 (0,35); 5,1724 (0,43); 5,1621 (0,46); 5,1534 (0,54); 5,1438 (0,83); 5,1346 (0,51); 5,1209 (0,63); 5,1153 (0,61); 5,111 (0,54); 5,102 (0,51); 5,0928 (0,75); 5,083 (0,44); 5,0741 (0,41); 5,0639 (0,36); 4,7968 (1,98); 4,7634 (2,08); 4,057 (0,46); 4,0391 (1,3); 4,0213 (1,3); 4,0033 (0,46); 3,7093 (0,34); 3,6957 (0,45); 3,6814 (0,43); 3,5678 (1,83); 3,5041 (0,99); 3,4873 (0,52); 3,4759 (1,43); 3,4602 (2,63); 3,4478 (1,15); 3,4319 (2,9); 3,4188 (1,59); 3,4113 (1,74); 3,3994 (3,61); 3,3804 (3,8); 3,3255 (1192,66); 3,2678 (4,37); 3,2383 (2,29); 3,1799 (0,57); 3,158 (0,73); 3,1386 (0,68); 3,1228 (0,38); 3,115 (0,53); 3,0958 (0,5); 2,6955 (0,54); 2,6744 (0,66); 2,6701 (0,87); 2,6658 (0,68); 2,5402 (2,51); 2,5098 (48,63); 2,5055 (88,38); 2,5011 (113,49); 2,4968 (79,56); 2,4926 (39,42); 2,3324 (0,72); 2,328 (0,9); 2,3233 (0,7); 2,2891 (0,44); 2,254 (16); 2,1141 (15,33); 2,0784 (2,2); 2,0685 (2,11); 2,0493 (0,36); 1,9867 (5,81); 1,7528 (0,85); 1,7248 (1,79); 1,6965 (1,6); 1,6689 (0,61); 1,2363 (0,37); 1,1928 (1,62); 1,175 (3,2); 1,1687 (0,35); 1,1571 (1,61); -0,0002 (2,74) |

| **Bsp. I-40, Solvent: DMSO-d₆** |
|---|
| 7,9577 (6,52); 7,6189 (0,43); 7,6129 (2,11); 7,6085 (2,73); 7,6024 (1,49); 7,5957 (2,16); 7,5894 (2,7); 7,4269 (0,83); 7,4161 (4,09); 7,408 (1,93); 7,3985 (2,27); 7,3852 (0,64); 7,304 (1,14); 7,1707 (2,48); 7,1581 (1,2); 7,0375 (1,19); 7,022 (2,76); 6,8981 (2,35); 6,886 (1,4); 5,7464 (3,91); 5,4477 (0,55); 5,4058 (1,77); 5,3612 (1,79); 5,319 (0,5); 4,3589 (0,59); 4,3256 (0,69); 4,2788 (1,13); 4,2505 (1,41); 4,2402 (1,49); 4,2115 (1,32); 3,9722 (0,59); 3,9431 (0,68); 3,6199 (0,39); 3,6051 (1,2); 3,5764 (1,32); 3,5642 (1,48); 3,5352 (1,32); 3,5043 (0,46); 3,4753 (0,56); 3,3986 (1,3); 3,3617 (3,06); 3,3065 (1733,11); 3,2839 (13,31); 3,2321 (0,55); 3,2289 (0,59); 3,1531 (1,14); 3,1135 (1,56); 3,0733 (0,9); 2,8591 (0,44); 2,829 (0,71); 2,796 (0,5); 2,6738 (1,65); 2,6691 (2,21); 2,6644 (1,61); 2,6605 (0,85); 2,5978 (0,5); 2,5391 (3,22); 2,5223 (9,32); 2,509 (123,76); 2,5046 (230,23); 2,5001 (300,24); 2,4957 (206,16); 2,4912 (97,47); 2,3358 (0,68); 2,3314 (1,44); 2,3267 (1,98); 2,3222 (1,35); 2,1199 (0,53); 2,0856 (1,01); 2,069 (1,42); 2,0495 (0,58); 1,7963 (0,44); 1,7736 (0,43); 1,7666 (0,42); 1,5648 (0,46); 1,5339 (0,43); 1,2357 (1); 0,3859 (16); 0,3712 (15,91); 0,008 (1,1); -0,0002 (26,36); -0,0086 (1,02) |

| **Bsp. I-41, Solvent: DMSO-d₆** |
|---|
| 8,008 (10,37); 7,9952 (3,98); 7,3121 (2,96); 7,3055 (0,93); 7,2928 (4,12); 7,2898 (3,66); 7,2788 (1,09); 7,2719 (3,78); 7,2657 (0,57); 7,0476 (2,67); 7,0284 (3,22); 7,0023 (3,63); 6,9676 (8,54); 6,9504 (6,29); 6,9468 (5,32); 6,9444 (4,44); 6,9342 (1,37); 6,9318 (1,55); 6,8591 (2,06); 6,8401 (1,72); 5,7458 (6,58); 5,0799 (0,4); 5,0662 (0,73); 5,0611 (0,67); 5,0508 (0,92); 5,0473 (0,96); 5,0386 (1,01); 5,0278 (0,68); 5,02 (0,69); 5,009 (0,44); 4,1742 (2,12); 4,1403 (2,7); 4,1233 (5,05); 4,1134 (3,64); 4,108 (3,43); 4,0952 (0,43); 4,0811 (0,41); 4,0569 (0,88); 4,0391 (2,53); 4,0213 (2,55); 4,0035 (0,88); 3,6095 (1,36); 3,5821 (1,54); 3,5664 (2,09); 3,539 (1,74); 3,3469 (4,33); 3,3131 (646,13); 3,2851 (4,56); 3,0142 (1,52); 2,9847 (2,7); 2,9562 (1,52); 2,6739 (0,49); 2,6693 (0,64); 2,6649 (0,48); 2,5394 (1,13); 2,5225 (2,97); 2,5091 (36,21); 2,5048 (67,26); 2,5003 (88,12); 2,4959 (62,47); 2,4915 (30,87); 2,3316 (0,55); 2,327 (0,69); 2,3225 (0,52); 2,234 (15,8); 2,2049 (0,46); 2,1124 (16); 2,0887 (1,92); 2,0823 (1,95); 2,0687 (1,51); 2,0562 (2,13); 1,9866 (11,26); 1,9079 (0,66); 1,7076 (0,65); 1,6984 (0,8); 1,6766 (1,61); 1,6682 (1,75); 1,6463 (1,64); 1,6381 (1,52); 1,6168 (0,66); 1,607 (0,53); 1,1927 (3,17); 1,1749 (6,3); 1,1571 (3,08); 0,0079 (0,86); -0,0002 (16,78); -0,0085 (0,74) |

| **Bsp. I-42, Solvent: DMSO-d₆** |
|---|
| 8,142 (16); 8,1312 (1,56); 7,7334 (0,58); 7,7175 (1,39); 7,7124 (1,39); 7,6966 (2,54); 7,6806 (1,52); 7,6757 (1,52); 7,6605 (0,67); 7,3354 (2,73); 7,3166 (4,35); 7,2925 (5,53); 7,27 (3,53); 7,2023 (5,92); 7,1746 (0,89); 7,1595 (3,33); 7,0693 (2,98); 7,041 (0,46); 7,0235 (7,73); 7,0138 (0,57); 6,8985 (6,67); 6,8877 (4,29); 5,7458 (5,34); 5,6063 (0,48); 5,5611 (9,69); 5,5156 (0,56); 5,4275 (0,5); 5,4083 (1,44); 5,3868 (1,18); 5,3638 (1,36); 5,3496 (0,87); 5,335 (0,95); 5,3206 (2,01); 5,2862 (1,63); 4,4709 (1,44); 4,4369 (1,56); 4,0572 (0,81); 4,0394 (2,42); 4,0216 (2,42); 4,0038 (0,86); 3,8169 (2,05); 3,7999 (0,53); 3,7883 (2,19); 3,7722 (3,11); 3,7436 (2,7); 3,7099 (0,43); 3,7057 (0,46); 3,6964 (0,55); 3,6818 (0,71); 3,6723 (0,43); 3,6686 (0,52); 3,6016 (4,12); 3,5872 (4,22); 3,5646 (3,46); 3,557 (5,3); 3,543 (3,38); 3,5288 (2,21); 3,5047 (0,58); 3,493 (0,61); 3,4753 (0,68); 3,4644 (0,66); 3,4481 (0,49); 3,3952 (2,2); 3,3636 (4,5); 3,3158 (1509,35); 3,2172 (0,62); 2,6794 (0,61); 2,6746 (1,12); 2,6702 (1,5); 2,6655 (1,13); 2,6609 (0,58); 2,5401 (2,32); 2,5232 (6,05); 2,5099 (83,72); 2,5056 (157,06); 2,5011 (205,87); 2,4967 (142,94); 2,4922 (68,63); 2,3367 (0,59); 2,3323 (1,07); 2,3278 (1,45); 2,3233 (1,07); 2,3188 (0,55); 2,2997 (0,55); 2,2171 (2,57); 2,1857 (2,9); 2,085 (1,01); 2,0692 (0,78); 2,0439 (0,33); 1,987 (10,45); 1,9489 (0,58); 1,9411 (0,69); 1,9114 (1,67); 1,8865 (1,28); 1,8814 (1,2); 1,8577 (0,54); 1,8486 (0,46); 1,7865 (0,62); 1,7775 (0,68); 1,756 (1,26); 1,7474 (1,35); 1,7242 (1,29); 1,7174 (1,19); 1,6949 (0,53); 1,6847 (0,42); 1,4048 (0,43); 1,2368 (1,27); 1,1929 (2,92); 1,1752 (5,71); 1,1574 (2,83); 0,9293 (0,39); 0,9127 (0,37); -0,0002 (3,79) |

| **Bsp. I-43, Solvent: CDCl₃** |
|---|
| 7,6396 (8,29) 7,2653 (16,83) 7,0027 (0,41) 6,9929 (1,02) 6,9898 (0,57) 6,9874 (0,63) 6,9832 (0,48) 6,9792 (1,3) 6,9784 (1,38) 6,9745 (2,16) 6,9705 (0,63) 6,9679 (0,7) 6,9649 (1,5) 6,9552 (0,72) 6,9185 (0,45) 6,9094 (2,89) 6,905 (0,38) 6,8965 (4,09) 6,8957 (3,91) 6,8874 (0,6) 6,8825 (5,04) 6,7908 (1,56) 6,7631 (4,12) 6,6715 (3,94) 6,5801 (1,88) 5,3029 (16) 5,191 (0,37) 5,1638 (3,38) 5,153 (3,41) 5,1258 (0,37) 5,1017 (0,37) 5,0929 (0,62) 5,0885 (0,5) 5,0847 (0,73) 5,0797 (0,78) 5,0759 (0,73) 5,0715 (0,81) 5,0678 (0,45) 5,0631 (0,64) 5,0545 (0,39) 4,5865 (0,6) 4,5641 (0,61) 4,3094 (0,95) 4,3014 (0,98) 4,2919 (1,85) 4,2839 (1,77) 4,2562 (1,6) 4,247 (1,58) 4,2387 (0,88) 4,2295 (0,85) 4,1278 (0,77) 4,1159 (0,78) 3,9317 (0,59) 3,9088 (0,63) 3,707 (0,49) 3,6131 (0,43) 3,5845 (2,98) 3,5756 (2,95) 3,5677 (2,99) 3,5624 (3,16) 3,547 (0,47) 3,3676 (0,64) 3,3606 (0,68) 3,3554 (1,14) 3,349 (1,37) 3,3423 (0,92) 3,3366 (1,18) 3,3307 (1,04) 3,3239 (0,4) 3,317 (0,49) 3,3124 (0,43) 2,9546 (0,39) 2,9499 (0,45) 2,9309 (0,77) 2,9124 (0,45) 2,9078 (0,41) 2,2934 (0,51) 2,2732 (0,58) 2,2117 (0,52) 2,1922 (0,57) 2,1895 (0,56) 2,048 (3,74) 1,8982 (0,47) 1,8918 (0,53) 1,8762 (0,5) 1,8701 (0,46) 1,8257 (0,51) 1,819 (0,56) 1,8037 (0,52) 1,7994 (0,42) 1,797 (0,46) 1,6249 (9,86) 1,4274 (2,17) 1,372 (0,35) 1,286 (0,65) 1,2847 (0,56) 1,2725 (1,19) 1,2605 (2,4) 1,2532 (1,74) 1,2487 (1,3) 0,0708 (0,55) -0,0002 (6,49) |

| **Bsp. I-44, Solvent: DMSO-d6** |
|---|
| 8.2506 (9.12); 7.3553 (2.18); 7.3500 (0.80); 7.3433 (0.36); 7.3370 (2.91); 7.3334 (3.30); 7.3276 (0.58); 7.3206 (1.89); 7.3150 (3.01); 7.3089 (0.41); 7.1879 (2.67); 7.1763 (1.30); 7.0919 (0.51); 7.0882 (2.73); 7.0856 (3.94); 7.0803 (1.12); 7.0687 (1.58); 7.0661 (3.12); 7.0638 (2.82); 7.0549 (1.42); 7.0403 (3.19); 7.0249 (5.74); 7.0183 (1.47); 7.0159 (0.85); 7.0000 (2.22); 6.9841 (0.60); 6.9816 (1.05); 6.9791 (0.60); 6.9167 (2.40); 6.9045 (1.55); 5.4715 (0.61); 5.4287 (2.01); 5.3883 (2.00); 5.3574 (8.19); 4.3828 (0.55); 4.3496 (0.58); 4.0551 (1.13); 4.0373 (3.48); 4.0195 (3.55); 4.0017 (1.59); 3.9628 (0.56); 3.4498 (0.37); 3.4304 (0.44); 3.4211 (0.83); 3.4119 (0.45); 3.3922 (0.44); 3.3477 (139.08); 3.3241 (0.74); 3.3149 (0.61); 3.3084 (0.62); 3.2776 (0.79); 3.2491 (0.47); 3.2426 (0.39); 3.0369 (0.56); 2.8757 (0.40); 2.8490 (1.10); 2.8188 (0.41); 2.5259 (0.34); 2.5212 (0.52); 2.5124 (12.90); 2.5079 (28.77); 2.5033 (39.38); 2.4987 (28.36); 2.4942 (13.50); 2.1643 (0.49); 2.1499 (0.37); 2.1322 (1.01); 2.0977 (0.56); 2.0770 (0.65); 1.9902 (16.00); 1.8471 (0.45); 1.8388 (0.51); 1.8166 (0.47); 1.8080 (0.44); 1.6172 (0.46); 1.6076 (0.51); 1.5862 (0.47); 1.5770 (0.46); 1.1923 (4.51); 1.1745 (9.14); 1.1566 (4.44); -0.0002 (3.31) |

| **Bsp. I-45, Solvent: DMSO-d6** |
|---|
| 8.0308 (9.22); 7.5207 (8.55); 7.5003 (10.00); 7.2220 (2.91); 7.2021 (3.90); 7.1814 (2.31); 7.0058 (1.50); 6.8686 (3.70); 6.7316 (1.72); 6.2984 (3.60); 5.7621 (12.53); 5.2691 (0.87); 5.2263 (2.59); 5.1773 (2.53); 5.1348 (0.86); 5.1172 (0.36); 5.1045 (0.55); 5.0985 (0.54); 5.0941 (0.48); 5.0887 (0.81); 5.0858 (0.76); 5.0764 (0.89); 5.0673 (0.50); 5.0582 (0.55); 5.0479 (0.38); 4.3886 (0.72); 4.3560 (0.75); 4.1736 (0.34); 4.1636 (0.49); 4.1475 (2.77); 4.1420 (3.02); 4.1378 (2.93); 4.1291 (2.43); 4.1159 (0.40); 4.1029 (0.43); 4.0550 (0.41); 4.0371 (1.22); 4.0193 (1.56); 4.0097 (0.69); 4.0016 (0.87); 3.9759 (0.73); 3.6430 (0.94); 3.6153 (1.08); 3.5998 (1.83); 3.5722 (1.52); 3.4991 (1.66); 3.4803 (1.67); 3.4558 (1.04); 3.4372 (1.03); 3.4096 (0.61); 3.4006 (0.52); 3.3911 (0.75); 3.3812 (1.33); 3.3724 (0.92); 3.3435 (254.94); 3.3200 (2.51); 3.2900 (0.62); 3.2592 (1.00); 3.2304 (0.54); 2.8557 (0.51); 2.8258 (0.91); 2.7979 (0.52); 2.6768 (0.42); 2.6721 (0.59); 2.6675 (0.43); 2.5423 (0.34); 2.5255 (0.97); 2.5207 (1.49); 2.5120 (31.41); 2.5076 (67.88); 2.5030 (91.55); 2.4985 (65.53); 2.4940 (31.35); 2.3343 (0.44); 2.3299 (0.61); 2.3251 (0.45); 2.1866 (16.00); 2.1275 (0.65); 2.1000 (1.36); 2.0769 (0.96); 2.0665 (0.75); 1.9901 (5.50); 1.7958 (0.57); 1.7872 (0.64); 1.7650 (0.57); 1.7562 (0.54); 1.5697 (0.55); 1.5601 (0.61); 1.5382 (0.56); 1.5289 (0.55); 1.1921 (1.45); 1.1743 (2.87); 1.1565 (1.41); 0.0080 (0.36); - 0.0002 (12.89); -0.0084 (0.48) |

| **Bsp. I-46, Solvent: DMSO-d6** |
|---|
| 8.7850 (0.39); 8.0073 (7.23); 7.3306 (2.51); 7.3112 (2.48); 7.3033 (1.15); 7.2991 (0.91); 7.2818 (1.30); 7.2780 (0.97); 7.1838 (1.24); 7.1699 (1.32); 7.1635 (1.35); 7.1493 (1.77); 7.1426 (0.76); 7.1286 (0.73); 6.4991 (4.56); 6.4895 (0.63); 5.7541 (0.33); 5.3538 (0.84); 5.3107 (2.49); 5.2568 (2.58); 5.2143 (0.90); 5.1956 (1.18); 5.0458 (0.46); 5.0404 (0.53); 5.0317 (0.76); 5.0265 (0.63); 5.0179 (0.81); 5.0108 (0.57); 5.0044 (0.45); 4.9993 (0.42); 4.9904 (0.35); 4.3857 (0.75); 4.3526 (0.78); 4.2880 (0.80); 4.2792 (0.83); 4.2600 (1.48); 4.2521 (1.41); 4.2045 (1.45); 4.1909 (1.46); 4.1775 (0.88); 4.1643 (0.79); 4.0561 (1.12); 4.0382 (3.43); 4.0205 (3.45); 4.0027 (1.46); 3.9903 (0.72); 3.9564 (0.76); 3.6150 (1.07); 3.5873 (1.11); 3.5718 (1.70); 3.5441 (1.48); 3.4415 (1.74); 3.4225 (1.96); 3.4097 (0.88); 3.3982 (1.68); 3.3892 (1.29); 3.3794 (2.72); 3.3535 (98.01); 3.3412 (164.99); 3.3374 (156.80); 3.2697 (0.96); 3.2413 (0.50); 3.2346 (0.43); 3.0434 (2.58); 2.8695 (0.53); 2.8553 (2.41); 2.8437 (0.91); 2.8404 (0.89); 2.8134 (0.50); 2.6720 (0.40); 2.5069 (49.52); 2.5030 (64.43); 2.4993 (45.67); 2.3290 (0.38); 2.2094 (16.00); 2.1928 (2.49); 2.1350 (0.63); 2.1054 (1.39); 2.0734 (0.92); 1.9888 (14.55); 1.8195 (0.61); 1.8119 (0.63); 1.7884 (0.57); 1.7842 (0.55); 1.5824 (0.56); 1.5720 (0.62); 1.5512 (0.58); 1.5417 (0.57); 1.3975 (0.41); 1.1927 (3.78); 1.1749 (7.52); 1.1571 (3.69); 0.0080 (0.62); -0.0002 (17.03); - 0.0085 (0.52) |

| **Bsp. I-47, Solvent: DMSO-d6** |
|---|
| 8.8013 (0.51); 7.9795 (3.46); 7.9764 (4.63); 7.3988 (0.41); 7.3951 (0.43); 7.3887 (0.56); 7.3850 (0.81); 7.3790 (0.72); 7.3752 (0.93); 7.3710 (0.72); 7.3652 (0.96); 7.3552 (0.54); 7.3516 (0.53); 7.2736 (0.90); 7.2697 (0.86); 7.1851 (1.96); 7.1810 (1.86); 7.1304 (1.31); 7.0966 (0.98); 7.0925 (0.95); 7.0786 (1.19); 7.0642 (1.69); 7.0611 (1.97); 7.0589 (1.94); 7.0405 (4.57); 7.0272 (1.33); 6.9497 (1.40); 6.9150 (4.06); 5.5819 (2.74); 5.5547 (2.96); 5.4587 (0.91); 5.4538 (0.79); 5.4303 (1.87); 5.4253 (1.61); 5.4151 (0.39); 5.3763 (2.08); 5.3478 (1.02); 5.3205 (0.59); 5.1530 (0.65); 5.1393 (0.84); 5.1352 (0.80); 5.1212 (0.70); 5.0697 (0.50); 5.0567 (0.66); 5.0528 (0.60); 5.0397 (0.51); 4.6528 (0.41); 4.3566 (0.88); 4.3396 (0.93); 4.0455 (1.27); 4.0337 (3.82); 4.0218 (3.84); 4.0100 (1.31); 3.9696 (0.88); 3.9511 (0.95); 3.7180 (0.71); 3.7085 (0.69); 3.6747 (0.68); 3.6678 (0.44); 3.6652 (0.72); 3.5054 (0.44); 3.4974 (0.57); 3.4884 (0.35); 3.4676 (0.70); 3.4590 (0.67); 3.4492 (0.67); 3.4303 (0.64); 3.4204 (1.15); 3.4021 (1.35); 3.3955 (0.91); 3.3752 (3.31); 3.3547 (484.69); 3.3313 (4.54); 3.2758 (0.66); 3.2548 (1.13); 3.2328 (0.61); 3.0365 (1.18); 2.8481 (1.47); 2.8213 (1.15); 2.8003 (0.61); 2.6181 (0.81); 2.6154 (1.05); 2.5064 (126.96); 2.5037 (163.63); 2.5011 (127.17); 2.3906 (0.80); 2.3879 (1.04); 2.3853 (0.81); 2.1145 (0.84); 2.0962 (1.12); 2.0872 (1.14); 2.0788 (1.44); 2.0600 (0.93); 1.9909 (16.00); 1.8226 (0.37); 1.8025 (0.86); 1.7967 (0.64); 1.7823 (0.80); 1.6326 (4.49); 1.5836 (0.49); 1.5614 (0.71); 1.5541 (0.75); 1.5477 (0.72); 1.5256 (5.75); 1.2342 (0.61); 1.1861 (4.16); 1.1741 (8.40); 1.1624 (4.15); -0.0002 (32.59) |

| **Bsp. I-48, Solvent: DMSO-d6** |
|---|
| 8.0015 (7.99); 7.1638 (0.39); 7.1585 (1.21); 7.1530 (5.26); 7.1466 (1.75); 7.1416 (1.97); 7.1363 (3.15); 7.1279 (4.52); 7.1205 (0.98); 7.1136 (0.40); 6.9998 (1.59); 6.8626 (3.72); 6.7255 (1.75); 6.2950 (3.61); 5.7551 (9.05); 5.2619 (0.78); 5.2193 (2.56); 5.1714 (2.58); 5.1293 (0.79); 5.0210 (0.36); 5.0151 (0.45); 5.0099 (0.56); 5.0018 (0.81); 4.9953 (0.70); 4.9875 (0.86); 4.9798 (0.59); 4.9742 (0.48); 4.9683 (0.45); 4.9600 (0.36); 4.3860 (0.72); 4.3526 (0.77); 4.3051 (0.85); 4.2970 (0.90); 4.2774 (1.67); 4.2691 (1.63); 4.2259 (1.59); 4.2116 (1.58); 4.1982 (0.91); 4.1839 (0.87); 4.0378 (0.32); 4.0200 (0.59); 4.0082 (0.68); 4.0026 (0.66); 3.9743 (0.74); 3.5941 (1.09); 3.5664 (1.26); 3.5510 (1.76); 3.5233 (1.48); 3.4068 (0.56); 3.3974 (0.42); 3.3875 (0.64); 3.3780 (1.25); 3.3690 (2.60); 3.3403 (73.45); 3.3361 (102.61); 3.3269 (95.68); 3.3066 (3.37); 3.2600 (1.17); 3.2314 (0.68); 3.2263 (0.63); 2.8580 (0.54); 2.8280 (0.93); 2.8008 (0.53); 2.6709 (0.38); 2.5242 (0.60); 2.5109 (18.55); 2.5064 (39.19); 2.5018 (54.10); 2.4972 (40.78); 2.4927 (20.90); 2.3285 (0.41); 2.1876 (16.00); 2.1320 (0.72); 2.0957 (1.45); 2.0733 (0.86); 1.9885 (1.37); 1.7948 (0.60); 1.7870 (0.65); 1.7650 (0.60); 1.7568 (0.57); 1.5715 (0.58); 1.5616 (0.64); 1.5398 (0.60); 1.5315 (0.58); 1.1923 (0.39); 1.1746 (0.76); 1.1568 (0.38); -0.0002 (7.60); -0.0085 (0.33) |

| **Bsp. I-49, Solvent: DMSO-d6** |
|---|
| 7.9731 (5.66); 7.9682 (9.39); 7.6879 (2.90); 7.6697 (3.42); 7.6311 (2.22); 7.6129 (2.85); 7.4823 (0.80); 7.4777 (1.17); 7.4733 (0.60); 7.4610 (3.28); 7.4419 (2.65); 7.4310 (2.65); 7.4270 (2.42); 7.4205 (1.11); 7.4103 (4.50); 7.3912 (3.84); 7.3827 (2.69); 7.3792 (1.57); 7.3726 (0.79); 7.3656 (1.77); 7.3562 (0.34); 7.3507 (0.34); 7.3475 (0.48); 7.3066 (1.54); 7.2972 (1.26); 7.1733 (3.60); 7.1640 (4.67); 7.1360 (7.05); 7.0402 (1.78); 7.0281 (4.71); 7.0243 (3.74); 6.9395 (5.75); 6.9030 (4.95); 6.8923 (2.59); 6.8886 (1.93); 5.7548 (8.18); 5.6411 (0.96); 5.6181 (1.28); 5.6135 (1.23); 5.5903 (1.00); 5.4667 (1.28); 5.4432 (2.56); 5.4400 (2.27); 5.4155 (1.80); 5.4080 (2.94); 5.3999 (2.41); 5.3653 (2.72); 5.3544 (2.15); 5.3222 (0.88); 5.3125 (1.08); 4.6342 (0.45); 4.6214 (1.01); 4.6080 (0.54); 4.3441 (1.10); 4.3157 (1.20); 4.0558 (1.16); 4.0380 (3.59); 4.0202 (3.65); 4.0024 (1.24); 3.9561 (0.93); 3.9273 (1.06); 3.7291 (0.50); 3.7244 (0.67); 3.7180 (0.73); 3.7133 (1.72); 3.7092 (1.36); 3.7014 (1.93); 3.6994 (2.31); 3.6805 (2.15); 3.6784 (2.09); 3.6706 (1.38); 3.6665 (1.79); 3.6619 (0.79); 3.6554 (0.69); 3.6507 (0.53); 3.5971 (0.77); 3.5683 (1.02); 3.5532 (1.37); 3.5258 (1.39); 3.5137 (0.58); 3.5105 (0.87); 3.5084 (0.86); 3.4970 (1.51); 3.4852 (1.06); 3.4737 (2.16); 3.4672 (0.57); 3.4619 (2.02); 3.4506 (0.67); 3.4459 (0.45); 3.4077 (1.06); 3.3844 (1.44); 3.3777 (1.28); 3.3641 (1.73); 3.3323 (241.93); 3.3294 (281.54); 3.2904 (1.63); 3.2742 (1.12); 3.2609 (1.64); 3.2452 (2.23); 3.2284 (1.22); 3.2175 (1.64); 3.1998 (0.68); 3.0367 (1.07); 2.9750 (1.00); 2.9520 (1.00); 2.9319 (0.84); 2.9090 (0.82); 2.8490 (1.24); 2.8414 (0.64); 2.8105 (1.09); 2.7986 (0.91); 2.7846 (0.71); 2.7688 (0.51); 2.6753 (0.41); 2.6707 (0.60); 2.6662 (0.43); 2.5241 (0.96); 2.5107 (30.74); 2.5062 (65.08); 2.5016 (89.61); 2.4970 (67.57); 2.4926 (34.57); 2.3328 (0.48); 2.3283 (0.67); 2.3237 (0.52); 2.0731 (2.29); 2.0604 (2.01); 1.9884 (16.00); 1.9088 (0.41); 1.8062 (0.46); 1.7764 (1.14); 1.7457 (1.06); 1.7159 (0.40); 1.5564 (0.37); 1.5354 (0.90); 1.5136 (0.86); 1.5051 (0.89); 1.4845 (0.52); 1.3975 (1.71); 1.2351 (0.66); 1.1923 (4.38); 1.1745 (8.74); 1.1567 (4.34); 0.0080 (0.70); -0.0002 (24.51); -0.0084 (1.06) |

| **Bsp. I-50, Solvent: DMSO-d6** |
|---|
| 8.7784 (0.43); 8.1552 (3.52); 8.0174 (0.67); 7.9948 (7.98); 7.7484 (0.40); 7.3284 (3.30); 7.3228 (3.56); 7.2037 (2.27); 7.1833 (3.23); 7.1635 (0.46); 7.1521 (5.05); 7.1361 (2.72); 7.1273 (4.48); 7.0891 (2.34); 7.0832 (2.38); 7.0689 (1.63); 7.0632 (1.64); 5.0213 (0.41); 5.0017 (0.69); 4.9948 (0.65); 4.9883 (0.79); 4.9812 (0.58); 4.9744 (0.44); 4.9602 (0.35); 4.3074 (0.81); 4.2992 (0.82); 4.2789 (1.55); 4.2708 (1.43); 4.2250 (1.54); 4.2110 (1.46); 4.1970 (0.91); 4.1828 (1.47); 4.1707 (1.70); 4.1382 (1.74); 4.0562 (0.63); 4.0384 (2.03); 4.0206 (2.02); 4.0028 (0.68); 3.5963 (1.06); 3.5685 (1.29); 3.5531 (1.72); 3.5254 (1.44); 3.4554 (0.37); 3.4304 (0.60); 3.3618 (1939.47); 3.3287 (3.96); 3.3093 (2.10); 3.2994 (1.03); 3.2899 (0.66); 3.0393 (1.17); 3.0083 (2.10); 2.9803 (1.16); 2.6777 (0.57); 2.6732 (0.71); 2.5425 (0.37); 2.5082 (86.24); 2.5041 (113.87); 2.5002 (80.49); 2.3303 (0.67); 2.1553 (16.00); 2.0914 (1.37); 2.0722 (2.01); 2.0656 (1.57); 1.9888 (8.43); 1.7017 (0.55); 1.6809 (1.23); 1.6730 (1.37); 1.6490 (1.24); 1.6424 (1.17); 1.6199 (0.48); 1.6142 (0.40); 1.4223 (1.53); 1.4056 (1.53); 1.3977 (0.86); 1.2884 (4.96); 1.2355 (0.46); 1.1928 (2.14); 1.1750 (4.39); 1.1573 (2.16); -0.0002 (6.48) |

| **Bsp. I-51, Solvent: DMSO-d6** |
|---|
| 8.1730 (3.55); 8.0112 (8.21); 7.3362 (1.06); 7.3338 (1.84); 7.3316 (1.48); 7.3264 (3.89); 7.3226 (4.53); 7.3184 (1.90); 7.3133 (1.57); 7.3095 (1.21); 7.3069 (0.95); 7.2953 (1.19); 7.2929 (0.97); 7.2007 (2.24); 7.1871 (2.80); 7.1777 (1.25); 7.1684 (1.23); 7.1640 (1.62); 7.1547 (1.77); 7.1502 (0.83); 7.1409 (0.76); 7.0883 (2.07); 7.0846 (1.98); 7.0748 (1.65); 7.0710 (1.60); 5.7635 (3.03); 5.0473 (0.34); 5.0415 (0.38); 5.0382 (0.44); 5.0347 (0.52); 5.0291 (0.76); 5.0259 (0.58); 5.0233 (0.54); 5.0200 (0.78); 5.0146 (0.51); 5.0109 (0.43); 5.0074 (0.40); 5.0017 (0.34); 4.2798 (0.93); 4.2744 (0.93); 4.2617 (1.38); 4.2563 (1.32); 4.1950 (1.37); 4.1859 (1.41); 4.1764 (1.36); 4.1677 (2.49); 4.1451 (1.64); 4.0457 (1.01); 4.0339 (3.06); 4.0221 (3.09); 4.0102 (1.02); 3.6031 (1.08); 3.5846 (1.26); 3.5745 (1.64); 3.5560 (1.38); 3.4351 (1.58); 3.4226 (1.62); 3.4064 (1.31); 3.3940 (1.35); 3.3887 (0.41); 3.3820 (0.61); 3.3549 (293.74); 3.3313 (1.71); 3.3227 (0.73); 3.3162 (0.46); 3.3098 (0.62); 3.3034 (0.32); 3.0293 (0.93); 3.0259 (1.06); 3.0063 (2.02); 2.9871 (1.08); 2.9839 (0.94); 2.9260 (0.80); 2.6185 (0.33); 2.6155 (0.45); 2.6126 (0.33); 2.5248 (0.75); 2.5217 (1.00); 2.5186 (1.18); 2.5097 (24.42); 2.5068 (51.37); 2.5037 (69.81); 2.5007 (51.03); 2.4979 (23.55); 2.3909 (0.33); 2.3879 (0.45); 2.3849 (0.32); 2.1545 (16.00); 2.0841 (1.34); 2.0780 (1.07); 2.0668 (1.44); 1.9905 (13.23); 1.6876 (0.45); 1.6681 (1.17); 1.6488 (1.13); 1.6325 (0.37); 1.6296 (0.40); 1.3967 (1.03); 1.1862 (3.56); 1.1744 (7.06); 1.1626 (3.50); -0.0002 (7.18) |

| **Bsp. I-52, Solvent: DMSO-d6** |
|---|
| 8.7996 (0.61); 7.9754 (5.31); 7.9534 (2.31); 7.3985 (0.44); 7.3948 (0.47); 7.3884 (0.61); 7.3848 (0.88); 7.3789 (0.81); 7.3749 (1.02); 7.3708 (0.80); 7.3651 (1.09); 7.3550 (0.62); 7.3514 (0.62); 7.0776 (1.25); 7.0589 (2.33); 7.0442 (2.29); 7.0407 (2.22); 7.0266 (1.42); 6.7571 (0.43); 6.5917 (1.38); 6.5685 (4.73); 5.7655 (10.34); 5.5818 (2.72); 5.5552 (3.11); 5.3656 (1.10); 5.3372 (2.03); 5.3333 (1.81); 5.3191 (0.37); 5.2673 (1.91); 5.2624 (1.61); 5.2390 (1.08); 5.2340 (0.90); 5.1544 (0.75); 5.1406 (0.95); 5.1367 (0.92); 5.1227 (0.79); 5.0713 (0.56); 5.0578 (0.80); 5.0541 (0.82); 5.0425 (1.54); 4.3765 (0.92); 4.3595 (1.06); 4.0456 (0.63); 4.0338 (1.91); 4.0220 (1.99); 4.0100 (1.29); 4.0015 (0.92); 3.9827 (1.03); 3.4494 (0.54); 3.4308 (0.66); 3.4207 (1.24); 3.4025 (1.26); 3.3888 (1.13); 3.3766 (3.60); 3.3562 (74.44); 3.3346 (2.75); 3.2758 (0.70); 3.2693 (0.62); 3.2553 (1.19); 3.2376 (0.69); 3.2328 (0.69); 2.9877 (0.36); 2.9461 (0.38); 2.9409 (0.44); 2.9349 (0.94); 2.9297 (0.99); 2.9236 (1.36); 2.9185 (1.28); 2.9123 (1.20); 2.9072 (1.04); 2.9009 (0.79); 2.8906 (14.64); 2.8498 (0.34); 2.8352 (0.69); 2.8142 (1.21); 2.7975 (0.60); 2.7929 (0.63); 2.7310 (12.45); 2.6154 (0.41); 2.5215 (1.21); 2.5066 (44.94); 2.5038 (59.07); 2.5011 (46.12); 2.3881 (0.38); 2.0849 (1.67); 2.0788 (2.06); 2.0633 (1.06); 1.9909 (7.93); 1.9104 (0.68); 1.8291 (0.39); 1.8093 (0.84); 1.8017 (0.66); 1.7965 (0.65); 1.7887 (0.77); 1.6339 (4.89); 1.5845 (0.35); 1.5791 (0.33); 1.5715 (0.38); 1.5581 (0.54); 1.5508 (0.78); 1.5265 (6.77); 1.2339 (0.51); 1.2080 (1.85); 1.1964 (2.53); 1.1944 (2.49); 1.1887 (6.87); 1.1838 (14.88); 1.1788 (14.72); 1.1727 (16.00); 1.1679 (10.90); 1.1626 (4.01); -0.0002 (7.66) |

| **Bsp. I-53, Solvent: DMSO-d6** |
|---|
| 8.6310 (7.22); 7.9902 (16.00); 7.7039 (3.91); 7.6837 (4.64); 7.5270 (6.00); 7.4870 (3.66); 7.4669 (3.02); 7.3731 (0.63); 7.3531 (3.40); 7.3436 (11.09); 7.3362 (9.49); 7.3294 (4.43); 7.3245 (1.31); 7.3090 (0.54); 7.2501 (0.36); 7.2381 (2.45); 7.2299 (2.17); 7.2227 (3.61); 7.2132 (2.69); 7.2099 (2.63); 7.2029 (2.62); 7.1926 (2.04); 7.1888 (1.78); 7.1786 (1.68); 7.0852 (5.99); 7.0717 (5.07); 6.9461 (2.92); 6.9339 (2.51); 5.7538 (8.66); 5.5873 (2.42); 5.5685 (2.81); 5.5587 (2.68); 5.5399 (2.45); 4.1755 (3.21); 4.1417 (3.48); 4.0564 (0.70); 4.0386 (2.11); 4.0208 (2.14); 4.0031 (0.71); 3.7266 (0.55); 3.7237 (0.60); 3.4715 (2.00); 3.4430 (2.35); 3.4273 (3.02); 3.3987 (2.94); 3.3577 (111.79); 3.3554 (117.89); 3.3539 (114.39); 3.3518 (120.56); 3.3434 (213.50); 3.3414 (232.75); 3.3200 (4.35); 3.3010 (1.08); 3.2915 (1.38); 3.2823 (0.76); 3.1794 (2.48); 3.1608 (2.48); 3.1352 (1.94); 3.1163 (1.91); 3.0522 (2.29); 3.0221 (4.23); 2.9932 (2.32); 2.6770 (0.40); 2.6724 (0.56); 2.6681 (0.37); 2.5257 (0.91); 2.5076 (63.89); 2.5034 (85.75); 2.4994 (59.51); 2.3348 (0.42); 2.3297 (0.60); 2.3257 (0.52); 2.0948 (2.79); 2.0691 (3.17); 1.9890 (9.26); 1.7089 (1.07); 1.7012 (0.84); 1.6794 (2.63); 1.6495 (2.53); 1.6278 (0.80); 1.6193 (0.99); 1.5833 (13.30); 1.5749 (13.50); 1.5053 (12.86); 1.4943 (12.96); 1.3969 (2.16); 1.1929 (2.42); 1.1751 (4.84); 1.1573 (2.41); -0.0002 (0.96) |

| **Bsp. I-54, Solvent: DMSO-d6** |
|---|
| 8.0036 (8.63); 7.1583 (1.23); 7.1527 (5.15); 7.1361 (2.98); 7.1278 (4.58); 6.4985 (4.34); 5.7556 (8.75); 5.3546 (0.85); 5.3114 (2.62); 5.2582 (2.54); 5.2157 (0.87); 5.0297 (0.33); 5.0208 (0.37); 5.0143 (0.44); 5.0081 (0.53); 5.0020 (0.75); 4.9954 (0.67); 4.9878 (0.79); 4.9823 (0.55); 4.9690 (0.41); 4.9604 (0.35); 4.3880 (0.72); 4.3540 (0.73); 4.3053 (0.82); 4.2973 (0.82); 4.2776 (1.57); 4.2694 (1.48); 4.2266 (1.54); 4.2125 (1.56); 4.1989 (0.89); 4.1847 (0.87); 4.0203 (0.37); 3.9896 (0.67); 3.9556 (0.71); 3.5947 (1.05); 3.5669 (1.16); 3.5514 (1.65); 3.5237 (1.42); 3.4103 (0.53); 3.4014 (0.39); 3.3903 (0.61); 3.3809 (1.14); 3.3700 (2.09); 3.3619 (0.70); 3.3506 (2.36); 3.3310 (62.48); 3.3225 (91.78); 3.3073 (2.09); 3.2688 (0.93); 3.2379 (0.51); 2.8681 (0.53); 2.8418 (0.88); 2.8115 (0.54); 2.6707 (0.49); 2.6660 (0.36); 2.5236 (0.88); 2.5056 (59.33); 2.5015 (79.02); 2.4975 (55.59); 2.3327 (0.37); 2.3284 (0.46); 2.2091 (16.00); 2.1359 (0.60); 2.1060 (1.29); 2.0737 (1.09); 1.9885 (1.50); 1.8212 (0.57); 1.8137 (0.61); 1.7912 (0.56); 1.7835 (0.50); 1.7647 (0.60); 1.7310 (0.48); 1.5728 (0.61); 1.5505 (0.54); 1.5420 (0.53); 1.4070 (0.69); 1.2371 (1.12); 1.1924 (0.42); 1.1744 (0.77); 1.1567 (0.40); 0.8545 (0.40); -0.0002 (4.58) |

| **Bsp. I-55, Solvent: DMSO-d6** |
|---|
| 8.1423 (0.33); 7.9992 (11.73); 7.5639 (1.57); 7.5483 (2.81); 7.5447 (3.12); 7.5253 (1.71); 7.3378 (0.67); 7.3139 (2.74); 7.3031 (2.46); 7.2890 (1.38); 7.2856 (1.42); 7.2754 (0.35); 7.2622 (2.57); 7.2371 (2.97); 7.2292 (2.76); 7.2194 (1.53); 7.2163 (1.64); 7.2100 (3.38); 7.1917 (1.57); 7.1881 (1.75); 7.1802 (4.86); 7.1673 (2.45); 7.0467 (2.32); 7.0316 (5.37); 6.9082 (5.58); 6.8957 (2.67); 5.4602 (1.22); 5.4175 (4.19); 5.3742 (4.04); 5.3320 (1.21); 4.8689 (0.53); 4.8529 (1.36); 4.8356 (1.57); 4.8270 (1.64); 4.8106 (1.46); 4.7941 (0.56); 4.3643 (1.29); 4.3299 (1.34); 4.0556 (1.18); 4.0380 (3.72); 4.0203 (3.88); 4.0026 (1.44); 3.9811 (1.12); 3.9460 (1.28); 3.5919 (1.99); 3.5660 (2.09); 3.5489 (2.70); 3.5228 (2.23); 3.5020 (0.33); 3.4797 (0.33); 3.4467 (0.41); 3.4080 (1.32); 3.3809 (3.06); 3.3371 (1390.17); 3.3180 (7.04); 3.3004 (4.23); 3.2856 (3.67); 3.2712 (5.01); 3.2569 (2.72); 3.2499 (3.58); 3.2345 (3.81); 3.2158 (1.56); 3.1999 (1.34); 3.0354 (0.40); 2.8663 (0.88); 2.8380 (1.67); 2.8113 (0.89); 2.6711 (1.39); 2.5702 (0.35); 2.5412 (0.65); 2.5064 (158.56); 2.5023 (216.00); 2.4984 (157.74); 2.4624 (0.68); 2.3287 (1.19); 2.1327 (1.11); 2.0926 (2.23); 2.0732 (2.98); 1.9885 (16.00); 1.9082 (0.41); 1.8336 (0.56); 1.8053 (1.05); 1.7816 (0.92); 1.7431 (0.46); 1.5982 (0.53); 1.5797 (1.02); 1.5698 (1.07); 1.5491 (1.05); 1.5390 (0.99); 1.5178 (0.42); 1.2347 (0.70); 1.1925 (4.22); 1.1747 (8.38); 1.1568 (4.15); - 0.0002 (5.83); -0.0082 (0.32); -2.2464 (0.34) |

| **Bsp. I-56, Solvent: DMSO-d6** |
|---|
| 8.7825 (0.32); 8.0179 (12.91); 7.5159 (9.57); 7.4956 (11.37); 7.2193 (3.37); 7.1995 (3.87); 7.1787 (2.66); 7.0487 (2.28); 7.0293 (2.77); 6.9971 (3.02); 6.8599 (1.76); 6.8371 (1.41); 5.7554 (16.00); 5.1162 (0.37); 5.1031 (0.56); 5.0977 (0.57); 5.0876 (0.79); 5.0754 (0.95); 5.0568 (0.57); 5.0459 (0.33); 4.1743 (1.74); 4.1664 (1.58); 4.1498 (3.73); 4.1448 (4.47); 4.1400 (4.59); 4.1319 (3.70); 4.1187 (0.63); 4.1060 (0.48); 4.0557 (0.45); 4.0378 (1.11); 4.0201 (1.09); 4.0025 (0.39); 3.6444 (1.03); 3.6170 (1.08); 3.6010 (1.92); 3.5736 (1.65); 3.5046 (1.74); 3.4863 (1.74); 3.4613 (1.12); 3.4425 (1.07); 3.3482 (1.28); 3.3233 (534.95); 3.2999 (2.64); 3.2820 (0.54); 3.0093 (1.07); 2.9810 (2.09); 2.9507 (1.19); 2.6751 (0.66); 2.6704 (0.87); 2.6655 (0.66); 2.5404 (0.52); 2.5237 (1.72); 2.5054 (98.12); 2.5013 (132.06); 2.3327 (0.64); 2.3282 (0.93); 2.3233 (0.69); 2.3189 (0.34); 2.2333 (13.61); 2.1105 (13.49); 2.0735 (2.40); 2.0466 (1.58); 1.9884 (4.83); 1.6922 (0.52); 1.6603 (1.27); 1.6355 (1.14); 1.6046 (0.43); 1.3972 (0.37); 1.2341 (0.39); 1.1923 (1.35); 1.1745 (2.64); 1.1567 (1.36); -0.0002 (11.99) |

| **Bsp. I-57, Solvent: DMSO-d6** |
|---|
| 8.1108 (6.85); 7.8454 (1.79); 7.8433 (1.83); 7.8371 (1.83); 7.8350 (1.78); 7.5308 (1.04); 7.5295 (1.06); 7.5250 (1.12); 7.2707 (0.83); 7.1990 (0.68); 7.1910 (1.04); 7.1822 (2.32); 7.1291 (0.89); 7.0936 (0.95); 7.0387 (2.35); 6.9484 (1.05); 6.9143 (2.04); 5.4958 (0.34); 5.4853 (0.36); 5.4772 (0.40); 5.4667 (0.39); 5.4534 (0.74); 5.4249 (1.54); 5.3746 (1.54); 5.3462 (0.71); 5.2298 (0.59); 4.3579 (0.45); 4.3357 (0.46); 4.0459 (1.13); 4.0340 (3.50); 4.0222 (3.52); 4.0103 (1.16); 3.9727 (0.43); 3.9497 (0.45); 3.7824 (0.78); 3.7632 (0.91); 3.7528 (1.06); 3.7336 (0.89); 3.5681 (12.73); 3.5063 (0.38); 3.4852 (1.05); 3.4747 (1.07); 3.4556 (0.95); 3.4451 (0.93); 3.3959 (0.39); 3.3829 (0.50); 3.3767 (0.90); 3.3702 (0.79); 3.3553 (58.34); 3.2743 (0.36); 3.2545 (0.62); 3.2354 (0.35); 2.8419 (0.33); 2.8243 (0.56); 2.8213 (0.57); 2.8040 (0.33); 2.5215 (0.33); 2.5184 (0.33); 2.5095 (9.34); 2.5065 (20.80); 2.5035 (28.96); 2.5004 (21.04); 2.4974 (9.45); 2.1189 (0.38); 2.0961 (0.53); 2.0849 (0.40); 2.0779 (0.54); 2.0603 (0.42); 1.9905 (16.00); 1.9102 (1.14); 1.8047 (0.55); 1.7982 (0.42); 1.7936 (0.39); 1.7829 (0.46); 1.7780 (0.37); 1.5616 (0.37); 1.5551 (0.40); 1.5412 (0.38); 1.5348 (0.37); 1.1861 (4.34); 1.1743 (9.14); 1.1625 (4.26); -0.0002 (1.66) |

| **Bsp. I-58, Solvent: DMSO-d6** |
|---|
| 7.9741 (10.68); 7.5994 (12.59); 7.5798 (16.00); 7.4404 (5.22); 7.4195 (7.27); 7.4002 (3.41); 7.3117 (2.70); 7.1783 (5.82); 7.1650 (3.06); 7.0449 (2.97); 7.0290 (6.28); 6.9129 (11.18); 6.8931 (3.04); 5.7463 (4.14); 5.7446 (4.22); 5.4578 (2.15); 5.4145 (6.34); 5.3688 (6.00); 5.3266 (2.21); 4.7793 (1.03); 4.7640 (2.75); 4.7471 (3.57); 4.7381 (3.22); 4.7211 (3.04); 4.7058 (1.23); 4.3599 (2.48); 4.3275 (2.69); 4.2864 (0.33); 4.0558 (0.74); 4.0393 (2.36); 4.0216 (2.43); 4.0041 (1.09); 3.9804 (2.39); 3.9467 (2.65); 3.9172 (0.35); 3.8185 (0.38); 3.8029 (0.34); 3.7661 (0.35); 3.7518 (0.41); 3.7184 (0.45); 3.7090 (0.48); 3.6884 (0.52); 3.6814 (0.56); 3.6175 (0.91); 3.5851 (3.66); 3.5699 (1.85); 3.5591 (4.03); 3.5414 (5.91); 3.5146 (7.54); 3.4567 (792.13); 3.4525 (775.96); 3.4490 (764.64); 3.4443 (811.54); 3.4411 (832.24); 3.4384 (891.58); 3.4332 (929.12); 3.4312 (921.69); 3.4272 (1171.48); 3.4247 (1194.84); 3.3798 (7.07); 3.3670 (8.02); 3.3492 (7.18); 3.3236 (3.90); 3.3057 (4.79); 3.2717 (3.51); 3.2423 (1.95); 3.2088 (0.77); 3.1925 (12.78); 3.1776 (12.81); 3.1418 (0.41); 3.0375 (0.62); 2.8724 (1.76); 2.8434 (3.28); 2.8137 (1.78); 2.6785 (1.25); 2.6740 (1.07); 2.5485 (0.55); 2.5131 (156.15); 2.5091 (213.73); 2.5054 (165.44); 2.3357 (1.32); 2.1272 (2.23); 2.0948 (4.63); 2.0600 (2.63); 1.9895 (9.39); 1.8285 (0.91); 1.8131 (0.82); 1.7981 (2.09); 1.7663 (1.89); 1.7441 (0.82); 1.5957 (0.85); 1.5654 (2.06); 1.5428 (1.88); 1.5150 (0.82); 1.3979 (0.49); 1.2349 (0.98); 1.1939 (2.47); 1.1760 (4.98); 1.1583 (2.39); -0.0002 (3.98) |

| **Bsp. I-59, Solvent: DMSO-d6** |
|---|
| 8.1517 (3.48); 8.0632 (0.35); 8.0190 (9.83); 7.5153 (9.19); 7.4950 (10.82); 7.3290 (3.34); 7.3233 (3.63); 7.2191 (3.13); 7.1995 (4.81); 7.1788 (3.89); 7.0877 (2.50); 7.0822 (2.36); 7.0675 (1.78); 7.0616 (1.68); 5.7545 (12.77); 5.1157 (0.36); 5.1035 (0.56); 5.0845 (0.77); 5.0756 (0.91); 5.0659 (0.52); 5.0578 (0.53); 5.0470 (0.40); 4.1721 (1.69); 4.1665 (1.57); 4.1496 (3.51); 4.1437 (4.36); 4.1401 (4.50); 4.1309 (3.51); 4.1179 (0.61); 4.1049 (0.53); 4.0559 (0.51); 4.0381 (1.45); 4.0203 (1.44); 4.0023 (0.50); 3.6435 (1.07); 3.6159 (1.10); 3.6004 (1.85); 3.5730 (1.60); 3.5043 (1.77); 3.4857 (1.78); 3.4671 (0.34); 3.4613 (1.16); 3.4423 (1.11); 3.3922 (0.43); 3.3785 (0.76); 3.3333 (811.15); 3.2917 (0.78); 3.2680 (0.33); 3.0390 (1.13); 3.0087 (2.01); 2.9805 (1.06); 2.6755 (0.60); 2.6711 (0.96); 2.6664 (0.64); 2.5412 (0.56); 2.5244 (1.75); 2.5063 (94.58); 2.5022 (126.35); 2.3331 (0.62); 2.3290 (0.81); 2.3241 (0.65); 2.1553 (16.00); 2.1413 (0.68); 2.0903 (1.30); 2.0734 (1.93); 2.0642 (1.54); 1.9886 (6.59); 1.7011 (0.49); 1.6791 (1.19); 1.6480 (1.16); 1.6222 (0.40); 1.2355 (0.34); 1.1925 (1.75); 1.1747 (3.55); 1.1569 (1.68); -0.0002 (5.54) |

| **Bsp. I-60, Solvent: DMSO-d6** |
|---|
| 8.1901 (4.01); 8.0230 (6.21); 7.6537 (1.35); 7.6496 (1.44); 7.6344 (1.46); 7.6302 (1.46); 7.4190 (0.75); 7.4147 (0.75); 7.4005 (1.02); 7.3977 (1.23); 7.3938 (1.02); 7.3797 (0.96); 7.3753 (0.91); 7.3126 (1.06); 7.1793 (2.49); 7.1659 (1.20); 7.1110 (1.83); 7.0911 (1.53); 7.0461 (1.21); 7.0299 (2.80); 7.0040 (1.01); 6.9852 (1.74); 6.9664 (0.88); 6.9085 (2.41); 6.8941 (1.34); 5.7526 (6.22); 5.4642 (0.55); 5.4214 (1.72); 5.3735 (1.77); 5.3308 (0.57); 5.1104 (0.40); 5.1059 (0.42); 5.0944 (0.63); 5.0859 (0.60); 5.0787 (0.39); 5.0745 (0.42); 5.0699 (0.38); 5.0669 (0.36); 4.3670 (0.54); 4.3327 (0.57); 4.2371 (0.68); 4.2287 (0.71); 4.2103 (1.30); 4.2021 (1.25); 4.1644 (1.18); 4.1522 (1.19); 4.1378 (0.69); 4.1255 (0.64); 4.0383 (0.73); 4.0206 (0.76); 4.0026 (0.41); 3.9883 (0.59); 3.9545 (0.56); 3.8560 (0.55); 3.8111 (16.00); 3.6233 (0.78); 3.5957 (0.91); 3.5802 (1.23); 3.5527 (1.04); 3.4191 (0.87); 3.4040 (2.24); 3.3875 (4.18); 3.3624 (92.37); 3.3512 (129.28); 3.2771 (0.82); 3.2479 (0.45); 3.2423 (0.41); 2.8799 (0.36); 2.8492 (0.66); 2.8223 (0.39); 2.5666 (1.05); 2.5258 (0.61); 2.5126 (12.13); 2.5081 (24.39); 2.5035 (32.41); 2.4989 (23.34); 2.4944 (11.24); 2.1417 (0.46); 2.1079 (0.98); 2.0727 (0.86); 1.9888 (3.23); 1.8254 (0.44); 1.8169 (0.45); 1.7951 (0.41); 1.7874 (0.39); 1.5915 (0.42); 1.5827 (0.45); 1.5617 (0.43); 1.5523 (0.41); 1.1927 (0.88); 1.1749 (1.77); 1.1571 (0.86); -0.0002 (0.46) |

| **Bsp. I-61, Solvent: DMSO-d6** |
|---|
| 8.0158 (16.00); 7.5246 (3.28); 7.5214 (3.50); 7.5046 (4.32); 7.5014 (4.32); 7.4864 (3.95); 7.4835 (4.05); 7.4667 (4.60); 7.4636 (4.60); 7.3719 (2.11); 7.3686 (2.08); 7.3530 (3.98); 7.3337 (2.32); 7.3304 (2.15); 7.3139 (2.76); 7.2433 (2.61); 7.2397 (2.56); 7.2238 (3.87); 7.2050 (1.78); 7.2017 (1.63); 7.1808 (6.06); 7.1678 (2.93); 7.0475 (3.03); 7.0319 (6.74); 6.9085 (6.43); 6.8961 (3.27); 5.7543 (5.95); 5.4614 (1.47); 5.4187 (4.83); 5.3754 (4.83); 5.3328 (1.47); 4.9400 (0.56); 4.9241 (1.73); 4.9075 (1.90); 4.8980 (1.85); 4.8818 (1.67); 4.8658 (0.67); 4.4445 (0.81); 4.4336 (0.65); 4.3655 (1.43); 4.3319 (1.49); 4.0558 (1.04); 4.0381 (3.31); 4.0203 (3.44); 4.0022 (1.32); 3.9825 (1.30); 3.9499 (1.48); 3.6217 (2.16); 3.5952 (2.35); 3.5781 (3.24); 3.5523 (2.72); 3.4128 (1.24); 3.3951 (2.88); 3.3812 (3.57); 3.3749 (2.46); 3.3482 (155.97); 3.3446 (161.44); 3.3368 (336.82); 3.3341 (282.93); 3.3179 (6.96); 3.3019 (7.36); 3.2840 (4.29); 3.2683 (3.50); 3.2388 (1.03); 2.8722 (0.94); 2.8688 (1.05); 2.8388 (1.79); 2.8123 (0.99); 2.6713 (0.69); 2.5414 (0.49); 2.5063 (91.38); 2.5024 (118.78); 2.4986 (83.75); 2.3291 (0.76); 2.3249 (0.55); 2.1308 (1.20); 2.0997 (2.55); 2.0728 (1.37); 2.0618 (1.43); 1.9886 (14.20); 1.8497 (0.45); 1.8380 (0.56); 1.8175 (1.16); 1.8084 (1.30); 1.7874 (1.40); 1.7789 (1.32); 1.7607 (0.75); 1.6448 (0.36); 1.6369 (0.36); 1.6127 (0.83); 1.6036 (0.74); 1.5819 (1.15); 1.5713 (1.19); 1.5507 (1.30); 1.5409 (1.30); 1.5209 (0.62); 1.5097 (0.59); 1.2343 (0.86); 1.2004 (0.50); 1.1925 (3.84); 1.1748 (7.58); 1.1677 (0.61); 1.1570 (3.75); 0.8691 (3.74); 0.8528 (3.48); 0.7939 (0.32); 0.7646 (0.56); 0.7353 (0.54); 0.7228 (0.36); 0.7136 (0.33); 0.6917 (0.36); 0.0078 (1.27); -0.0002 (34.68); -0.0085 (1.01) |

| **Bsp. I-62, Solvent: DMSO-d6** |
|---|
| 7.9843 (7.69); 7.4082 (0.36); 7.3929 (0.85); 7.3873 (0.78); 7.3722 (1.61); 7.3665 (0.65); 7.3568 (0.72); 7.3514 (0.93); 7.3368 (0.46); 7.0958 (2.66); 7.0751 (2.26); 7.0669 (2.74); 7.0461 (2.22); 6.4963 (4.35); 5.7538 (3.14); 5.3460 (0.85); 5.3037 (2.46); 5.2488 (2.35); 5.2046 (1.83); 5.1859 (1.32); 5.1754 (1.18); 5.1573 (1.09); 4.6257 (0.41); 4.3851 (0.71); 4.3527 (0.77); 4.0557 (0.53); 4.0380 (1.53); 4.0203 (1.63); 4.0021 (0.59); 3.9823 (0.71); 3.9469 (0.74); 3.9434 (0.70); 3.7127 (0.51); 3.7093 (0.42); 3.6994 (0.79); 3.6784 (0.77); 3.6706 (0.46); 3.6665 (0.60); 3.5679 (0.38); 3.5098 (0.46); 3.4967 (0.69); 3.4848 (0.57); 3.4735 (1.08); 3.4669 (1.33); 3.4620 (1.02); 3.4500 (0.47); 3.4385 (1.15); 3.4231 (1.57); 3.3942 (2.01); 3.3524 (251.72); 3.3488 (278.34); 3.3401 (691.55); 3.3184 (4.36); 3.2838 (0.76); 3.2491 (1.00); 3.2223 (0.53); 3.1764 (1.14); 3.1573 (1.10); 3.1324 (0.85); 3.1138 (0.77); 2.8909 (0.49); 2.8457 (0.47); 2.8139 (0.90); 2.7879 (0.57); 2.7313 (0.40); 2.6761 (0.78); 2.6718 (1.03); 2.6670 (0.78); 2.5418 (0.55); 2.5248 (1.82); 2.5068 (116.17); 2.5026 (156.51); 2.3336 (0.72); 2.3295 (1.01); 2.3249 (0.81); 2.2041 (16.00); 2.1931 (1.02); 2.1216 (0.64); 2.0888 (1.27); 2.0730 (2.10); 2.0523 (0.74); 1.9885 (6.66); 1.9089 (1.17); 1.7996 (0.56); 1.7707 (0.56); 1.5581 (0.59); 1.5530 (0.59); 1.5281 (0.58); 1.4917 (0.39); 1.4631 (6.84); 1.3924 (3.59); 1.3848 (6.31); 1.3778 (3.61); 1.2358 (0.41); 1.1925 (1.83); 1.1747 (3.61); 1.1569 (1.71); -0.0002 (4.58) |

| **Bsp. I-63, Solvent: CDCl3** |
|---|
| 7.6098 (7.85); 7.5909 (2.40); 7.5889 (2.40); 7.5777 (2.52); 7.5756 (2.47); 7.4195 (1.95); 7.4171 (2.02); 7.4063 (2.51); 7.4040 (2.47); 7.3148 (1.36); 7.3127 (1.35); 7.3021 (2.28); 7.3004 (2.25); 7.2895 (1.26); 7.2874 (1.20); 7.2637 (43.73); 7.1104 (1.34); 7.1079 (1.36); 7.0974 (2.07); 7.0954 (2.07); 7.0849 (1.15); 7.0824 (1.10); 6.9749 (1.55); 6.8831 (3.28); 6.7914 (1.68); 6.7670 (4.29); 6.6735 (3.77); 6.5822 (1.83); 5.3038 (16.00); 5.1920 (0.48); 5.1648 (3.98); 5.1539 (4.02); 5.1267 (0.48); 5.0974 (0.40); 4.9503 (0.42); 4.9429 (0.49); 4.9393 (0.55); 4.9325 (0.89); 4.9246 (0.83); 4.9176 (0.89); 4.9108 (0.54); 4.9071 (0.50); 4.8998 (0.43); 4.5937 (0.80); 4.5712 (0.82); 4.1286 (0.49); 4.1168 (0.50); 3.9299 (0.75); 3.9072 (0.80); 3.5917 (1.29); 3.5745 (1.35); 3.5630 (1.94); 3.5458 (1.80); 3.4942 (0.52); 3.4332 (2.09); 3.4218 (2.74); 3.4132 (1.78); 3.4044 (1.58); 3.3982 (2.03); 3.3934 (1.89); 3.3910 (2.04); 3.3689 (0.40); 3.3624 (0.80); 3.3538 (0.90); 3.3505 (1.02); 3.3438 (1.54); 3.3373 (1.54); 3.3253 (0.86); 3.3158 (0.69); 3.3113 (0.55); 3.0978 (0.43); 3.0384 (1.85); 3.0233 (1.87); 3.0161 (1.73); 3.0060 (0.44); 3.0010 (1.67); 2.9467 (0.52); 2.9423 (0.58); 2.9234 (1.01); 2.9046 (0.57); 2.9003 (0.51); 2.2913 (0.69); 2.2707 (0.77); 2.2095 (0.70); 2.1874 (0.76); 2.0496 (2.20); 1.8875 (0.68); 1.8681 (0.68); 1.8393 (0.38); 1.8320 (0.38); 1.8186 (0.68); 1.8125 (0.74); 1.7973 (0.71); 1.7918 (0.67); 1.7781 (0.37); 1.7714 (0.36); 1.6383 (0.68); 1.2733 (0.66); 1.2615 (1.27); 1.2496 (0.77); 0.0052 (0.84); -0.0002 (26.19); -0.0057 (0.85) |

| **Bsp. I-64, Solvent: DMSO-d6** |
|---|
| 11.1389 (6.68); 10.2551 (0.65); 10.2533 (0.65); 8.1882 (4.93); 8.0408 (1.31); 8.0320 (7.82); 8.0274 (1.54); 7.6855 (0.40); 7.6765 (1.51); 7.6722 (1.64); 7.6657 (0.61); 7.6573 (1.67); 7.6530 (1.59); 7.3772 (0.78); 7.3728 (0.80); 7.3587 (1.10); 7.3549 (1.33); 7.3520 (1.19); 7.3379 (1.28); 7.3336 (1.13); 7.3221 (0.38); 7.3124 (1.56); 7.1791 (3.44); 7.1668 (1.79); 7.0953 (2.10); 7.0757 (1.69); 7.0459 (1.72); 7.0307 (4.22); 7.0118 (0.35); 6.9921 (1.10); 6.9734 (1.99); 6.9545 (1.24); 6.9070 (3.50); 6.8949 (2.01); 5.7547 (16.00); 5.4595 (0.67); 5.4167 (2.24); 5.3938 (0.33); 5.3760 (2.34); 5.3330 (0.72); 5.3255 (1.01); 5.1267 (0.32); 5.1136 (0.54); 5.1095 (0.50); 5.1043 (0.55); 5.0996 (0.71); 5.0868 (0.77); 5.0773 (0.56); 5.0693 (0.49); 5.0604 (0.37); 4.3595 (0.70); 4.3230 (0.86); 4.3028 (0.32); 4.2179 (0.61); 4.2091 (0.71); 4.1913 (1.49); 4.1826 (1.54); 4.1662 (1.45); 4.1531 (1.37); 4.1395 (0.66); 4.1263 (0.68); 4.0557 (0.53); 4.0380 (1.61); 4.0202 (1.64); 4.0023 (0.63); 3.9819 (0.68); 3.9485 (0.75); 3.6327 (0.79); 3.6051 (1.16); 3.5895 (1.28); 3.5677 (0.44); 3.5620 (1.03); 3.4196 (0.63); 3.4128 (0.56); 3.3860 (2.38); 3.3765 (1.19); 3.3686 (1.96); 3.3328 (509.45); 3.2708 (1.30); 3.2501 (0.53); 3.2407 (0.70); 3.2181 (0.34); 3.1622 (3.57); 3.1537 (0.71); 3.0588 (2.55); 3.0414 (0.38); 2.8746 (0.54); 2.8434 (0.96); 2.8169 (0.56); 2.6756 (0.38); 2.6710 (0.53); 2.6664 (0.38); 2.5243 (1.46); 2.5110 (30.40); 2.5065 (61.38); 2.5019 (82.11); 2.4973 (59.35); 2.4927 (28.92); 2.3333 (0.43); 2.3287 (0.57); 2.3240 (0.43); 2.1363 (0.60); 2.1018 (1.30); 2.0733 (2.32); 1.9885 (7.32); 1.8256 (0.55); 1.8189 (0.61); 1.7976 (0.59); 1.7882 (0.54); 1.5894 (0.55); 1.5797 (0.65); 1.5589 (0.58); 1.5502 (0.61); 1.1924 (2.11); 1.1746 (4.16); 1.1568 (2.08); 0.0080 (0.34); -0.0002 (9.00); -0.0085 (0.34) |

| **Bsp. I-65, Solvent: DMSO-d6** |
|---|
| 8.0235 (6.08); 7.5161 (6.69); 7.4958 (7.72); 7.2197 (2.12); 7.1995 (3.43); 7.1790 (1.64); 6.4994 (4.54); 5.7532 (5.60); 5.3529 (0.85); 5.3098 (2.42); 5.2560 (2.39); 5.2132 (0.82); 5.1175 (0.32); 5.1037 (0.57); 5.0972 (0.53); 5.0872 (0.84); 5.0758 (0.94); 5.0691 (0.59); 5.0600 (0.56); 5.0475 (0.36); 5.0357 (0.62); 4.3876 (0.77); 4.3546 (0.83); 4.1760 (0.39); 4.1659 (0.46); 4.1500 (2.75); 4.1449 (3.00); 4.1410 (2.73); 4.1321 (2.31); 4.1194 (0.37); 4.1070 (0.38); 4.0381 (0.73); 4.0203 (0.67); 3.9913 (0.72); 3.9583 (0.81); 3.6427 (0.87); 3.6147 (1.02); 3.5990 (1.75); 3.5713 (1.60); 3.5002 (1.71); 3.4816 (1.73); 3.4567 (1.21); 3.4387 (1.26); 3.4099 (1.51); 3.3647 (154.46); 3.3613 (151.04); 3.3506 (146.41); 3.3460 (167.37); 3.3384 (190.97); 3.2700 (1.29); 3.2409 (0.71); 3.0429 (0.41); 2.8687 (0.49); 2.8556 (0.59); 2.8396 (0.93); 2.8126 (0.54); 2.6715 (0.76); 2.5411 (0.36); 2.5029 (117.60); 2.3294 (0.69); 2.3248 (0.58); 2.2518 (1.14); 2.2090 (16.00); 2.1942 (0.76); 2.1333 (0.68); 2.1044 (1.41); 2.0750 (0.82); 1.9886 (2.71); 1.8122 (0.62); 1.7891 (0.61); 1.7812 (0.56); 1.5730 (0.62); 1.5523 (0.62); 1.2357 (0.41); 1.1927 (0.72); 1.1750 (1.38); 1.1567 (0.70); -0.0002 (5.76) |

| **Bsp. I-66, Solvent: DMSO-d6** |
|---|
| 7.9863 (5.17); 7.3935 (0.44); 7.3878 (0.40); 7.3726 (0.86); 7.3673 (0.34); 7.3574 (0.43); 7.3519 (0.52); 7.3083 (0.80); 7.1751 (1.79); 7.1651 (0.90); 7.0963 (1.41); 7.0755 (1.23); 7.0674 (1.49); 7.0542 (0.60); 7.0466 (1.27); 7.0421 (1.11); 7.0291 (2.11); 6.9051 (1.72); 6.8932 (1.00); 5.7543 (16.00); 5.4551 (0.38); 5.4126 (1.29); 5.3679 (1.24); 5.3250 (0.38); 5.3030 (0.69); 5.2057 (0.55); 5.1871 (0.65); 5.1774 (0.63); 5.1586 (0.57); 4.3630 (0.36); 4.3313 (0.39); 3.9762 (0.32); 3.9424 (0.37); 3.7010 (0.35); 3.6974 (0.46); 3.6883 (0.44); 3.6734 (0.62); 3.6635 (0.40); 3.6592 (0.41); 3.5681 (0.67); 3.4680 (0.44); 3.4393 (0.51); 3.4234 (0.68); 3.3947 (0.84); 3.3733 (0.42); 3.3641 (0.72); 3.3340 (192.46); 3.2813 (0.32); 3.2511 (0.48); 3.1774 (0.57); 3.1587 (0.55); 3.1336 (0.42); 3.1152 (0.41); 2.8495 (0.35); 2.8163 (0.44); 2.5199 (0.47); 2.5066 (21.11); 2.5024 (28.41); 2.4990 (19.28); 2.0860 (1.38); 2.0736 (0.57); 2.0489 (0.36); 1.9887 (0.35); 1.9092 (0.46); 1.4646 (3.61); 1.3933 (1.82); 1.3861 (3.35); 1.3792 (1.95); -0.0002 (0.87) |

| **Bsp. I-67, Solvent: DMSO-d6** |
|---|
| 7.9996 (6.17); 7.3639 (1.45); 7.3546 (4.84); 7.3472 (4.14); 7.3406 (2.05); 7.2496 (0.98); 7.2408 (0.84); 7.2334 (0.71); 7.2243 (0.87); 7.2141 (1.05); 7.2037 (0.81); 7.1998 (0.87); 7.1899 (0.65); 6.5076 (4.49); 5.5954 (1.09); 5.5779 (1.32); 5.5676 (1.27); 5.5489 (1.21); 5.3553 (0.89); 5.3132 (2.38); 5.2587 (2.26); 5.2158 (0.85); 4.3949 (0.73); 4.3639 (0.82); 4.0665 (0.50); 4.0488 (1.32); 4.0310 (1.33); 4.0133 (0.52); 3.9936 (0.65); 3.9611 (0.78); 3.4960 (0.48); 3.4786 (1.23); 3.4498 (1.59); 3.4340 (2.17); 3.3808 (422.45); 3.3742 (862.74); 3.3717 (915.50); 3.2940 (0.97); 3.2616 (1.06); 3.2334 (0.78); 3.1819 (1.21); 3.1641 (1.21); 3.1383 (0.94); 3.1201 (0.95); 2.9019 (0.59); 2.8594 (0.53); 2.8281 (0.90); 2.8013 (0.57); 2.7426 (0.46); 2.6882 (0.63); 2.6835 (0.94); 2.6792 (0.72); 2.5533 (0.44); 2.5371 (1.50); 2.5189 (104.35); 2.5147 (142.57); 2.5106 (100.47); 2.3505 (0.33); 2.3457 (0.70); 2.3410 (0.88); 2.3372 (0.66); 2.2153 (16.00); 2.1305 (0.68); 2.1268 (0.61); 2.0978 (1.37); 2.0827 (0.87); 2.0682 (0.79); 1.9993 (5.76); 1.9198 (0.58); 1.8119 (0.59); 1.7846 (0.52); 1.5908 (6.34); 1.5825 (6.37); 1.5408 (0.67); 1.5129 (6.00); 1.5018 (6.11); 1.2457 (0.44); 1.2034 (1.56); 1.1856 (2.96); 1.1680 (1.52) |

| **Bsp. I-68, Solvent: DMSO-d6** |
|---|
| 8.6287 (8.28); 7.9844 (14.62); 7.7299 (0.34); 7.7026 (4.24); 7.6822 (5.10); 7.5250 (6.60); 7.4978 (0.46); 7.4859 (4.04); 7.4659 (3.35); 7.4247 (0.51); 7.4076 (0.72); 7.3928 (1.78); 7.3878 (1.70); 7.3723 (3.25); 7.3562 (1.74); 7.3516 (2.06); 7.3360 (1.02); 7.2243 (2.65); 7.2076 (2.37); 7.0954 (5.58); 7.0847 (6.64); 7.0674 (8.64); 7.0458 (4.65); 6.9458 (2.81); 6.9329 (2.39); 5.8615 (0.33); 5.7541 (14.73); 5.6743 (0.34); 5.2049 (2.46); 5.1867 (2.82); 5.1767 (2.70); 5.1581 (2.53); 4.1729 (3.66); 4.1392 (3.96); 4.0559 (0.69); 4.0383 (2.08); 4.0204 (2.19); 4.0030 (0.88); 3.7222 (0.48); 3.5682 (1.52); 3.4697 (2.10); 3.4412 (2.39); 3.4254 (3.25); 3.3971 (3.40); 3.3402 (1408.83); 3.3179 (7.32); 3.2890 (1.69); 3.2433 (0.39); 3.2143 (0.40); 3.1824 (2.47); 3.1646 (2.51); 3.1393 (2.06); 3.1205 (1.95); 3.0504 (2.60); 3.0201 (4.88); 2.9910 (2.61); 2.6715 (1.54); 2.5417 (0.63); 2.5066 (179.46); 2.5026 (243.49); 2.3289 (1.58); 2.3254 (1.32); 2.2121 (0.51); 2.0994 (3.03); 2.0918 (3.21); 2.0730 (3.38); 2.0654 (3.74); 1.9887 (8.88); 1.7063 (1.21); 1.6764 (2.86); 1.6468 (2.87); 1.6152 (1.13); 1.4655 (16.00); 1.4358 (0.43); 1.3877 (14.92); 1.2345 (0.61); 1.1926 (2.38); 1.1748 (4.75); 1.1570 (2.38); -0.0002 (3.15); -2.6302 (0.34) |

| **Bsp. I-69, Solvent: DMSO-d6** |
|---|
| 9.6020 (0.42); 7.9924 (11.69); 7.3735 (0.34); 7.3537 (2.03); 7.3442 (6.69); 7.3369 (5.57); 7.3300 (2.80); 7.3253 (0.89); 7.3088 (1.99); 7.2398 (1.39); 7.2317 (1.14); 7.2236 (1.07); 7.2151 (1.32); 7.2044 (1.47); 7.1941 (1.25); 7.1906 (1.24); 7.1799 (1.47); 7.1754 (4.30); 7.1655 (2.05); 7.0422 (2.03); 7.0294 (4.76); 6.9056 (4.03); 6.8936 (2.31); 5.7544 (16.00); 5.5862 (1.37); 5.5674 (1.63); 5.5579 (1.56); 5.5388 (1.45); 5.4553 (0.89); 5.4128 (3.04); 5.3983 (0.68); 5.3685 (2.96); 5.3262 (0.93); 4.3651 (0.87); 4.3314 (0.93); 4.0383 (0.76); 4.0204 (0.73); 3.9777 (0.78); 3.9430 (0.87); 3.6970 (0.91); 3.5680 (0.38); 3.4688 (1.18); 3.4396 (1.31); 3.4241 (1.67); 3.3957 (2.11); 3.3755 (1.00); 3.3661 (1.65); 3.3569 (1.61); 3.3348 (500.06); 3.2834 (0.79); 3.2522 (1.18); 3.2240 (0.67); 3.1729 (1.42); 3.1541 (1.44); 3.1285 (1.09); 3.1099 (1.10); 2.8498 (0.68); 2.8216 (1.06); 2.7926 (0.63); 2.6761 (0.33); 2.6715 (0.45); 2.6671 (0.33); 2.5245 (0.72); 2.5066 (52.78); 2.5026 (69.97); 2.3292 (0.48); 2.3246 (0.35); 2.1213 (0.80); 2.0875 (1.54); 2.0739 (1.22); 2.0538 (0.86); 1.9887 (3.27); 1.9094 (0.54); 1.8010 (0.65); 1.7693 (0.65); 1.7434 (0.34); 1.5818 (7.86); 1.5735 (8.33); 1.5319 (0.79); 1.5031 (7.64); 1.4920 (7.64); 1.2346 (0.32); 1.1926 (0.83); 1.1748 (1.74); 1.1571 (0.86); -0.0002 (1.53) |

| **Bsp. I-70, Solvent: DMSO-d6** |
|---|
| 8.0094 (9.59); 7.4134 (1.85); 7.4122 (1.87); 7.4004 (2.20); 7.2742 (1.24); 7.2293 (1.56); 7.2171 (2.76); 7.2063 (1.86); 7.1934 (1.13); 7.1919 (0.99); 7.1856 (2.98); 7.1442 (1.48); 7.1424 (1.50); 7.1303 (3.35); 7.1196 (0.87); 7.1178 (0.82); 7.0971 (1.42); 7.0400 (3.48); 6.9497 (1.57); 6.9161 (3.14); 5.7632 (7.74); 5.4588 (1.14); 5.4304 (2.43); 5.3775 (2.40); 5.3491 (1.13); 4.8549 (0.35); 4.8439 (0.91); 4.8374 (0.49); 4.8337 (0.94); 4.8267 (0.93); 4.8229 (0.50); 4.8164 (0.88); 4.8054 (0.37); 4.3611 (0.73); 4.3390 (0.75); 3.9770 (0.68); 3.9542 (0.72); 3.5839 (1.24); 3.5665 (1.47); 3.5553 (1.70); 3.5380 (1.41); 3.4073 (0.35); 3.4011 (0.63); 3.3944 (0.49); 3.3883 (0.96); 3.3821 (1.40); 3.3756 (1.72); 3.3555 (379.88); 3.3320 (1.56); 3.3082 (1.78); 3.2964 (2.72); 3.2862 (1.81); 3.2796 (1.75); 3.2734 (2.25); 3.2680 (2.31); 3.2638 (2.89); 3.2448 (0.59); 3.2408 (0.50); 3.2079 (1.79); 3.1969 (1.83); 3.1854 (1.23); 3.1744 (1.19); 2.8570 (0.45); 2.8529 (0.53); 2.8352 (0.91); 2.8323 (0.93); 2.8150 (0.54); 2.8110 (0.45); 2.6184 (0.35); 2.6153 (0.48); 2.6123 (0.33); 2.5246 (0.98); 2.5215 (1.29); 2.5184 (1.48); 2.5096 (26.12); 2.5066 (55.05); 2.5036 (74.81); 2.5005 (54.16); 2.4976 (24.50); 2.3907 (0.36); 2.3878 (0.48); 2.3847 (0.36); 2.3048 (16.00); 2.1224 (0.63); 2.1028 (0.76); 2.0835 (0.72); 2.0776 (4.16); 2.0641 (0.70); 1.9904 (0.71); 1.8085 (0.59); 1.8025 (0.65); 1.7878 (0.62); 1.7821 (0.58); 1.5685 (0.60); 1.5621 (0.65); 1.5481 (0.63); 1.5419 (0.59); 1.2337 (0.33); 1.1743 (0.41); 0.0053 (0.46); -0.0002 (13.65); -0.0057 (0.41) |

| **Bsp. I-71, Solvent: DMSO-d6** |
|---|
| 9.9015 (0.55); 8.6461 (0.49); 8.0101 (16.00); 7.4198 (3.98); 7.4159 (4.30); 7.4001 (4.46); 7.3962 (4.50); 7.3217 (1.97); 7.3177 (1.97); 7.3090 (3.29); 7.3010 (3.77); 7.2826 (2.96); 7.2785 (2.72); 7.2738 (0.91); 7.1906 (4.81); 7.1873 (5.06); 7.1756 (7.08); 7.1699 (4.28); 7.1665 (3.76); 7.1592 (3.36); 7.1404 (0.40); 7.0424 (3.25); 7.0362 (1.07); 7.0231 (7.12); 6.9906 (2.62); 6.9872 (2.61); 6.9716 (4.10); 6.9684 (3.99); 6.9524 (2.30); 6.9490 (2.11); 6.9008 (6.67); 6.8872 (3.58); 5.7453 (13.54); 5.4561 (1.33); 5.4129 (4.75); 5.3684 (4.65); 5.3258 (1.31); 5.1117 (0.74); 5.0899 (1.87); 5.0851 (2.12); 5.0807 (1.61); 5.0711 (1.88); 5.0623 (1.17); 5.0576 (1.05); 5.0532 (1.01); 5.0440 (0.72); 4.3620 (1.52); 4.3307 (1.61); 4.2639 (1.74); 4.2550 (1.80); 4.2369 (3.94); 4.2280 (3.71); 4.2047 (3.83); 4.1911 (3.77); 4.1777 (1.85); 4.1641 (1.72); 4.0571 (0.37); 4.0393 (0.89); 4.0216 (0.90); 4.0041 (0.62); 3.9847 (1.48); 3.9510 (1.56); 3.6204 (2.35); 3.5929 (2.62); 3.5774 (3.65); 3.5681 (0.82); 3.5501 (3.18); 3.5101 (0.52); 3.4749 (0.71); 3.4183 (2.62); 3.4092 (5.43); 3.3998 (3.60); 3.3909 (7.47); 3.3812 (4.39); 3.3657 (7.73); 3.3212 (555.51); 3.2467 (1.87); 2.8779 (1.03); 2.8506 (1.89); 2.8207 (1.00); 2.6753 (0.46); 2.6705 (0.59); 2.6660 (0.49); 2.5401 (0.96); 2.5100 (35.91); 2.5057 (65.72); 2.5013 (84.54); 2.4969 (58.55); 2.4926 (28.03); 2.3327 (0.37); 2.3280 (0.52); 2.3233 (0.37); 2.1394 (1.35); 2.1044 (2.69); 2.0690 (1.65); 1.9870 (3.13); 1.8441 (0.58); 1.8220 (1.16); 1.8140 (1.22); 1.7916 (1.10); 1.7853 (1.05); 1.7609 (0.45); 1.6114 (0.68); 1.5895 (1.16); 1.5819 (1.27); 1.5604 (1.11); 1.5530 (1.07); 1.5310 (0.45); 1.5219 (0.36); 1.2372 (0.57); 1.1929 (0.94); 1.1752 (1.78); 1.1573 (0.88); 0.0078 (0.38); -0.0002 (7.13) |

| **Bsp. I-72, Solvent: DMSO-d6** |
|---|
| 10.3781 (4.04); 10.3763 (4.00); 8.0115 (6.15); 7.4096 (1.22); 7.4055 (1.34); 7.3896 (1.53); 7.3855 (1.59); 7.3118 (1.00); 7.2981 (1.16); 7.2939 (1.32); 7.2785 (2.40); 7.2743 (1.99); 7.2396 (1.42); 7.2379 (1.42); 7.2198 (1.83); 7.2181 (1.86); 7.2001 (0.74); 7.1983 (0.79); 7.1785 (2.29); 7.1667 (1.12); 7.0453 (1.13); 7.0307 (2.60); 6.9065 (2.24); 6.8949 (1.29); 5.7551 (10.32); 5.4587 (0.50); 5.4159 (1.69); 5.3731 (1.72); 5.3306 (0.50); 5.0836 (0.34); 5.0797 (0.38); 5.0706 (0.56); 5.0650 (0.48); 5.0559 (0.62); 5.0466 (0.38); 5.0374 (0.32); 4.3625 (0.52); 4.3297 (0.55); 4.3011 (0.47); 4.2919 (0.54); 4.2736 (1.56); 4.2643 (1.58); 4.2582 (1.60); 4.2434 (1.42); 4.2306 (0.49); 4.2158 (0.49); 4.0379 (0.34); 4.0201 (0.33); 3.9833 (0.49); 3.9483 (0.53); 3.8683 (16.00); 3.8339 (0.70); 3.5966 (0.71); 3.5689 (0.90); 3.5534 (1.20); 3.5259 (0.96); 3.4139 (0.62); 3.4005 (1.49); 3.3939 (0.89); 3.3821 (1.82); 3.3749 (1.23); 3.3563 (2.86); 3.3306 (450.97); 3.3073 (2.93); 3.2938 (0.97); 3.2701 (0.73); 3.2386 (0.40); 3.2310 (0.36); 2.8690 (0.35); 2.8409 (0.63); 2.8112 (0.35); 2.6755 (0.42); 2.6708 (0.59); 2.6663 (0.41); 2.5410 (0.40); 2.5240 (1.66); 2.5108 (33.29); 2.5063 (66.94); 2.5017 (89.03); 2.4972 (64.16); 2.4926 (31.01); 2.3330 (0.41); 2.3286 (0.58); 2.3240 (0.41); 2.1345 (0.44); 2.1001 (0.91); 2.0735 (1.54); 2.0645 (0.53); 1.9885 (1.39); 1.8152 (0.40); 1.8074 (0.43); 1.7844 (0.40); 1.7771 (0.37); 1.5826 (0.40); 1.5735 (0.43); 1.5516 (0.40); 1.5427 (0.37); 1.1924 (0.40); 1.1745 (0.79); 1.1567 (0.40); 0.0080 (0.33); -0.0002 (8.61) |

| **Bsp. I-73, Solvent: DMSO-d6** |
|---|
| 10.2509 (8.57); 8.0336 (13.13); 7.6869 (2.35); 7.6841 (3.68); 7.6793 (2.30); 7.6742 (2.59); 7.6714 (3.91); 7.6670 (2.76); 7.6655 (2.61); 7.6625 (1.79); 7.6533 (2.07); 7.6502 (1.55); 7.2722 (2.19); 7.2667 (4.06); 7.2528 (3.78); 7.1836 (4.45); 7.1273 (2.04); 7.1112 (2.20); 7.0988 (4.10); 7.0953 (2.48); 7.0864 (2.06); 7.0369 (5.02); 6.9465 (2.32); 6.9139 (5.17); 5.7604 (16.00); 5.4571 (1.70); 5.4286 (3.70); 5.3769 (3.63); 5.3485 (1.69); 5.1617 (0.56); 5.1537 (0.81); 5.1507 (0.84); 5.1478 (0.76); 5.1431 (1.28); 5.1367 (1.25); 5.1324 (0.76); 5.1295 (0.81); 5.1264 (0.76); 5.1244 (0.76); 5.1183 (0.55); 4.3608 (1.13); 4.3407 (2.45); 4.3351 (2.29); 4.3230 (3.02); 4.3173 (2.75); 4.2973 (2.82); 4.2890 (2.86); 4.2795 (1.51); 4.2713 (1.38); 4.0461 (0.67); 4.0343 (2.07); 4.0224 (2.06); 4.0106 (0.70); 3.9803 (1.06); 3.9575 (1.14); 3.6365 (1.68); 3.6181 (1.96); 3.6078 (2.55); 3.5895 (2.13); 3.4563 (2.40); 3.4451 (2.48); 3.4276 (2.06); 3.4164 (2.45); 3.4075 (1.05); 3.4010 (1.60); 3.3948 (2.58); 3.3870 (2.47); 3.3605 (693.55); 3.3372 (7.93); 3.3168 (0.37); 3.2911 (0.90); 3.2713 (1.54); 3.2522 (0.88); 2.8621 (0.82); 2.8418 (1.49); 2.8244 (0.87); 2.6188 (0.41); 2.6158 (0.56); 2.6129 (0.40); 2.5658 (1.65); 2.5250 (0.92); 2.5219 (1.20); 2.5187 (1.51); 2.5099 (31.29); 2.5070 (65.04); 2.5040 (88.14); 2.5010 (63.51); 2.4981 (29.07); 2.3913 (0.39); 2.3882 (0.55); 2.3852 (0.37); 2.1353 (0.97); 2.1152 (1.17); 2.0955 (1.12); 2.0765 (4.57); 1.9901 (8.99); 1.8413 (0.36); 1.8347 (0.44); 1.8211 (0.89); 1.8149 (0.98); 1.8008 (0.92); 1.7947 (0.87); 1.7805 (0.39); 1.6049 (0.39); 1.5981 (0.46); 1.5844 (0.91); 1.5780 (1.01); 1.5639 (0.98); 1.5577 (0.93); 1.5440 (0.41); 1.5372 (0.35); 1.1864 (2.37); 1.1745 (4.71); 1.1627 (2.35); -0.0002 (1.38) |

| **Bsp. I-74, Solvent: DMSO-d6** |
|---|
| 7.9998 (16.00); 7.3340 (2.20); 7.3303 (3.55); 7.3265 (3.65); 7.3209 (1.78); 7.3137 (2.97); 7.3073 (6.46); 7.3031 (4.55); 7.2976 (2.42); 7.2936 (1.93); 7.2763 (2.71); 7.2724 (2.08); 7.1813 (3.15); 7.1745 (6.21); 7.1673 (3.40); 7.1594 (4.91); 7.1467 (3.48); 7.1399 (1.63); 7.1259 (1.43); 7.0415 (2.90); 7.0229 (6.59); 6.9002 (5.66); 6.8870 (3.22); 5.7459 (14.51); 5.4534 (1.22); 5.4109 (4.33); 5.3665 (4.18); 5.3244 (1.21); 5.0584 (0.67); 5.0446 (0.95); 5.0303 (1.48); 5.0255 (1.25); 5.0167 (1.58); 5.0025 (0.92); 4.9896 (0.71); 4.3622 (1.36); 4.3290 (1.46); 4.2876 (1.66); 4.2788 (1.64); 4.2600 (2.92); 4.2523 (2.73); 4.2076 (2.80); 4.1934 (2.82); 4.1796 (1.65); 4.1669 (1.56); 4.0390 (0.39); 4.0211 (0.45); 3.9842 (1.35); 3.9492 (1.39); 3.6751 (0.34); 3.6494 (0.44); 3.6353 (0.44); 3.6138 (2.25); 3.5860 (2.48); 3.5707 (3.70); 3.5431 (3.28); 3.5300 (0.75); 3.5133 (0.76); 3.4407 (4.22); 3.4217 (4.90); 3.3972 (5.11); 3.3860 (4.90); 3.3783 (6.05); 3.3152 (2722.35); 3.2720 (4.76); 3.2435 (1.69); 3.2236 (0.75); 2.8810 (0.88); 2.8753 (0.94); 2.8441 (1.63); 2.8172 (0.97); 2.6745 (0.99); 2.6700 (1.51); 2.6654 (1.10); 2.5398 (1.99); 2.5096 (85.73); 2.5053 (161.11); 2.5008 (211.76); 2.4964 (147.17); 2.4919 (70.94); 2.3322 (1.04); 2.3275 (1.50); 2.3227 (0.99); 2.1368 (1.17); 2.1045 (2.42); 2.0690 (5.38); 1.9867 (1.44); 1.8375 (0.50); 1.8088 (1.08); 1.7850 (1.02); 1.7582 (0.39); 1.6188 (0.46); 1.6072 (0.54); 1.5873 (1.09); 1.5785 (1.18); 1.5568 (1.02); 1.5250 (0.44); 1.5173 (0.44); 1.3521 (1.60); 1.2986 (0.48); 1.2590 (0.70); 1.2362 (1.76); 1.2305 (1.74); 1.1926 (0.42); 1.1750 (0.84); 1.1572 (0.40); 0.0079 (0.88); -0.0002 (21.43); -0.0085 (1.03) |

| **Bsp. I-75, Solvent: DMSO-d6** |
|---|
| 8.0148 (14.18); 7.5454 (3.29); 7.5394 (3.42); 7.5182 (3.28); 7.5122 (3.46); 7.3537 (1.82); 7.3496 (2.18); 7.3444 (1.69); 7.3316 (2.48); 7.3277 (2.93); 7.3222 (2.33); 7.3087 (2.53); 7.2102 (3.28); 7.1877 (5.48); 7.1754 (5.80); 7.1654 (2.98); 7.1596 (2.94); 7.0421 (2.71); 7.0234 (6.08); 6.9000 (5.45); 6.8875 (3.13); 5.7469 (7.15); 5.4528 (1.11); 5.4111 (4.13); 5.3688 (4.11); 5.3262 (1.11); 5.0919 (0.58); 5.0774 (0.97); 5.0740 (0.94); 5.0644 (1.47); 5.0592 (1.25); 5.0551 (1.08); 5.0500 (1.61); 5.0404 (1.01); 5.0323 (0.85); 5.0223 (0.66); 4.3640 (1.29); 4.3313 (1.36); 4.2484 (0.86); 4.2387 (1.12); 4.2211 (4.00); 4.2106 (6.55); 4.1953 (3.63); 4.1829 (1.04); 4.1684 (0.99); 4.0568 (1.22); 4.0390 (3.71); 4.0213 (3.78); 4.0034 (1.50); 3.9847 (1.28); 3.9495 (1.33); 3.6105 (1.88); 3.5831 (2.15); 3.5673 (2.88); 3.5400 (2.37); 3.4158 (1.24); 3.4058 (1.06); 3.3964 (1.59); 3.3872 (2.49); 3.3778 (1.88); 3.3677 (1.83); 3.3584 (2.56); 3.3409 (5.73); 3.3040 (1216.35); 3.2802 (16.23); 3.2429 (2.24); 3.1765 (0.60); 3.1632 (0.48); 2.8739 (0.96); 2.8447 (1.67); 2.8168 (0.95); 2.6737 (0.95); 2.6691 (1.25); 2.6645 (0.94); 2.5391 (1.66); 2.5087 (68.90); 2.5044 (127.36); 2.5000 (165.63); 2.4956 (117.33); 2.4913 (58.43); 2.3312 (0.98); 2.3267 (1.27); 2.3221 (0.96); 2.1379 (1.23); 2.1042 (2.51); 2.0693 (4.98); 1.9867 (16.00); 1.9076 (0.33); 1.8510 (0.47); 1.8414 (0.57); 1.8199 (1.08); 1.8128 (1.13); 1.7892 (1.08); 1.7824 (1.01); 1.7607 (0.50); 1.6198 (0.48); 1.6103 (0.59); 1.5873 (1.08); 1.5797 (1.13); 1.5589 (1.06); 1.5493 (1.03); 1.5292 (0.49); 1.4089 (0.88); 1.2363 (0.91); 1.1927 (4.44); 1.1749 (8.68); 1.1571 (4.30); 0.0079 (1.04); -0.0002 (19.61); -0.0084 (0.98) |

### NMR-Daten von Zwischenprodukten

| **XVIII-1, Solvent: DMSO-d₆** |
|---|
| 11,3471; (1,52); 8,172; (1,42); 7,9592; (1,36); 7,8249; (0,34); 4,3287; (0,42); 3,8907; (0,38); 3,4588; (0,32); 3,4461; (0,33); 3,4413; (0,34); 3,4286; (0,32); 3,3175; (28,88); 2,5112; (1,77); 2,5069; (3,23); 2,5024; (4,14); 2,498; (2,82); 2,4937; (1,34); 2,1258; (0,65); 2,1156; (0,51); 2,08; (0,61); 2,0703; (0,43); 1,4232; (15); 1,0757; (0,76); 1,0582; (1,49); 1,0408; (0,73); -0,0001; (0,44) |

| **XVIIa-1, Solvent: DMSO-d₆** |
|---|
| 8,1017; (1,85); 7,4021; (1,98); 7,3911; (3,22); 4,3294; (0,68); 4,3132; (0,65); 3,3029; (160,66); 3,2792; (0,64); 3,2322; (0,4); 3,2048; (0,39); 3,1655; (5,27); 3,0649; (0,35); 3,0441; (0,33); 2,5224; (0,6); 2,5177; (0,89); 2,509; (11,67); 2,5046; (22,95); 2,5001; (31,05); 2,4956; (21,24); 2,4911; (9,97); 2,1179; (0,36); 2,1066; (0,54); 2,0963; (0,44); 2,0694; (0,39); 2,0619; (0,39); 1,4187; (16); 1,4084; (2,51); 1,3985; (0,52); -0,0001; (2,86) |

| **XIII-1, Solvent: DMSO-d₆** |
|---|
| 8,7012; (0,41); 8,6788; (0,46); 8,2106; (0,39); 7,8892; (0,36); 7,8851; (0,71); 7,3969; (0,55); 7,3945; (0,46); 3,5678; (16); 3,3917; (0,44); 3,3857; (0,5); 3,352; (32,76); 3,2112; (1,19); 3,1689; (1,13); 2,5241; (0,36); 2,5194; (0,51); 2,5107; (4,97); 2,5063; (9,71); 2,5017; (13,23); 2,4973; (9,21); 2,4928; (4,44); -0,0001; (0,55) |

| **XIX-1, Solvent: DMSO-d₆** |
|---|
| 8,0704 (2,17) 8,0602 (0,91) 8,0576 (1,02) 8,0398 (1,13) 8,0364 (0,91) 7,7159 (0,59) 7,6974 (0,42) 7,6169 (0,8) 7,5972 (1,14) 7,5786 (0,53) 6,2127 (0,4) 6,1939 (0,44) 6,1859 (0,55) 6,167 (0,41) 4,0405 (0,93) 4,0227 (1,12) 4,0048 (0,54) 3,9945 (0,4) 3,7686 (0,75) 3,7607 (0,71) 3,7499 (0,66) 3,7338 (0,66) 3,3119 (20,55) 3,2882 (0,36) 3,2489 (0,36) 2,5113 (1,65) 2,507 (2,94) 2,5026 (3,72) 2,4982 (2,58) 2,494 (1,25) 2,0498 (0,41) 2,0447 (0,43) 2,0183 (0,47) 1,9881 (3,35) 1,5754 (0,38) 1,5657 (0,46) 1,5447 (0,37) 1,5355 (0,35) 1,4071 (16) 1,1939 (0,94) 1,1761 (1,85) 1,1583 (0,91) |

| **XVIIa-2, Solvent: DMSO-d₆** |
|---|
| 8,047; (2,03); 7,6197; (0,48); 7,6157; (0,57); 7,6024; (0,75); 7,5963; (0,84); 7,5539; (0,36); 7,5423; (1,46); 7,5258; (0,64); 4,0577; (0,47); 4,0399; (1,41); 4,0221; (1,66); 4,0044; (0,66); 3,9887; (0,37); 3,6486; (0,44); 3,6199; (0,38); 3,4795; (0,42); 3,4634; (0,42); 3,3124; (43,45); 3,2894; (0,33); 3,2336; (0,34); 2,5103; (2,76); 2,5061; (4,94); 2,5016; (6,28); 2,4973; (4,35); 2,493; (2,1); 2,0378; (0,39); 2,0326; (0,39); 2,0056; (0,48); 1,995; (0,53); 1,9873; (5,79); 1,5619; (0,41); 1,5512; (0,39); 1,5299; (0,37); 1,521; (0,33); 1,4071; (16); 1,1933; (1,6); 1,1755; (3,14); 1,1577; (1,55) |

| **XIII-2, Solvent: DMSO-d₆** |
|---|
| 8,0948 (1,15) 7,6043 (0,41) 7,5982 (0,46) 7,5462 (0,81) 7,5295 (0,35) 3,7555 (4,16) 3,7106 (0,32) 3,7027 (0,34) 3,568 (16) 2,5115 (2,77) 2,5072 (4,96) 2,5028 (6,33) 2,4984 (4,4) 2,4941 (2,13) |

| **XVIIa-3, Solvent: DMSO-d₆** |
|---|
| 7,9965; (1,95); 7,3093; (0,61); 7,29; (0,89); 7,2874; (0,69); 7,2691; (0,73); 6,9635; (1,28); 6,9566; (0,44); 6,9478; (1,02); 6,9422; (0,92); 6,94; (0,86); 4,1172; (1); 4,1073; (0,72); 4,1023; (0,68); 4,022; (0,38); 3,9952; (0,37); 3,5547; (0,42); 3,5274; (0,36); 3,3355; (0,7); 3,3131; (24,98); 3,2925; (0,59); 3,2736; (0,45); 3,2505; (0,35); 2,5097; (3); 2,5054; (5,43); 2,5009; (6,96); 2,4965; (4,84); 2,4922; (2,32); 2,0521; (0,41); 2,0467; (0,42); 2,0204; (0,47); 1,5784; (0,37); 1,5699; (0,43); 1,5484; (0,38); 1,5402; (0,36); 1,4092; (16); 1,398; (2,76) |

| **XX-1, Solvent: DMSO-d₆** |
|---|
| 7,9956; (1,6); 4,0236; (0,34); 3,9917; (0,35); 3,7017; (0,71); 3,697; (0,71); 3,6886; (0,72); 3,6843; (0,68); 3,5531; (0,4); 3,5262; (0,35); 3,3051; (109,24); 3,2639; (0,68); 3,253; (0,35); 3,2465; (0,58); 3,2203; (0,38); 3,203; (0,34); 2,5393; (0,34); 2,509; (10,79); 2,5046; (19,36); 2,5002; (24,75); 2,4958; (17,13); 2,4914; (8,19); 2,0463; (0,39); 2,0141; (0,44); 1,5714; (0,38); 1,5621; (0,42); 1,5401; (0,37); 1,5313; (0,34); 1,4276; (0,48); 1,4085; (16); 1,3932; (0,48); -0,0002; (3,27) |

| **VII-1, Solvent: DMSO-d₆** |
|---|
| 9,9015; (0,41); 8,7771; (0,43); 8,6466; (0,37); 8,4922; (1,2); 8,0162; (16); 7,3089; (3,15); 7,1757; (7,16); 7,1598; (3,62); 7,0423; (3,54); 7,0239; (7,95); 6,9004; (7,3); 6,888; (4,36); 5,7464; (9,68); 5,454; (1,5); 5,4111; (5,43); 5,3686; (5,57); 5,3259; (1,5); 5,3071; (0,57); 5,2908; (0,33); 5,0077; (0,76); 4,9949; (1,71); 4,9906; (1,21); 4,981; (1,79); 4,9778; (2,27); 4,968; (2,21); 4,955; (1,25); 4,9508; (1,82); 4,9381; (0,82); 4,7915; (0,65); 4,7778; (0,7); 4,3649; (1,66); 4,3327; (1,77); 4,0571; (0,76); 4,0392; (2,28); 4,0213; (2,36); 4,0035; (1,07); 3,9843; (1,6); 3,9502; (1,76); 3,7346; (0,64); 3,7215; (0,79); 3,708; (6,91); 3,7035; (7,1); 3,6949; (7,19); 3,6909; (6,95); 3,6768; (0,91); 3,6643; (0,62); 3,6075; (2,66); 3,5805; (2,87); 3,5639; (3,72); 3,5369; (3,21); 3,4118; (1,38); 3,4015; (1,19); 3,3924; (1,88); 3,3832; (2,9); 3,3737; (2); 3,3637; (1,89); 3,354; (2,74); 3,3079; (1047,19); 3,2847; (11,75); 3,2757; (9,27); 3,2584; (6,42); 3,232; (4,78); 3,2147; (3,72); 3,1537; (0,53); 3,1047; (0,36); 3,0888; (0,33); 3,0369; (1,23); 2,8709; (1,21); 2,849; (2,15); 2,8415; (2,17); 2,8137; (1,21); 2,6738; (0,91); 2,6693; (1,25); 2,6649; (0,95); 2,5393; (2,24); 2,509; (74,52); 2,5047; (135,64); 2,5003; (174,36); 2,496; (122,78); 2,4917; (60,93); 2,3314; (1,08); 2,3271; (1,38); 2,3225; (1,07); 2,1336; (1,71); 2,0999; (3,31); 2,0691; (3,17); 2,0389; (0,35); 1,9868; (10,09); 1,9079; (0,39); 1,8398; (0,79); 1,819; (1,48); 1,81; (1,51); 1,7884; (1,43); 1,7789; (1,3); 1,7589; (0,64); 1,6063; (0,77); 1,5765; (1,53); 1,5548; (1,4); 1,5467; (1,34); 1,526; (0,6); 1,3986; (0,46); 1,2746; (0,44); 1,2375; (0,57); 1,1927; (2,82); 1,1749; (5,44); 1,1571; (2,73); -0,0002; (8,76); -0,0084; (0,49) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

In der Tabelle sind alle NMR-Daten sowohl zu den Endverbindungen wie auch zu den Zwischenprodukten aufgeführt

### Verwendungsbeispiele

### Beispiel A

### Phytophthora-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100% rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen und nicht erfindungsgemäßen Verbindungen (mit * gekennzeichnet) bei einer Konzentration an Wirkstoff von 100ppm einen Wirkungsgrad von 70% oder mehr.

| **Bsp.** | **Eff.%** |
|---|---|
| I-1* | 100 |
| I-2* | 100 |
| I-3* | 100 |
| I-4 | 100 |
| I-5 | 100 |
| I-6* | 100 |
| I-7* | 100 |
| I-8 | 100 |
| I-9 | 100 |
| I-10* | 100 |
| I-11 | 95 |
| I-12* | 100 |
| I-13* | 100 |
| I-14* | 95 |
| I-15 | 95 |
| I-16 | 100 |
| I-17 | 95 |
| I-18 | 95 |
| I-19 | 100 |
| I-20 | 95 |
| I-21 | 95 |
| I-22 | 95 |
| I-23* | 95 |
| I-24 | 100 |
| I-25 | 100 |
| I-26* | 100 |
| I-27 | 90 |
| I-28 | 100 |
| I-29* | 100 |
| I-30 | 95 |
| I-31 | 100 |
| I-32 | 94 |
| I-33* | 100 |
| I-34* | 100 |
| I-35 | 95 |
| I-36* | 100 |
| I-37* | 100 |
| I-38* | 100 |
| I-39 | 100 |
| I-40* | 100 |
| I-41* | 100 |
| I-42 | 95 |
| I-43* | 95 |
| I-44* | 95 |
| I-45* | 93 |
| I-46* | 95 |
| I-47 | 93 |
| I-49 | 97 |
| I-50* | 92 |
| I-51* | 95 |
| I-52 | 95 |
| I-53 | 91 |
| I-54* | 100 |
| I-55* | 94 |
| I-56* | 94 |
| I-57 | 95 |
| I-58* | 94 |
| I-59* | 94 |
| I-60* | 94 |
| I-61* | 91 |
| I-62 | 91 |
| I-63* | 91 |
| I-64* | 95 |
| I-65* | 84 |
| I-66 | 94 |
| I-67 | 94 |
| I-68 | 94 |
| I-69 | 94 |
| I-70* | 95 |
| I-71* | 100 |
| I-72* | 95 |
| I-73* | 90 |
| I-74* | 100 |

### Beispiel B

### Plasmopara-Test (Rebe) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen und nicht erfindungsgemäßen Verbindungen (mit * gekennzeichnet) bei einer Konzentration an Wirkstoff von 10ppm einen Wirkungsgrad von 70% oder mehr.

| **Bsp.** | **Eff.%** |
|---|---|
| I-1* | 100 |
| I-2* | 97 |
| I-3* | 100 |
| I-4 | 97 |
| I-5 | 100 |
| I-6* | 96 |
| I-7* | 96 |
| I-8 | 93 |
| I-9 | 100 |
| I-10* | 93 |
| I-12* | 70 |
| I-16 | 87 |
| I-17 | 100 |
| I-18 | 100 |
| I-19 | 94 |
| I-20 | 93 |
| I-21 | 85 |
| I-22 | 100 |
| I-23* | 89 |
| I-24 | 94 |
| I-25 | 94 |
| I-26* | 96 |
| I-27 | 92 |
| I-28 | 98 |
| I-29* | 78 |
| I-30 | 100 |
| I-31 | 85 |
| I-32 | 78 |
| I-34* | 96 |
| I-35 | 100 |
| I-36* | 100 |
| I-37* | 97 |
| I-39 | 78 |
| I-40* | 92 |
| I-41* | 94 |
| I-42 | 100 |
| I-43* | 100 |
| I-44* | 100 |
| I-45* | 100 |
| I-46* | 98 |
| I-47 | 95 |
| I-48* | 96 |
| I-50* | 97 |
| I-51* | 93 |
| I-54* | 97 |
| I-55* | 100 |
| I-56* | 100 |
| I-57 | 100 |
| I-58* | 100 |
| I-59* | 100 |
| I-61* | 93 |
| I-62 | 96 |
| I-63* | 96 |
| I-64* | 73 |
| I-65* | 100 |
| I-66 | 100 |
| I-67 | 100 |
| I-68 | 96 |
| I-69 | 95 |
| I-71* | 100 |
| I-72* | 81 |
| I-74* | 100 |
| I-75* | 98 |

### Beispiel C

### Peronospora Test (Rapsölsamen) / Saatgutbehandlung

Der Test wurde unter Gewächshaus-Bedingungen durchgeführt.

Rapsölsamen behandelt mit einer erfindungsgemäßen aktiven Verbindung bzw. einer Kombination von erfindungsgemäßen aktiven Verbindungen gelöst in N-methyl-2-pyrrolidon und verdünnt mit Wasser zu der gewünschten Dosis wurden in 6*6cm grosse Gefäße ausgesät. Die Gefäße enthalten 4 cm einer 1:1 Mischung aus dampfbehandelter Felderde und Sand. Die Pflanzen wuchsen bei 10°C.

14 Tage alte Pflanzen wurden mit einen wässrigen Sporensuspension von ***Peronospora brassicae*** inokuliert. Die Gefäße mit den Pflanzen wurden im Gewächshaus 7 Tage bei einer Temperatur von 15°C und einer relativen Luftfeuchte von 100% inkubiert.

Die Beurteilung des Tests beinhaltete die Evaluation der infizierten Blattfläche pro Pflanze. 0% bedeutet dabei eine Effizienz die derjenigen der Kontrolle entspricht, währenddem eine Effizienz von 100% aussagt, dass keine Krankheit beobachtet wird.

In diesem Test zeigten folgende Verbindungen eine Effizienz von 70% und höher bei einer Dosis von 50 g/dt der aktiven erfindungsgemäßen Verbindung bzw. nicht erfindungsgemäßen Verbindung (mit * gekennzeichnet).

| **Bsp.** | **Eff.%** |
|---|---|
| I-2* | 95 |
| I-3* | 98 |
| I-9 | 97 |
| I-36* | 78 |
| I-43* | 98 |

## Patentansprüche

1. Verbindungen der Formel **(I),** 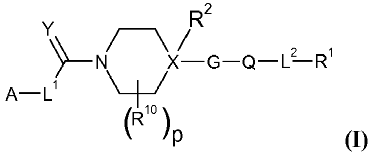 in welcher die Restedefinitionen folgende Bedeutungen haben:
A steht für Phenyl, das bis zu fünf Substituenten enthalten kann,
wobei die Substituenten unabhängig voneinander ausgewählt sind aus Z^{A-1}, oder
A steht für ein gegebenenfalls benzokondensiertes, unsubstituiertes oder substituiertes 5- oder 6-gliedriges Heteroaryl, das bis zu vier Substituenten enthalten kann, wobei die Substituenten am Kohlenstoff unabhängig voneinander ausgewählt sind aus Z^{A-2} und die Substituenten am Stickstoff unabhängig voneinander ausgewählt sind aus Z^{A-3},
Z^{A-1} stehen gleich oder verschieden und unabhängig voneinander für Wasserstoff, Halogen, Hydroxy, Thioxy, Nitro, Cyano, -C(=O)H, -C(=O)OH,, Alkyl, Alkenyl, Alkinyl, Halogenalkyl, Halogenalkenyl, Halogenalkinyl, Cycloalkyl, Cycloalkenyl, Halogencycloalkyl, Halogencycloalkenyl, Hydroxyalkyl, Cyanoalkyl, Formylalkyl, Alkoxyalkyl, Halogenalkoxyalkyl, Cycloalkoxyalkyl, Alkinyloxyalkyl, Alkylthioalkyl, Alkylsulfinylalkyl, Alkylaminoalkyl, Halogenalkylaminoalkyl, Cycloalkylaminoalkyl, Dialkylaminoalkyl, Alkylcarbonylalkyl, Alkylsulfonylalkyl, Alkylcycloalkyl, Alkylcycloalkenyl, Alkoxy, Alkylcycloalkylalkyl, Halogencycloalkoxy, Alkylthio, Halogenalkylthio, Cycloalkylthio, Alkinylthio, Alkenyloxy, Alkinyloxy, Halogenalkoxy, Halogenalkenyloxy, Halogenalkinyloxy, Cycloalkoxy, Alkoxyalkoxy, Cycloalkylalkoxy, Alkylcarbonyloxy, Halogenalkylcarbonyloxy, Cycloalkylcarbonyloxy, Cycloalkylamino, Alkylcarbonylamino, Cycloalkylcarbonylamino, Alkoxycarbonylamino, Alkylsulfonylamino, Halogenalkylsulfonylamino, Phenylsulfonylamino, Cycloalkylalkyl, Halogencycloalkylalkyl, Cycloalkylcycloalkyl, Alkoxyalkoxyalkyl, Alkylaminocarbonyloxy, Alkylcarbonylalkoxy, Cycloalkylaminocarbonyl, Cycloalkylalkoxycarbonyl, Alkylsulfinyl, Halogenalkylsulfinyl, Alkylsulfonyl, Halogenalkylsulfonyl, Cycloalkylsulfonyl, Alkylcarbonyl, Halogenalkylcarbonyl, Cycloalkylcarbonyl, Alkoxycarbonyl, Cycloalkoxycarbonyl, Trialkylsilyl, -SF₅, Phenyl, -C(=O)NR³R⁴ oder -NR³R⁴,
Z^{A-2} und R^{G1} stehen gleich oder verschieden und unabhängig voneinander für Wasserstoff, Halogen, Hydroxy, Thioxy, Nitro, Cyano, -C(=O)H, -C(=O)OH, Alkyl, Alkenyl, Alkinyl, Halogenalkyl, Halogenalkenyl, Halogenalkinyl, Cycloalkyl, Halogencycloalkyl, Hydroxyalkyl, Formylalkyl, Alkoxyalkyl, Alkylcarbonylalkyl, Alkylcycloalkyl, Alkoxy, Alkylcycloalkylalkyl, Alkylthio, Halogenalkylthio, Alkinylthio, Alkenyloxy, Alkinyloxy, Halogenalkoxy, Alkoxyalkoxy, Alkylcarbonyloxy, Halogenalkylcarbonyloxy, Cycloalkylcarbonylamino, Alkylsulfonylamino, Halogenalkylsulfonylamino, Phenylsulfonylamino, Cycloalkylalkyl, Halogencycloalkylalkyl, Cycloalkylcycloalkyl, Alkoxycarbonyloxy, Alkylcarbonylthio, Alkylsulfinyl, Halogenalkylsulfinyl, Alkylsulfonyl, Halogenalkylsulfonyl, Alkylcarbonyl, Halogenalkylcarbonyl, Alkoxycarbonyl, Alkylamionocarbonyloxy, -C(=O)NR³R⁴ oder -NR³R⁴,
Z^{A-3}, R^{G2} und Z² stehen gleich oder verschieden und unabhängig voneinander für Wasserstoff, -C(=O)H, -C(=O)NR³R⁴, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Alkoxyalkyl, Alkylsulfonyl, Halogenalkylsulfonyl, Cycloalkylsulfonyl, Phenylsulfonyl, Alkylcarbonyl, Halogenalkylcarbonyl, Alkoxycarbonyl, Halogenalkoxycarbonyl, Cycloalkoxycarbonyl, Phenyl oder Benzyl,
R³ und R⁴ stehen gleich oder verschieden und unabhängig voneinander für Wasserstoff, Alkyl, Alkenyl, Alkinyl, Halogenalkyl, Cycloalkyl, Benzyl oder Phenyl,
L¹ steht für NR^{L12} oder C(R^{L11})₂,
R^{L11} steht gleich oder verschieden und unabhängig voneinander für Wasserstoff, Halogen, Hydroxy, Cyano, -C(=O)H, -C(=O)OH, Alkyl, Alkenyl, Alkinyl, Halogenalkyl, Halogenalkenyl, Halogenalkinyl, Alkoxyalkyl, Alkylthioalkyl, Alkylaminoalkyl, Dialkylaminoalkyl, Alkoxy, Alkylthio, Halogenalkylthio, Halogenalkoxy, Alkylcarbonyloxy, Alkylcarbonylamino, Alkylcarbonylthio, Alkylsulfonyl, Halogenalkylsulfonyl, Alkylcarbonyl, Halogenalkylcarbonyl, Alkoxycarbonyl, Trialkylsilyloxy, -NR³R⁴ oder -C(=O)NR³R⁴,
oder die beiden Reste R^{L11} bilden zusammen mit dem Kohlenstoffatom, an das sie gebunden sind, einen Cyclopropylring, oder
die beiden Reste R^{L11} stehen für =CH₂, =COR³, =NOR³ oder =CHN(R⁹)₂,
R^{L12} steht für Wasserstoff, -C(=O)H, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Cycloalkylaminocarbonyl, Halogenalkylaminocarbonyl, Alkylsulfonyl, Halogenalkylsulfonyl, Cycloalkylsulfonyl, Alkylcarbonyl, Halogenalkylcarbonyl, Alkoxycarbonyl, Halogenalkoxycarbonyl, Cycloalkoxycarbonyl, Alkylaminocarbonyl, Dialkylaminocarbonyl, Phenyl oder Benzyl,
R⁹ steht für Alkyl, Alkenyl, Alkinyl, Halogenalkyl, Cycloalkyl, Benzyl oder Phenyl,
Y steht für Schwefel oder Sauerstoff,
X steht für Kohlenstoff oder Stickstoff,
R² steht für Wasserstoff, Alkyl, Alkenyl, Halogenalkyl, Alkoxy, Halogen, Cyano oder Hydroxy,
R¹⁰ steht für Oxo, Alkyl, Alkenyl, Halogenalkyl, Alkoxy, Halogen, Cyano oder Hydroxy,
p steht für 0 bis 2,
G steht für ein unsubstituiertes oder substituiertes 5-gliedriges Heteroaryl, wobei die Substituenten am Kohlenstoff unabhängig voneinander ausgewählt sind aus R^{G1} und die Substituenten am Stickstoff unabhängig voneinander ausgewählt sind aus R^{G2},
Q steht für gesättigtes oder teilweise oder vollständig ungesättigtes, unsubstituiertes oder substituiertes 5-gliedriges Heterocyclyl, wobei ein oder mehrere Substituenten R⁵ gleich oder verschieden unabhängig voneinander ausgewählt sind aus,
R⁵ steht gleich oder verschieden unabhängig voneinander für:
verbunden mit Kohlenstoff des 5-gliedrigen Heterocyclyl von Q:
Wasserstoff, Oxo, Halogen, Cyano, Hydroxy, Nitro, -CHO, -C(=O)OH, -C(=O)NH₂,-C(=O)NR³R⁴, -NR³R⁴, Alkyl, Alkenyl, Alkinyl. Halogenalkyl, Halogenalkenyl, Halogenalkinyl, Cycloalkyl, Halogencycloalkyl, Alkylcycloalkyl, Cycloalkylalkyl, Cycloalkylcycloalkyl, Halogencycloalkylalkyl, Alkylcycloalkylalkyl, Cycloalkenyl, Halogencycloalkenyl, Alkoxyalkyl, Halogenalkoxyalkyl, Cycloalkoxyalkyl, Alkoxyalkoxyalkyl, Alkylthioalkyl, Formylalkyl, Alkylcarbonylalkyl, Alkylsulfinylalkyl, Alkylsulfonylalkyl, Alkylaminoalkyl, Dialkylaminoalkyl, Halogenalkylaminoalkyl, Cycloalkylaminoalkyl, Alkylcarbonyl, Halogenalkylcarbonyl, Cycloalkylcarbonyl, Alkoxycarbonyl, Cycloalkoxycarbonyl, Cycloalkylalkoxycarbonyl, Cycloalkylaminocarbonyl, Hydroxyalkyl, Alkoxy, Halogenalkoxy, Cycloalkoxy, Halogencycloalkoxy, Cycloalkylalkoxy, Alkenyloxy, Halogenalkenyloxy, Alkinyloxy, Halogenalkinyloxy, Alkoxyalkoxy, Alkylcarbonyloxy, Halogenalkylcarbonyloxy, Cycloalkylcarbonyloxy, Alkylcarbonylalkoxy, Alkylthio, Halogenalkylthio, Cycloalkylthio, Alkylsulfinyl, Halogenalkylsulfinyl, Alkylsulfonyl, Halogenalkylsulfonyl, Cycloalkylsulfonyl, Trialkylsilyl, Alkylsulfonylamino, Halogenalkylsulfonylamino,
verbunden mit Stickstoff, des 5-gliedrigen Heterocyclyl von Q:
Wasserstoff, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Phenyl, Benzyl, Alkylsulfonyl, -C(=O)H, Alkoxycarbonyl oder Alkylcarbonyl, 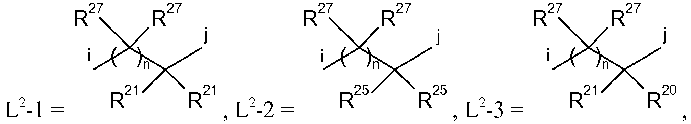 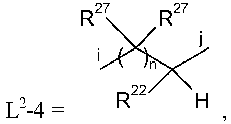
wobei die Bindung, die mit "i" identifiziert ist, direkt an Q gebunden ist, und wobei die Bindung, die mit "j" identifiziert ist, direkt an R¹ gebunden ist,
m steht für 0 bis 2,
n steht für 0 bis 4,
R²⁰ steht für Halogen oder Hydroxy,
R²¹ steht gleich oder verschieden und unabhängig voneinander für Alkyl, Halogenalkyl, Alkoxy, Halogenalkoxy, Alkenyl, Halogenalkenyl, Alkinyl, Halogenalkinyl, Alkenyloxy Halogenalkenyloxy, Alkinyloxy, Halogenalkinyloxy, oder Alkylcarbonyloxy,
R²² steht für Hydroxy, Halogen, Alkoxy, Halogenalkoxy, Alkenyl, Halogenalkenyl, Alkinyl, Halogenalkinyl, Alkenyloxy Halogenalkenyloxy, Alkinyloxy, Halogenalkinyloxy, Alkylcarbonyloxy oder Cycloalkyl,
R²³ steht für Wasserstoff, -C(=O)H, Alkyl, Alkenyl, Alkinyl, Alkoxyalkyl, Alkylsulfonyl, Halogenalkylsulfonyl, Alkylcarbonyl, Halogenalkylcarbonyl, Alkoxycarbonyl, Halogenalkoxycarbonyl, Cyclopropyl, Benzyl oder Phenylsulfonyl,
R²⁴ steht gleich oder verschieden und unabhängig voneinander für Wasserstoff, Cyano, Alkyl, Cycloalkyl, Halogenalkyl, Alkenyl, Halogenalkenyl, Alkinyl, Halogenalkinyl, Phenyl oder Benzyl,
R²⁵ steht gleich oder verschieden unabhängig voneinander für Fluor, Brom, Chlor,
R²⁶ steht für Wasserstoff, Hydroxy, Pyridinyl, Alkyl, Halogenalkyl oder Alkoxy,
R²⁷ steht jeweils unabhängig voneinander und gleich oder verschieden für Wasserstoff, Alkyl, Alkoxy, Alkenyloxy oder Alkinyloxy,
unter der Voraussetzung, daß wenn n größer 1, jeweils ein L² Rest maximal zwei R²⁷ enhalten darf, die von Wasserstoff verschieden sind,
R¹ steht für ein gegebenenfalls ein- oder mehrfach und gleich oder verschieden durch Z¹ substituiertes Phenyl, Benzyl oder Naphthalenyl, oder für ein gegebenenfalls benzokondensiertes, unsubstituiertes oder substituiertes 5- oder 6-gliedriges Heteroaryl, wobei die Substituenten am Kohlenstoff unabhängig voneinander ausgewählt aus Z¹ sind und die Substituenten am Stickstoff unabhängig voneinander ausgewählt sind aus Z²,
oder
R¹ steht für einen von 3- bis 7-gliedrigen nicht-aromatischen carbocyclischen Ring, für einen 5-, 6- oder 7-gliedrigen nicht-aromatischen Heterocyclylrest oder für einen von 8- bis 11-gliedrigen carbocyclischen oder heterocyclischen bicyclischen Ring, wobei die Substituenten am Kohlenstoff unabhängig voneinander ausgewählt sind aus Oxo, Thioxo oder Z¹ und die Substituenten am Stickstoff unabhängig voneinander ausgewählt sind aus Z²,
Z¹ stehen für Wasserstoff, Halogen, Hydroxy, Thioxy, Nitro, Cyano, -C(=O)H, -C(=O)OH, Alkyl, Alkenyl, Alkinyl, Halogenalkyl, Halogenalkenyl, Halogenalkinyl, Cycloalkyl, Cycloalkenyl, Halogencycloalkyl, Halogencycloalkenyl, Hydroxyalkyl, Alkoxyalkyl, Halogenalkoxyalkyl, Cycloalkoxyalkyl, Alkylthioalkyl, Alkylsulfinylalkyl, Alkylaminoalkyl, Halogenalkylaminoalkyl, Cycloalkylaminoalkyl, Dialkylaminoalkyl, Alkoxycarbonylalkyl, Alkylsulfonylalkyl, Alkylcycloalkyl, Alkoxy, Alkylcycloalkylalkyl, Halogencycloalkoxy, Alkylthio, Halogenalkylthio, Alkinylthio, Alkenyloxy, Alkinyloxy, Halogenalkoxy, Halogenalkenyloxy, Halogenalkinyloxy, Cycloalkoxy, Alkoxyalkoxy, Cycloalkylalkoxy, Alkylcarbonyloxy, Halogenalkylcarbonyloxy, Cycloalkylcarbonyloxy, Halogencycloalkylcarbonyloxy, Alkylsulfonyloxy, Halogenalkylsulfonyloxy, Phenylsulfonyloxy, Alkylcarbonylamino, Halogenalkylcarbonylamino, Alkylsulfonylamino, Halogenalkylsulfonylamino, Phenylsulfonylamino, Cycloalkylalkyl, Halogencycloalkylalkyl, Cycloalkylcycloalkyl, Alkoxyalkoxyalkyl, Alkylcarbonylalkoxy, Cycloalkylcarbonyl, Cycloalkylthio, Cycloalkylaminocarbonyl, Cycloalkylalkoxycarbonyl, Alkenylthio, Alkylcarbonylthio, Alkylsulfinyl, Halogenalkylsulfinyl, Alkylsulfonyl, Halogenalkylsulfonyl, Cycloalkylsulfonyl, Alkylcarbonyl, Halogenalkylcarbonyl, Alkoxycarbonyl, Halogenalkoxycarbonyl, Cycloalkoxycarbonyl, Trialkylsilyl, Trialkylsilyloxy, -SF₅, Cycloalkylalkylamino, Dialkylaminothiocarbonyl, Alkoxycarbonylamino, Alkoxyalkylaminocarbonyl, Halogenalkoxyamino, Cycloalkylalkylaminoalkyl, Dialkylaminocarbonylamino, Alkoxyhalogenalkoxy, Alkylthiocarbonyloxy, Halogenalkoxyalkoxy, Dialkylaminosulfonyl, Alkoxyhalogenalkyl, Alkylaminosulfonyl, Dialkoxyalkyl, Halogenalkoxyhalogenalkyl, -NHC(=O)H, Halogencycloalkenyloxyalkyl, Alkylthiocarbonyl, Alkoxyalkenyl, -SO₂NHCN, -NHCN, -Halogenalkylsulfonylaminocarbonyl, Alkylaminothiocarbonylamino, Alkylaminothiocarbonyl, Alkylaminocarbonylalkylamino, Alkoxyalkinyl, Halogendialkylaminoalkyl, Cyanoalkyl, Alkoxyalkylcarbonyl, Alkoxycarbonylalkoxy, Alkoxyamino, Alkoxyalkoxycarbonyl, Cycloalkenyloxyalkyl, Dialkylaminothiocarbonylamino, Alkylsulfonylaminocarbonyl, Halogenalkoxyhalogenalkoxy, Halogencycloalkoxyalkyl, -C(=O)NHCN, -N=C(R⁹)₂,-NR³R⁴, -C(=O)NR³R⁴, -C(=N-OR⁷)R⁸ oder -L³Z³,
R⁷ steht für Wasserstoff, Alkyl, Halogenalkyl, Benzyl oder Z³,
R⁸ steht für Wasserstoff, Alkyl, Halogenalkyl, Cycloalkylalkyl, Cycloalkyl, Alkylcycloalkyl, Halogenalkylcycloalkyl, Alkoxylalkyl, Halogenalkoxyalkyl, Benzyl oder Phenyl,
L³ steht für eine direkte Bindung, -CH₂-, -C(=O)-, Schwefel, Sauerstoff, -C(=O)O-,-C(=O)NH-, -OC(=O)- oder -NHC(=O)-,
Z³ steht für einen Phenylrest oder einen 5- oder 6-gliedrigen Heteroarylrest, der bis zu drei Substituenten enthalten kann, wobei die Substituenten unabhängig voneinander ausgewählt sind aus folgender Liste:
Substituenten am Kohlensoff: Halogen, Cyano, Nitro, Hydroxy, Amino, -SH, Alkyl, Alkenyl, Alkinyl, Halogenalkyl, Halogenalkenyl, Halogenalkinyl, Cycloalkyl, Halogencycloalkyl, Alkoxyalkyl, Alkylcarbonyl, Halogenalkylcarbonyl, Alkoxycarbonyl, Alkoxy, Halogenalkoxy, Cycloalkoxy, Halogencycloalkoxy, Alkenyloxy, Alkinyloxy, Alkoxyalkoxy, Alkylthio, Halogenalkylthio, Alkylsulfinyl, Halogenalkylsulfinyl, Alkylsulfonyl, Halogenalkylsulfonyl, Trisilylalkyl oder Phenyl, Substituenten am Stickstoff: Wasserstoff, -C(=O)H, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Alkoxyalkyl, Alkylsulfonyl, Halogenalkylsulfonyl, Cycloalkylsulfonyl, Phenylsulfonyl, Alkylcarbonyl, Halogenalkylcarbonyl, Alkoxycarbonyl, Halogenalkoxycarbonyl, Cycloalkoxycarbonyl, -C(=O)NR³R⁴, Phenyl oder Benzyl,
sowie Salze, Metallkomplexe und N-Oxide der Verbindungen der Formel **(I).**

2. Verbindungen der Formel [I] nach Anspruch 1,
in welcher die Symbole folgende Bedeutungen haben,
A steht für Phenyl, das bis zu zwei Substituenten enthalten kann, wobei die Substituenten unabhängig voneinander ausgewählt sind aus folgender Liste: Fluor, Brom, Iod, Chlor, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio oder Trifluormethylthio, oder
A steht für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten gleich oder verschieden und unabhängig voneinander ausgewählt sind aus folgender Liste:
Substituenten am Kohlenstoff:
Fluor, Chlor, Brom, Iod, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio, Trifluormethylthio oder Phenyl,
Substituenten am Stickstoff:
Methyl, Ethyl, Propyl, 1-Methylethyl, Methylsulfonyl, Trifluormethylsulfonyl, Methylcarbonyl, Trifluormethylcarbonyl, Chlormethylcarbonyl, 2,2-Trifluorethyl, 2,2-Difluorethyl, 2,2-Dichlor-2-fluorethyl, 2-Chlor-2-difluorethyl oder 2-Chlor-2-fluorethyl.
R^{L11} steht für Wasserstoff, Methyl, Ethyl oder Cyclopropyl, oder die beiden Reste R^{L11} bilden zusammen mit dem Kohlenstoffatom, an das sie gebunden sind, einen Cyclopropylring oder die beiden Reste R^{L11} stehen für =CHN(R⁹)₂,
R^{L12} steht für Wasserstoff, C₁-C₄-Alkyl, C₁-C₄-Halogenalkyl, C₃-C₈-Cycloalkyl, C₁-C₄-Alkylsulfonyl, C₁-C₄-Alkoxycarbonyl,
R⁹ steht gleich oder verschieden und unabhängig voneinander für C₁-C₆-Alkyl, C₂-C₆-Alkenyl, C₂-C₆-Alkinyl, C₁-C₆-Halogenalkyl, C₃-C₈-Cycloalkyl, Benzyl oder Phenyl
R² steht für Wasserstoff, Fluor, Chlor, Brom oder Hydroxy,
R¹⁰ steht für Fluor, Chlor, Brom oder Hydroxy,
p steht für 0 oder 1,
G steht für 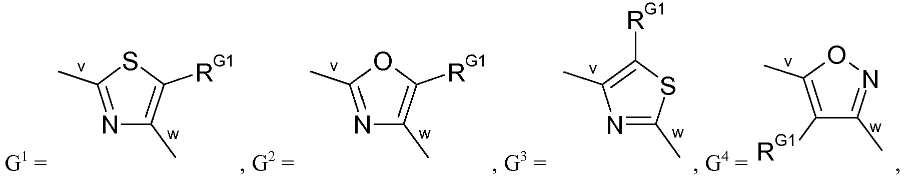 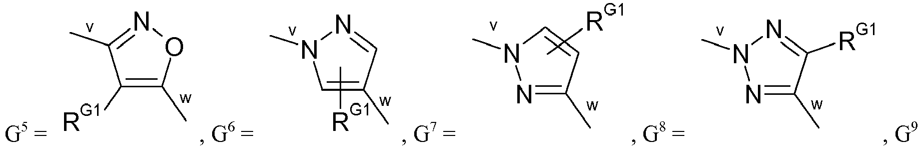 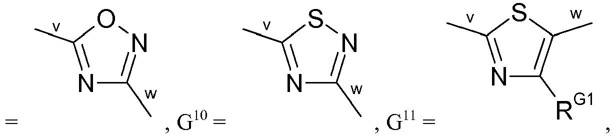
wobei die Bindung, die mit "v" identifiziert ist, direkt an X gebunden ist, und wobei die Bindung, die mit "w" identifiziert ist, direkt an Q gebunden ist,
R^{G1} steht für Wasserstoff,
Q steht für 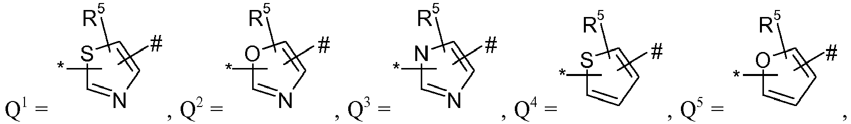 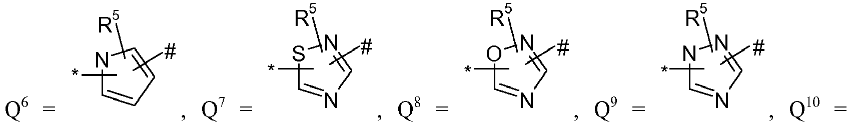 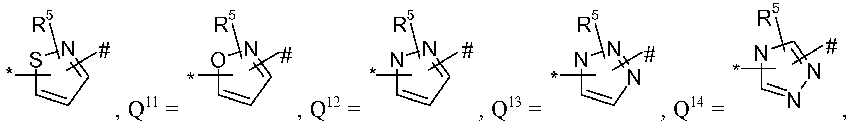 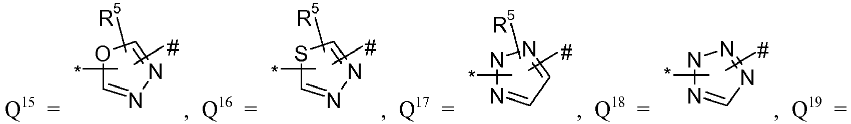 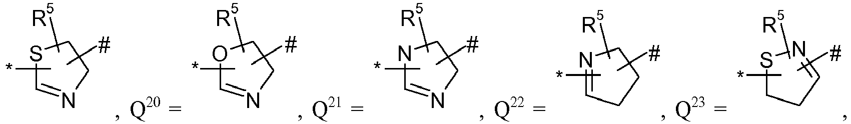 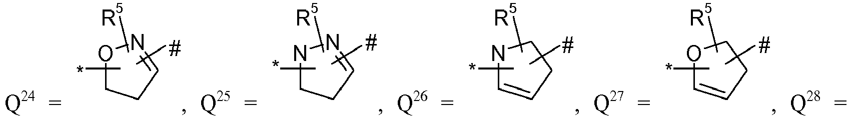 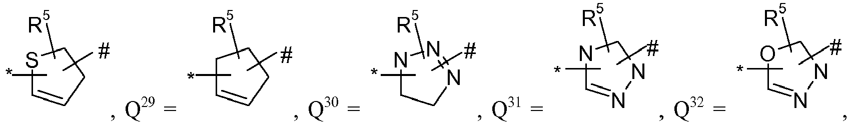 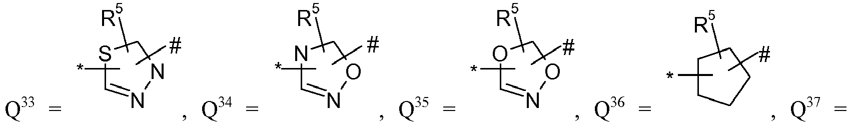 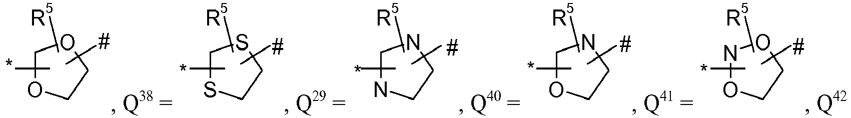 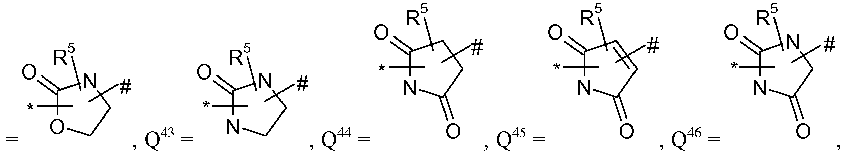
wobei die Bindung, die mit "*" identifiziert ist, direkt an G ist und gleichzeitig die Bindung, die mit "#" identifiziert ist, direkt an L² gebunden ist oder wobei die Bindung, die mit "*" identifiziert ist, direkt an L² gebunden ist, und gleichzeitig die Bindung, die mit "#" identifiziert ist, direkt an G gebunden ist,
R⁵ steht gleich oder verschieden unabhängig voneinander für
verbunden mit Kohlenstoff des 5-gliedriges Heterocyclyl von Q:
Wasserstoff, Cyano, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₃-C₈-Halogencycloalkyl, C₁-C₄-Alkyl-C₃-C₈-cycloalkyl, C₃-C₈-Cycloalkyl-C₁-C₄-alkyl, C₁-C₄-Alkoxy-C₁-C₄-alkyl, C₃-C₈-Cycloalkoxy-C₁-C₄-alkyl, C₁-C₄-Alkoxy-C₁-C₄-alkoxy-C₁-C₄-alkyl, C₁-C₄-Alkylthio-C₁-C₄-alkyl, C₁-C₆-Alkoxy, C₁-C₆-Halogenalkoxy, C₃-C₈-Cycloalkoxy, C₃-C₈-Halogencycloalkoxy, C₃-C₈-Cycloalkyl-C₁-C₄-alkoxy, C₂-C₆-Alkenyloxy, C₂-C₆-Halogenalkenyloxy, C₂-C₆-Alkinyloxy, C₂-C₆-Halogenalkinyloxy, C₁-C₆-Alkoxy-C₁-C₄-alkoxy, C₁-C₆-Alkylcarbonyloxy, C₁-C₆-Halogenalkylcarbonyloxy, C₄-C₈-Cycloalkylcarbonyloxy, C₁-C₆-Alkylcarbonyl-C₁-C₆-alkoxy, C₁-C₆-Alkylthio, C₁-C₆-Halogenalkylthio, C₃-C₈-Cycloalkylthio,
verbunden mit Stickstoff des 5-gliedriges Heterocyclyl von Q:
Wasserstoff, -C(=O)H, C₁-C₃-Alkyl, C₁-C₆-Alkylcarbonyl, C₁-C₆-Alkoxycarbonyl oder Benzyl,
R³ und R⁴ stehen gleich oder verschieden und unabhängig voneinander für Methyl, Ethyl, Propyl, 1-Methylethyl, Butyl oder 1,1-Dimethylethyl,
L² steht für 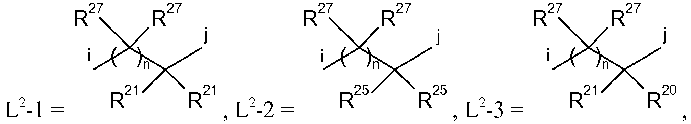 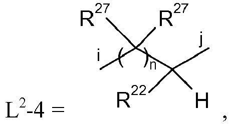 wobei die Bindung, die mit "i" identifiziert ist, direkt an Q gebunden ist, und wobei die Bindung, die mit "j" identifiziert ist, direkt an R¹ gebunden ist,
n steht für 0 bis 2,
m steht für 0 oder 2,
R¹ steht für unsubstituiertes oder substituiertes C₅-C₆-Cycloalkenyl, C₃-C₈-Cycloalkyl, wobei die Substituenten unabhängig voneinander ausgewählt sind aus Z¹⁻¹ oder
R¹ steht für unsubstituiertes oder substituiertes Phenyl, wobei die Substituenten unabhängig voneinander ausgewählt sind aus Z¹⁻² oder
R¹ steht für unsubstituiertes oder substituiertes, Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 2,3-Dihydro-1H-inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, wobei die Substituenten unabhängig voneinander ausgewählt aus Z¹⁻³ sind, oder
R¹ steht für einen unsubstituierten oder substituierten 5- oder 6-gliedrigen Heteroarylrest, wobei die Substituenten am Kohlenstoff unabhängig voneinander ausgewählt sind aus Z¹⁻⁴, und die Substituenten am Stickstoff unabhängig voneinander ausgewählt sind aus Z², oder
R¹ steht für benzokondensiertes unsubstituiertes oder substituiertes 5- oder 6- gliedriges Heteroaryl, wobei die Substituenten am Kohlenstoff unabhängig voneinander ausgewählt aus Z¹⁻⁵ sind, und die Substituenten am Stickstoff unabhängig voneinander ausgewählt sind aus Z², oder
R¹ steht für unsubstituiertes oder substituiertes C₅-C₁₅-Heterocyclyl, wobei die Substituenten am Kohlenstoff unabhängig voneinander ausgewählt sind aus Z¹⁻⁶ und die Substituenten am Stickstoff unabhängig voneinander ausgewählt sind aus Z²,
Z¹⁻¹ stehen gleich oder verschieden unabhängig voneinander bevorzugt für Cyano, Halogen, -C(=O)H, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylcarbonyloxy, C₁-C₆-Halogenalkylcarbonyloxy, C₁-C₆-Alkylthio oder C₁-C₆-Halogenalkylthio,
Z¹⁻² stehen gleich oder verschieden unabhängig voneinander für Halogen, Cyano, Hydroxy, Thio, Nitro, -C(=O)H, -COOH, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₃-C₈-Cycloalkenyl, C₃-C₈-Halogencycloalkenyl, C₁-C₆-Alkoxy-C₁-C₆-alkyl, C₁-C₆-Alkylcarbonyl, C₁-C₆-Halogenalkylcarbonyl, C₃-C₈-Cycloalkylcarbonyl, C₁-C₆-Alkoxycarbonyl, C₃-C₈-Cycloalkoxycarbonyl, C₃-C₈-Cycloalkylaminocarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Halogenalkenyloxy, C₂-C₆-Alkinyloxy, C₂-C₆-Halogenalkinyloxy, C₁-C₄-Alkoxy-C₁-C₄-alkoxy, C₁-C₆-Alkylcarbonyloxy, C₁-C₆-Halogenalkylcarbonyloxy, C₃-C₈-Cycloalkylcarbonyloxy, C₁-C₆-Alkylcarbonyl-C₁-C₄-alkoxy, C₁-C₆-Alkylthio, C₁-C₆-Halogenalkylthio, C₃-C₆-Cycloalkylthio, C₁-C₆-Alkylsulfonyl, C₁-C₆-Halogenalkylsulfonyl, C₃-C₈-Cycloalkylsulfonyl, C₁-C₆-Alkylsulfonyloxy, C₁-C₆-Halogenalkylsulfonyloxy, Phenylsulfonyloxy, C₁-C₆-Alkylsulfonylamino, C₁-C₆-Halogenalkylsulfonylamino, Tri(C₁-C₄)alkylsilyl, Tri(C₁-C₄)alkylsilyloxy oder -C(=N-OR⁷)R⁸,
Z¹⁻³ und Z¹⁻⁵ stehen gleich oder verschieden unabhängig voneinander für Halogen, Cyano, Nitro, -C(=O)H, -NR³R⁴, -C(=O)NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₄-Alkoxy-C₁-C₄-alkyl, C₁-C₆-Alkylcarbonyl, C₁-C₆-Alkoxycarbonyl, C₁-C₄-Alkoxy, C₁-C₄-Halogenalkoxy, C₁-C₆-Alkylcarbonyloxy, C₁-C₆-Alkylcarbonylthio, C₁-C₄-Alkylthio, C₁-C₄-Halogenalkylthio, C₁-C₄-Alkylsulfonyl, C₁-C₄-Halogenalkylsulfonyl,
Z¹⁻⁴ stehen gleich oder verschieden unabhängig voneinander für Halogen, Cyano, Hydroxy, Thio, Nitro, -C(=O)H, -C(=O)OH, -C(=O)NR³R⁴, -NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₃-C₈-Cycloalkenyl, C₃-C₈-Halogencycloalkenyl, C₁-C₆-Alkoxy-C₁-C₆-alkyl, C₁-C₆-Alkylcarbonyl, C₁-C₆-Halogenalkylcarbonyl, C₃-C₈-Cycloalkylcarbonyl, C₁-C₆-Alkoxycarbonyl, C₃-C₈-Cycloalkoxycarbonyl, C₃-C₁₀-Cycloalkylaminocarbonyl, C₁-C₆-Alkoxy, C₁-C₆-Halogenalkoxy, C₃-C₈-Cycloalkoxy, C₃-C₈-Halogencycloalkoxy, C₂-C₆-Alkenyloxy, C₂-C₆-Halogenalkenyloxy, C₂-C₆-Alkinyloxy, C₂-C₆-Halogenalkinyloxy, C₁-C₄-Alkoxy-C₁-C₄-alkoxy, C₁-C₆-Alkylcarbonyloxy, C₁-C₆-Halogenalkylcarbonyloxy, C₃-C₈-Cycloalkylcarbonyloxy, C₁-C₆-Alkylcarbonyl-C₁-C₄-alkoxy, C₁-C₆-Alkylthio, C₁-C₆-Halogenalkylthio, C₃-C₆-Cycloalkylthio, C₁-C₆-Alkylsulfonyl, C₁-C₆-Halogenalkylsulfonyl, C₃-C₈-Cycloalkylsulfonyl, C₁-C₆-Alkylsulfonyloxy, C₁-C₆-Halogenalkylsulfonyloxy, Phenylsulfonyloxy, C₁-C₆-Alkylsulfonylamino oder C₁-C₆-Halogenalkylsulfonylamino,
Z¹⁻⁶ stehen gleich oder verschieden unabhängig voneinander für Cyano, Halogen, -C(=O)H, -C(=O)NR³R⁴, Phenyl, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, C₁-C₆-Alkoxy, C₁-C₆-Halogenalkoxy, C₂-C₆-Alkenyloxy, C₂-C₆-Alkinyloxy, C₁-C₆-Alkylthio, -NR³R⁴, C₁-C₆-Alkylcarbonyl, C₁-C₆-Alkoxycarbonyl, C₁-C₆-Alkylcarbonyloxy oder C₁-C₆-Halogenalkylthio,
Z² steht gleich oder verschieden unabhängig voneinander für Wasserstoff, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₄-alkoxy-C₁-C₄-alkyl, Phenyl, Benzyl, C₁-C₄-Halogenalkylsulfonyl, C₁-C₆-Alkoxycarbonyl, C₁-C₆-Halogenalkoxycarbonyl, Phenylsulfonyl, C₁-C₄-Alkylsulfonyl, -C(=O)H, oder C₁-C₃-Alkylcarbonyl,
R⁷ steht für Wasserstoff, Methyl oder Ethyl,
R⁸ steht für Wasserstoff, C₁-C₆-Alkyl, C₁-C₆-Halogenalkyl, C₃-C₈-Cycloalkyl-C₁-C₄-alkyl, C₃-C₈-Cycloalkyl, C₁-C₄-Alkyl-C₃-C₈-cycloalkyl, C₁-C₄-Halogenalkyl-C₃-C₈-cycloalkyl, C₁-C₄-Alkoxyl-C₁-C₄-alkyl, C₁-C₄-Halogenalkoxy-C₁-C₄-alkyl, Benzyl oder Phenyl,
L³ steht bevorzugt für eine direkte Bindung, -CH₂-, Schwefel, Sauerstoff, -C(=O)O-,-C(=O)NH-, -OC(=O)- oder -NHC(=O)-, besonders bevorzugt für eine direke Bindung,
Z³⁻¹ steht bevorzugt für einen Phenylrest oder einen 5- oder 6-gliedrigen Heteroarylrest, der bis zu zwei Substituenten enthalten kann, wobei die Substituenten unabhängig voneinander ausgewählt sind aus folgender Liste:
Substituenten am Kohlenstoff:
Halogen, Cyano, Nitro, Hydroxy, Amino, -SH, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₁-C₄-Alkoxyalkyl, C₁-C₆-Alkylcarbonyl, C₁-C₆-Halogenalkylcarbonyl, C₁-C₆-Alkoxycarbonyl, C₁-C₄-Alkoxy, C₁-C₄-Halogenalkoxy, C₂-C₆-Alkenyloxy, C₂-C₆-Alkinyloxy, C₁-C₄-Alkylthio, C₁-C₄-Halogenalkylthio, C₁-C₄-Alkylsulfonyl, C₁-C₄-Halogenalkylsulfonyl oder C₁-C₄-Alkylamino, Di(C₁-C₄-alkyl)amino,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C1-C4-alkoxy-C₁-C₄-alkyl, Phenyl, Benzyl, C₁-C₄-Halogenalkylsulfonyl, C₁-C₆-Alkoxycarbonyl, C₁-C₆-Halogenalkoxycarbonyl, Phenylsulfonyl, C₁-C₄-Alkylsulfonyl, -C(=O)H, oder C₁-C₃-Alkylcarbonyl,
sowie Salze, Metallkomplexe und N-Oxide der Verbindungen der Formel **(I)**.

3. Verbindungen der Formel [I] nach ein der vorherigen Ansprüche,
in welcher die Symbole folgende Bedeutungen haben,
A steht für Pyrazol-1-yl, der bis zu zwei Substituenten enthalten kann, wobei die Substituenten unabhängig voneinander ausgewählt sind aus folgender Liste: Methyl, Ethyl, Chlor, Brom, Fluor, Difluormethyl oder Trifluormethyl, oder
A steht für Phenyl, der bis zu zwei Substituenten enthalten kann, wobei die Substituenten unabhängig voneinander ausgewählt sind aus folgender Liste: Methyl, Ethyl, Iod, Chlor, Brom, Fluor, Methoxy, Ethoxy, Difluormethyl oder Trifluormethyl.
L¹ steht für CH₂,
Y steht für Sauerstoff,
X steht für Kohlenstoff,
R² steht für Wasserstoff oder Fluor,
p steht für 0,
G steht für 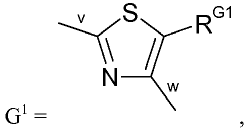
Q steht für 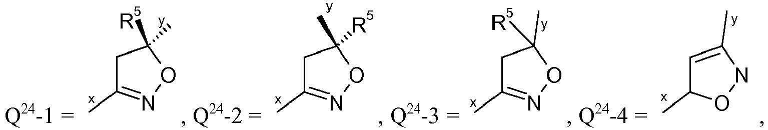 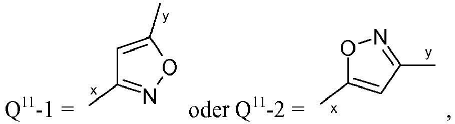
wobei die Bindung, die mit "x" identifiziert ist, direkt an G gebunden ist, und wobei die Bindung, die mit "y" identifiziert ist, direkt an L² gebunden ist,
R⁵ für Wasserstoff, Cyano, Methyl, Trifluomethyl, Difluormethyl oder Methoxymethyl,
m steht für 0,
n steht für 0,
R²⁰ steht für Halogen oder Hydroxy,
R²¹ steht gleich oder verschieden und unabhängig voneinander für Methyl, Ethyl, Ethenyl, Ethinyl, Trifluormethyl, Difluormethyl, Methylcarbonyloxy, Ethylcarbonyloxy, Methoxy, Ethoxy, Propoxy, 1-Methylethoxy, 2-Propenyloxy, 2-Propinyloxy oder Trifluormethoxy,
R²² steht für Hydroxy, Chlor, Fluor, Brom, Iod, Methoxy, Ethyoxy, Propoxy, 1-Methylethoxy, Butoxy, , Trifluormethyl, Difluormethyl, Methylcarbonyloxy, Ethylcarbonyloxy, 1-Ethenyloxy, 2-Propenyloxy, 2-Propinyoxy oder Trifluormethoxy,
R²³ steht für Wasserstoff, Methyl, Ethyl, Propyl, 1-Methylethyl, Methylcarbonyl, Ethylcarbonyl, 1,1 -Dimethylethyloxycarbonyl, -C(=O)H, Phenylsulfonyl, Methylsulfonyl, Trifluormethylsulfonyl oder Benzyl,
R²⁴ steht für Wasserstoff,
R²⁵ steht für Fluor,
R²⁶ steht gleich oder verschieden und unabhängig voneinander Methyl, Ethyl, Methoxy oder Ethoxy,
R²⁷ steht für Wasserstoff,
R¹ steht für Cyclopentenyl, Cyclohexenyl, Cyclopentyl, Cyclohexyl oder Cycloheptyl, welche jeweils bis zu zwei Substituenten enthalten kann, wobei die Substituenten unabhängig voneinander ausgewählt sind aus folgender Liste: Methyl, Ethyl, Methoxy, Ethoxy, Trifluormethoxy, Ethinyl, 2-Propenyloxy, 2-Propinyloxy, Methylcarbonyloxy, Ethylcarbonyloxy, Trifluorcarbonyloxy, Methylthio, Ethylthio oder Trifluormethylthio, oder
R¹ steht für Phenyl, das bis zu drei Substituenten enthalten kann, wobei die Substituenten unabhängig voneinander ausgewählt sind aus folgender Liste: Fluor, Chlor, Brom, Iod, Cyano, Nitro, Hydroxy, Amino, Thio, -(C=O)H, Methyl, Ethyl, Propyl, 1-Methylethyl, Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylcarbonylamino, Trifluoralkylcarbonylamino, Chlormethylcarbonylamino, Methylthio, Ethylthio, Methylsulfinyl, Methylsulfonyl, Methylsulfonyloxy, Trifluorsulfonyloxy, Methylsulfonylamino, Trifluoromethylsulfonylamino,-C(=N-OH)H, -C(=N-OCH₃)H, -C(=N-OCH₂CH₃)H, -C(=N-OH)CH₃,-C(=N-OCH₃)CH₃, -C(=N-OCH₂CH₃)CH₃ oder Trimethylsilyloxy, oder
R¹ steht für unsubstituiertes oder substituiertes, Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetra-hydronaphthalen-1-yl, 5,6,7,8-Tetrahydronaphthalen-2-yl, Decalin-1-yl, Decalin-2-yl, 1H-Inden-1-yl, 2,3-Dihydro-1H-inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, wobei die Substituenten unabhängig voneinander ausgewählt aus folgender Liste ausgewählt sind: Methyl, Methoxy, Cyano, Fluor, Chlor, Brom, Iod, oder
R¹ steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Cyano, Nitro, Hydroxy, Amino, Thio, -(C=O)H, Methyl, Ethyl, Propyl, 1-Methylethyl, Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, Propoxy, 1-Methylethoxy, 1,1-Dimethylethoxy, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 1-Ethenyloxy, 2-Propenyloxy, 2-Propinyloxy, Methylcarbonyloxy, Trifluoralkylcarbonyloxy, Chlormethylcarbonyloxy, Methylcarbonylamino, Trifluoralkylcarbonylamino, Chlormethylcarbonylamino, Methylthio, Ethylthio, Methylsulfinyl, Methylsulfonyl, Methylsulfonyloxy, Trifluorsulfonyloxy, Methylsulfonylamino, oder Trifluoromethylsulfonylamino,
Substituenten am Stickstoff: Methyl, Ethyl, Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl, oder
R¹ steht für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl, oder
R¹ für Piperidin-1-yl, Piperidin-2-yl, Piperidin-3-yl, Piperidin-4-yl, Piperazin-1-yl, Piperazin-2-yl, Piperazin-3-yl, Morpholin-1-yl, Morpholin-2-yl, Morpholin-3-yl, Tetrahydropyran-2-yl, Tetrahydropyran-3-yl, Tetrahydropyran-4-yl, 1,2,3,4-Tetrahydrochinolin-1-yl, 1,2,3,4-Tetrahydroisochinolin-2-yl, 1,2,3,4-Tetrahydrochinoxalin-1-yl, Indolin-1-yl, Isoindolin-2-yl, Decahydrochinolin-1-yl oder Decahydroisochinolin-2-yl, welche jeweils bis zu zwei Substituenten enthalten kann, wobei die Substituenten unabhängig voneinander ausgewählt sind aus folgender Liste:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, Propyl, -C(=O)H, Methylcarbonyl, Trifluormethylcarbonyl, Chlormethylcarbonyl, Methylsulfonyl, Trifluormethylsulfonyl, Phenylsulfonyl, Phenyl oder 2-Propinyl,
sowie Salze, Metallkomplexe und N-Oxide der Verbindungen der Formel **(I).**

4. Verfahren zur Bekämpfung von pflanzenpathogenen Schadpilzen, **dadurch gekennzeichnet, dass** man Verbindungen der Formel (I) gemäß einem der vorherigen Ansprüche, auf die pflanzenpathogenen Schadpilze und/oder deren Lebensraum ausbringt.

5. Mittel zur Bekämpfung von pflanzenpathogenen Schadpilzen, **gekennzeichnet durch** einen Gehalt an mindestens einer Verbindung der Formel (I) gemäß einem der Ansprüche 1 oder 3, neben Streckmitteln und/oder oberflächenaktiven Stoffen.

6. Verwendung von mindestens einer Verbindung der Formel (I), gemäß einem der Ansprüche 1 bis 3 bzw. eines Mittels gemäß Anspruch 5 zur Bekämpfung von pflanzenpathogenen Schadpilzen.

7. Verfahren zur Herstellung von Mitteln zur Bekämpfung von pflanzenpathogenen Schadpilzen gemäß Anspruch 5, **dadurch gekennzeichnet, dass** Verbindungen der Formel (I) gemäß einem der Ansprüche 1 oder 3 mit Streckmitteln und/oder oberflächenaktiven Stoffen vermischt.

8. Verwendung von Verbindungen der Formel (I) gemäß einem der Ansprüche 1 bis 3 zur Behandlung von Saatgut.

9. Verwendung von Verbindungen der Formel (I) gemäß einem der Ansprüche 1 bis 3 zur Behandlung von transgenen Pflanzen.

10. Verwendung von Verbindungen der Formel (I) gemäß einem der Ansprüche 1 bis 3 zur Behandlung von transgenem Saatgut.

11. Verbindungen der Formel **(XVIIa),** 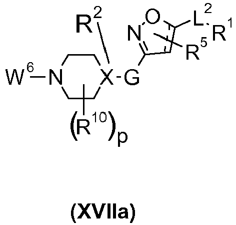 bei denen
W⁶ für Acetyl, C₁-C₄ Alkoxycarbonyl, Benzyl oder Benzyloxycarbonyl steht,
und in denen die Symbole X, G, L², p, R¹, R², R⁵ und R¹⁰ eine Bedeutung haben wie in einem der Ansprüche 1 bis 3 angegeben ist, sowie Salze, Metallkomplexe und N-Oxide davon.

12. Verbindungen der Formel **(XXXIII),** 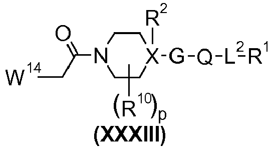 bei denen
W¹⁴ für Aldehyde, Ketone, Ester, Carbonsäuren, Amide, Thioamide, Nitrile, Alkohole, Thiole, Hydrazine, Oxime, Amidine, Amideoxidme, Olefine, Acetylene, Halide, Alkylhalide, Methansulfonate, Trifluormethansulfonate, Borsäure, Boronate steht,
und in denen die Symbole W¹⁴, X, L², R¹, R², R¹⁰, Q, p und G eine Bedeutung haben wie in einem der Ansprüche 1 bis 3 angegeben ist, sowie Salze, Metallkomplexe und N-Oxide davon.

13. Verbindungen der Formel **(XIIIa),** 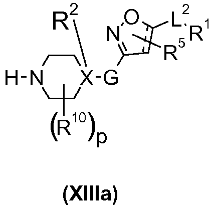 und in denen die Symbole X, L², p, G, R¹, R², R⁵ und R¹⁰ eine Bedeutung haben wie in einem der Ansprüche 1 bis 3 angegeben ist, sowie Salze, Metallkomplexe und N-Oxide davon.

## Claims

1. Compounds of the formula **(I)** 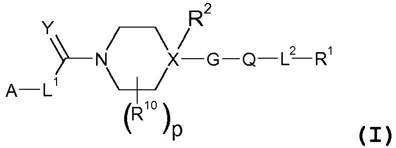 in which the radicals are each defined as follows:
A is phenyl which may contain up to five substituents,
where the substituents are each independently selected from Z^{A-1},
or
A is an optionally benzofused unsubstituted or substituted 5- or 6-membered heteroaryl which may contain up to four substituents, where the substituents on carbon are each independently selected from Z^{A-2} and the substituents on nitrogen are each independently selected from Z^{A-3},
Z^{A-1} are the same or different and are each independently hydrogen, halogen, hydroxyl, thiol, nitro, cyano, -C(=O)H, -C(=O)OH, alkyl, alkenyl, alkynyl, haloalkyl, haloalkenyl, haloalkynyl, cycloalkyl, cycloalkenyl, halocycloalkyl, halocycloalkenyl, hydroxyalkyl, cyanoalkyl, formylalkyl, alkoxyalkyl, haloalkoxyalkyl, cycloalkoxyalkyl, alkynyloxyalkyl, alkylthioalkyl, alkylsulphinylalkyl, alkylaminoalkyl, haloalkylaminoalkyl, cycloalkylaminoalkyl, dialkylaminoalkyl, alkylcarbonylalkyl, alkylsulphonylalkyl, alkylcycloalkyl, alkylcycloalkenyl, alkoxy, alkylcycloalkylalkyl, halocycloalkoxy, alkylthio, haloalkylthio, cycloalkylthio, alkynylthio, alkenyloxy, alkynyloxy, haloalkoxy, haloalkenyloxy, haloalkynyloxy, cycloalkoxy, alkoxyalkoxy, cycloalkylalkoxy, alkylcarbonyloxy, haloalkylcarbonyloxy, cycloalkylcarbonyloxy, cycloalkylamino, alkylcarbonylamino, cycloalkylcarbonylamino, alkoxycarbonylamino, alkylsulphonylamino, haloalkylsulphonylamino, phenylsulphonylamino, cycloalkylalkyl, halocycloalkylalkyl, cycloalkylcycloalkyl, alkoxyalkoxyalkyl, alkylaminocarbonyloxy, alkylcarbonylalkoxy, cycloalkylaminocarbonyl, cycloalkylalkoxycarbonyl, alkylsulphinyl, haloalkylsulphinyl, alkylsulphonyl, haloalkylsulphonyl, cycloalkylsulphonyl, alkylcarbonyl, haloalkylcarbonyl, cycloalkylcarbonyl, alkoxycarbonyl, cycloalkoxycarbonyl, trialkylsilyl, -SF₅, phenyl, -C(=O)NR³R⁴ or -NR³R⁴,
Z^{A-2} and R^{G1} are the same or different and are each independently hydrogen, halogen, hydroxyl, thioxy, nitro, cyano, -C(=O)H, -C(=O)OH, alkyl, alkenyl, alkynyl, haloalkyl, haloalkenyl, haloalkynyl, cycloalkyl, halocycloalkyl, hydroxyalkyl, formylalkyl, alkoxyalkyl, alkylcarbonylalkyl, alkylcycloalkyl, alkoxy, alkylcycloalkylalkyl, alkylthio, haloalkylthio, alkynylthio, alkenyloxy, alkynyloxy, haloalkoxy, alkoxyalkoxy, alkylcarbonyloxy, haloalkylcarbonyloxy, cycloalkylcarbonylamino, alkylsulphonylamino, haloalkylsulphonylamino, phenylsulphonylamino, cycloalkylalkyl, halocycloalkylalkyl, cycloalkylcycloalkyl, alkoxycarbonyloxy, alkylcarbonylthio, alkylsulphinyl, haloalkylsulphinyl, alkylsulphonyl, haloalkylsulphonyl, alkylcarbonyl, haloalkylcarbonyl, alkoxycarbonyl, alkylamionocarbonyloxy, -C(=O)NR³R⁴ or -NR³R⁴,
Z^{A-3}, R^{G2} and Z² are the same or different and are each independently hydrogen, -C(=O)H, -C(=O)NR³R⁴, alkyl, alkenyl, alkynyl, haloalkyl, haloalkenyl, haloalkynyl, cycloalkyl, halocycloalkyl, alkylcycloalkyl, cycloalkylalkyl, alkoxyalkyl, alkylsulphonyl, haloalkylsulphonyl, cycloalkylsulphonyl, phenylsulphonyl, alkylcarbonyl, haloalkylcarbonyl, alkoxycarbonyl, haloalkoxycarbonyl, cycloalkoxycarbonyl, phenyl or benzyl,
R³ and R⁴ are the same or different and are each indepently hydrogen, alkyl, alkenyl, alkynyl, haloalkyl, cycloalkyl, benzyl or phenyl,
L¹ is NR^{L12} or C(R^{L11})₂,
R^{L11} are the same or different and are each independently hydrogen, halogen, hydroxyl, cyano, - C(=O)H, -C(=O)OH, alkyl, alkenyl, alkynyl, haloalkyl, haloalkenyl, haloalkynyl, alkoxyalkyl, alkylthioalkyl, alkylaminoalkyl, dialkylaminoalkyl, alkoxy, alkylthio, haloalkylthio, haloalkoxy, alkylcarbonyloxy, alkylcarbonylamino, alkylcarbonylthio, alkylsulphonyl, haloalkylsulphonyl, alkylcarbonyl, haloalkylcarbonyl, alkoxycarbonyl, trialkylsilyloxy, -NR³R⁴ or -C(=O)NR³R⁴,
or the two R^{L11} radicals, together with the carbon atom to which they are bonded, form a cyclopropyl ring, or
the two R^{L11} radicals are =CH₂, =COR³, =NOR³ or =CHN(R⁹)₂,
R^{L12} is hydrogen, -C(=O)H, alkyl, alkenyl, alkynyl, haloalkyl, haloalkenyl, haloalkynyl, cycloalkyl, halocycloalkyl, alkylcycloalkyl, cycloalkylalkyl, cycloalkylaminocarbonyl, haloalkylaminocarbonyl, alkylsulphonyl, haloalkylsulphonyl, cycloalkylsulphonyl, alkylcarbonyl, haloalkylcarbonyl, alkoxycarbonyl, haloalkoxycarbonyl, cycloalkoxycarbonyl, alkylaminocarbonyl, dialkylaminocarbonyl, phenyl or benzyl,
R⁹ is alkyl, alkenyl, alkynyl, haloalkyl, cycloalkyl, benzyl or phenyl,
Y is sulphur or oxygen,
X is carbon or nitrogen,
R² is hydrogen, alkyl, alkenyl, haloalkyl, alkoxy, halogen, cyano or hydroxyl,
R¹⁰ is oxo, alkyl, alkenyl, haloalkyl, alkoxy, halogen, cyano or hydroxyl,
p is 0 to 2,
G is an unsubstituted or substituted 5-membered heteroaryl, where the substituents on carbon are each independently selected from R^{G1} and the substituents on nitrogen are each independently selected from R^{G2},
Q is saturated or partly or fully unsaturated, unsubstituted or substituted 5-membered heterocyclyl, where one or more substituents R⁵ are the same or different and are each independently selected from,
R⁵ is the same or different and is independently:
bonded to carbon of the 5-membered heterocyclyl of Q:
hydrogen, oxo, halogen, cyano, hydroxyl, nitro, - CHO, -C(=O)OH, -C(=O)NH₂, -C(=O)NR³R⁴, -NR³R⁴, alkyl, alkenyl, alkynyl, haloalkyl, haloalkenyl, haloalkynyl, cycloalkyl, halocycloalkyl, alkylcycloalkyl, cycloalkylalkyl, cycloalkylcycloalkyl, halocycloalkylalkyl, alkylcycloalkylalkyl, cycloalkenyl, halocycloalkenyl, alkoxyalkyl, haloalkoxyalkyl, cycloalkoxyalkyl, alkoxyalkoxyalkyl, alkylthioalkyl, formylalkyl, alkylcarbonylalkyl, alkylsulphinylalkyl, alkylsulphonylalkyl, alkylaminoalkyl, dialkylaminoalkyl, haloalkylaminoalkyl, cycloalkylaminoalkyl, alkylcarbonyl, haloalkylcarbonyl, cycloalkylcarbonyl, alkoxycarbonyl, cycloalkoxycarbonyl, cycloalkylalkoxycarbonyl, cycloalkylaminocarbonyl, hydroxyalkyl, alkoxy, haloalkoxy, cycloalkoxy, halocycloalkoxy, cycloalkylalkoxy, alkenyloxy, haloalkenyloxy, alkynyloxy, haloalkynyloxy, alkoxyalkoxy, alkylcarbonyloxy, haloalkylcarbonyloxy, cycloalkylcarbonyloxy, alkylcarbonylalkoxy, alkylthio, haloalkylthio, cycloalkylthio, alkylsulphinyl, haloalkylsulphinyl, alkylsulphonyl, haloalkylsulphonyl, cycloalkylsulphonyl, trialkylsilyl, alkylsulphonylamino, haloalkylsulphonylamino,
bonded to nitrogen of the 5-membered heterocyclyl of Q:
hydrogen, alkyl, alkenyl, alkynyl, haloalkyl, haloalkenyl, haloalkynyl, cycloalkyl, halocycloalkyl, alkylcycloalkyl, cycloalkylalkyl, phenyl, benzyl, alkylsulphonyl, -C(=O)H, alkoxycarbonyl or alkylcarbonyl, 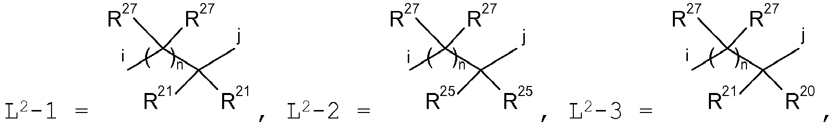 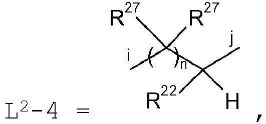 where the bond identified by "i" is bonded directly to Q, and where the bond identified by "j" is bonded directly to R¹,
m is 0 to 2,
n is 0 to 4,
R²⁰ is halogen or hydroxyl,
R²¹ is the same or different and is independently alkyl, haloalkyl, alkoxy, haloalkoxy, alkenyl, haloalkenyl, alkynyl, haloalkynyl, alkenyloxy, haloalkenyloxy, alkynyloxy, haloalkynyloxy, or alkylcarbonyloxy,
R²² is hydroxyl, halogen, alkoxy, haloalkoxy, alkenyl, haloalkenyl, alkynyl, haloalkynyl, alkenyloxy, haloalkenyloxy, alkynyloxy, haloalkynyloxy, alkylcarbonyloxy or cycloalkyl,
R²³ is hydrogen, -C(=O)H, alkyl, alkenyl, alkynyl, alkoxyalkyl, alkylsulphonyl, haloalkylsulphonyl, alkylcarbonyl, haloalkylcarbonyl, alkoxycarbonyl, haloalkoxycarbonyl, cyclopropyl, benzyl or phenylsulphonyl,
R²⁴ is the same or different and is independently hydrogen, cyano, alkyl, cycloalkyl, haloalkyl, alkenyl, haloalkenyl, alkynyl, haloalkynyl, phenyl or benzyl,
R²⁵ is the same or different and is independently fluorine, bromine, chlorine,
R²⁶ is hydrogen, hydroxyl, pyridinyl, alkyl, haloalkyl or alkoxy,
R²⁷ is the same or different and is in each case independently hydrogen, alkyl, alkoxy, alkenyloxy or alkynyloxy, with the proviso that, when n is greater than 1, one L² radical in each case may contain not more than two R²⁷ different from hydrogen,
R¹ is a phenyl, benzyl or naphthalenyl optionally mono- or polysubstituted identically or differently by Z¹ , or an optionally benzofused, unsubstituted or substituted 5- or 6-membered heteroaryl, where the substituents on carbon are each independently selected from Z¹ and the substituents on nitrogen are each independently selected from Z²,
or
R¹ is a 3- to 7-membered nonaromatic carbocyclic ring, a 5-, 6- or 7-membered nonaromatic heterocyclyl radical or an 8- to 11-membered carbocyclic or heterocyclic bicyclic ring, where the substituents on carbon are each independently selected from oxo, thioxo or Z¹ and the substituents on nitrogen are each independently selected from Z²,
Z¹ are each hydrogen, halogen, hydroxyl, thioxy, nitro, cyano, -C(=O)H, -C(=O)OH, alkyl, alkenyl, alkynyl, haloalkyl, haloalkenyl, haloalkynyl, cycloalkyl, cycloalkenyl, halocycloalkyl, halocycloalkenyl, hydroxyalkyl, alkoxyalkyl, haloalkoxyalkyl, cycloalkoxyalkyl, alkylthioalkyl, alkylsulphinylalkyl, alkylaminoalkyl, haloalkylaminoalkyl, cycloalkylaminoalkyl, dialkylaminoalkyl, alkoxycarbonylalkyl, alkylsulphonylalkyl, alkylcycloalkyl, alkoxy, alkylcycloalkylalkyl, halocycloalkoxy, alkylthio, haloalkylthio, alkynylthio, alkenyloxy, alkynyloxy, haloalkoxy, haloalkenyloxy, haloalkynyloxy, cycloalkoxy, alkoxyalkoxy, cycloalkylalkoxy, alkylcarbonyloxy, haloalkylcarbonyloxy, cycloalkylcarbonyloxy, halocycloalkylcarbonyloxy, alkylsulphonyloxy, haloalkylsulphonyloxy, phenylsulphonyloxy, alkylcarbonylamino, haloalkylcarbonylamino, alkylsulphonylamino, haloalkylsulphonylamino, phenylsulphonylamino, cycloalkylalkyl, halocycloalkylalkyl, cycloalkylcycloalkyl, alkoxyalkoxyalkyl, alkylcarbonylalkoxy, cycloalkylcarbonyl, cycloalkylthio, cycloalkylaminocarbonyl, cycloalkylalkoxycarbonyl, alkenylthio, alkylcarbonylthio, alkylsulphinyl, haloalkylsulphinyl, alkylsulphonyl, haloalkylsulphonyl, cycloalkylsulphonyl, alkylcarbonyl, haloalkylcarbonyl, alkoxycarbonyl, haloalkoxycarbonyl, cycloalkoxycarbonyl, trialkylsilyl, trialkylsilyloxy, -SF₅, cycloalkylalkylamino, di-alkylaminothiocarbonyl, alkoxycarbonylamino, alkoxyalkylaminocarbonyl, haloalkoxyamino, cycloalkylalkylaminoalkyl, dialkylaminocarbonylamino, alkoxyhaloalkoxy, alkylthiocarbonyloxy, haloalkoxyalkoxy, dialkylaminosulphonyl, alkoxyhaloalkyl, alkylaminosulphonyl, dialkoxyalkyl, haloalkoxyhaloalkyl, -NHC(=O)H, halocycloalkenyloxyalkyl, alkylthiocarbonyl, alkoxyalkenyl, -SO₂NHCN, -NHCN, haloalkylsulphonylaminocarbonyl, alkylaminothiocarbonylamino, alkylaminothiocarbonyl, alkylaminocarbonylalkylamino, alkoxyalkynyl, halodialkylaminoalkyl, cyanoalkyl, alkoxyalkylcarbonyl, alkoxycarbonylalkoxy, alkoxyamino, alkoxyalkoxycarbonyl, cycloalkenyloxyalkyl, dialkylaminothiocarbonylamino, alkylsulphonylaminocarbonyl, haloalkoxyhaloalkoxy, halocycloalkoxyalkyl, -C(=O)NHCN, -N=C(R⁹)₂, -NR³R⁴, -C(=O)NR³R⁴,-C(=N-OR⁷)R⁸ or -L³Z³,
R⁷ is hydrogen, alkyl, haloalkyl, benzyl or Z³,
R⁸ is hydrogen, alkyl, haloalkyl, cycloalkylalkyl, cycloalkyl, alkylcycloalkyl, haloalkylcycloalkyl, alkoxylalkyl, haloalkoxyalkyl, benzyl or phenyl,
L³ is a direct bond, -CH₂-, -C(=O)-, sulphur, oxygen, -C(=O)O-, -C(=O)NH-, -OC(=O)- or -NHC(=O)-,
Z³ is a phenyl radical or a 5- or 6-membered heteroaryl radical which may contain up to three substituents, where the substituents are each independently selected from the following list: substituents on carbon: halogen, cyano, nitro, hydroxyl, amino, -SH, alkyl, alkenyl, alkynyl, haloalkyl, haloalkenyl, haloalkynyl, cycloalkyl, halocycloalkyl, alkoxyalkyl, alkylcarbonyl, haloalkylcarbonyl, alkoxycarbonyl, alkoxy, haloalkoxy, cycloalkoxy, halocycloalkoxy, alkenyloxy, alkynyloxy, alkoxyalkoxy, alkylthio, haloalkylthio, alkylsulphinyl, haloalkylsulphinyl, alkylsulphonyl, haloalkylsulphonyl, trisilylalkyl or phenyl,
and substituents on nitrogen: hydrogen, -C(=O)H, alkyl, alkenyl, alkynyl, haloalkyl, haloalkenyl, haloalkynyl, cycloalkyl, halocycloalkyl, alkylcycloalkyl, cycloalkylalkyl, alkoxyalkyl, alkylsulphonyl, haloalkylsulphonyl, cycloalkylsulphonyl, phenylsulphonyl, alkylcarbonyl, haloalkylcarbonyl, alkoxycarbonyl, haloalkoxycarbonyl, cycloalkoxycarbonyl, -C(=O)NR³R⁴, phenyl or benzyl,
and salts, metal complexes and N-oxides of the compounds of the formula **(I).**

2. Compounds of the formula [I] according to Claim 1, in which the symbols are each defined as follows:
A is phenyl which may contain up to two substituents, where the substituents are each independently selected from the following list: fluorine, bromine, iodine, chlorine, cyano, nitro, methyl, ethyl, propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, propoxy, methoxy, trifluoromethoxy, difluoromethoxy, 1-methylethylthio, methylthio, ethylthio, propylthio, difluoromethylthio or trifluoromethylthio, or
A is a heteroaromatic radical selected from the following group: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrimidin-2-yl, pyrimidin-4-yl or pyrimidin-5-yl, which may contain up to two substituents, where the substituents are the same or different and are each independently selected from the following list:
substituents on carbon:
fluorine, chlorine, bromine, iodine, cyano, nitro, methyl, ethyl, propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, propoxy, methoxy, trifluoromethoxy, difluoromethoxy, 1-methylethylthio, methylthio, ethylthio, propylthio, difluoromethylthio, trifluoromethylthio or phenyl,
substituents on nitrogen:
methyl, ethyl, propyl, 1-methylethyl, methylsulphonyl, trifluoromethylsulphonyl, methylcarbonyl, trifluoromethylcarbonyl, chloromethylcarbonyl, 2,2-trifluoroethyl, 2,2-difluoroethyl, 2,2-dichloro-2-fluoroethyl, 2-chloro-2-difluoroethyl or 2-chloro-2-fluoroethyl.
R^{L11} is hydrogen, methyl, ethyl or cyclopropyl, or the two R^{L11} radicals, together with the carbon atom to which they are bonded, form a cyclopropyl ring, or the two R^{L11} radicals are =CHN(R⁹)₂,
R^{L12} is hydrogen, C₁-C₄-alkyl, C₁-C₄-haloalkyl, C₃-C₈-cycloalkyl, C₁-C₄-alkylsulphonyl, C₁-C₄-alkoxycarbonyl,
R⁹ is the same or different and is independently C₁-C₆-alkyl, C₂-C₆-alkenyl, C₂-C₆-alkynyl, C₁-C₆-haloalkyl, C₃-C₈-cycloalkyl, benzyl or phenyl
R² is hydrogen, fluorine, chlorine, bromine or hydroxyl,
R¹⁰ is fluorine, chlorine, bromine or hydroxyl,
p is 0 or 1,
G is 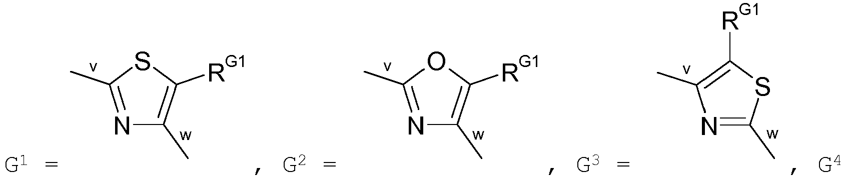 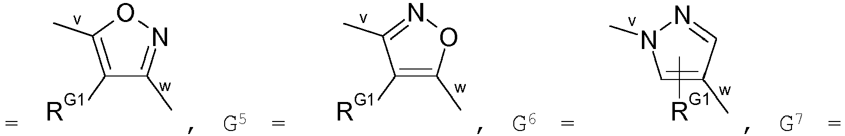 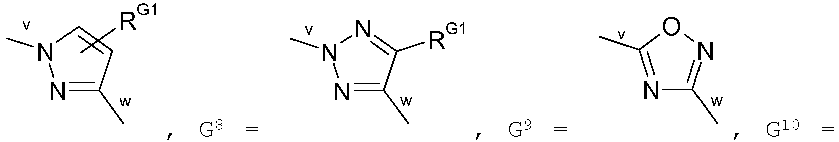 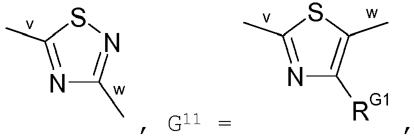
where the bond identified by "v" is bonded directly to X and where the bond identified by "w" is bonded directly to Q,
R^{G1} is hydrogen,
Q is 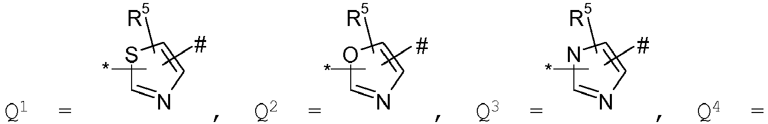 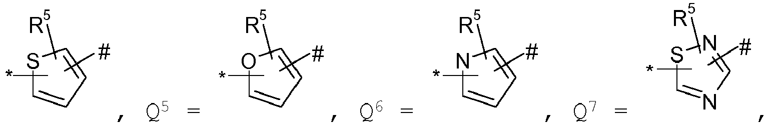 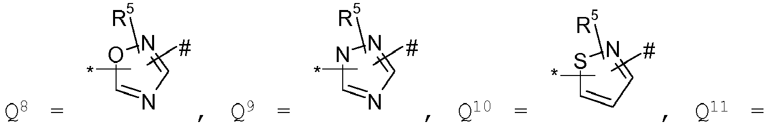 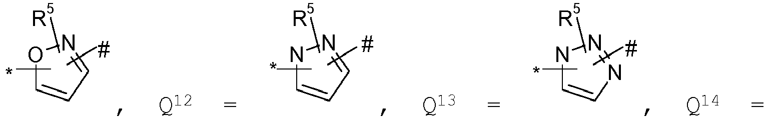 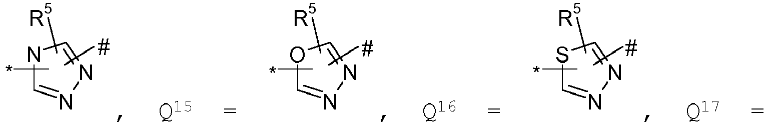 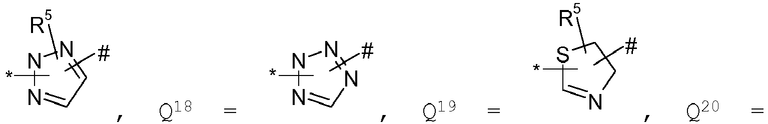 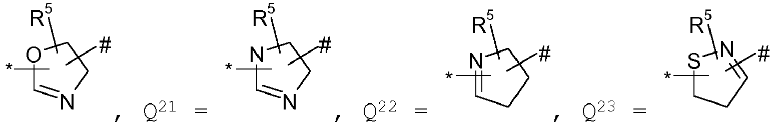 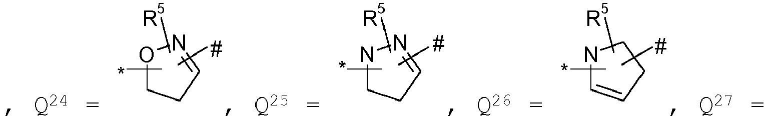 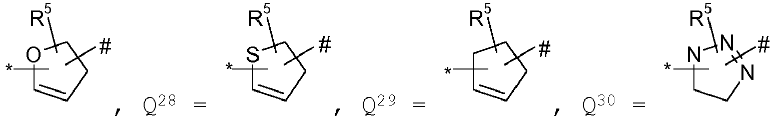 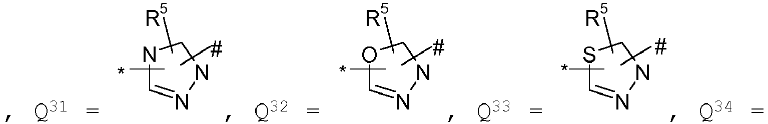 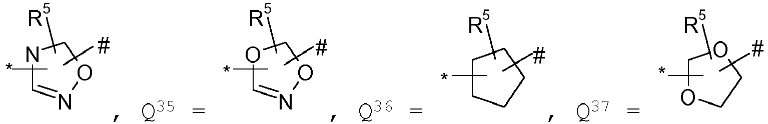 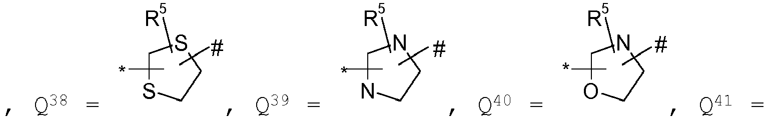 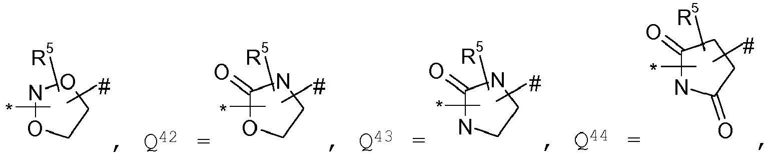 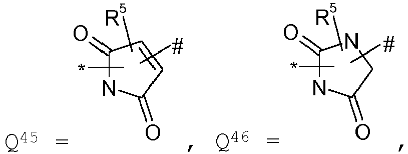 where the bond identified by "*" is bonded directly to G and, at the same time, the bond identified by "#" is bonded directly to L², or where the bond identified by "*" is bonded directly to L² and, at the same time, the bond identified by "#" is bonded directly to G,
R⁵ is the same or different and is independently
bonded to carbon of the 5-membered heterocyclyl of Q:
hydrogen, cyano, -NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₃-C₈-halocycloalkyl, C₁-C₄-alkyl-C₃-C₈-cycloalkyl, C₃-C₈-cycloalkyl-C₁-C₄-alkyl, C₁-C₄-alkoxy-C₁-C₄-alkyl, C₃-C₈-cycloalkoxy-C₁-C₄-alkyl, C₁-C₄-alkoxy-C₁-C₄-alkoxy-C₁-C₄-alkyl, C₁-C₄-alkylthio-C₁-C₄-alkyl, C₁-C₆-alkoxy, C₁-C₆-haloalkoxy, C₃-C₈-cycloalkoxy, C₃-C₈-halocycloalkoxy, C₃-C₈-cycloalkyl-C₁-C₄-alkoxy, C₂-C₆-alkenyloxy, C₂-C₆-haloalkenyloxy, C₂-C₆-alkynyloxy, C₂-C₆-haloalkynyloxy, C₁-C₆-alkoxy-C₁-C₄-alkoxy, C₁-C₆-alkylcarbonyloxy, C₁-C₆-haloalkylcarbonyloxy, C₄-C₈-cycloalkylcarbonyloxy, C₁-C₆-alkylcarbonyl-C₁-C₆-alkoxy, C₁-C₆-alkylthio, C₁-C₆-haloalkylthio, C₃-C₈-cycloalkylthio,
bonded to nitrogen of the 5-membered heterocyclyl of Q:
hydrogen, -C(=O)H, C₁-C₃-alkyl, C₁-C₆-alkylcarbonyl, C₁-C₆-alkoxycarbonyl or benzyl,
R³ and R⁴ are the same or different and are each independently methyl, ethyl, propyl, 1-methylethyl, butyl or 1,1-dimethylethyl,
L² is 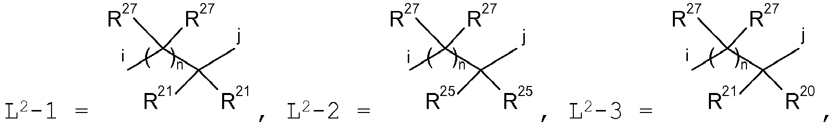 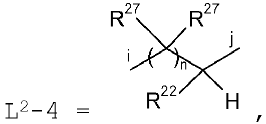 where the bond identified by "i" is bonded directly to Q, and where the bond identified by "j" is bonded directly to R¹,
n is 0 to 2,
m is 0 or 2,
R¹ is unsubstituted or substituted C₅-C₆-cycloalkenyl, C₃-C₈-cycloalkyl, where the substituents are each independently selected from Z¹⁻¹ or
R¹ is unsubstituted or substituted phenyl, where the substituents are each independently selected from Z¹⁻² or
R¹ is unsubstituted or substituted, naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 2,3-dihydro-1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl, where the substituents are each independently selected from Z¹⁻³, or
R¹ is an unsubstituted or substituted 5- or 6-membered heteroaryl radical where the substituents on carbon are each independently selected from Z¹⁻⁴, and the substituents on nitrogen are each independently selected from Z², or
R¹ is benzofused unsubstituted or substituted 5- or 6- membered heteroaryl, where the substituents on carbon are each independently selected from Z¹⁻⁵, and the substituents on nitrogen are each independently selected from Z², or
R¹ is unsubstituted or substituted C₅-C₁₅-heterocyclyl, where the substituents on carbon are each independently selected from Z¹⁻⁶ and the substituents on nitrogen are each independently selected from Z²,
Z¹⁻¹ are the same or different and are each independently preferably cyano, halogen, -C(=O)H, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, hydroxyl, oxo, C₁-C₆-alkoxy, C₁-C₆-haloalkoxy, C₂-C₆-alkenyloxy, C₂-C₆-alkynyloxy, C₁-C₆-alkylcarbonyloxy, C₁-C₆-haloalkylcarbonyloxy, C₁-C₆-alkylthio or C₁-C₆-haloalkylthio,
Z¹⁻² are the same or different and are each independently halogen, cyano, hydroxyl, thio, nitro, -C(=O)H, -COOH, -C(=O)NR³R⁴, -NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₃-C₈-cycloalkenyl, C₃-C₈-halocycloalkenyl, C₁-C₆-alkoxy-C₁-C₆-alkyl, C₁-C₆-alkylcarbonyl, C₁-C₆-haloalkylcarbonyl, C₃-C₈-cycloalkylcarbonyl, C₁-C₆-alkoxycarbonyl, C₃-C₈-cycloalkoxycarbonyl, C₃-C₈-cycloalkylaminocarbonyl, C₁-C₆-alkoxy, C₁-C₆-haloalkoxy, C₃-C₈-cycloalkoxy, C₃-C₈-halocycloalkoxy, C₂-C₆-alkenyloxy, C₂-C₆-haloalkenyloxy, C₂-C₆-alkynyloxy, C₂-C₆-haloalkynyloxy, C₁-C₄-alkoxy-C₁-C₄-alkoxy, C₁-C₆-alkylcarbonyloxy, C₁-C₆-haloalkylcarbonyloxy, C₃-C₈-cycloalkylcarbonyloxy, C₁-C₆-alkylcarbonyl-C₁-C₄-alkoxy, C₁-C₆-alkylthio, C₁-C₆-haloalkylthio, C₃-C₆-cycloalkylthio, C₁-C₆-alkylsulphonyl, C₁-C₆-haloalkylsulphonyl, C₃-C₈-cycloalkylsulphonyl, C₁-C₆-alkylsulphonyloxy, C₁-C₆-haloalkylsulphonyloxy, phenylsulphonyloxy, C₁-C₆-alkylsulphonylamino, C₁-C₆-haloalkylsulphonylamino, tri(C₁-C₄)alkylsilyl, tri(C₁-C₄)alkylsilyloxy or -C(=N-OR⁷)R⁸,
Z¹⁻³ and Z¹⁻⁵ are the same or different and are each independently halogen, cyano, nitro, -C(=O)H,-NR³R⁴, -C(=O)NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₄-alkoxy-C₁-C₄-alkyl, C₁-C₆-alkylcarbonyl, C₁-C₆-alkoxycarbonyl, C₁-C₄-alkoxy, C₁-C₄-haloalkoxy, C₁-C₆-alkylcarbonyloxy, C₁-C₆-alkylcarbonylthio, C₁-C₄-alkylthio, C₁-C₄-haloalkylthio, C₁-C₄-alkylsulphonyl, C₁-C₄-haloalkylsulphonyl,
Z¹⁻⁴ are the same or different and are each independently halogen, cyano, hydroxyl, thio, nitro, -C(=O)H, -C(=O)OH, -C(=O)NR³R⁴, -NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₃-C₈-cycloalkenyl, C₃-C₈-halocycloalkenyl, C₁-C₆-alkoxy-C₁-C₆-alkyl, C₁-C₆-alkylcarbonyl, C₁-C₆-haloalkylcarbonyl, C₃-C₈-cycloalkylcarbonyl, C₁-C₆-alkoxycarbonyl, C₃-C₈-cycloalkoxycarbonyl, C₃-C₁₀-cycloalkylaminocarbonyl, C₁-C₆-alkoxy, C₁-C₆-haloalkoxy, C₃-C₈-cycloalkoxy, C₃-C₈-halocycloalkoxy, C₂-C₆-alkenyloxy, C₂-C₆-haloalkenyloxy, C₂-C₆-alkynyloxy, C₂-C₆-haloalkynyloxy, C₁-C₄-alkoxy-C₁-C₄-alkoxy, C₁-C₆-alkylcarbonyloxy, C₁-C₆-haloalkylcarbonyloxy, C₃-C₈-cycloalkylcarbonyloxy, C₁-C₆-alkylcarbonyl-C₁-C₄-alkoxy, C₁-C₆-alkylthio, C₁-C₆-haloalkylthio, C₃-C₆-cycloalkylthio, C₁-C₆-alkylsulphonyl, C₁-C₆-haloalkylsulphonyl, C₃-C₈-cycloalkylsulphonyl, C₁-C₆-alkylsulphonyloxy, C₁-C₆-haloalkylsulphonyloxy, phenylsulphonyloxy, C₁-C₆-alkylsulphonylamino or C₁-C₆-haloalkylsulphonylamino,
Z¹⁻⁶ are the same or different and are each independently cyano, halogen, -C(=O)H, -C(=O)NR³R⁴, phenyl, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, C₂-C₆-alkenyloxy, C₂-C₆-alkynyloxy, C₁-C₆-alkylthio, -NR³R⁴, C₁-C₆-alkylcarbonyl, C₁-C₆-alkoxycarbonyl, C₁-C₆-alkylcarbonyloxy or C₁-C₆-haloalkylthio,
Z² is the same or different and is independently hydrogen, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₄-alkoxy-C₁-C₄-alkyl, phenyl, benzyl, C₁-C₄-haloalkylsulphonyl, C₁-C₆-alkoxycarbonyl, C₁-C₆-haloalkoxycarbonyl, phenylsulphonyl, C₁-C₄-alkylsulphonyl, -C(=O)H, or C₁-C₃-alkylcarbonyl,
R⁷ is hydrogen, methyl or ethyl,
R⁸ is hydrogen, C₁-C₆-alkyl, C₁-C₆-haloalkyl, C₃-C₈-cycloalkyl-C₁-C₄-alkyl, C₃-C₈-cycloalkyl, C₁-C₄-alkyl-C₃-C₈-cycloalkyl, C₁-C₄-haloalkyl-C₃-C₈-cycloalkyl, C₁-C₄-alkoxyl-C₁-C₄-alkyl, C₁-C₄-haloalkoxy-C₁-C₄-alkyl, benzyl or phenyl,
L³ is preferably a direct bond, -CH₂-, sulphur, oxygen, -C(=O)O-, -C(=O)NH-, -OC(=O)- or -NHC(=O)-, more preferably a direct bond,
Z³⁻¹ is preferably a phenyl radical or a 5- or 6-membered heteroaryl radical which may contain up to two substituents, where the substituents are each independently selected from the following list:
substituents on carbon:
halogen, cyano, nitro, hydroxyl, amino, -SH, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₁-C₄-alkoxyalkyl, C₁-C₆-alkylcarbonyl, C₁-C₆-haloalkylcarbonyl, C₁-C₆-alkoxycarbonyl, C₁-C₄-alkoxy, C₁-C₄-haloalkoxy, C₂-C₆-alkenyloxy, C₂-C₆-alkynyloxy, C₁-C₄-alkylthio, C₁-C₄-haloalkylthio, C₁-C₄-alkylsulphonyl, C₁-C₄-haloalkylsulphonyl or C₁-C₄-alkylamino, di(C₁-C₄-alkyl)amino,
substituents on nitrogen: C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₄-alkoxy-C₁-C₄-alkyl, phenyl, benzyl, C₁-C₄-haloalkylsulphonyl, C₁-C₆-alkoxycarbonyl, C₁-C₆-haloalkoxycarbonyl, phenylsulphonyl, C₁-C₄-alkylsulphonyl,-C(=O)H, or C₁-C₃-alkylcarbonyl,
and salts, metal complexes and N-oxides of the compounds of the formula **(I).**

3. Compounds of the formula [I] according to either of the preceding claims,
in which the symbols are each defined as follows:
A is pyrazol-1-yl which may contain up to two substituents, where the substituents are each independently selected from the following list: methyl, ethyl, chlorine, bromine, fluorine, difluoromethyl and trifluoromethyl, or
A is phenyl which may contain up to two substituents, where the substituents are each independently selected from the following list: methyl, ethyl, iodine, chlorine, bromine, fluorine, methoxy, ethoxy, difluoromethyl and trifluoromethyl,
L¹ is CH₂,
Y is oxygen,
X is carbon,
R² is hydrogen or fluorine,
p is 0,
G is 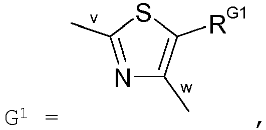
Q is 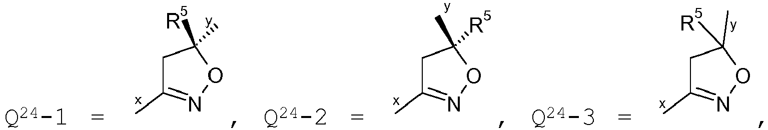 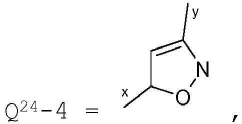 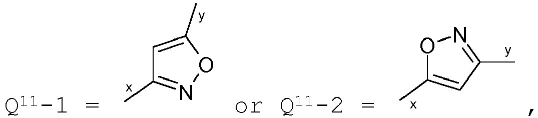 where the bond identified by "x" is bonded directly to G, and where the bond identified by "y" is bonded directly to L²,
R⁵ is hydrogen, cyano, methyl, trifluoromethyl, difluoromethyl or methoxymethyl,
m is 0,
n is 0,
R²⁰ is halogen or hydroxyl,
R²¹ is the same or different and is independently methyl, ethyl, ethenyl, ethynyl, trifluoromethyl, difluoromethyl, methylcarbonyloxy, ethylcarbonyloxy, methoxy, ethoxy, propoxy, 1-methylethoxy, 2-propenyloxy, 2-propynyloxy or trifluoromethoxy,
R²² is hydroxyl, chlorine, fluorine, bromine, iodine, methoxy, ethoxy, propoxy, 1-methylethoxy, butoxy, trifluoromethyl, difluoromethyl, methylcarbonyloxy, ethylcarbonyloxy, 1-ethenyloxy, 2-propenyloxy, 2-propynyloxy or trifluoromethoxy,
R²³ is hydrogen, methyl, ethyl, propyl, 1-methylethyl, methylcarbonyl, ethylcarbonyl, 1,1-dimethylethyloxycarbonyl, -C(=O)H, phenylsulphonyl, methylsulphonyl, trifluoromethylsulphonyl or benzyl,
R²⁴ is hydrogen,
R²⁵ is fluorine,
R²⁶ is the same or different and is independently methyl, ethyl, methoxy or ethoxy,
R²⁷ is hydrogen,
R¹ is cyclopentenyl, cyclohexenyl, cyclopentyl, cyclohexyl or cycloheptyl, each of which may contain up to two substituents, where the substituents are each independently selected from the following list: methyl, ethyl, methoxy, ethoxy, trifluoromethoxy, ethynyl, 2-propenyloxy, 2-propynyloxy, methylcarbonyloxy, ethylcarbonyloxy, trifluorocarbonyloxy, methylthio, ethylthio or trifluoromethylthio, or
R¹ is phenyl which may contain up to three substituents, where the substituents are each independently selected from the following list: fluorine, chlorine, bromine, iodine, cyano, nitro, hydroxyl, amino, thio, -(C=O)H, methyl, ethyl, propyl, 1-methylethyl, butyl, 1,1-dimethylethyl, 1,2-dimethylethyl, ethenyl, ethynyl, trifluoromethyl, difluoromethyl, trichloromethyl, dichloromethyl, cyclopropyl, methoxy, ethoxy, propoxy, 1-methylethoxy, 1,1-dimethylethoxy, methylcarbonyl, ethylcarbonyl, trifluoromethylcarbonyl, methoxycarbonyl, ethoxycarbonyl, propoxycarbonyl, 1-methylethoxycarbonyl, 1,1-dimethylethoxycarbonyl, 1-ethenyloxy, 2-propenyloxy, 2-propynyloxy, methylcarbonyloxy, trifluoroalkylcarbonyloxy, chloromethylcarbonyloxy, methylcarbonylamino, trifluoroalkylcarbonylamino, chloromethylcarbonylamino, methylthio, ethylthio, methylsulphinyl, methylsulphonyl, methylsulphonyloxy, trifluorosulphonyloxy, methylsulphonylamino, trifluoromethylsulphonylamino, -C(=N-OH)H, -C(=N-OCH₃)H,-C(=N-OCH₂CH₃)H, -C(=N-OH)CH₃, -C(=N-OCH₃)CH₃,-C(=N-OCH₂CH₃)CH₃ or trimethylsilyloxy, or
R¹ is unsubstituted or substituted, naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 2,3-dihydro-1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl, where the substituents are each independently selected from the following list: methyl, methoxy, cyano, fluorine, chlorine, bromine, iodine, or
R¹ is furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1,2,3-triazol-4-yl, 1,2,4-triazol-1-yl, 1,2,4-triazol-3-yl, 1,2,4-triazol-4-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl or pyrazin-2-yl, each of which may contain up to two substituents, where the substituents are each independently selected from the following list:
substituents on carbon: fluorine, chlorine, bromine, iodine, cyano, nitro, hydroxyl, amino, thio, -(C=O)H, methyl, ethyl, propyl, 1-methylethyl, butyl, 1,1-dimethylethyl, 1,2-dimethylethyl, ethenyl, ethynyl, trifluoromethyl, difluoromethyl, trichloromethyl, dichloromethyl, cyclopropyl, methoxy, ethoxy, propoxy, 1-methylethoxy, 1,1-dimethylethoxy, methylcarbonyl, ethylcarbonyl, trifluoromethylcarbonyl, methoxycarbonyl, ethoxycarbonyl, propoxycarbonyl, 1-methylethoxycarbonyl, 1,1-dimethylethoxycarbonyl, 1-ethenyloxy, 2-propenyloxy, 2-propynyloxy, methylcarbonyloxy, trifluoroalkylcarbonyloxy, chloromethylcarbonyloxy, methylcarbonylamino, trifluoroalkylcarbonylamino, chloromethylcarbonylamino, methylthio, ethylthio, methylsulphinyl, methylsulphonyl, methylsulphonyloxy, trifluorosulphonyloxy, methylsulphonylamino, or trifluoromethylsulphonylamino,
substituents on nitrogen: methyl, ethyl, propyl, - C(=O)H, methylcarbonyl, trifluoromethylcarbonyl, chloromethylcarbonyl, methylsulphonyl, trifluoromethylsulphonyl, phenylsulphonyl, phenyl or 2-propynyl, or
R¹ is indol-1-yl, indol-2-yl, indol-3-yl, indol-4-yl, indol-5-yl, indol-6-yl, indol-7-yl, benzimidazol-1-yl, benzimidazol-2-yl, benzimidazol-4-yl, benzimidazol-5-yl, indazol-1-yl, indazol-3-yl, indazol-4-yl, indazol-5-yl, indazol-6-yl, indazol-7-yl, indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,3-benzoxazol-2-yl, 1,3-benzoxazol-4-yl, 1,3-benzoxazol-5-yl, 1,3-benzoxazol-6-yl, 1,3-benzoxazol-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl, each of which may contain up to two substituents, where the substituents are each independently selected from the following list:
substituents on carbon: fluorine, chlorine, bromine, iodine, methyl, methoxy, 2-propynyloxy, 2-propenyloxy,
substituents on nitrogen: methyl, ethyl, propyl, - C(=O)H, methylcarbonyl, trifluoromethylcarbonyl, chloromethylcarbonyl, methylsulphonyl, trifluoromethylsulphonyl, phenylsulphonyl, phenyl or 2-propynyl, or
R¹ is piperidin-1-yl, piperidin-2-yl, piperidin-3-yl, piperidin-4-yl, piperazin-1-yl, piperazin-2-yl, piperazin-3-yl, morpholin-1-yl, morpholin-2-yl, morpholin-3-yl, tetrahydropyran-2-yl, tetrahydropyran-3-yl, tetrahydropyran-4-yl, 1,2,3,4-tetrahydroquinolin-1-yl, 1,2,3,4-tetrahydroisoquinolin-2-yl, 1,2,3,4-tetrahydroquinoxalin-1-yl, indolin-1-yl, isoindolin-2-yl, decahydroquinolin-1-yl oder decahydroisoquinolin-2-yl, each of which may contain up to two substituents, where the substituents are each independently selected from the following list: substituents on carbon: fluorine, chlorine, bromine, iodine, methyl, methoxy, 2-propynyloxy, 2-propenyloxy,
substituents on nitrogen: methyl, ethyl, propyl,-C(=O)H, methylcarbonyl, trifluoromethylcarbonyl, chloromethylcarbonyl, methylsulphonyl, trifluoromethylsulphonyl, phenylsulphonyl, phenyl or 2-propynyl,
and salts, metal complexes and N-oxides of the compounds of the formula **(I).**

4. Method for controlling phytopathogenic harmful fungi, **characterized in that** compounds of the formula (I) according to any of the preceding claims are applied to the phytopathogenic harmful fungi and/or their habitat.

5. Composition for controlling phytopathogenic harmful fungi, **characterized by** a content of at least one compound of the formula (I) according to any of Claims 1 to 3, in addition to extenders and/or surfactants.

6. Use of at least one compound of the formula (I) according to any of Claims 1 to 3 or of a composition according to Claim 5 for controlling phytopathogenic harmful fungi.

7. Process for producing compositions for controlling phytopathogenic harmful fungi according to Claim 5, **characterized in that** compounds of the formula (I) according to any of Claims 1 to 3 are mixed with extenders and/or surfactants.

8. Use of compounds of the formula (I) according to any of Claims 1 to 3 for treatment of seed.

9. Use of compounds of the formula (I) according to any of Claims 1 to 3 for treatment of transgenic plants.

10. Use of compounds of the formula (I) according to any of Claims 1 to 3 for treatment of transgenic seed.

11. Compounds of the formula **(XVIIa)** 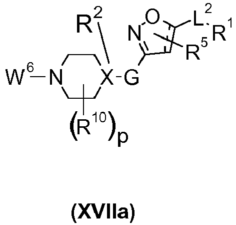 in which
W⁶ is acetyl, C₁-C₄ alkoxycarbonyl, benzyl or benzyloxycarbonyl,
and in which the symbols X, G, L², p, R¹, R², R⁵ and R¹⁰ are each as defined in any of Claims 1 to 3, and salts, metal complexes and N-oxides thereof.

12. Compounds of the formula **(XXXIII)** 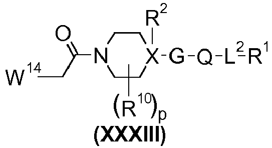 in which
W¹⁴ represents aldehydes, ketones, esters, carboxylic acids, amides, thioamides, nitriles, alcohols, thiols, hydrazines, oximes, amidines, amide oximes, olefins, acetylenes, halides, alkyl halides, methanesulphonates, trifluoromethane-sulphonates, boric acid, boronates,
and in which the symbols W¹⁴, X, L², R¹, R², R¹⁰, Q, p and G are each as defined in any of Claims 1 to 3, and salts, metal complexes and N-oxides thereof.

13. Compounds of the formula **(XIIIa)** 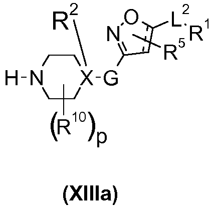 and in which the symbols X, L², p, G, R¹, R², R⁵ and R¹⁰ are each as defined in any of Claims 1 to 3, and salts, metal complexes and N-oxides thereof.

## Revendications

1. Composés de formule (I) 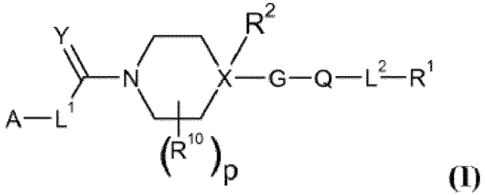 dans laquelle les radicaux ont les significations suivantes :
A représente phényle, qui peut contenir jusqu'à cinq substituants,
les substituants étant choisis indépendamment les uns des autres parmi Z^{A-1},
ou
A représente un hétéroaryle à 5 ou 6 chaînons non substitué ou substitué, éventuellement benzocondensé, qui peut contenir jusqu'à quatre substituants, les substituants sur le carbone étant choisis indépendamment les uns des autres parmi Z^{A-2} et les substituants sur l'azote étant choisis indépendamment les uns des autres parmi Z^{A-3},
les Z^{A-1} représentent, de manière identique ou différente et indépendamment les uns des autres, hydrogène, halogène, hydroxy, thioxy, nitro, cyano, - C(=O)H, -C(=O)OH, alkyle, alcényle, alcynyle, halogénoalkyle, halogénoalcényle, halogénoalcynyle, cycloalkyle, cycloalcényle, halogénocycloalkyle, halogénocycloalcényle, hydroxyalkyle, cyanoalkyle, formylalkyle, alcoxyalkyle, halogénoalcoxyalkyle, cycloalcoxyalkyle, alcynyloxyalkyle, alkylthioalkyle, alkylsulfinylalkyle, alkylaminoalkyle, halogénoalkylaminoalkyle, cycloalkylaminoalkyle, dialkylaminoalkyle, alkylcarbonylalkyle, alkylsulfonylalkyle, alkylcycloalkyle, alkylcycloalcényle, alcoxy, alkylcycloalkylalkyle, halogénocycloalcoxy, alkylthio, halogénoalkylthio, cycloalkylthio, alcynylthio, alcényloxy, alcynyloxy, halogénoalcoxy, halogénoalcényloxy, halogénoalcynyloxy, cycloalcoxy, alcoxyalkoxy, cycloalkylalcoxy, alkylcarbonyloxy, halogénoalkylcarbonyloxy, cycloalkylcarbonyloxy, cycloalkylamino, alkylcarbonylamino, cycloalkylcarbonylamino, alcoxycarbonylamino, alkylsulfonylamino, halogénoalkylsulfonylamino, phénylsulfonylamino, cycloalkylalkyle, halogénocycloalkylalkyle, cycloalkylcycloalkyle, alcoxyalkoxyalkyle, alkylaminocarbonyloxy, alkylcarbonylalkoxy, cycloalkylaminocarbonyle, cycloalkylalcoxycarbonyle, alkylsulfinyle, halogénoalkylsulfinyle, alkylsulfonyle, halogénoalkylsulfonyle, cycloalkylsulfonyle, alkylcarbonyle, halogénoalkylcarbonyle, cycloalkylcarbonyle, alcoxycarbonyle, cycloalcoxycarbonyle, trialkylsilyle, -SF₅, phényle,
-C(=O)NR³R⁴ ou -NR³R⁴,
Z^{A-2} et R^{G1} représentent, de manière identique ou différente et indépendamment les uns des autres, hydrogène, halogène, hydroxy, thioxy, nitro, cyano, -C(=O)H, -C(=O)OH, alkyle, alcényle, alcynyle, halogénoalkyle, halogénoalcényle, halogénoalcynyle, cycloalkyle, halogénocycloalkyle, hydroxyalkyle, formylalkyle, alcoxyalkyle, alkylcarbonylalkyle, alkylcycloalkyle, alcoxy, alkylcycloalkylalkyle, alkylthio, halogénoalkylthio, alcynylthio, alcényloxy, alcynyloxy, halogénoalcoxy, alcoxyalcoxy, alkylcarbonyloxy, halogénoalkylcarbonyloxy, cycloalkylcarbonylamino, alkylsulfonylamino, halogénoalkylsulfonylamino, phénylsulfonylamino, cycloalkylalkyle, halogénocycloalkylalkyle, cycloalkylcycloalkyle, alcoxycarbonyloxy, alkylcarbonylthio, alkylsulfinyle, halogénoalkylsulfinyle, alkylsulfonyle, halogénoalkylsulfonyle, alkylcarbonyle, halogénoalkylcarbonyle, alcoxycarbonyle, alkylamionocarbonyloxy, -C(=O)NR³R⁴ ou -NR³R⁴, Z^{A-3}, R^{G2} et Z² représentent, de manière identique ou différente et indépendamment les uns des autres, hydrogène, -C(=O)H, -C(=O)NR³R⁴, alkyle, alcényle, alcynyle, halogénoalkyle, halogénoalcényle, halogénoalcynyle, cycloalkyle, halogénocycloalkyle, alkylcycloalkyle, cycloalkylalkyle, alcoxyalkyle, alkylsulfonyle, halogénoalkylsulfonyle, cycloalkylsulfonyle, phénylsulfonyle, alkylcarbonyle, halogénoalkylcarbonyle, alcoxycarbonyle, halogénoalcoxycarbonyle, cycloalcoxycarbonyle, phényle ou benzyle,
R³ et R⁴ représentent, de manière identique ou différente et indépendamment les uns des autres, hydrogène, alkyle, alcényle, alcynyle, halogénoalkyle, cycloalkyle, benzyle ou phényle,
L¹ représente NR^{L12} ou C(R^{L11})₂,
les R^{L11} représentent, de manière identique ou différente et indépendamment les uns des autres, hydrogène, halogène, hydroxy, cyano, -C(=O)H, -C(=O)OH, alkyle, alcényle, alcynyle, halogénoalkyle, halogénoalcényle, halogénoalcynyle, alcoxyalkyle, alkylthioalkyle, alkylaminoalkyle, dialkylaminoalkyle, alcoxy, alkylthio, halogénoalkylthio, halogénoalcoxy, alkylcarbonyloxy, alkylcarbonylamino, alkylcarbonylthio, alkylsulfonyle, halogénoalkylsulfonyle, alkylcarbonyle, halogénoalkylcarbonyle, alcoxycarbonyle, trialkylsilyloxy, -NR³R⁴ ou -C(=O)NR³R⁴,
ou les deux radicaux R^{L11} forment ensemble avec l'atome de carbone auquel ils sont reliés un cycle cyclopropyle, ou
les deux radicaux R^{L11} représentent =CH₂, =COR³, =NOR³ ou =CHN(R⁹)₂,
R^{L12} représente hydrogène, -C(=O)H, alkyle, alcényle, alcynyle, halogénoalkyle, halogénoalcényle, halogénoalcynyle, cycloalkyle, halogénocycloalkyle, alkylcycloalkyle, cycloalkylalkyle, cycloalkylaminocarbonyle, halogénoalkylaminocarbonyle, alkylsulfonyle, halogénoalkylsulfonyle, cycloalkylsulfonyle, alkylcarbonyle, halogénoalkylcarbonyle, alcoxycarbonyle, halogénoalcoxycarbonyle, cycloalcoxycarbonyle, alkyl-aminocarbonyle, dialkylaminocarbonyle, phényle ou benzyle,
R⁹ représente alkyle, alcényle, alcynyle, halogénoalkyle, cycloalkyle, benzyle ou phényle,
Y représente soufre ou oxygène,
X représente carbone ou azote,
R² représente hydrogène, alkyle, alcényle, halogénoalkyle, alcoxy, halogène, cyano ou hydroxy,
R¹⁰ représente oxo, alkyle, alcényle, halogénoalkyle, alcoxy, halogène, cyano ou hydroxy,
p représente 0 à 2,
G représente un hétéroaryle à 5 chaînons non substitué ou substitué, les substituants sur le carbone étant choisis indépendamment les uns des autres parmi R^{G1} et les substituants sur l'azote étant choisis indépendamment les uns des autres parmi R^{G2},
Q représente un hétérocyclyle à 5 chaînons saturé ou partiellement ou entièrement insaturé, non substitué ou substitué, un ou plusieurs substituants R⁵ étant choisis, de manière identique ou différente, indépendamment les uns des autres,
les R⁵ représentent, de manière identique ou différente, indépendamment les uns des autres :
reliés avec le carbone de l'hétérocyclyle à 5 chaînons de Q :
hydrogène, oxo, halogène, cyano, hydroxy, nitro, -CHO, -C(=O)OH, -C(=O)NH₂, -C(=O)NR³R⁴, -NR³R⁴, alkyle, alcényle, alcynyle, halogénoalkyle, halogénoalcényle, halogénoalcynyle, cycloalkyle, halogénocycloalkyle, alkylcycloalkyle, cycloalkylalkyle, cycloalkylcycloalkyle, halogénocycloalkylalkyle, alkylcycloalkylalkyle, cycloalcényle, halogénocycloalcényle, alcoxyalkyle, halogénoalcoxyalkyle, cycloalcoxyalkyle, alcoxyalcoxyalkyle, alkylthioalkyle, formylalkyle, alkylcarbonylalkyle, alkylsulfinylalkyle, alkylsulfonylalkyle, alkylaminoalkyle, dialkylaminoalkyle, halogénoalkylaminoalkyle, cycloalkylaminoalkyle, alkylcarbonyle, halogénoalkylcarbonyle, cycloalkylcarbonyle, alcoxycarbonyle, cycloalcoxycarbonyle, cycloalkylalcoxycarbonyle, cycloalkylaminocarbonyle, hydroxyalkyle, alcoxy, halogénoalcoxy, cycloalcoxy, halogénocycloalcoxy, cycloalkylalcoxy, alcényloxy, halogénoalcényloxy, alcynyloxy, halogénoalcynyloxy, alcoxyalcoxy, alkylcarbonyloxy, halogénoalkylcarbonyloxy, cycloalkylcarbonyloxy, alkylcarbonylalcoxy, alkylthio, halogénoalkylthio, cycloalkylthio, alkylsulfinyle, halogénoalkylsulfinyle, alkylsulfonyle, halogénoalkylsulfonyle, cycloalkylsulfonyle, trialkylsilyle, alkylsulfonylamino, halogénoalkylsulfonylamino,
reliés avec l'azote de l'hétérocyclyle à 5 chaînons de Q :
hydrogène, alkyle, alcényle, alcynyle, halogénoalkyle, halogénoalcényle, halogénoalcynyle, cycloalkyle, halogénocycloalkyle, alkylcycloalkyle, cycloalkylalkyle, phényle, benzyle, alkylsulfonyle, -C(=O)H, alcoxycarbonyle ou alkylcarbonyle, 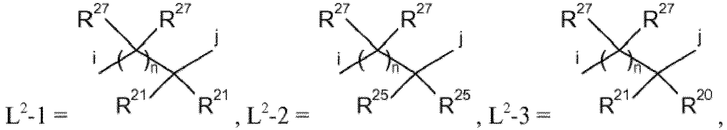 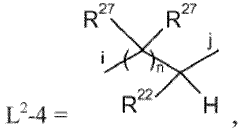
la liaison qui est identifiée avec « i » étant directement reliée à Q et la liaison qui est identifiée avec « j » étant directement reliée à R¹,
m représente 0 à 2,
n représente 0 à 4,
R²⁰ représente halogène ou hydroxy,
les R²¹ représentent, de manière identique ou différente et indépendamment les uns des autres, alkyle, halogénoalkyle, alcoxy, halogénoalcoxy, alcényle, halogénoalcényle, alcynyle, halogénoalcynyle, alcényloxy halogénoalcényloxy, alcynyloxy, halogénoalcynyloxy ou alkylcarbonyloxy,
R²² représente hydroxy, halogène, alcoxy, halogénoalcoxy, alcényle, halogénoalcényle, alcynyle, halogénoalcynyle, alcényloxy halogénoalcényloxy, alcynyloxy, halogénoalcynyloxy, alkylcarbonyloxy ou cycloalkyle,
R²³ représente hydrogène, -C(=O)H, alkyle, alcényle, alcynyle, alcoxyalkyle, alkylsulfonyle, halogénoalkylsulfonyle, alkylcarbonyle, halogénoalkylcarbonyle, alcoxycarbonyle, halogénoalcoxycarbonyle, cyclopropyle, benzyle ou phénylsulfonyle,
les R²⁴ représentent, de manière identique ou différente et indépendamment les uns des autres, hydrogène, cyano, alkyle, cycloalkyle, halogénoalkyle, alcényle, halogénoalcényle, alcynyle, halogénoalcynyle, phényle ou benzyle,
les R²⁵ représentent, de manière identique ou différente et indépendamment les uns des autres, fluor, brome, chlore,
R²⁶ représente hydrogène, hydroxy, pyridinyle, alkyle, halogénoalkyle ou alcoxy,
les R²⁷ représentent, chacun indépendamment les uns des autres et de manière identique ou différente, hydrogène, alkyle, alcoxy, alcényloxy ou alcynyloxy, à condition que lorsque n est supérieur à 1, un radical
L² puisse contenir au plus deux R²⁷ qui sont différents de l'hydrogène,
R¹ représente un phényle, benzyle ou naphtalényle, éventuellement substitué une ou plusieurs fois et de manière identique ou différente, par Z¹, ou un hétéroaryle à 5 ou 6 chaînons, non substitué ou substitué, éventuellement benzocondensé, les substituants sur le carbone étant choisis indépendamment les uns des autres parmi Z¹ et les substituants sur l'azote étant choisis indépendamment les uns des autres parmi Z²,
ou
R¹ représente un cycle carbocyclique non aromatique de 3 à 7 chaînons, un radical hétérocyclyle non aromatique à 5, 6 ou 7 chaînons, ou un cycle bicyclique carbocyclique ou hétérocyclique de 8 à 11 chaînons, les substituants sur le carbone étant choisis indépendamment les uns des autres parmi oxo, thioxo ou Z¹, et les substituants sur l'azote étant choisis indépendamment les uns des autres parmi Z²,
les Z¹ représentent hydrogène, halogène, hydroxy, thioxy, nitro, cyano, -C(=O)H, -C(=O)OH, alkyle, alcényle, alcynyle, halogénoalkyle, halogénoalcényle, halogénoalcynyle, cycloalkyle, cycloalcényle, halogénocycloalkyle, halogénocycloalcényle, hydroxyalkyle, alcoxyalkyle, halogénoalcoxyalkyle, cycloalcoxyalkyle, alkylthioalkyle, alkylsulfinylalkyle, alkylaminoalkyle, halogénoalkylaminoalkyle, cycloalkylaminoalkyle, dialkylaminoalkyle, alcoxycarbonylalkyle, alkylsulfonylalkyle, alkylcycloalkyle, alcoxy, alkylcycloalkylalkyle, halogénocycloalcoxy, alkylthio, halogénoalkylthio, alcynylthio, alcényloxy, alcynyloxy, halogénoalcoxy, halogénoalcényloxy, halogénoalcynyloxy, cycloalcoxy, alcoxyalcoxy, cycloalkylalcoxy, alkylcarbonyloxy, halogénoalkylcarbonyloxy, cycloalkylcarbonyloxy, halogénocycloalkylcarbonyloxy, alkylsulfonyloxy, halogénoalkylsulfonyloxy, phénylsulfonyloxy, alkylcarbonylamino, halogénoalkylcarbonylamino, alkylsulfonylamino, halogénoalkylsulfonylamino, phénylsulfonylamino, cycloalkylalkyle, halogénocycloalkylalkyle, cycloalkylcycloalkyle, alcoxyalcoxyalkyle, alkylcarbonylalcoxy, cycloalkylcarbonyle, cycloalkylthio, cycloalkylaminocarbonyle, cycloalkylalcoxycarbonyle, alcénylthio, alkylcarbonylthio, alkylsulfinyle, halogénoalkylsulfinyle, alkylsulfonyle, halogénoalkylsulfonyle, cycloalkylsulfonyle, alkylcarbonyle, halogénoalkylcarbonyle, alcoxycarbonyle, halogénoalcoxycarbonyle, cycloalcoxycarbonyle, trialkylsilyle, trialkylsilyloxy, -SF₅, cycloalkylalkylamino, dialkylaminothiocarbonyle, alcoxycarbonylamino, alcoxyalkylaminocarbonyle, halogénoalcoxyamino, cycloalkylalkylaminoalkyle, dialkylaminocarbonylamino, alcoxyhalogénoalcoxy, alkylthiocarbonyloxy, halogénoalcoxyalcoxy, dialkylaminosulfonyle, alcoxyhalogénoalkyle, alkylaminosulfonyle, dialcoxyalkyle, halogénoalcoxyhalogénoalkyle, -NHC(=O)H, halogénocycloalcényloxyalkyle, alkylthiocarbonyle, alcoxyalcényle, - SO₂NHCN, -NHCN, -halogénoalkylsulfonylaminocarbonyle, alkylaminothiocarbonylamino, alkylaminothiocarbonyle, alkylaminocarbonylalkylamino, alcoxyalcynyle, halogénodialkylaminoalkyle, cyanoalkyle, alcoxyalkylcarbonyle, alcoxycarbonylalcoxy, alcoxyamino, alcoxyalcoxycarbonyle, cycloalcényloxyalkyle, dialkylaminothiocarbonylamino, alkylsulfonylaminocarbonyle, halogénoalcoxyhalogénoalcoxy, halogénocycloalcoxyalkyle,-C(=O)NHCN, -N=C(R⁹)₂, -NR³R⁴, -C(=O)NR³R⁴, -C(=N-OR⁷)R⁸ ou -L³Z³,
R⁷ représente hydrogène, alkyle, halogénoalkyle, benzyle ou Z³,
R⁸ représente hydrogène, alkyle, halogénoalkyle, cycloalkylalkyle, cycloalkyle, alkylcycloalkyle, halogénoalkylcycloalkyle, alcoxylalkyle, halogénoalcoxyalkyle, benzyle ou phényle,
L³ représente une liaison directe, -CH₂-, -C(=O)-, soufre, oxygène, -C(=O)O-, -C(=O)NH-, -OC(=O)- ou -NHC(=O)-,
Z³ représente un radical phényle ou un radical hétéroaryle à 5 ou 6 chaînons, qui peut contenir jusqu'à trois substituants, les substituants étant choisis indépendamment les uns des autres dans la liste suivante :
substituants sur le carbone : halogène, cyano, nitro, hydroxy, amino, -SH, alkyle, alcényle, alcynyle, halogénoalkyle, halogénoalcényle, halogénoalcynyle, cycloalkyle, halogénocycloalkyle, alcoxyalkyle, alkylcarbonyle, halogénoalkylcarbonyle, alcoxycarbonyle, alcoxy, halogénoalcoxy, cycloalcoxy, halogénocycloalcoxy, alcényloxy, alcynyloxy, alcoxyalcoxy, alkylthio, halogénoalkylthio, alkylsulfinyle, halogénoalkylsulfinyle, alkylsulfonyle, halogénoalkylsulfonyle, trisilylalkyle ou phényle,
substituants sur l'azote :
hydrogène, -C(=O)H, alkyle, alcényle, alcynyle, halogénoalkyle, halogénoalcényle, halogénoalcynyle, cycloalkyle, halogénocycloalkyle, alkylcycloalkyle, cycloalkylalkyle, alcoxyalkyle, alkylsulfonyle, halogénoalkylsulfonyle, cycloalkylsulfonyle, phénylsulfonyle, alkylcarbonyle, halogénoalkylcarbonyle, alcoxycarbonyle, halogénoalcoxycarbonyle, cycloalcoxycarbonyle, -C(=O)NR³R⁴, phényle ou benzyle,
ainsi que les sels, complexes métalliques et N-oxydes des composés de formule (I).

2. Composés de formule (I) selon la revendication 1, dans laquelle les symboles ont les significations suivantes :
A représente phényle, qui peut contenir jusqu'à deux substituants, les substituants étant choisis indépendamment les uns des autres dans la liste suivante :
fluor, brome, iode, chlore, cyano, nitro, méthyle, éthyle, propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, propoxy, méthoxy, trifluorométhoxy, difluorométhoxy, 1-méthyléthylthio, méthylthio, éthylthio, propylthio, difluorométhylthio ou trifluorométhylthio, ou
A représente un radical hétéroaromatique choisi dans le groupe suivant : furan-2-yle, furan-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrimidin-2-yle, pyrimidin-4-yl ou pyrimidin-5-yle, qui peut contenir jusqu'à deux substituants, les substituants étant choisis, de manière identique ou différente et indépendamment les uns des autres, dans la liste suivante :
substituants sur le carbone :
fluor, chlore, brome, iode, cyano, nitro, méthyle, éthyle, propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, propoxy, méthoxy, trifluorométhoxy, difluorométhoxy, 1-méthyléthylthio, méthylthio, éthylthio, propylthio, difluorométhylthio, trifluorométhylthio ou phényle,
substituants sur l'azote :
méthyle, éthyle, propyle, 1-méthyléthyle, méthylsulfonyle, trifluorométhylsulfonyle, méthylcarbonyle, trifluorométhylcarbonyle, chlorométhylcarbonyle, 2,2-trifluoroéthyle, 2,2-difluoroéthyle, 2,2-dichloro-2-fluoroéthyle, 2-chloro-2-difluoroéthyle ou 2-chloro-2-fluoroéthyle,
R^{L11} représente hydrogène, méthyle, éthyle ou cyclopropyle,
ou les deux radicaux R^{L11} forment ensemble avec l'atome de carbone auquel ils sont reliés un cycle cyclopropyle, ou
les deux radicaux R^{L11} représentent =CHN(R⁹)₂,
R^{L12} représente hydrogène, alkyle en C₁-C₄, halogénoalkyle en C₁-C₄, cycloalkyle en C₃-C₈, alkylsulfonyle en C₁-C₄, alcoxycarbonyle en C₁-C₄,
les R⁹ représentent, de manière identique ou différente et indépendamment les uns des autres, alkyle en C₁-C₆, alcényle en C₂-C₆, alcynyle en C₂-C₆, halogénoalkyle en C₁-C₆, cycloalkyle en C₃-C₈, benzyle ou phényle,
R² représente hydrogène, fluor, chlore, brome ou hydroxy,
R¹⁰ représente fluor, chlore, brome ou hydroxy,
p représente 0 ou 1,
G représente 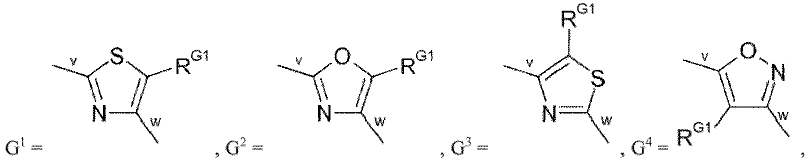 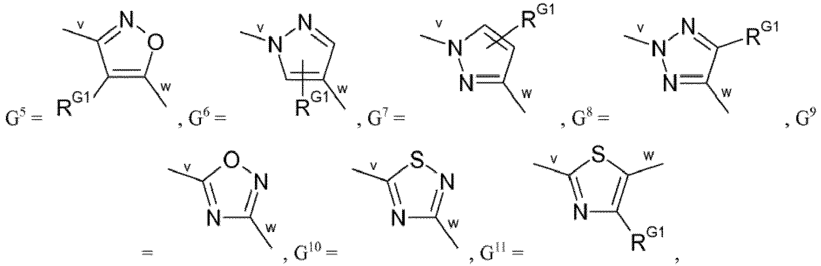
la liaison qui est identifiée avec « v » étant directement reliée à X et la liaison qui est identifiée avec « w » étant directement reliée à Q,
R^{G1} représente hydrogène,
Q représente 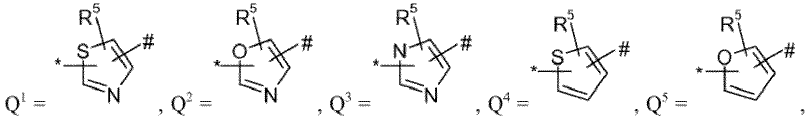 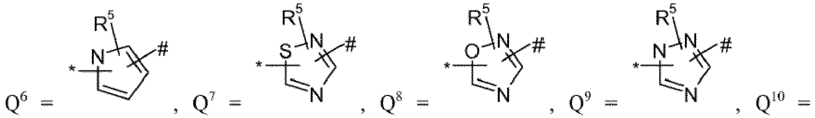 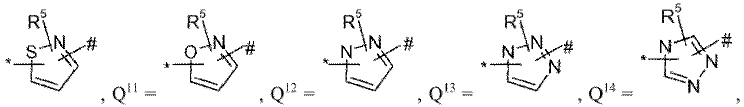 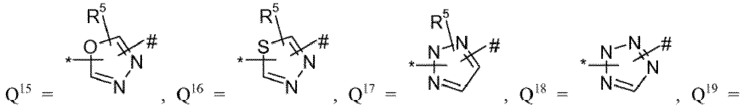 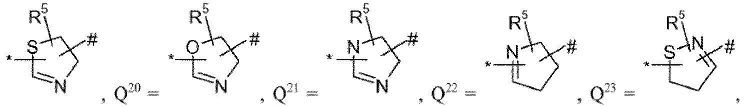 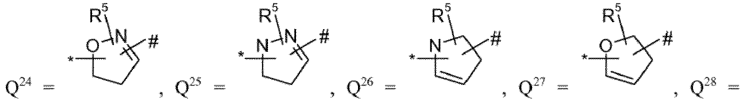 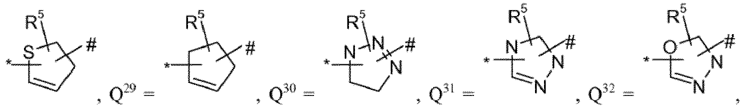 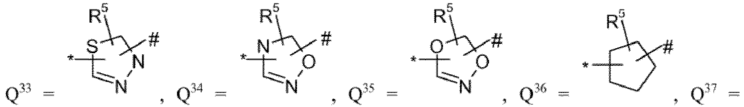 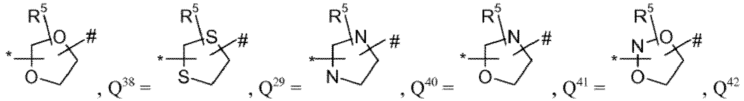 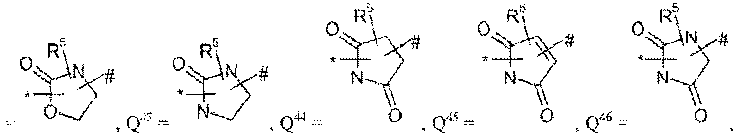
la liaison qui est identifiée avec « * » étant directement reliée à G et simultanément la liaison qui est identifiée avec « # » étant directement reliée à L², ou la liaison qui est identifiée avec « * » étant directement reliée à L² et simultanément la liaison qui est identifiée avec « # » étant directement reliée à G, les R⁵ représentent, de manière identique ou différente, indépendamment les uns des autres,
reliés avec le carbone de l'hétérocyclyle à 5 chaînons de Q :
hydrogène, cyano, -NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₈, halogénocycloalkyle en C₃-C₈, halogénocycloalkyle en C₃-C₈, alkyle en C₁-C₄-cycloalkyle en C₃-C₈, cycloalkyle en C₃-C₈-alkyle en C₁-C₄, alcoxy en C₁-C₄-alkyle en C₁-C₄, cycloalcoxy en C₃-C₈-alkyle en C₁-C₄, alcoxy en C₁-C₄-alcoxy en C₁-C₄-alkyle en C₁-C₄, alkylthio en C₁-C₄-alkyle en C₁-C₄, alcoxy en C₁-C₆, halogénoalcoxy en C₁-C₆, cycloalcoxy en C₃-C₈, halogénocycloalcoxy en C₃-C₈, cycloalkyle en C₃-C₈-alcoxy en C₁-C₄, alcényloxy en C₂-C₆, halogénoalcényloxy en C₂-C₆, alcynyloxy en C₂-C₆, halogénoalcynyloxy en C₂-C₆, alcoxy en C₁-C₆-alcoxy en C₁-C₄, alkylcarbonyloxy en C₁-C₆, halogénoalkylcarbonyloxy en C₁-C₆, cycloalkylcarbonyloxy en C₄-C₈, alkylcarbonyle en C₁-C₆-alcoxy en C₁-C₆, alkylthio en C₁-C₆, halogénoalkylthio en C₁-C₆, cycloalkylthio en C₃-C₈,
reliés avec l'azote de l'hétérocyclyle à 5 chaînons de Q :
hydrogène, -C(=O)H, alkyle en C₁-C₃, alkylcarbonyle en C₁-C₆, alcoxycarbonyle en C₁-C₆ ou benzyle, R³ et R⁴ représentent, de manière identique ou différente et indépendamment l'un de l'autre, méthyle, éthyle, propyle, 1-méthyléthyle, butyle ou 1,1-diméthyléthyle,
L² représente 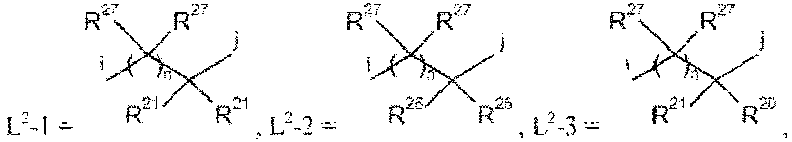 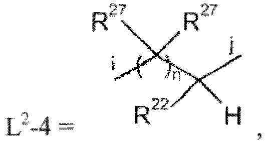
la liaison qui est identifiée avec « i » étant reliée directement à Q et la liaison qui est identifiée avec « j » étant reliée directement à R¹,
n représente 0 à 2,
m représente 0 ou 2,
R¹ représente cycloalcényle en C₅-C₆ non substitué ou substitué, cycloalkyle en C₃-C₈, les substituants étant choisis indépendamment les uns des autres parmi Z¹⁻¹, ou
R¹ représente phényle non substitué ou substitué, les substituants étant choisis indépendamment les uns des autres parmi Z¹⁻², ou
R¹ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, 1H-indén-1-yle, 2,3-dihydro-1H-indén-1-yle, 1H-indén-2-yle, 1H-indén-3-yle, 1H-indén-4-yle, 1H-indén-5-yle, 1H-indén-6-yle, 1H-indén-7-yle, indan-1-yle, indan-2-yle, indan-3-yle, indan-4-yle ou indan-5-yle, non substitué ou substitué, les substituants étant choisis indépendamment les uns des autres parmi Z¹⁻³, ou
R¹ représente un radical hétéroaryle à 5 ou 6 chaînons non substitué ou substitué, les substituants sur le carbone étant choisis indépendamment les uns des autres parmi Z¹⁻⁴, et les substituants sur l'azote étant choisis indépendamment les uns des autres parmi Z², ou R¹ représente un radical hétéroaryle à 5 ou 6 chaînons benzocondensé non substitué ou substitué, les substituants sur le carbone étant choisis indépendamment les uns des autres parmi Z¹⁻⁵, et les substituants sur l'azote étant choisis indépendamment les uns des autres parmi Z², ou
R¹ représente un hétérocyclyle en C₅-C₁₅ non substitué ou substitué, les substituants sur le carbone étant choisis indépendamment les uns des autres parmi Z¹⁻⁶, et les substituants sur l'azote étant choisis indépendamment les uns des autres parmi Z²,
les Z¹⁻¹ représentent de préférence, de manière identique ou différente, indépendamment les uns des autres, cyano, halogène, -C(=O)H, alkyle en C₁-C₆, halogénoalkyle en C₁-C₆, cycloalkyle en C₃-C₆, alcényle en C₂-C₆, halogénoalcényle en C₂-C₆, alcynyle en C₂-C₆, halogénoalcynyle en C₂-C₆, hydroxy, oxo, alcoxy en C₁-C₆, halogénoalcoxy en C₁-C₆, alcényloxy en C₂-C₆, alcynyloxy en C₂-C₆, alkylcarbonyloxy en C₁-C₆, halogénoalkylcarbonyloxy en C₁-C₆, alkylthio en C₁-C₆ ou halogénoalkylthio en C₁-C₆,
les Z¹⁻² représentent, de manière identique ou différente, indépendamment les uns des autres, halogène, cyano, hydroxy, thio, nitro, -C(=O)H, -COOH, -C(=O)NR³R⁴, -NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₈, halogénocycloalkyle en C₃-C₈, cycloalcényle en C₃-C₈, halogénocycloalcényle en C₃-C₈, alcoxy en C₁-C₆-alkyle en C₁-C₆, alkylcarbonyle en C₁-C₆, halogénoalkylcarbonyle en C₁-C₆, cycloalkylcarbonyle en C₃-C₈, alcoxycarbonyle en C₁-C₆, cycloalcoxycarbonyle en C₃-C₈, cycloalkylaminocarbonyle en C₃-C₈, alcoxy en C₁-C₆, halogénoalcoxy en C₁-C₆, cycloalcoxy en C₃-C₈, halogénocycloalcoxy en C₃-C₈, alcényloxy en C₂-C₆, halogénoalcényloxy en C₂-C₆, alcynyloxy en C₂-C₆, halogénoalcynyloxy en C₂-C₆, alcoxy en C₁-C₄-alcoxy en C₁-C₄, alkylcarbonyloxy en C₁-C₆, halogénoalkylcarbonyloxy en C₁-C₆, cycloalkylcarbonyloxy en C₃-C₈, alkylcarbonyle en C₁-C₆-alcoxy en C₁-C₄, alkylthio en C₁-C₆, halogénoalkylthio en C₁-C₆, cycloalkylthio en C₃-C₆, alkylsulfonyle en C₁-C₆, halogénoalkylsulfonyle en C₁-C₆, cycloalkylsulfonyle en C₃-C₈, alkylsulfonyloxy en C₁-C₆, halogénoalkylsulfonyloxy en C₁-C₆, phénylsulfonyloxy, alkylsulfonylamino en C₁-C₆, halogénoalkylsulfonylamino en C₁-C₆, tri-alkylsilyle en C₁-C₄, tri-alkylsilyloxy en C₁-C₄ ou -C(=N-OR⁷)R⁸,
les Z¹⁻³ et Z¹⁻⁵ représentent, de manière identique ou différente, indépendamment les uns des autres, halogène, cyano, nitro, -C(=O)H, -NR³R⁴, -C(=O)NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, alcoxy en C₁-C₄-alkyle en C₁-C₄, alkylcarbonyle en C₁-C₆, alcoxycarbonyle en C₁-C₆, alcoxy en C₁-C₄, halogénoalcoxy en C₁-C₄, alkylcarbonyloxy en C₁-C₆, alkylcarbonylthio en C₁-C₆, alkylthio en C₁-C₄, halogénoalkylthio en C₁-C₄, alkylsulfonyle en C₁-C₄, halogénoalkylsulfonyle en C₁-C₄, les Z¹⁻⁴ représentent, de manière identique ou différente, indépendamment les uns des autres, halogène, cyano, hydroxy, thio, nitro, -C(=O)H,-C(=O)OH, -C(=O)NR³R⁴, -NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₈, halogénocycloalkyle en C₃-C₈, cycloalcényle en C₃-C₈, halogénocycloalcényle en C₃-C₈, alcoxy en C₁-C₆-alkyle en C₁-C₆, alkylcarbonyle en C₁-C₆, halogénoalkylcarbonyle en C₁-C₆, cycloalkylcarbonyle en C₃-C₈, alcoxycarbonyle en C₁-C₆, cycloalcoxycarbonyle en C₃-C₈, cycloalkylaminocarbonyle en C₃-C₁₀, alcoxy en C₁-C₆, halogénoalcoxy en C₁-C₆, cycloalcoxy en C₃-C₈, halogénocycloalcoxy en C₃-C₈, alcényloxy en C₂-C₆, halogénoalcényloxy en C₂-C₆, alcynyloxy en C₂-C₆, halogénoalcynyloxy en C₂-C₆, alcoxy en C₁-C₄-alcoxy en C₁-C₄, alkylcarbonyloxy en C₁-C₆, halogénoalkylcarbonyloxy en C₁-C₆, cycloalkylcarbonyloxy en C₃-C₈, alkylcarbonyle en C₁-C₆-alcoxy en C₁-C₄, alkylthio en C₁-C₆, halogénoalkylthio en C₁-C₆, cycloalkylthio en C₃-C₆, alkylsulfonyle en C₁-C₆, halogénoalkylsulfonyle en C₁-C₆, cycloalkylsulfonyle en C₃-C₈, alkylsulfonyloxy en C₁-C₆, halogénoalkylsulfonyloxy en C₁-C₆, phénylsulfonyloxy, alkylsulfonylamino en C₁-C₆ ou halogénoalkylsulfonylamino en C₁-C₆,
les Z¹⁻⁶ représentent, de manière identique ou différente, indépendamment les uns des autres, cyano, halogène, -C(=O)H, -C(=O)NR³R⁴, phényle, alkyle en C₁-C₆, halogénoalkyle en C₁-C₆, cycloalkyle en C₃-C₆, alcényle en C₂-C₆, halogénoalcényle en C₂-C₆, alcynyle en C₂-C₆, halogénoalcynyle en C₂-C₆, alcoxy en C₁-C₆, halogénoalcoxy en C₁-C₆, alcényloxy en C₂-C₆, alcynyloxy en C₂-C₆, alkylthio en C₁-C₆, -NR³R⁴, alkylcarbonyle en C₁-C₆, alcoxycarbonyle en C₁-C₆, alkylcarbonyloxy en C₁-C₆ ou halogénoalkylthio en C₁-C₆,
les Z² représentent, de manière identique ou différente, indépendamment les uns des autres, hydrogène, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, alcoxy en C₁-C₄-alkyle en C₁-C₄, phényle, benzyle, halogénoalkylsulfonyle en C₁-C₄, alcoxycarbonyle en C₁-C₆, halogénoalcoxycarbonyle en C₁-C₆, phénylsulfonyle, alkylsulfonyle en C₁-C₄,-C(=O)H ou alkylcarbonyle en C₁-C₃,
R⁷ représente hydrogène, méthyle ou éthyle,
R⁸ représente hydrogène, alkyle en C₁-C₆, halogénoalkyle en C₁-C₆, cycloalkyle en C₃-C₈-alkyle en C₁-C₄, cycloalkyle en C₃-C₈, alkyle en C₁-C₄-cycloalkyle en C₃-C₈, halogénoalkyle en C₁-C₄-cycloalkyle en C₃-C₈, alcoxy en C₁-C₄-alkyle en C₁-C₄, halogénoalcoxy en C₁-C₄-alkyle en C₁-C₄, benzyle ou phényle,
L³ représente de préférence une liaison directe, -CH₂-, soufre, oxygène, -C(=O)O-, -C(=O)NH-, -OC(=O)- ou-NHC(=O)-, de manière particulièrement préférée une liaison directe,
Z³⁻¹ représente de préférence un radical phényle ou un radical hétéroaryle à 5 ou 6 chaînons, qui peut contenir jusqu'à deux substituants, les substituants étant choisis indépendamment les uns des autres dans la liste suivante :
substituants sur le carbone :
halogène, cyano, nitro, hydroxy, amino, -SH, alkyle en C₁-C₄, alcényle en C₂-C₄, alcynyle en C₂-C₄, halogénoalkyle en C₁-C₄, halogénoalcényle en C₂-C₄, halogénoalcynyle en C₂-C₄, alcoxyalkyle en C₁-C₄, alkylcarbonyle en C₁-C₆, halogénoalkylcarbonyle en C₁-C₆, alcoxycarbonyle en C₁-C₆, alcoxy en C₁-C₄, halogénoalcoxy en C₁-C₄, alcényloxy en C₂-C₆, alcynyloxy en C₂-C₆, alkylthio en C₁-C₄, halogénoalkylthio en C₁-C₄, alkylsulfonyle en C₁-C₄, halogénoalkylsulfonyle en C₁-C₄ ou alkylamino en C₁-C₄, di(alkyle en C₁-C₄)amino,
substituants sur l'azote : alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, alcoxy en C₁-C₄-alkyle en C₁-C₄, phényle, benzyle, halogénoalkylsulfonyle en C₁-C₄, alcoxycarbonyle en C₁-C₆, halogénoalcoxycarbonyle en C₁-C₆, phénylsulfonyle, alkylsulfonyle en C₁-C₄, -C(=O)H ou alkylcarbonyle en C₁-C₃,
ainsi que les sels, complexes métalliques et N-oxydes des composés de formule (I).

3. Composés de formule (I) selon l'une quelconque des revendications précédentes,
dans laquelle les symboles ont les significations suivantes :
A représente pyrazol-1-yle, qui peut contenir jusqu'à deux substituants, les substituants étant choisis indépendamment les uns des autres dans la liste suivante :
méthyle, éthyle, chlore, brome, fluor, difluorométhyle ou trifluorométhyle, ou
A représente phényle, qui peut contenir jusqu'à deux substituants, les substituants étant choisis indépendamment les uns des autres dans la liste suivante :
méthyle, éthyle, iode, chlore, brome, fluor, méthoxy, éthoxy, difluorométhyle ou trifluorométhyle.
L¹ représente CH₂,
Y représente oxygène,
X représente carbone,
R² représente hydrogène ou fluor,
p représente 0,
G représente 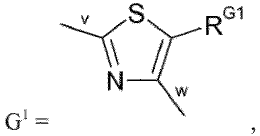
Q représente 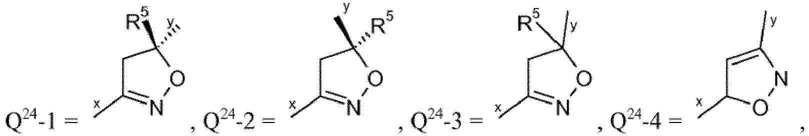 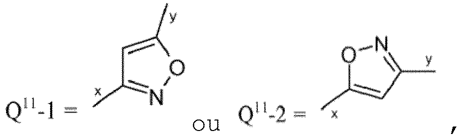
la liaison qui est identifiée par « x » étant directement reliée à G et la liaison qui est identifiée par « y » étant directement reliée à L²,
R⁵ représente hydrogène, cyano, méthyle, trifluorométhyle, difluorométhyle ou méthoxyméthyle,
m représente 0,
n représente 0,
R²⁰ représente halogène ou hydroxy,
les R²¹ représentent, de manière identique ou différente et indépendamment les uns des autres, méthyle, éthyle, éthényle, éthynyle, trifluorométhyle, difluorométhyle, méthylcarbonyloxy, éthylcarbonyloxy, méthoxy, éthoxy, propoxy, 1-méthyléthoxy, 2-propényloxy, 2-propinyloxy ou trifluorométhoxy,
R²² représente hydroxy, chlore, fluor, brome, iode, méthoxy, éthoxy, propoxy, 1-méthyléthoxy, butoxy, trifluorométhyle, difluorométhyle, méthylcarbonyloxy, éthylcarbonyloxy, 1-éthényloxy, 2-propényloxy, 2-propinyloxy ou trifluorométhoxy,
R²³ représente hydrogène, méthyle, éthyle, propyle, 1-méthyléthyle, méthylcarbonyle, éthylcarbonyle, 1,1-diméthyléthyloxycarbonyle, -C(=O)H, phénylsulfonyle, méthylsulfonyle, trifluorométhylsulfonyle ou benzyle,
R²⁴ représente hydrogène,
R²⁵ représente fluor,
les R²⁶ représentent, de manière identique ou différente et indépendamment les uns des autres, méthyle, éthyle, méthoxy ou éthoxy,
R²⁷ représente hydrogène,
R¹ représente cyclopentényle, cyclohexényle, cyclopentyle, cyclohexyle ou cycloheptyle, qui peuvent chacun contenir jusqu'à deux substituants, les substituants étant choisis indépendamment les uns des autres dans la liste suivante : méthyle, éthyle, méthoxy, éthoxy, trifluorométhoxy, éthinyle, 2-propényloxy, 2-propinyloxy, méthylcarbonyloxy, éthylcarbonyloxy, trifluorocarbonyloxy, méthylthio, éthylthio ou trifluorométhylthio, ou
R¹ représente phényle, qui peut contenir jusqu'à trois substituants, les substituants étant choisis indépendamment les uns des autres dans la liste suivante : fluor, chlore, brome, iode, cyano, nitro, hydroxy, amino, thio, -(C=O)H, méthyle, éthyle, propyle, 1-méthyléthyle, butyle, 1,1-diméthyléthyle, 1,2-diméthyléthyle, éthényle, éthinyle, trifluorométhyle, difluorométhyle, trichlorométhyle, dichlorométhyle, cyclopropyle, méthoxy, éthoxy, propoxy, 1-méthyléthoxy, 1,1-diméthyléthoxy, méthylcarbonyle, éthylcarbonyle, trifluorométhylcarbonyle, méthoxycarbonyle, éthoxycarbonyle, propoxycarbonyle, 1-méthyléthoxycarbonyle, 1,1-diméthyléthoxycarbonyle, 1-éthényloxy, 2-propényloxy, 2-propinyloxy, méthylcarbonyloxy, trifluoroalkylcarbonyloxy, chlorométhylcarbonyloxy, méthylcarbonylamino, trifluoroalkylcarbonylamino, chlorométhylcarbonylamino, méthylthio, éthylthio, méthylsulfinyle, méthylsulfonyle, méthylsulfonyloxy, trifluorosulfonyloxy, méthylsulfonylamino, trifluoroométhylsulfonylamino, C(=N-OH)H, -C(=N-OCH₃)H, -C(=N-OCH₂CH₃)H, -C(=N-OH)CH₃, C(=N-OCH₃)CH₃, -C(=N-OCH₂CH₃)CH₃ ou triméthylsilyloxy, ou
R¹ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, 1H-indén-1-yle, 2,3-dihydro-1H-indén-1-yle, 1H-indén-2-yle, 1H-indén-3-yle, 1H-indén-4-yle, 1H-indén-5-yle, 1H-indén-6-yle, 1H-indén-7-yle, indan-1-yle, indan-2-yle, indan-3-yle, indan-4-yl ou indan-5-yle, non substitué ou substitué, les substituants étant choisis indépendamment les uns des autres dans la liste suivante : méthyle, méthoxy, cyano, fluoré, chlore, brome, iode, ou
R¹ représente furan-2-yle, furan-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, 1,2,4-triazol-4-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle ou pyrazin-2-yle, qui peuvent chacun contenir jusqu'à deux substituants, les substituants étant choisis indépendamment les uns des autres dans la liste suivante :
substituants sur le carbone : fluor, chlore, brome, iode, cyano, nitro, hydroxy, amino, thio, -(C=O)H, méthyle, éthyle, propyle, 1-méthyléthyle, butyle, 1,1-diméthyléthyle, 1,2-diméthyléthyle, éthényle, éthinyle, trifluorométhyle, difluorométhyle, trichlorométhyle, dichlorométhyle, cyclopropyle, méthoxy, éthoxy, propoxy, 1-méthyléthoxy, 1,1-diméthyléthoxy, méthylcarbonyle, éthylcarbonyle, trifluorométhylcarbonyle, méthoxycarbonyle, éthoxycarbonyle, propoxycarbonyle, 1-méthyléthoxycarbonyle, 1,1-diméthyléthoxycarbonyle, 1-éthényloxy, 2-propényloxy, 2-propinyloxy, méthylcarbonyloxy, trifluoroalkylcarbonyloxy, chlorométhylcarbonyloxy, méthylcarbonylamino, trifluoroalkylcarbonylamino, chlorométhylcarbonylamino, méthylthio, éthylthio, méthylsulfinyle, méthylsulfonyle, méthylsulfonyloxy, trifluorosulfonyloxy, méthylsulfonylamino ou trifluorométhylsulfonylamino,
substituants sur l'azote : méthyle, éthyle, propyle,-C(=O)H, méthylcarbonyle, trifluorométhylcarbonyle, chlorométhylcarbonyle, méthylsulfonyle, trifluorométhylsulfonyle, phénylsulfonyle, phényle ou 2-propinyle, ou
R¹ représente indol-1-yle, indol-2-yle, indol-3-yle, indol-4-yle, indol-5-yle, indol-6-yle, indol-7-yle, benzimidazol-1-yle, benzimidazol-2-yle, benzimidazol-4-yle, benzimidazol-5-yle, indazol-1-yle, indazol-3-yle, indazol-4-yle, indazol-5-yle, indazol-6-yle, indazol-7-yle, indazol-2-yle, 1-benzofuran-2-yle, 1-benzofuran-3-yle, 1-benzofuran-4-yle, 1-benzofuran-5-yle, 1-benzofuran-6-yle, 1-benzofuran-7-yle, 1-benzothiophén-2-yle, 1-benzothiophén-3-yle, 1-benzothiophén-4-yle, 1-benzothiophén-5-yle, 1-benzothiophén-6-yle, 1-benzothiophén-7-yle, 1,3-benzothiazol-2-yle, 1,3-benzothiazol-4-yle, 1,3-benzothiazol-5-yle, 1,3-benzothiazol-6-yle, 1,3-benzothiazol-7-yle, 1,3-benzoxazol-2-yle, 1,3-benzoxazol-4-yle, 1,3-benzoxazol-5-yle, 1,3-benzoxazol-6-yle, 1,3-benzoxazol-7-yle, quinolin-2-yle, quinolin-3-yle, quinolin-4-yle, quinolin-5-yle, quinolin-6-yle, quinolin-7-yle, quinolin-8-yle, isoquinolin-1-yle, isoquinolin-3-yle, isoquinolin-4-yle, isoquinolin-5-yle, isoquinolin-6-yle, isoquinolin-7-yle ou isoquinolin-8-yle, qui peuvent chacun contenir jusqu'à deux substituants, les substituants étant choisis indépendamment les uns des autres dans la liste suivante :
substituants sur le carbone : fluor, chlore, brome, iode, méthyle, méthoxy, 2-propinyloxy, 2-propényloxy, substituants sur l'azote : méthyle, éthyle, propyle,-C(=O)H, méthylcarbonyle, trifluorométhylcarbonyle, chlorométhylcarbonyle, méthylsulfonyle, trifluorométhylsulfonyle, phénylsulfonyle, phényle ou 2-propinyle, ou
R¹ représente pipéridin-1-yle, pipéridin-2-yle, pipéridin-3-yle, pipéridin-4-yle, pipérazin-1-yle, pipérazin-2-yle, pipérazin-3-yle, morpholin-1-yle, morpholin-2-yle, morpholin-3-yle, tétrahydropyran-2-yle, tétrahydropyran-3-yle, tétrahydropyran-4-yle, 1,2,3,4-tétrahydroquinolin-1-yle, 1,2,3,4-tétrahydroisoquinolin-2-yle, 1,2,3,4-tétrahydroquinoxalin-1-yle, indolin-1-yle, isoindolin-2-yle, décahydroquinolin-1-yle ou décahydroisoquinolin-2-yle, qui peuvent chacun contenir jusqu'à deux substituants, les substituants étant choisis indépendamment les uns des autres dans la liste suivante :
substituants sur le carbone : fluor, chlore, brome, iode, méthyle, méthoxy, 2-propinyloxy, 2-propényloxy,
substituants sur l'azote : méthyle, éthyle, propyle,-C(=O)H, méthylcarbonyle, trifluorométhylcarbonyle, chlorométhylcarbonyle, méthylsulfonyle, trifluorométhylsulfonyle, phénylsulfonyle, phényle ou 2-propinyle,
ainsi que les sels, complexes métalliques et N-oxydes des composés de formule (I).

4. Procédé de lutte contre des champignons nocifs phytopathogènes, **caractérisé en ce que** des composés de formule (I) selon l'une quelconque des revendications précédentes sont appliqués sur les champignons nocifs phytopathogènes et/ou leur habitat.

5. Agent de lutte contre des champignons nocifs phytopathogènes, **caractérisé par** une teneur en au moins un composé de formule (I) selon l'une quelconque des revendications 1 ou 3, outre des extendeurs et/ou des substances tensioactives.

6. Utilisation d'au moins un composé de formule (I) selon l'une quelconque des revendications 1 ou 3, ou d'un agent selon la revendication 5 pour lutter contre des champignons nocifs phytopathogènes.

7. Procédé de fabrication d'agents de lutte contre des champignons nocifs phytopathogènes selon la revendication 5, **caractérisé en ce que** des composés de formule (I) selon l'une quelconque des revendications 1 ou 3 sont mélangés avec des extendeurs et/ou des substances tensioactives.

8. Utilisation de composés de formule (I) selon l'une quelconque des revendications 1 à 3 pour le traitement de graines.

9. Utilisation de composés de formule (I) selon l'une quelconque des revendications 1 à 3 pour le traitement de plantes transgéniques.

10. Utilisation de composés de formule (I) selon l'une quelconque des revendications 1 à 3 pour le traitement de graines transgéniques.

11. Composés de formule (XVIIa) 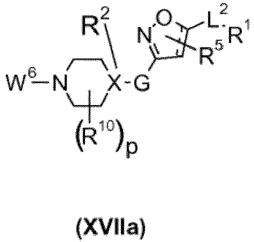 dans laquelle
W⁶ représente acétyle, alcoxycarbonyle en C₁-C₄, benzyle ou benzyloxycarbonyle,
et dans laquelle les symboles X, G, L², p, R¹, R², R⁵ et R¹⁰ ont une signification telle qu'indiquée dans l'une quelconque des revendications 1 à 3, ainsi que leurs sels, complexes métalliques et N-oxydes.

12. Composés de formule (XXXIII) 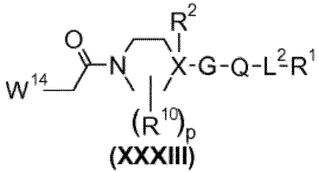 dans laquelle
W¹⁴ représente des aldéhydes, des cétones, des esters, des acides carboxyliques, des amides, des thioamides, des nitriles, des alcools, des thiols, des hydrazines, des oximes, des amidines, des amidoximes, des oléfines, des acétylènes, des halogénures, des halogénures d'alkyle, des méthane-sulfonates, des trifluorométhane-sulfonates, l'acide borique, des boronates,
et dans laquelle les symboles W¹⁴, X, L², R¹, R², R¹⁰, Q, p et G ont une signification telle qu'indiquée dans l'une quelconque des revendications 1 à 3, ainsi que leurs sels, complexes métalliques et N-oxydes.

13. Composés de formule (XIIIa) 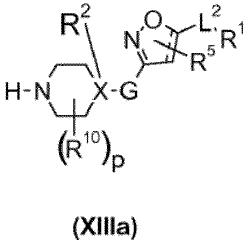 dans laquelle les symboles X, L², p, G, R¹, R², R⁵ et R¹⁰ ont une signification telle qu'indiquée dans l'une quelconque des revendications 1 à 3, ainsi que leurs sels, complexes métalliques et N-oxydes.
